(19) Europäisches Patentamt
European Patent Office
Office européen des brevets

(11) EP 1 704 240 B1

# (12) EUROPEAN PATENT SPECIFICATION

(45) Date of publication and mention of the grant of the patent:
**24.10.2007 Bulletin 2007/43**

(21) Application number: **04806534.6**

(22) Date of filing: **23.12.2004**

(51) Int Cl.:
***C12P 7/64*** *(2006.01)*

(86) International application number:
**PCT/IB2004/004374**

(87) International publication number:
**WO 2005/066351 (21.07.2005 Gazette 2005/29)**

## (54) ENZYMATIC TREATMENT OF OILS

ENZYMATISCHE BEHANDLUNG VON ÖLEN

TRAITEMENT ENZYMATIQUE D'HUILES

(84) Designated Contracting States:
**AT BE BG CH CY CZ DE DK EE ES FI FR GB GR HU IE IS IT LI LT LU MC NL PL PT RO SE SI SK TR**

(30) Priority:
**24.12.2003 GB 0330016**
**15.01.2004 WOPCT/IB2004/000655**
**16.07.2004 GB 0416023**
**26.07.2004 US 898775**

(43) Date of publication of application:
**27.09.2006 Bulletin 2006/39**

(60) Divisional application:
**07017066.7**

(73) Proprietor: **DANISCO A/S**
**1001 Copenhagen K. (DK)**

(72) Inventors:
- **KRISTENSEN, Anna Cecilie Jentoft**
  **8000 Arhus C (DK)**
- **WASSELL, Paul**
  **Hastings, East Sussex TN34 2PH (GB)**
- **MIKKELSEN, Jorn Dalgaard**
  **2650 Hividovre (DK)**
- **SOE, Jorn Borch**
  **8381 Tilst (DK)**

(74) Representative: **Williams, Aylsa**
**D Young & Co**
**120 Holborn**
**London EC1N 2DY (GB)**

(56) References cited:
**US-A- 5 219 744**

- **UPTON ET AL: "A new family of lipolytic enzymes ?" TRENDS IN BIOCHEMICAL SCIENCES, vol. 20, May 1995 (1995-05), pages 178-179, XP004222260**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

FIELD OF INVENTION

The present invention relates to a novel method for the enzymatic removal and/or reduction of diglyceride (preferably 1,2-diacylglyceride) from an edible oil.

TECHNICAL BACKGROUND

Oils and fats consist of complex mixtures of triacylglycerols (TAGs), diacylglycerols (DAGs), free fatty acids and other minor components. The crystallisation of these mixtures depends on the characteristics of the TAGs (structure, chain length, saturation compared with unsaturation, and the like) and the interaction of these TAGs with each other. Regarding the presence of DAGs, previous studies have shown that they have significant effect on the physical properties of oils and fats. These vary from rate of crystallisation, polymorphism changes, melting point, crystal size and habits (Siew, 2001).

In most oils that are extracted from oilseeds, the effect of DAGs is less pronounced, as DAGs are only present in small quantities. In primarily palm oil and olive oil, which are oils containing high natural amounts of DAGs, however, the quality of these oils suffers if DAGs are present therein.

Palm oil obtained from oil palm (*Elaeis guineensis*) is commercially important edible oil. Palm oil has been a prominent fat and oil resource for the food industry due to several advantageous properties, such as high productivity, low price, high thermal and oxidative stability and plasticity at room temperature. In addition, compared with other vegetable oils, palm oil is a rich source of the anti-oxidant vitamin E.

A typical chemical composition of refined palm oil is about 93% triglycerides, 6% diglycerides and 1% monoglycerides (MAGs) (Okiy, 1977).

When palm oil crystallizes, a complex 3-dimensional network of the present components is formed. In the theory it is described that the bigger the diversity of the building blocks (TAGs, DAGs and MAGS) in the network, the more complicated the network will be and the slower the crystallization will happen (Jacobsberg & Ho, 1976). This theory was confirmed by Drozdowski (1994). Furthermore, his studies showed that the more the fatty acid composition variation in the triacylglycerol molecule, the more difficult was the transition between the different crystal phases.

As previously mentioned, a high content of diglycerides in palm oil affects its crystallization properties (Okiy *et al.,* 1978, Okiy, 1978).

The presence of diglycerides in such oils is disadvantageous. In particular, diglycerides in edible oils (in particular palm oil) can lead to a low quality oil.

The problems relating to the diglyceride content in palm oil and other edible oils and fat have been the subject of many studies and different solutions to attempt to overcome the problem of too much diglyceride can be found in the literature.

The Japanese enzyme producer Amano on their home page (Amano Enzyme Inc., 2004), recommend an enzymatic process to remove diglyceride in fats and oils. This process is based on the use of an enzyme LIPASE G "AMANO" 50 which is able to degrade diglycerides to free fatty acids and glycerol. This enzyme is a diglyceride (DAG) and/or monoglyceride (MAG) hydrolyzing. The free fatty acids produced are removed by vacuum distillation or fractional crystallisation.

EP 0 558 112 describes a process for the enzymatic hydrolysis of residual diglycerides in triglyceride preparations in emulsions. The process is based on the hydrolysis of diglyceride with Lipase G from Amano, Japan (*supra*). The process was enhanced by making the enzymatic reaction in an emulsion for the degradation of diglyceride to fatty acids and glycerol. The water phase is separated after reaction and the enzyme is partly reused.

JP 62061590 teaches a hard butter containing low amounts of diglyceride, which is manufactured by treating oils or fats with a partially glyceride-specific enzyme (e.g., a lipase) in the presence of a catalytic amount of water and by a lipase that is a 1,3-specific enzyme in the presence of fatty acids, fatty acid esters, or other glyceride oils or fats. The product is hard butter especially suitable for use as a cacao butter substitute. Thus, lipase G and *Rhizopus deremer* lipase (1,3-specific enzyme) were mixed with diatomaceous earth and granulated. The granules was mixed with palm medium melting point fraction (5.7% diglyceride, acid value 0.25) and water (10% with regard to the partial glyceride-specific enzyme). The mixture was stirred at room temp. for 1 h, and the enzymes and water removed to give a hard butter containing 1.2% diglyceride (acid value 10.5).

The prior art thus teaches ways to reduce or remove the content of diglyceride in palm oil and other edible oils by enzymatic reactions. These processes rely on the hydrolysis of diglyceride with a specific diglyceride hydrolysing lipase during formation of free fatty acids and glycerol. The free fatty acids can then be removed by means of different processes like vacuum distillation or fractionation.

The disadvantage of using a specific diglyceride hydrolysing enzyme is the disadvantageous formation of free

fatty acids. These free fatty acids have to be removed from the palm oil. Thus, the formation of free fatty acids is often considered as loss of product.

To overcome the problems with the removal of free fatty acid and the loss of product caused by the free fatty acid formation we have found a new method to overcome the problems with high diglyceride content in palm oil and other vegetable oils.

Enzymatic removal of diglycerides from palm oil has been taught by use of lipases, which are typically 1,3 specific triacylglycerol hydrolyzing enzymes (E.C. 3.1.1.3) (for example see JP 62061590 or EP 0 652 289). WO00/05396 teaches *inter alia* treatment of a food material which may comprise glycerol with a lipase to effect glycerolysis in a low water environment.

However, both 1,3 specific triacylglycerol hydrolyzing enzymes (lipases) and DAG/MAG hydrolyzing enzymes result in a significant increase in free fatty acid in the oil, and also result in the hydrolysis of monoglycerides.

However, in some vegetable oils for some applications for example it may be desirable to increase the monoglyceride content of the oil as this provides emulsifier functionality. Thus, in one aspect it is preferable to reduce diglyceride content without decreasing the monoglyceride content. In another aspects it may preferably to reduce both the diglyceride and monoglyceride content.

Lipase enzymes can also result in a detrimental increase in DAG due to the hydrolysis of triacylglycerol (TAG), the bulk lipid present in food oils.

In W02000/36114, US2003/0028923 and US2003/0074695 the transformation of a plant with a nucleic acid having a sequence which encodes a diacylglycerol acyltransferase (DGAT) enzyme or an antisense sequence thereto is taught. The DGAT enzyme taught in these documents catalyses the final step in the "Kennedy pathway" where a diacylglycerol (DAG) is combined with the acyl groups of acyl CoA to form a triglyceride (TAG). Thus, these documents teach the production of transgenic plants with modified TAG compositions and/or contents. The DGAT enzymes taught in these documents are not lipid acyl transferases and/or diglyceride:glycerol acyltransferases in accordance with the present invention.

In particular, DGAT's require the presence of acyl CoA or fatty acid CoA to function. Acyl CoA is not suitable for use commercially for treating edible oils as it is prohibitively expensive. However, these enzymes will not work without the present of acyl CoA. In addition enzyme reactions relying on fatty acid-CoA are very difficult to control industrially.

In addition, all of these documents teach that it is often desirable to reduce the expression of DGAT enzymes in a plant, thus to reduce the amount of TAG in the plant. This contrasts sharply with the present invention which ultimately requires the preservation and/or production of TAG whilst reducing diglycerides (DAG) in an edible oil.

WO03/100044 teaches a phospholipids:diacylglycerol acyltransferase (PDAT) which catalyses the formation of triglycerides (TAGs) by an acyltransfer from phospholipids (lecithin) to *inter alia* diacylglycerols (DAGs). PDATs require phospholipid as the acyl donor. This contrasts sharply with the present invention where the acyl donor is DAG. This document does not teach the removal of DAG from edible oils using a lipid acyltransferase in accordance with the present invention.

## SUMMARY OF THE INVENTION

It has been found that the use of fatty-acid CoA independent lipid acyltransferases as defined herein, specifically fatty-acid CoA independent diglyceride:glycerol acyltransferases, results in the selective reduction and/or removal of diglycerides (preferably 1,2-diglycerides) from edible oils.

The term "selective" as used herein means that in an edible oil environment the enzyme utilizes diglycerides (DAGs), preferably 1,2-diglycerides, as a substrate preferentially to either triacylglycerides (TAGs) or monoglycerides (MAGs). Thus, diglycerides can be removed and/or reduced from the edible oil whilst leaving the amount of triglyceride in the oil unchanged (or substantially unchanged). The amount of monoglycerides in the oil either remains unchanged (or substantially changed) or may increase. In some applications, the amound of monoglyceride in the oil may be reduced.

In an aspect of the present invention there is provided a method of reducing and/or removing diglyceride from an edible oil, comprising a) admixing an edible oil with an acyl acceptor substrate and a fatty-acid CoA independent diglyceride:glycerol acyltransferase, wherein the fatty-acid CoA independent diglyceride:glycerol acyltransferase is characterized as an enzyme which in an edible oil can transfer an acyl group from a diglyceride to glycerol wherein the CoA independent diglyceride:glycerol acyltransferase according to the present invention is an acyltransferase which comprises the amino acid sequence motif GDSX, wherein X is one or more of the following amino acid residues L, A, V, I, F, Y, H, Q, T, N, M or S.

Suitably, the method according to the present invention may further comprise adding the treated edible oil or a portion thereof to one or more food constituents to formulate a foodstuff, such as margarine or spread for example.

In a further aspect, the present invention yet further provides the use of a fatty-acid CoA independent diglyceride: glycerol acyltransferase characterized as an enzyme which in an edible oil can transfer an acyl group from a diglyceride to glycerol, in the manufacture of a foodstuff, for improving the crystallization properties of the foodstuff, wherein, the

CoA independent diglyceride:glycerol acyltransferase according to the present invention is an acyltransferase which comprises the amino acid sequence motif GDSX, wherein X is one or more of the following amino acid residues L, A, V, I,F,Y,H,Q,T,N,M or S.

In another aspect the present invention provides the use of a fatty-acid CoA independent diglyceride:glycerol acyltransferase characterized as an enzyme which in an edible oil can transfer an acyl group from a diglyceride to glycerol, in the manufacture of an edible oil, for reducing and/or removing (preferably selectively reducing and/or removing) diglyceride from said edible oil wherein the CoA independent diglyceride:glycerol acyltransferase according to the present invention is an acyltransferase which comprises the amino acid sequence motif GDSX, wherein X is one or more of the following amino acid residues L, A, V, I, Y, H, Q, T, N, M or S.

The use of a fatty-acid CoA independent diglyceride glycerol acyltransferase characterized as an enzyme which in an edible oil can transfer an acyl group from a diglyceride to glycerol, in the manufacture of a foodstuff for reducing and/or removing (preferably selectively reducing and/or removing) diglyceride from said foodstuff is taught herein.

The use of a fatty-acid CoA independent diglyceride:glycerol acyltransferase characterized as an enzyme which in an edible oil can transfer an acyl group from a diglyceride to glycerol, in the manufacture of an edible oil for improving the crystallization properties of the edible oil is also taught herein.

## DETAILED DISCLOSURE OF INVENTION

The terms "fatty-acid CoA independent lipid acyltransferase" and "fatty-acid CoA independent diglyceride: glycerol acyltransferase" as used herein means an enzyme which has acyltransferase activity (generally classified as E.C. 2.3.1.x in accordance with the Enzyme Nomenclature Recommendations (1992) of the Nomenclature Committee of the International Union of Biochemistry and Molecular Biology), whereby the enzyme is capable of transferring an acyl group from a diglyceride to one or more acceptor substrates).

Thus, the "fatty-acid CoA independent lipid acyltransferase" or "fatty-acid CoA independent diglyceride:glycerol acyltransferase" is an enzyme which has acyltransferase activity (generally classified as E.C. 2.3.1.x), but which is not a diacylglycerol acyltransferase (DGAT) or a phospholipid:diacylglycerol acyltransferase (PDAT). DGATs are typically classified as E.C. 2.3.1.20. PDATs are typically classified as E.C. 2.3.1.158. Thus, the fatty-acid CoA independent lipid acyltransferase or fatty-acid CoA independent diglyceride:glycerol acyltransferase is an enzyme which has acyltransferase activity, but which is not any enzyme classified as E.C. 2.3.1.20 or E.C. 2.3.1.158.

The fatty-acid CoA independent lipid acyltransferase enzyme or fatty-acid CoA independent diglyceride:glycerol acyltransferase enzyme is one which is capable in an edible oil of transferring an acyl group from DAG to glycerol. Thus, the reaction catalysed by the enzyme according to the present invention is:

$$\text{Diglyceride (DAG) + glycerol} \rightarrow \text{2 monoglycerides (MAGs)}$$

This contrasts sharply with enzymes known as diacylglycerol acyltransferases (or diacylglycerol *O*-acyltransferase) (DGATs) (such enzymes are classified as E.C. 2.3.1.20) which catalyse the final step in the Kennedy process, namely:

$$\text{1,2-DAG + acyl CoA} \rightarrow \text{CoA + triacylglycerol (TAG)}$$

For the avoidance of doubt, DGATs are not fatty-acid CoA independent lipid acyltransferases or fatty-acid CoA independent diglyceride:glycerol acyltransferases in accordance with the present invention.

The reaction catalysed by the enzyme according to the present invention also contrasts sharply with that catalysed by enzymes known as phospholipids:diacylglycerol acyltransferase (PDAT), namely:

$$\text{Phospholipids (such as lecithin) + 1,2.DAG} \rightarrow \text{triacylglycerol (TAG) + lysophospholipid}$$

For the avoidance of doubt, PDATs are not fatty-acid CoA independent lipid acyltransferases or fatty-acid CoA independent diglyceride:glycerol acyltransferases in accordance with the present invention.

Preferably, the fatty-acid CoA independent lipid acyltransferase or fatty-acid CoA independent diglyceride: glycerol acyltransferase is an enzyme classified as E.C. 2.3.1.73.

Suitably, the fatty-acid CoA independent diglyceride:glycerol acyltransferase may be membrane independent, i.e. may be a protein that is not, in its natural environment, associated with a membrane by the presence of a membrane anchor or membrane spanning domain.

For the avoidance of doubt, the fatty-acid CoA independent lipid acyltransferase is not an enzyme taught in any one of WO03/100044, W02000/36114, US2003/0028923 or US2003/0074695.

As well as having acyltransferase activity the enzyme may have lipase activity, e.g. phospholipase activity,

(generally classified as E.C. 3.1.1.x).

The fatty-acid CoA independent lipid acyltransferase is a fatty-acid CoA independent diglyceride:glycerol acyltransferase. These terms may be used interchangeably herein.

The fatty-acid CoA independent diglyceride:glycerol acyltransferase is an acyltransferase which comprises the amino acid sequence motif GDSX, wherein X is one or more of the following amino acid residues L, A, V, I, F, Y, H, Q, T, N, M or S.

The term "diglyceride:glycerol acyltransferase" as used herein is synonymous with the term "fatty-acid CoA independent diglyceride:glycerol acyltransferase".

For the avoidance of doubt, the term "fatty-acid CoA" is the same as the terms "Acyl-CoA", "fatty-acid enzyme CoA" and "Acyl-Co enzyme A". These terms may be used interchangeably herein.

The edible oil used in a process or use according to the present invention may be in the form of a crude oil or may be a refined oil.

In one embodiment, preferably the amount of diglyceride is reduced rather than completely removed.

The term "reduced" as used herein means that the amount of diglyceride in an edible oil treated with the enzyme in accordance with the present invention is less than the amount of diglyceride in the edible oil before enzyme treatment.

Preferably, diglycerides are not completely removed from the edible oil.

In some applications, such as the use of the treated oil in margarines and/or shortening, the amount of diglycerides, particularly 1,2 diglycerides, should be reduced to a point

where the crystallisation speed of the fat blend produces small beta-crystals. The amount of 1,2-diglyceride to the amount of total diglyceride in the palm oil depends on the age and storage conditions of the oil. For commercial oils the ratio of 1,3 diglyceride: 1,2 diglyceride is 1.8:3.3. The removal of 1,2 glycerides will have the most impact on the crystallisation properties.

As will be readily apparent to the skilled person the reduction in the amount of diglyceride can be controlled by the reaction time and temperature of the reaction. In a flow reactor with an immobilised enzyme the reaction may be controlled by the flow rate.

The lipid acyltransferase for use in the methods and/or uses of the present invention is capable of transferring an acyl group from a diglyceride to an acyl acceptor, wherein the acyl acceptor is any compound comprising a hydroxy group (-OH).

Suitably, the term "diglyceride" as used herein means one or more of 1,2-diglyceride or 1,3-diglyceride. Preferably, the diglyceride is a 1,2-diglyceride.

The terms "diglyceride" and "diacylglycerol" are used herein interchangeably.

The term "diglyceride" does not encompass digalactosyldiglyceride (DGDG) and/or lecithin, e.g. phosphatidylcholine.

The acyl acceptor is one which is soluble in an edible oil.

The acyl acceptor is glycerol.

Thus, in one embodiment the present invention provides a method of reducing and/or removing diglyceride from an edible oil, comprising a) admixing an edible oil with both glycerol and a fatty-acid CoA independent diglyceride: glycerol acyltransferase, wherein the fatty-acid CoA independent diglyceride:glycerol acyltransferase is characterized as an enzyme which possesses acyl transferase activity and which comprises the amino acid sequence motif GDSX, wherein X is one or more of the following amino acid residues L, A, V, I, F, Y, H, Q, T, N, M or S.

Preferably, the fatty-acid CoA independent lipid acyltransferase is capable of cleaving the acyl bond between a fatty acid residue(s) and a glycerol backbone of a lipid substrate, wherein preferably the lipid substrate is a diglyceride, preferably 1,2-diglyceride.

Preferably, the fatty-acid CoA independent lipid acyltransferase does not act on triglycerides and/or monoglycerides. In other words, preferably the fatty-acid CoA independent lipid acyltransferase is selective for diglycerides, preferably selective for 1,2-diglycerides. This lipid substrate may be referred to herein as the "lipid acyl donor".

Thus in accordance with the present invention, one or more of the following advantageous properties can be achieved: reduction in diglyceride content of an edible oil; a reduction in the diglyceride content of an edible oil without a reduction in the triglyceride content of the edible oil; a reduction in the diglyceride content of the edible oil without an increase in the monoglyceride content; a reduction in the diglyceride content of the edible oil with an increase in the monoglyceride content; a reduction of diglyceride and a reduction in monoglyceride content of an edible oil; a reduction in the diglyceride content of the edible oil without a significant increase in the fatty acid content in the edible oil.

Preferably, the fatty-acid CoA independent lipid acyltransferase performs an alcoholysis reaction (glycerolysis) by transferring a fatty acid acyl group from the diglyceride (preferably the 1,2-DAG) to an alcohol (glycerol) thereby producing two monoglyceride molecules, i.e. one from the diglyceride and one from the glycerol together with the accepted acyl group.

Preferably, X of the GDSX motif is L. Thus, preferably the enzyme comprises the amino acid sequence motif GSDL.

The GDSX motif is comprised of four conserved amino acids. Preferably, the serine within the motif is a catalytic serine of the lipid acyltransferase enzyme. Suitably, the seine of the GDSX motif may be in a position corresponding to Ser-16 in *Aeromonas hydrophila* lipolytic enzyme taught in Brumlik & Buckley (Journal of Bacteriology Apr. 1996, Vol. 178, No. 7, p 2060-2064).

To determine if a protein has the GDSX motif, the sequence is preferably compared with the hidden markov model profiles (HMM profiles) of the pfam database.

Pfam is a database of protein domain families. Pfam contains curated multiple sequence alignments for each family as well as profile hidden Markov models (profile HMMs) for identifying these domains in new sequences, An introduction to Pfam can be found in Bateman A et al. (2002) Nucleic Acids Res. 30; 276-280. Hidden Markov models are used in a number of databases that aim at classifying proteins, for review see Bateman A and Haft DH (2002) Brief Bioinform 3; 236-2 45.
http://www.ncbi.nlm.nih.gov/entrez/query.fcgi?cmd=Retrieve&db=PubMed&list uids =12230032&dopt=Abstract
http://www.ncbi.nlm.nih.gov/entrez/query.fcgi?cmd=Retrieve&db=PubMed&list uids =11752314&dopt=Abstract

For a detailed explanation of hidden Markov models and how they are applied in the Pfam database see Durbin R, Eddy S, and Krogh A (1998) Biological sequence analysis; probabilistic models of proteins and nucleic acids. Cambridge University Press, ISBN 0-521-62041-4. The Hammer software package can be obtained from Washington University, St Louis, USA.

Alternatively, the GDSX motif can be identified using the Hammer software package, the instructions are provided in Durbin R, Eddy S, and Krogh A (1998) Biological sequence analysis; probabilistic models of proteins and nucleic acids. Cambridge University Press, ISBN 0-521-62041-4 and the references therein, and the HMMER2 profile provided within this specification.

The PFAM database can be accessed, for example, through several servers which are currently located at the following websites.
http://www.sanger.ac.uk/Software/Pfam/index.shtml
http://pfam.wustl.edu/
http://pfam.jouy.inra.fr/
http://pfam.cgb.ki.se/

The database offers a search facility where one can enter a protein sequence. Using the default parameters of the database the protein sequence will then be analysed for the presence of Pfam domains. The GDSX domain is an established domain in the database and as such its presence in any query sequence will be recognised . The database will return the alignment of the Pfam00657 consensus sequence to the query sequence:

A multiple alignment, including *Aeromonas salmonicida* or *Aeromonas hydrophila* can be obtained by:

a) manual
obtain an alignment of the protein of interest with the Pfam00657 consensus sequence and obtain an alignment of P10480 with the Pfam00657 consensus sequence following the procedure described above;
or

b) through the database
After identification of the Pfam00657 consensus sequence the database offers the option to show an alignment of the query sequence to the seed alignment of the Pfam00657 consensus sequence. P10480 is part of this seed alignment and is indicated by GCAT_AERHY. Both the query sequence and P10480 will be displayed in the same window.

The *Aeromonas hydrophila* reference sequence:

The residues of *Aeromonas hydrophila* GDSX lipase are numbered in the NCBI file P10480, the numbers in this text refer to the numbers given in that file which in the present invention is used to determine specific amino acids residues which, in a preferred embodiment are present in the lipid acyltransferase enzymes of the invention.

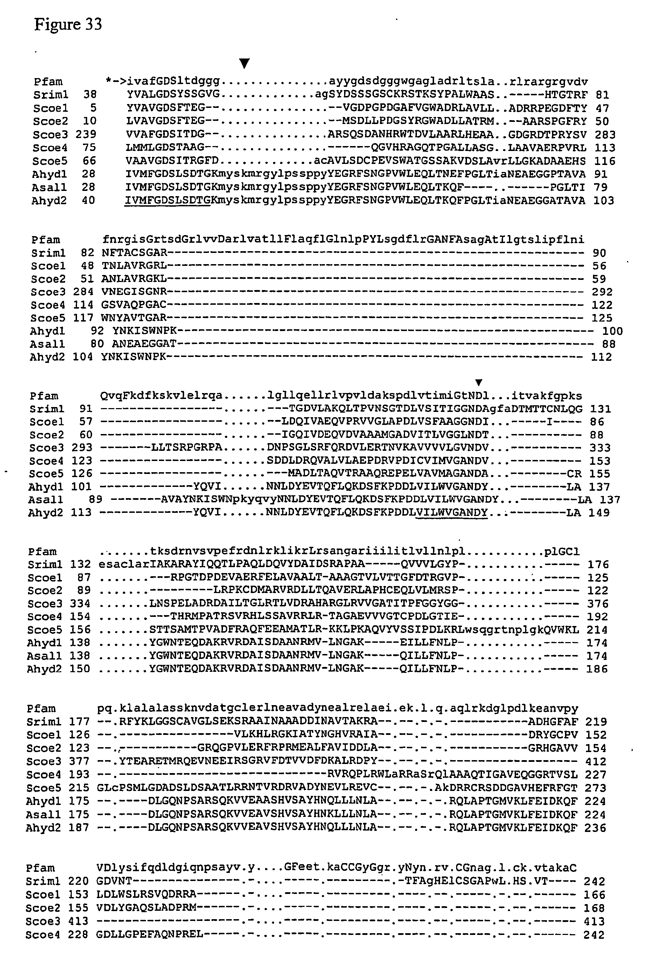

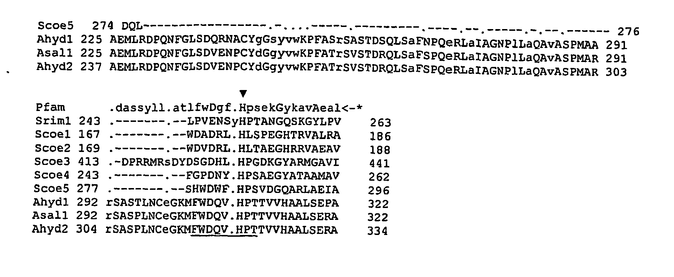

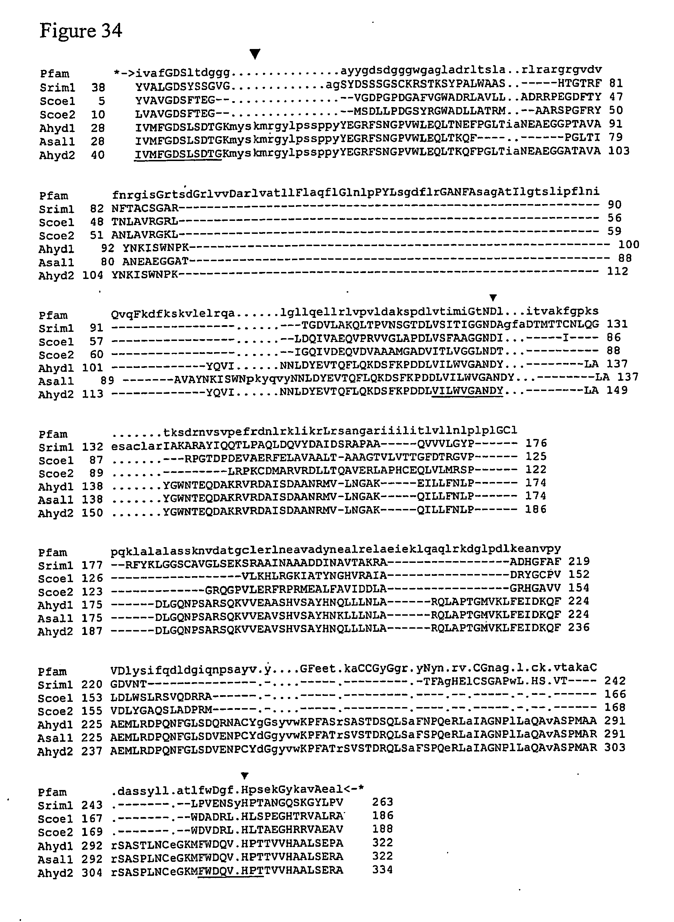

**The Pfam alignment was performed (Figure 33 and 34):**

The following conserved residues can be recognised and in a preferable embodiment may be present in the enzymes for use in the compositions and methods of the invention;

Block 1 - GDSX block
hid hid hid hid Gly Asp Ser hid

(continued)

| | | | | | | | |
|---|---|---|---|---|---|---|---|
| Block 1 - GDSX block | | | | | | | |
| 28 | 29 | 30 | 31 | 32 | 33 | 34 | 35 |
| Block 2 - GANDY block | | | | | | | |
| hid | Gly | hid | Asn | Asp | hid | | |
| 130 | 131 | 132 | 133 | 134 | 135 | | |
| Block 3 - HPT block | | | | | | | |
| His | | | | | | | |
| 309 | | | | | | | |

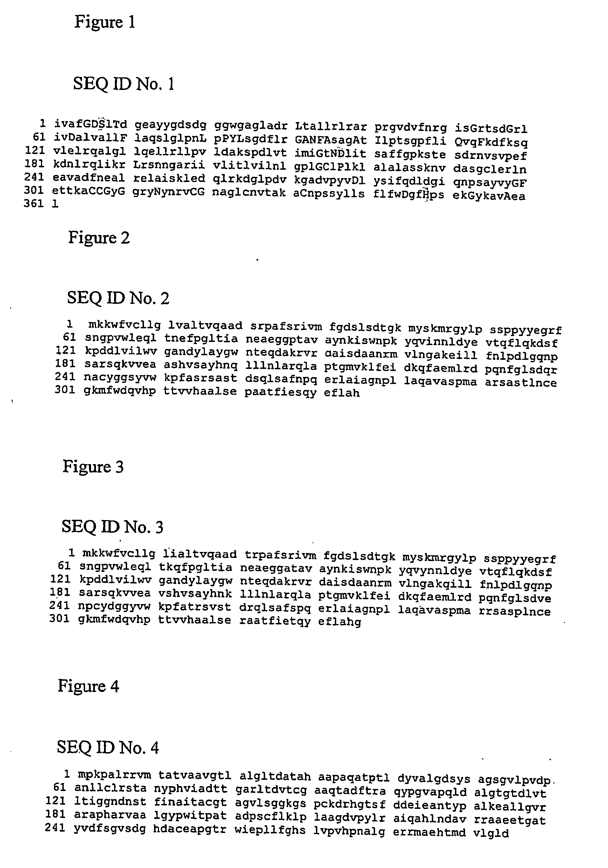

Where 'hid' means a hydrophobic residue selected from Met, Ile, Leu, Val, Ala, Gly, Cys, His, Lys, Trp, Tyr, Phe.
Preferably the lipid acyltransferase enzyme for use in the compositions/methods of the invention can be aligned using the Pfam00657 consensus sequence.
Preferably, a positive match with the hidden markov model profile (HMM profile) of the pfam00657 domain family indicates the presence of the GDSL or GDSX domain according to the present invention.
Preferably when aligned with the Pfam00657 consensus sequence the lipid acyltransferase for use in the compositions/methods of the invention have at least one, preferably more than one, preferably more than two, of the following, a GDSx block, a GANDY block, a HPT block. Suitably, the lipid acyltransferase may have a GDSx block and a GANDY block. Alternatively, the enzyme may have a GDSx block and a HPT block. Preferably the enzyme comprises at least a GDSx block.
Preferably, when aligned with the Pfam00657 consensus sequence the enzyme for use in the compositions/ methods of the invention have at least one, preferably more than one, preferably more than two, preferably more than three, preferably more than four, preferably more than five, preferably more than six, preferably more than seven, preferably more than eight, preferably more than nine, preferably more than ten, preferably more than eleven, preferably more than twelve, preferably more than thirteen, preferably more than fourteen, of the following amino acid residues when compared to the reference *A.hydrophilia* polypeptide sequence, namely SEQ ID No. 32: 28hid, 29hid, 30hid, 31hid, 32gly, 33Asp, 34Ser, 35hid, 130hid, 131Gly, 132Hid, 133Asn, 134Asp, 135hid, 309His
The pfam00657 GDSX domain is a unique identifier which distinguishes proteins possessing this domain from other enzymes.
The pfam00657 consensus sequence is presented in Figure 1 as SEQ ID No. 1. This is derived from the identification of the pfam family 00657, database version 6, which may also be referred to as pfam00657.6 herein.
The consensus sequence may be updated by using further releases of the pfam database.
For example, Figures 33 and 34 show the pfam alignment of family 00657, from database version 11, which may also be referred to as pfam00657.11 herein.
The presence of the GDSx, GANDY and HPT blocks are found in the pfam family 00657 from both releases of the database. Future releases of the pfam database can be used to identify the pfam family 00657.
Preferably, the fatty-acid CoA independent lipid acyltransferase enzyme may be characterised using the following criteria:

(i) the enzyme possesses acyl transferase activity which may be defined as ester transfer activity whereby the acyl part of an original ester bond of a lipid acyl donor is transferred to acyl acceptor to form a new ester;
(ii) the enzyme comprises the amino acid sequence motif GDSX, wherein X is one or more of the following amino acid residues L, A, V, I, F, Y, H,Q,T,N,MorS.;
(iii) the enzyme comprises His-309 or comprises a histidine residue at a position corresponding to His-309 in the *Aeromonas hydrophila* lipolytic enzyme shown in Figure 2 (SEQ ID No. 2 or SEQ ID No. 32).

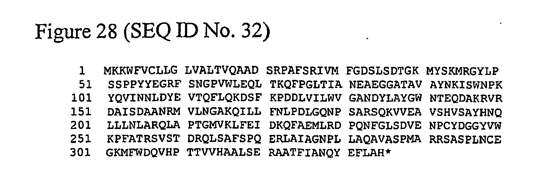

Preferably, the amino acid residue of the GDSX motif is L.
In SEQ ID No. 2 or SEQ ID No. 32 the first 18 amino acid residues form a signal sequence. His-309 of the full length sequence, that is the protein including the signal sequence, equates to His-291 of the mature part of the protein, i.e. the sequence without the signal sequence.
Preferably, the fatty-acid CoA independent lipid acyltransferase enzyme comprises the following catalytic triad: Ser-34, Asp-134 and His-309 or comprises a serine residue, an aspartic acid residue and a histidine residue, respectively, at positions corresponding to Ser-34, Asp-134 and His-309 in the *Aeromonas hydrophila* lipolytic enzyme shown in Figure 2 (SEQ ID No. 2) or Figure 28 (SEQ ID No. 32). As stated above, in the sequence shown in SEQ ID No. 2 or

SEQ ID No. 32 the first 18 amino acid residues form a signal sequence. Ser-34, Asp-134 and His-309 of the full length sequence, that is the protein including the signal sequence, equate to Ser-16, Asp-116 and His-291 of the mature part of the protein, i.e. the sequence without the signal sequence. In the pfam00657 consensus sequence, as given in Figure 1 (SEQ ID No. 1) the active site residues correspond to Ser-7, Asp-157 and His-348.

Preferably, the fatty-acid CoA independent lipid acyltransferase enzyme may be characterised using the following criteria:

(i) the enzyme possesses acyl transferase activity which may be defined as ester transfer activity whereby the acyl part of an original ester bond of a first lipid acyl donor is transferred to an acyl acceptor to form a new ester; and
(ii) the enzyme comprises at least Gly-32, Asp-33, Ser-34, Asp-134 and His-309 or comprises glycine, aspartic acid, serine, aspartic acid and histidine residues at positions corresponding to Gly-32, Asp-33, Ser-34, Asp-134 and His-309, respectively, in the *Aeromonas hydrophila* lipolytic enzyme shown in Figure 2 (SEQ ID No. 2) or Figure 28 (SEQ ID No. 32).

Suitably, the fatty-acid CoA independent lipid acyltransferase enzyme may be obtainable, preferably obtained, from organisms from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* and *Candida.*

Suitably, the fatty-acid CoA independent lipid acyltransferase enzyme may be obtainable, preferably obtained, from one or more of the following organisms: *Aeromonas hydrophila, Aeromonas salmonicida, Streptomyces coelicolor, Streptomyces rimosus, Mycobacterium, Streptococcus pyogenes, Lactococcus lactis, Streptococcus pyogenes, Streptococcus thermophilus, Lactobacillus helveticus, Desulfitobacterium dehalogenans, Bacillus sp, Campylobacter jejuni, Vibrionaceae, Xylella fastidiosa, Sulfolobus solfataricus, Saccharomyces cerevisiae, Aspergillus terreus, Schizosaccharomyces pombe*, *Listeria innocua, Listeria monocytogenes, Neisseria meningitidis, Mesorhizobium loti, Ralstonia solanacearum, Xanthomonas campestris, Xanthomonas axonopodis and Candida parapsilosis.*

In one aspect, preferably the fatty-acid CoA independent lipid acyltransferase enzyme is obtainable, preferably obtained, from one or more *of Aeromonas hydrophila or Aeromonas salmonicida.*

Suitably, the fatty-acid CoA independent lipid acyltransferase enzyme comprises one or more of the following amino acid sequences:

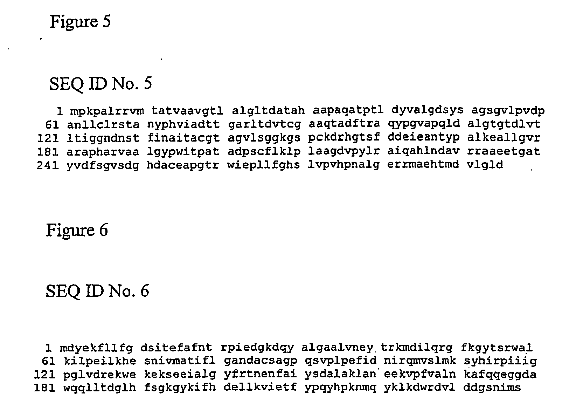

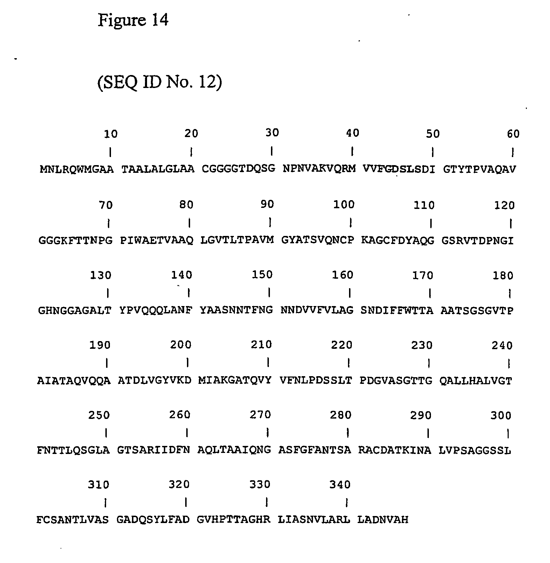

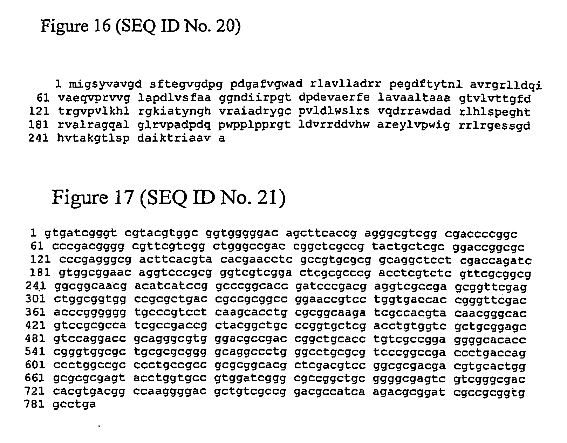

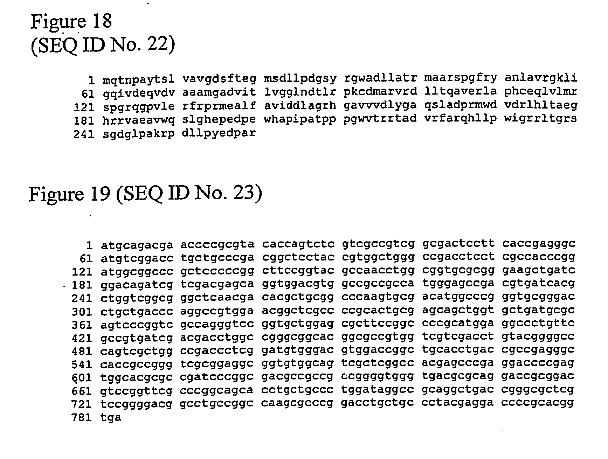

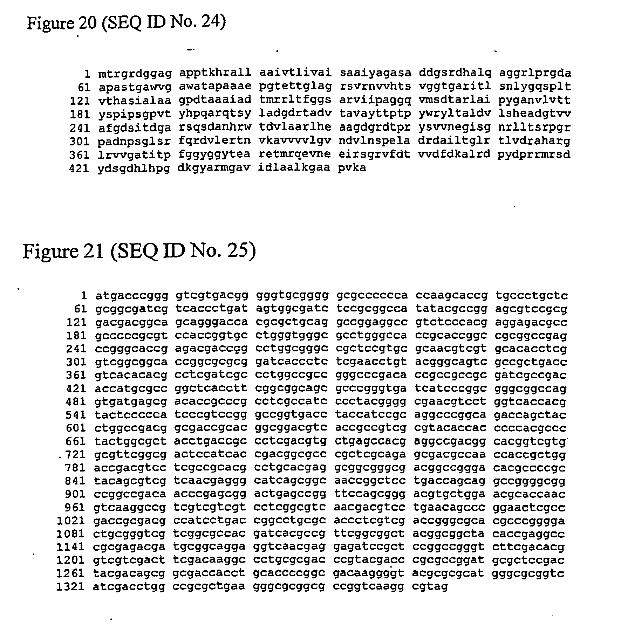

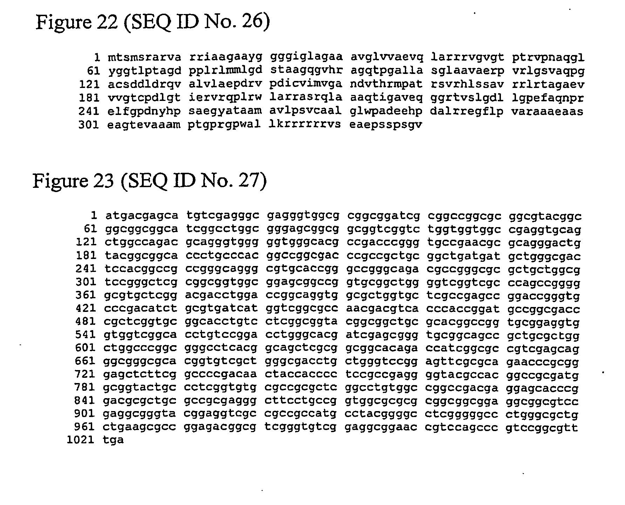

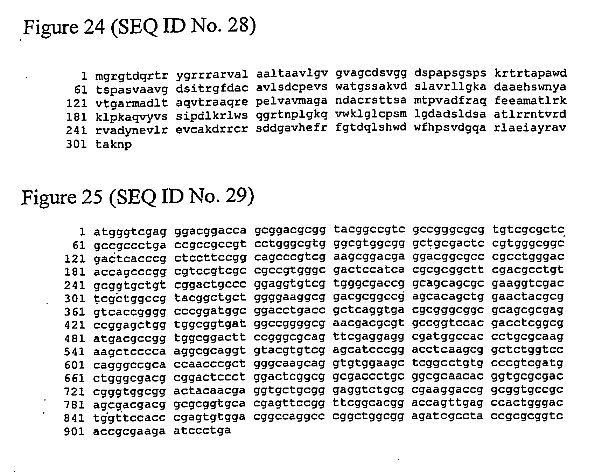

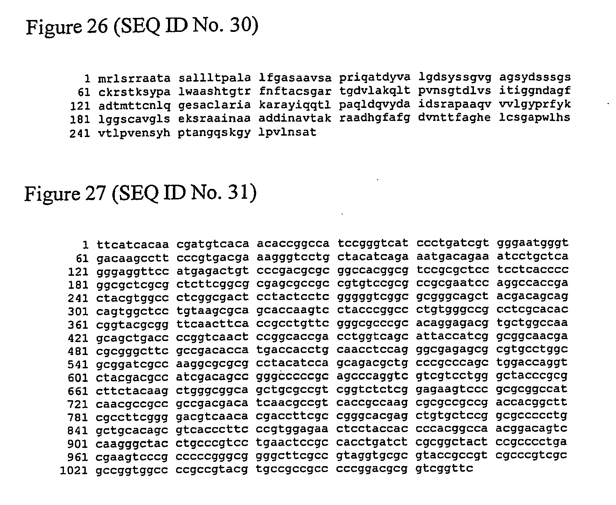

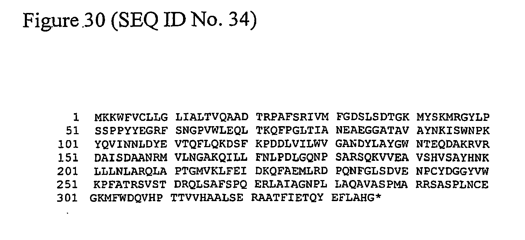

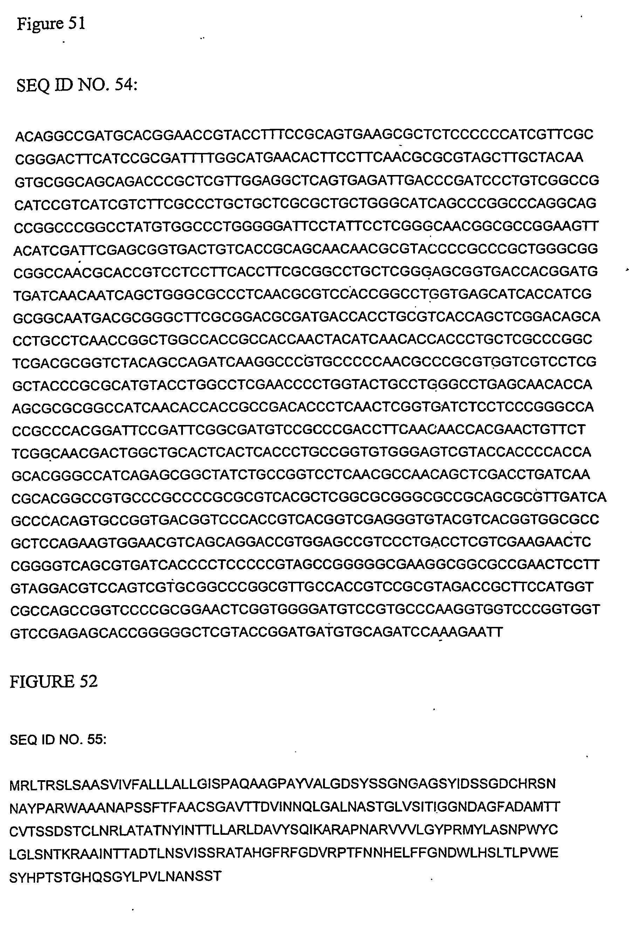

(i) the amino acid sequence shown as SEQ ID No. 2 (see Figure 2)
(ii) the amino acid sequence shown as SEQ ID No. 3 (see Figure 3)
(iii) the amino acid sequence shown as SEQ ID No. 4 (see Figure 4)
(iv) the amino acid sequence shown as SEQ ID No. 5 (see Figure 5)
(v) the amino acid sequence shown as SEQ ID No. 6 (see Figure 6)
(vi) the amino acid sequence shown as SEQ ID No. 12 (see Figure 14)
(vii) the amino acid sequence shown as SEQ ID No. 20 (Figure 16)
(viii) the amino acid sequence shown as SEQ ID No. 22 (Figure 18)
(ix) the amino acid sequence shown as SEQ ID No. 24 (Figure 20)
(x) the amino acid sequence shown as SEQ ID No. 26 (Figure 22)
(xi) the amino acid sequence shown as SEQ ID No. 28 (Figure 24)
(xii) the amino acid sequence shown as SEQ ID No. 30 (Figure 26)
(xiii) the amino acid sequence shown as SEQ ID No. 32 (Figure 28)
(xiv) the amino acid sequence shown as SEQ ID No. 34 (Figure 30)
*(xv)* the amino acid sequence shown as SEQ ID No. 55 (Figure 52)
*(xvi)* the amino acid sequence shown as SEQ ID No. 58
*(xvii)* the amino acid sequence shown as SEQ ID No. 60
*(xviii)* the amino acid sequence shown as SEQ ID No. 61
*(xix)* the amino acid sequence shown as SEQ ID No. 63
(xx) the amino acid sequence shown as SEQ ID No. 65
(xxi) the amino acid sequence shown as SEQ ID No. 67
(xxii) the amino acid sequence shown as SEQ ID No. 70 or
(xxiii) an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32, SEQ ID No. 34, SEQ ID No. 55, SEQ ID No. 58, SEQ ID No. 60, SEQ ID No. 61, SEQ ID No. 63, SEQ ID No: 65, SEQ ID No. 67 or SEQ ID No. 70.

Suitably, the fatty-acid CoA independent lipid acyltransferase enzyme comprises either the amino acid sequence

shown as SEQ ID No. 2 or as SEQ ID No. 3 or SEQ ID No. 32 or SEQ ID No. 34 or comprises an amino acid sequence which has 75% or more, preferably 80% or more, preferably 85% or more, preferably 90% or more, preferably 95% or more, identity with the amino acid sequence shown as SEQ ID No. 2 or the amino acid sequence shown as SEQ ID No. 3 or the amino acid sequence shown as SEQ ID No. 32 or the amino acid sequence shown as SEQ ID No. 34.

For the purposes of the present invention, the degree of identity is based on the number of sequence elements which are the same. The degree of identity may be suitably determined by means of computer programs known in the art, such as GAP provided in the GCG program package (Program Manual for the Wisconsin Package, Version 8, August 1994, Genetics Computer Group, 575 Science Drive, Madison, Wisconsin, US53711) (Needleman & Wunsch (1970), J. of Molecular Biology 48, 443-45) using the following settings for polypeptide sequence comparison: GAP creation penalty of 3.0 and GAP extension penalty of 0.1.

Suitably the fatty-acid CoA independent lipid acyltransferase enzyme comprises an amino acid sequence which has 80% or more, preferably 85% or more, more preferably 90% or more and even more preferably 95% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32, SEQ ID No. 34, SEQ ID No. 55, SEQ ID No. 58, SEQ ID No. 60, SEQ ID No. 61, SEQ ID No. 63, SEQ ID No. 65, SEQ ID No. 67 or SEQ ID No. 70.

Suitably, the fatty-acid CoA independent lipid acyltransferase enzyme comprises one or more of the following amino acid sequences:

(a) an amino acid sequence shown as amino acid residues 1-100 of SEQ ID No. 2 or SEQ ID No. 32;
(b) an amino acid sequence shown as amino acids residues 101-200 of SEQ ID No. 2 or SEQ ID No. 32;
(c) an amino acid sequence shown as amino acid residues 201-300 of SEQ ID No. 2 or SEQ ID No. 32; or
(d) an amino acid sequence which has 75% or more, preferably 85% or more, more preferably 90% or more, even more preferably 95% or more identity to any one of the amino acid sequences defined in (a)-(c) above.

Suitably, the fatty-acid CoA independent lipid acyltransferase enzyme comprises one or more of the following amino acid sequences:

(a) an amino acid sequence shown as amino acid residues 28-39 of SEQ ID No. 2 or SEQ ID No. 32;
(b) an amino acid sequence shown as amino acids residues 77-88 of SEQ ID No. 2 or SEQ ID No. 32;
(c) an amino acid sequence shown as amino acid residues 126-136 of SEQ ID No. 2 or SEQ ID No. 32;
(d) an amino acid sequence shown as amino acid residues 163-175 of SEQ ID No. 2 or SEQ ID No. 32;
(e) an amino acid sequence shown as amino acid residues 304-311 of SEQ ID No. 2 or SEQ ID No. 32; or
(f) an amino acid sequence which has 75% or more, preferably 85% or more, more preferably 90% or more, even more preferably 95% or more identity to any one of the amino acid sequences defined in (a)-(e) above.

Suitably, the fatty-acid CoA independent lipid acyltransferase enzyme may comprise an amino acid sequence produced by the expression or one or more of the following nucleotide sequences:

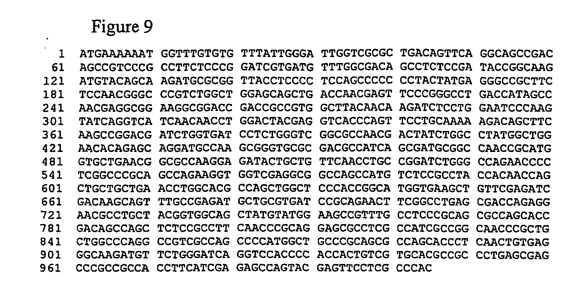

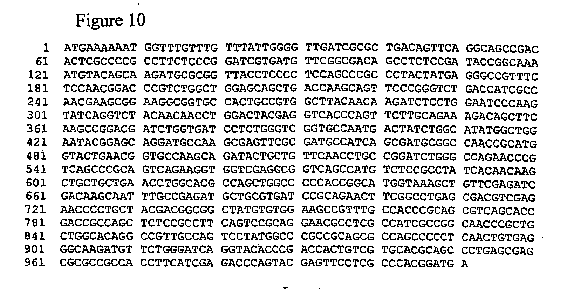

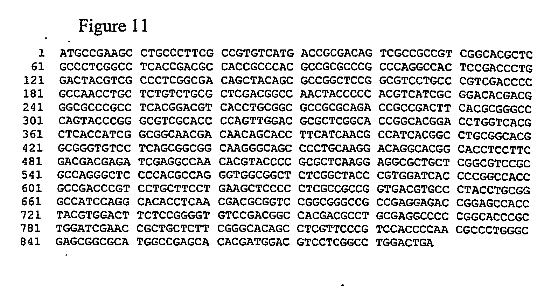

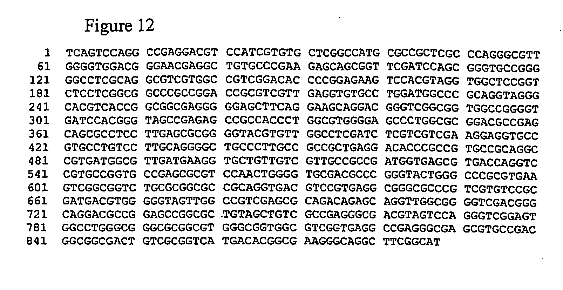

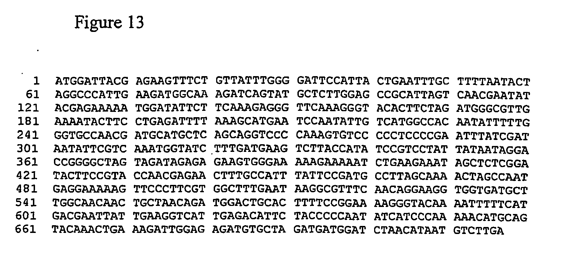

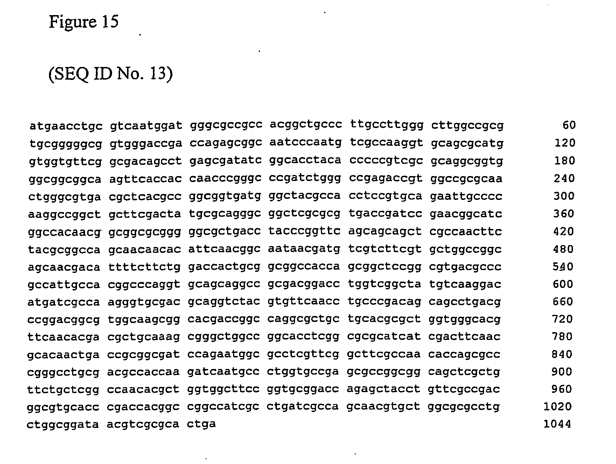

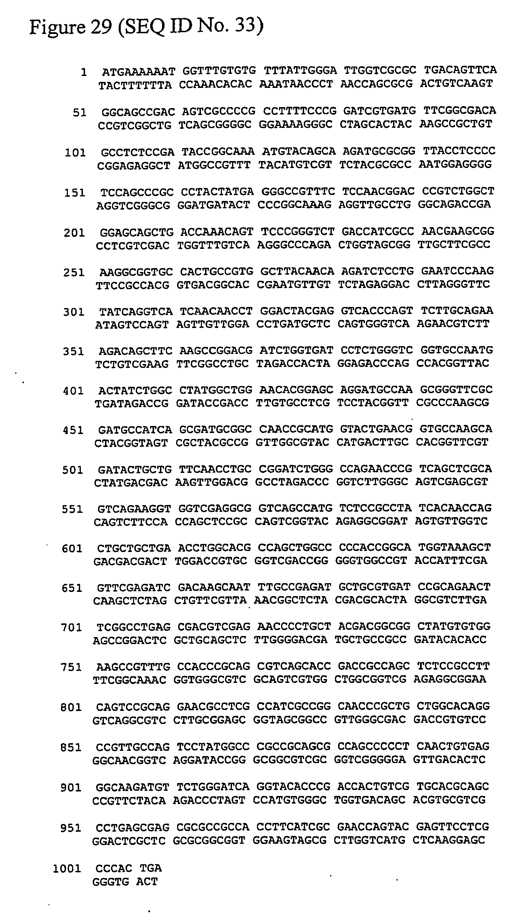

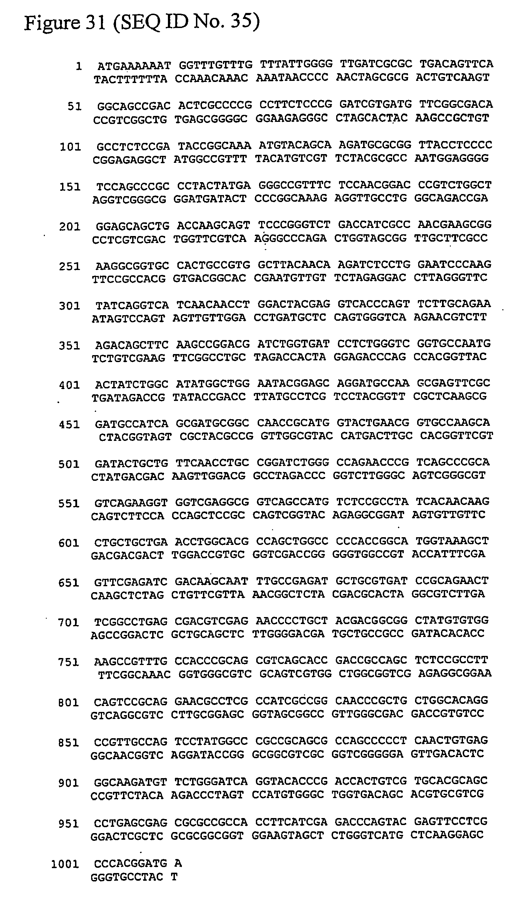

(a) the nucleotide sequence shown as SEQ ID No. 7 (see Figure 9);
(b) the nucleotide sequence shown as SEQ ID No. 8 (see Figure 10);
(c) the nucleotide sequence shown as SEQ ID No. 9 (see Figure 11);
(d) the nucleotide sequence shown as SEQ ID No. 10 (see Figure 12);
(e) the nucleotide sequence shown as SEQ ID No. 11 (see Figure 13);
(f) the nucleotide sequence shown as SEQ ID No. 13 (see Figure 15);
(g) the nucleotide sequence shown as SEQ ID No. 21 (see Figure 17);
(h) the nucleotide sequence shown as SEQ ID No. 23 (see Figure 19);
(i) the nucleotide sequence shown as SEQ ID No. 25 (see Figure 21);
(j) the nucleotide sequence shown as SEQ ID No. 27 (see Figure 23);
(k) the nucleotide sequence shown as SEQ ID No. 29 (see Figure 25);
(l) the nucleotide sequence shown as SEQ ID No. 31 (see Figure 27);
(m)the nucleotide sequence shown as SEQ ID No. 33 (see Figure 29);
(n) the nucleotide sequence shown as SEQ ID No. 35 (see Figure 31);
(o) the nucleotide sequence shown as SEQ ID No. 54 (Figure 51);
(p) the nucleotide sequence shown as SEQ ID No. 59;
(q) the nucleotide sequence shown as SEQ ID No. 62;
(r) the nucleotide sequence shown as SEQ ID No. 64;
(s) the nucleotide sequence shown as SEQ ID No. 66;

(t) the nucleotide sequence shown as SEQ ID No. 68

(u) the nucleotide sequence shown as SEQ ID No. 69 or

(v) a nucleotide sequence which has 75% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33, SEQ ID No. 35, SEQ ID No. 54, SEQ ID No. 59, SEQ ID No. 62, SEQ ID No. 64, SEQ ID No. 66, SEQ ID No. 68 or SEQ ID No. 69.

Suitably the nucleotide sequence may have 80% or more, preferably 85% or more, more preferably 90% or more and even more preferably 95% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33 or SEQ ID No. 35, SEQ ID No. 54, SEQ ID No. 59, SEQ ID No. 62, SEQ ID No. 64, SEQ ID No. 66, SEQ ID No. 68 or SEQ ID No. 69.

In one aspect, the fatty-acid CoA independent lipid acyltransferase may be a lecithin:cholesterol acyltransferases (LCAT) or variant thereof (for example a variant made by molecular evolution)

Suitable LCATs are known in the art and may be obtainable from one or more of the following organisms for example: mammals, rat, mice, chickens, *Drosophila melanogaster,* plants, including *Arabidopsis* and *Oryza sativa,* nematodes, fungi and yeast.

In one embodiment the fatty-acid CoA independent lipid acyltransferase enzyme may be the lipid acyltransferase obtainable, preferably obtained, from the *E. coli* strains TOP 10 harbouring pPet12aAhydro and pPet12aASalmo deposited by Danisco A/S of Langebrogade 1, DK-1001 Copenhagen K, Denmark under the Budapest Treaty on the International Recognition of the Deposit of Microorganisms for the purposes of Patent Procedure at the National Collection of Industrial, Marine and Food Bacteria (NCIMB) 23 St. Machar Street, Aberdeen Scotland, GB on 22 December 2003 under accession numbers NICMB 41204 and NCIMB 41205, respectively.

Preferably, when carrying out a method according to the present invention the product is produced without increasing or substantially increasing the free fatty acids in the foodstuff.

The term "transferase" as used herein is interchangeable with the term "lipid acyltransferase".

Suitably, the fatty-acid CoA independent lipid acyltransferase as defined herein catalyses one or more of the following reactions: interesterification, transesterification, alcoholysis, hydrolysis.

The term "interesterification" refers to the enzymatic catalysed transfer of acyl groups between a lipid donor and lipid acceptor, wherein the lipid donor is not a free acyl group.

The term "transesterification" as used herein means the enzymatic catalysed transfer of an acyl group from a lipid donor (other than a free fatty acid) to an acyl acceptor (other than water).

As used herein, the term "alcoholysis" refers to the enzymatic cleavage of a covalent bond of an acid derivative by reaction with an alcohol ROH so that one of the products combines with the H of the alcohol and the other product combines with the OR group of the alcohol.

As used herein, the term "alcohol" refers to an alkyl compound containing a hydroxyl group.

As used herein, the term "hydrolysis" refers to the enzymatic catalysed transfer of an acyl group from a lipid to the OH group of a water molecule. Acyl transfer which results from hydrolysis requires the separation of the water molecule.

The term "without increasing or without substantially increasing the free fatty acids" as used herein means that preferably the lipid acyl transferase according to the present invention has 100% transferase activity (i.e. transfers 100% of the acyl groups from an acyl donor onto the acyl acceptor, with no hydrolytic activity) in an edible oil environment; however, the enzyme may transfer less than 100% of the acyl groups present in the lipid acyl donor to the acyl acceptor. In which case, preferably the acyltransferase activity accounts for at least 30%, more preferably at least 40%, more preferably at least 50%, more preferably at least 70%, more preferably at least 80%, more preferably at least 90% and more preferably at least 98% of the total enzyme activity. The % transferase activity (i.e. the transferase activity as a percentage of the total enzymatic activity) may be determined by the following protocol:

*Protocol for the determination of % acyltransferase activity:*

An edible oil to which a fatty-acid CoA independent lipid acyltransferase has been added may be extracted following the enzymatic reaction with $CHCl_3$:$CH_3OH$ 2:1 and the organic phase containing the lipid material is isolated and analysed by GLC according to the procedure detailed herein below. From the GLC analyses the amount of free fatty acids and diglycerides are determined. A control edible oil to which no enzyme according to the present invention has been added, is analysed in the same way.

*Calculation:*

From the results of the GLC analyses the increase in free fatty acids and the decrease in diglycerides can be calculated:

$$\Delta \text{ \% fatty acid} = \text{\% Fatty acid(enzyme)} - \text{\% fatty acid(control)};$$

Mv Fa = average molecular weight of the fatty acids;

$$\Delta \text{ \% diglyceride} = \text{\% Diglyceride(control)} - \text{\% Diglyceride(enzyme)};$$

Mv Di= average molecular weight of diglyceride;
The transferase activity is calculated as a percentage of the total enzymatic activity:

$$\text{\% transferase activity} = \frac{((\Delta \text{ \% Di/(Mv Di)} - \Delta \text{ \% Fa/(Mv FA))} \times 100}{\Delta \text{ \% Di/(Mv Di)} - \Delta \text{ \% Fa/(Mv Fa)} + \Delta \text{ \% Fa/(Mv Fa)}}$$

$$\text{\% transferase activity} = \; = \frac{((\Delta \text{ \% Di/(Mv Di)} - \Delta \text{ \% Fa/(Mv FA))} \times 100}{\Delta \text{ \% Di/(Mv Di)}}$$

If the free fatty acids are increased in the edible oil they are preferably not increased substantially, i.e. to a significant degree. By this we mean, that the increase in free fatty acid does not adversely affect the quality of the edible oil.

In some aspects of the present invention, the term "without substantially increasing free fatty acids" as used herein means that the amount of free fatty acid in an edible oil treated with an lipid acyltransferase is less than the amount of free fatty acid produced in an edible oil or composition when an enzyme other than a lipid acyltransferase had been used, such as for example as compared with the amount of free fatty acid produced when a conventional lipase e.g. *Pseudomonas cepacia* lipase (Lipase PS, Amano Japan) or *Rhizopus oryzae* lipase (LipaseF, Amano Japan).

Suitably, any glycerol remaining in the edible oil after the reaction has taken place may be removed, e.g. by centrifugation or vacuum distillation.

Optionally, the enzyme may be removed from the edible oil after the enzymatic reaction has taken place. Alternatively, the enzyme may simply be deactivated and left in the edible oil. Suitably, the enzyme may be deactivated by heating for example.

In one embodiment the fatty-acid CoA independent lipid acyltransferase for use in the methods of the present invention may be immobilised. When it is the case that the enzyme is immobilised an admixture comprising an acyl acceptor and the edible oil may be passed through a column for example comprising the immobilised enzyme. By immobilising the enzyme it is possible to easily reuse it.

Suitably the immobilised enzyme may be used in a flow reactor or in a batch reactor containing a reaction mixture which comprises an acyl acceptor and an edible oil as a two-phase system. The reaction mixture may be optionally stirred or sonicated. Once the reaction has reached equilibrium for example, the reaction mixture and the immobilised enzyme may be separated. Suitably, excess acyl acceptor (such as excess glycerol) may be removed after the reaction, e.g. by centrifugation or vacuum distillation.

Immobilised lipid acyltransferase can be prepared using immobilisation techniques known in the art. There are numerous methods of preparing immobilised enzymes, which will be apparent to a person skilled in the art (for example the techniques referred to in EP 0 746 608; or Balcao V.M., Paiva A.L., Malcata F.X., Enzyme Microb Technol. 1996 May 1;18(6):392-416; or Retz M.T., Jaeger K.E. Chem Phys Lipids. 1998 Jun;93(1-2):3-14; Bornscheuer U.T., Bessler C, Srinivas R, Krishna S.H. Trends Biotechnol. 2002 Oct; 20(10):433-7; Plou et al, J. Biotechnology 92 (2002) 55-66; Warmuth et al., 1992. Bio Forum 9, 282-283; Ferrer et al., 2000. J. Chem. Technol. Biotechnol. 75, 1-8; or Christensen et al., 1998. Nachwachsende Rohstoff 10, 98-105; Petersen and Christenen, 2000, Applied Biocatalysis. Harwood Academic Publishers, Amsterdam. Techniques which may be used herein include covalent coupling to Eupergit C, adsorption on polypropylene and silica-granulation for example.

Preferably, the edible oil is any edible oil containing a diglyceride, preferably a significant amount of diglyceride.

Preferably, the edible oil is one or more of the following oils: oils extracted from or derived from palm oil, palm olein, palm stearin, palm mid fraction or any palm oil fraction or olive oil.

More preferably, the edible oil is palm oil and/or palm olein and/or palm stearin.

With regard to the admixing of the edible oil, the acyl acceptor substrate and the lipid acyltransferase, as a person skilled in the art would readily appreciate this can be done in any combination and/or order. By way of example only, the acyl acceptor substrate may be admixed with the edible oil followed by addition of the lipid acyltransferase. Alternatively, the edible oil may be admixed with the lipid acyltransferase followed by addition of the acyl acceptor substrate. Alternatively, the lipid acyltransferase and acyl acceptor substrate may be admixed followed by admixing of the enzyme/substrate mixture with the edible oil. Alternatively, of course, all three substances (namely the edible oil, the acyl acceptor substrate and the lipid acyltransferase) may be admixed simultaneously.

Preferably the method is carried out at a temperature above the melting point of the edible oil.

Suitably the method may be carried out at a temperature of between 30-50°C, preferably between 35-45°C, preferably between 40-45°C.

Preferably, the enzyme is added to crude or refined edible oil. Preferably the present invention does not encompass the enzyme treatment of an edible oil when admixed with water containing constituents. Thus, the present invention envisages the treatment of an edible oil *per se,* i.e. in a low water environment.

Although water may be added to the oil prior or during the method of the present invention, in a most preferable embodiment no water is added. If water is present in the edible oil, preferably there is less than 10% water present, more preferably less than 7.5%, less than 5%, less than 1%, less than 0.5%, less than 0.4%, less than 0.3%, less than 0.2% or less than 0.1% water present. Suitably, between 0.1. and 1% water may be present in the edible oil.

In one embodiment the edible oil used in a process according to the present invention may be supplemented with one or more acyl donors and/or may be supplemented with one or more acyl acceptors and/or may be supplemented with both one or more acyl acceptors and one or more acyl donors. By "supplemented with" means that either the acyl donor and/or the acyl acceptor is not naturally present in the edible oil and is added at the same time, immediately after and/or immediately before bringing the edible into contact with the lipid acyltransferase in accordance with the present invention.

Suitably, the supplementary acyl donor may be other than a DAG. Suitably the supplemental acyl donor may be for example a phospholipid (e.g. lecithin).

Suitably, the supplementary acyl acceptor may be glycerol. Suitably, however it may be an acyl acceptor other than glycerol, for example a plant sterol and/or plant stanol for instance. Suitably, the supplementary acyl acceptor may be a combination of glycerol and one or more further acyl acceptors.

The use of an oil supplemented with a phospholipid allows for an additional emulsifier (lyso-phospholipid) to be produced in the oil. The use of an oil supplemented with a plant sterol and/or plant stanol allows for a plant sterol ester and/or a plant stanol ester to be produced in the edible both. Both plant sterol esters and plant stanol esters have been reported to have blood serum cholesterol reducing effects when incorporated into the diet.

Enzymatic interesterification using immobilised lipases, such as Lipozyme® TL IM, a 1,3 specific lipase (Novozymes, Denmark), is used to reduce the amount of trans fatty acids in oils for dietary use and/or for modifying the melting characteristics of edible oils and fats.

During interesterification, one or two of the polyunsaturated fatty acids in the triglycerides of the food oil can be replaced with a fatty acid from another oil low in trans fatty acids, such as palm oil. This transfer of fatty acids from palm oil allows the modification of the melting point of the food oil without introduction of the trans fatty acids. The immobilisation of lipases is described in US5776741, US4798793 and US5156963. US6284501 describes the interesterification of phospholipids.

An immobilised transferase can be produced using the same technology as used for lipases.

In one embodiment, the fatty-acid CoA independent lipid acyl transferase as described herein can be used in combination with a interesterification lipase. The lipase interesterification and the acyl transferase steps are preferable carried out separately.

In a further aspect, the fatty-acid CoA independent lipid acyl transferase as described herein can be used in combination with conventional crystallisation inhibitors.

In a further aspect, the present invention provides a method of improving the crystallization properties in a foodstuff comprising an edible oil, comprising admixing an edible oil with an acyl acceptor substrate and a fatty-acid CoA independent diglyceride:glycerol acyltransferase as described herein and optionally a further crystallization inhibitor.

In one embodiment the present invention provides a fatty-acid CoA independent diglyceride:glycerol acyltransferase in the preparation of a foodstuff comprising an edible oil for improving the crystallization properties of the foodstuff, wherein the CoA independent diglyceride:glycerol acyltransferase is an acyltransferase which comprises the amino acid sequence motif GDSX, wherein X is one or more of the following amino acid residues L, A, V, I, F, Y, H, Q, T, N, M or S.

Suitably one or more further crystallization inhibitors may optionally be added.

The present invention yet further provides the use of a fatty-acid CoA independent diglyceride:glycerol acyltransferase as described herein in the manufacture of a foodstuff comprising an edible oil for improving the crystallization properties of said foodstuff.

In another aspect, the present invention provides the use of a fatty-acid CoA independent diglyceride:glycerol acyltransferase as described herein in combination with a further crystallization inhibitor in the manufacture of a foodstuff comprising an edible oil for improving the crystallization properties of said foodstuff.

The further crystallization inhibitor may be any conventional crystallization inhibitor such as one or more of sorbitan tristearates, lecithins, PGE or polysorbates for example.

ADVANTAGES

In the present invention by use of the method taught herein, and in particular by selection of the enzymes taught herein for use in the claimed method, for removing or reducing diglyceride from or in an edible oil, a selective reduction in diglyceride over mono- and di-glyceride can be effected, without a decrease in triglycerides and/or a significant increase in free fatty acids (FFAs).

The fact that the present method can be carried out without substantially increasing free fatty acid content of the edible oil overcomes the problem of loss of product.

If a crude palm oil is treated with a conventional lipase it is necessary to remove the free fatty acid during the oil refining, but if a refined palm oil is treated with the lipid acyltransferase according to the present invention (thus negating the need to treat the crude palm oil with a conventional lipase) it is not necessary to remove the fatty acids because the content of free fatty acid does not increase substantially.

In addition or alternatively, the present invention results in the removal and/or reduction of diglycerides from or in an edible oil, with no significant decrease in monoglyceride levels.

In addition or alternatively, the present invention results in the removal and/or reduction of diglycerides from or in an edible oil, whilst increasing monoglyceride levels in the edible oil. This contrasts prior art enzymes which utilise monoglyceride as a substrate and therefore reduce the amount of monoglyceride in the edible oil.

The present invention is advantageous as it does not require the addition of fatty acid CoA. This contrasts sharply with the acyl CoA dependent DGATs. Rather the enzyme according to the present invention relies on the presence (and addition if necessary) of glycerol - which is cheap commodity.

COMMERCIAL RELEVANCE OF REMOVING/REDUCING DIGLYCERIDE IN PALM OIL

Diglycerides retard the crystallisation in palm based margarines and shortenings

For many years, we have seen a steady increase in the growth of palm oil consumption, partially because of localised availability, partially because of economics, and primarily because of performance. Presence of Palm oil in Margarine / shortening type products is found to enhance the β' quality of an oil blend. However, the use of palm oil was previously restricted to the use of palm stearine, and palm olein, with some use of palm kernels and its fractions. Today, this is more diverse, because of confectionery and food processors wanting very specific melting profiles.

With customers trying to increase the use of palm oil in formulations, the industry is seeing more crystallization issues than ever before. Where it is necessary to add "pro-crystal" promoters to initiate crystallization in low trans or no-trans formulations thereby preventing or delaying post crystal formation before point of use. Also increased interest in anti-crystallisers, such as Sorbitan Tristearates, lecithins, PGE, Polysorbates, which will slow, delay post-crystal growth has been seen. This is a symptom of high diglyceride presence.

The commercial benefits of the present invention may be one or more of the following:

a) reduces the need to add additional monoglyceride to the edible oil during processing and/or later during use. Monoglyceride is a widely used emulsifier in food systems.
b) decreases in diglyceride from about 6 - 8% to about 4-5%, and even more.
c) increases the flexibility on plant (factory) capacity - hence reduction in production overheads.
d) allows for major decreases in post-crystallisation issues.
e) in some formulations, it would be possible to remove some fully saturated oil triglycerides, because we reduce the need for crystal promotion. In this regard manufacturers conventionally add fully saturated triglycerides to formulations in order to "promote" crystal formation and overcome undesirable delayed crystal formation in the product post-sale. Such crystal formation is a symptom of having a significant amount of palm oil or its components in a formulation, which would contain concentrations of diglycerides sufficient to delay crystal growth to the desired crystal type (i.e. preferably beta-prime in the case of margarines and shortenings) during manufacture. However, by use of palm oil treated in accordance with the present invention, the diglyceride content is sufficiently reduced within a given blend or formulation so that the need for additional fully saturated triglyceride addition to aid crystal

development becomes less important.

f) allows greater diversification towards palm based blends, with increased liquid oil costs, and without major process changes.

g) allows for replacement of anti-crystallisers and/or partial replacement of anti-crystallisers.

B Trans fatty acid

Today, we see legislation and public opinion against the use of trans acids in food products (Fødevareministeriet, Denmark 2003). So, this has led to performance problems. Manufacturers where possible may wish to switch to palm based solutions because of the high levels ofC:16:0 and C:18:0 isomers found within this oil type. The result is that we are beginning to see increased consumption of palm in economies that previously did not use palm.

C Crystallisation inhibition of diglycerides in confectionery products

Cocoa Butter Equivalents (CBEs) are formulated from speciality fats such as fractionated palm oil, in particular palm mid fractions (PMF), fractionated shea fat as well as many other exotic fats. For cost reasons it is favourable to include as much as possible PMF in the CBE. In fact many CBEs may only contain palm fractions. The most delicate aspect of chocolate manufacture is the crystallisation of the fat. Diglycerides will have a negative impact on the crystallisation, for example demoulding can be a serious problem (Siew, 2001).

D Crystal, quality

Triglyceride quality greatly affects performance, and this is evident, from examples, of a typical trans acid containing oil formula for standard 82% fat table margarine, against trans free version containing a typical interesterified palm stearine fraction and palm kernel as hard stock, both having similar SFC profile. Reduced speed and quality of crystallization-can result in a symptom known as "oiling out." In turn this calls for the need of crystal promoters, such as hard MAGs. Further, delay in crystal stabilized crystal formation leads to symptom known as "sandiness" whereby transformation of desired crystal type eventually reverts to the more stable Beta form, and so gives sandy texture. This symptom is particular problem for Industrial type products.

SOME USES

**Fat Modification**

During the chemical production of fats for use in high fat solid food products such as margarines, spreads, confectionery/chocolates, trans fatty acids are introduced into the food oil during a process of hydrogenation (hardening). This allows for the modification of the melting temperature of the food oil to ensure a suitable consistency of the final food product, e.g. a table margarine, which is solid but spreadable at room temperature, or a chocolate which is solid during storage, but melts in the mouth.

However, chemical production uses large quantities of solvents such as hexane which are considered hazardous to the environment and health and requires complete removal of the solvent prior to use of the modified oil/fat as a food ingredient.

In many countries the use of partial hydrogenation for food production is being limited by legislation and regulatory controls, and many major food producers are now switching to alternative low-trans alternatives.

The oil prepared by the process of the invention can be used as an ingredient for margarine and/or spreads.

**Cocoa Butter Replacements/Equivalents**

Cocoa butter contains a unique composition which provides a solid confectionery which melts in the mouth. In order to substitute cocoa butter using cheaper alternatives, plant oils have been hydrogenated to produce trans fatty acids, thereby raising the melting point of the food oil to provide a similar solid confectionery which melts at body temperature. Such modified plant oils are known as cocoa butter equivalents (CBEs) or, in the case where the modified fats enhance the characteristics of the chocolate product, cocoa butter replacements (CBRs). One major problem with CBEs and CBRs is that the hydrogenation of plant oils produces trans fats which are considered to be detrimental to health and temperature. This has lead to the use of low trans plant oils, or fractions thereof, which have a higher melting temperature. Palm oil and palm oil fractions are considered advantageous in this respect as a major component low in trans CBRs/CBEs.

Cocoa butter replacement (CBR) fats are used to provide preferable characteristics to chocolate products such

as non-tempering, reduced post hardening, stability, bloom resistant. It is particularly desirable to use non-lauric/non-trans fats such as palm oil. The crystallisation properties of the fats used in CBRs play a key role in ensuring a suitable balance between the product melting in the mouth whilst retaining the above preferable characteristics. The use of low-trans fats, such as palm oil, in CBR blends is particularly desirable for health reasons. The use of palm oil in CBRs is described in EP0293194.

The oil prepared in accordance with a process of the present invention can be used as an ingredient for chocolate, for example within a fat blend as a cocoa butter replacement and/or equivalent.

SEQUENCES OF SOME DIGLYCERIDE:GLYCEROL ACYLTRANSFERASE ENZYMES FOR USE IN ACCORDANCE WITH THE PRESENT INVENTION

Suitable diglyceride:glycerol acyltransferase enzymes for use in accordance with the present invention and/or in the methods of the present invention may comprise any one of the following amino acid sequences and/or be encoded by the following nucleotide sequences:

Termobifida\fusca GDSx 548 aa

SEQ ID No. 58

ZP_00058717

```
  1 mlphpagerg evgaffallv gtpqdrrlrl echetrplrg rcgcgerrvp pltlpgdgvl
 61 cttsstrdae tvwrkhlqpr pdggfrphlg vgcllagqgs pgvlwcgreg crfevcrrdt
121 pglsrtrngd ssppfragws lppkcgeisq sarktpavpr ysllrtdrpd gprgrfvgsg
181 praatrrrlf lgipalvlvt altlvlavpt gretlwrmwc eatqdwclgv pvdsrgqpae
241 dgeflllspv qaatwgnyya lgdsyssgdg ardyypgtav kggcwrsana ypelvaeayd
301 faghlsflac sgqrgyamld aidevgsqld wnsphtslvt igiggndlgf stvlktcmvr
361 vplldskact dqedairkrm akfettfeel isevrtrapd arilvvgypr ifpeeptgay
421 ytltasnqrw lnetiqefnq qlaeavavhd eeiaasggvg svefvdvyha ldgheigsde
481 pwvngvqlrd latgvtvdrs tfhpnaaghr avgervieqi etgpgrplya tfavvagatv
541 dtlagevg
```

SEQ ID No. 59

nt

1 ggtggtgaac cagaacaccc ggtcgtcggc gtgggcgtcc aggtgcaggt gcaggttctt
61 caactgctcc agcaggatgc cgccgtggcc gtgcacgatg gccttgggca ggcctgtggt
121 ccccgacgag tacagcaccc atagcggatg gtcgaacggc agcggggtga actccagttc
181 cgcgccttcg cccgcggctt cgaactccgc ccaggacagg gtgtcggcga cagggccgca
241 gcccaggtac ggcaggacga cggtgtgctg caggctgggc atgccgtcgc gcagggcttt
301 gagcacgtca cggcggtcga agtccttacc gccgtagcgg tagccgtcca cggccagcag
361 cactttcggt tcgatctgcg cgaaccggtc gaggacgctg cgcaccccga agtcggggga
421 acaggacgac caggtcgcac cgatcgcggc gcaggcgagg aatgcggccg tcgcctcggc
481 gatgttcggc aggtaggcca cgacccggtc gccggggccc accccgaggc tgcggagggc
541 cgcagcgatc gcggcggtgc gggtccgcag ttctccccag gtccactcgg tcaacggccg
601 gagttcggac gcgtgccgga tcgccacggc tgatgggtca cggtcgcgga agatgtgctc
661 ggcgtagttg agggtggcgc cggggaacca gacggcgccg ggcatggcgt cggaggcgag
721 cactgtggtg tacggggtgg cggcgcgcac ccggtagtac tcccagatcg cggaccagaa
781 tccttcgagg tcggttaccg accagcgcca cagtgcctcg tagtccggtg cgtccacacc
841 gcggtgctcc cgcacccagc gggtgaacgc ggtgaggttg gcgcgttctt tgcgctcctc
901 gtcgggactc cacaggatcg gcggctgcgg cttgagtgtc atgaaacgcg accccttcgt
961 ggacggtgcg gatgcggtga gcgtcgggtg cctcccctaa cgctccccgg tgacggagtg
1021 ttgtgcacca catctagcac gcgggacgcg gaaaccgtat ggagaaaaca cctacaaccc
1081 cggccggacg gtgggtttcg gccacactta ggggtcgggt gcctgcttgc cgggcagggc
1141 agtcccgggg tgctgtggtg cgggcgggag ggctgtcgct tcgaggtgtg ccggcgggac
1201 actccgggcc tcagccgtac ccgcaacggg gacagttctc ctcccttccg ggctggatgg
1261 tcccttcccc cgaaatgcgg cgagatctcc cagtcagccc ggaaaacacc cgctgtgccc
1321 aggtactctt tgcttcgaac agacaggccg gacggtccac gggggaggtt tgtgggcagc
1381 ggaccacgtg cggcgaccag acgacggttg ttcctcggta tccccgctct tgtacttgtg
1441 acagcgctca cgctggtctt ggctgtcccg acggggcgcg agacgctgtg gcgcatgtgg
1501 tgtgaggcca cccaggactg gtgcctgggg gtgccggtcg actcccgcgg acagcctgcg
1561 gaggacggcg agtttctgct gctttctccg gtccaggcag cgacctgggg gaactattac
1621 gcgctcgggg attcgtactc ttcgggggac ggggcccgcg actactatcc cggcaccgcg
1681 gtgaagggcg gttgctggcg gtccgctaac gcctatccgg agctggtcgc cgaagcctac
1741 gacttcgccg gacacttgtc gttcctggcc tgcagcggcc agcgcggcta cgccatgctt
1801 gacgctatcg acgaggtcgg ctcgcagctg gactggaact cccctcacac gtcgctggtg
1861 acgatcggga tcggcggcaa cgatctgggg ttctccacgg ttttgaagac ctgcatggtg
1921 cgggtgccgc tgctggacag caaggcgtgc acggaccagg aggacgctat ccgcaagcgg
1981 atggcgaaat tcgagacgac gtttgaagag ctcatcagcg aagtgcgcac ccgcgcgccg
2041 gacgcccgga tccttgtcgt gggctacccc cggatttttc cggaggaacc gaccggcgcc
2101 tactacacgc tgaccgcgag caaccagcgg tggctcaacg aaaccattca ggagttcaac
2161 cagcagctcg ccgaggctgt cgcggtccac gacgaggaga ttgccgcgtc gggcggggtg
2221 ggcagcgtgg agttcgtgga cgtctaccac gcgttggacg gccacgagat cggctcggac

2281 gagccgtggg tgaacggggt gcagttgcgg gacctcgcca ccggggtgac tgtggaccgc
2341 agtaccttcc accccaacgc cgctgggcac cgggcggtcg gtgagcgggt catcgagcag
2401 atcgaaaccg gcccgggccg tccgctctat gccactttcg cggtggtggc gggggcgacc
2461 gtggacactc tcgcgggcga ggtggggtga cccggcttac cgtccggccc gcaggtctgc
2521 gagcactgcg gcgatctggt ccactgccca gtgcagttcg tcttcggtga tgaccagcgg
2581 cggggagagc cggatcgttg agccgtgcgt gtctttgacg agcacacccc gctgcaggag
2641 ccgttcgcac agttctcttc cggtggccag agtcgggtcg acgtcgatcc cagcccacag
2701 gccgatgctg cgggccgcga ccacgccgtt gccgaccagt tggtcgaggc gggcgcgcag
2761 cacgggggcg agggcgcgga catggtccag gtaagggccg tcgcggacga ggctcaccac
2821 ggcagtgccg accgcgcagg cgagggcgtt gccgccgaag gtgctgccgt gctggccggg
2881 gcggatcacg tcgaagactt ccgcgtcgcc taccgccgcc gccacgggca ggatgccgcc
2941 gcccagcgct ttgccgaaca ggtagatatc ggcgtcgact ccgctgtggt cgcaggcccg

//

Termobifida\fusca\ - GDSx

SEQ ID No. 60

1 vgsgpraatr rrlflgipal vlvtaltlvl avptgretlw rmwceatqdw clgvpvdsrg
61 qpaedgefll lspvqaatwg nyyalgdsys sgdgardyyp gtavkggcwr sanaypelva
121 eaydfaghls flacsgqrgy amldaidevg sqldwnspht slvtigiggn dlgfstvlkt
181 cmvrvplld s kactdqedai rkrmakfett feelisevrt rapdarilvv gyprifpeep
241 tgayytltas nqrwlnetiq efnqqlaeav avhdeeiaas ggvgsvefvd vyhaldghei
301 gsdepwvngv qlrdlatgvt vdrstfhpna aghravgerv ieqietgpgr plyatfavva
361 gatvdtlage vg

Corynebacterium\effciens\ GDSx 300 aa

SEQ ID No. 61

1 mrttviaasa llllagcadg areetagapp gessggiree gaeastsitd vyialgdsya
61 amggrdqplr gepfclrssg nypellhaev tdltcqgavt gdlleprtlg ertlpaqvda
121 ltedttlvtl siggndlgfg evagcireri agenaddcvd llgetigeql dqlppqldrv
181 heairdragd aqvvvtgylp lvsagdcpel gdvseadrrw aveltgqine tvreaaerhd
241 alfvlpddad ehtscappqq rwadiqgqqt dayplhptsa gheamaaavr dalglepvqp
//

SEQ ID No. 62
nt

1 ttctggggtg ttatggggtt gttatcggct cgtcctgggt ggatcccgcc aggtggggta
61 ttcacggggg acttttgtgt ccaacagccg agaatgagtg ccctgagcgg tgggaatgag
121 gtgggcgggg ctgtgtcgcc atgagggggc ggcgggctct gtggtgcccc gcgacccccg
181 gccccggtga gcggtgaatg aaatccggct gtaatcagca tcccgtgccc accccgtcgg
241 ggaggtcagc gcccggagtg tctacgcagt cggatcctct cggactcggc catgctgtcg
301 gcagcatcgc gctcccgggt cttggcgtcc ctcggctgtt ctgcctgctg tccctggaag
361 gcgaaatgat caccggggag tgatacaccg gtggtctcat cccggatgcc cacttcggcg
421 ccatccggca attcgggcag ctccgggtgg aagtaggtgg catccgatgc gtcggtgacg
481 ccatagtggg cgaagatctc atcctgctcg agggtgctca ggccactctc cggatcgata
541 tcgggggcgt ccttgatggc gtccttgctg aaaccgaggt gcagcttgtg ggcttccaat
601 ttcgcaccac ggagcgggac gaggctggaa tgacggccga agagcccgtg gtggacctca

661 acgaaggtgg gtagtcccgt gtcatcattg aggaacacgc cctccaccgc acccagcttg
721 tggccggagt tgtcgtaggc gctggcatcc agaagggaaa cgatctcata tttgtcggtg
781 tgctcagaca tgatcttcct ttgctgtcgg tgtctggtac taccacggta gggctgaatg
841 caactgttat ttttctgtta ttttaggaat tggtccatat cccacaggct ggctgtggtc
901 aaatcgtcat caagtaatcc ctgtcacaca aaatgggtgg tgggagccct ggtcgcggtt
961 ccgtgggagg cgccgtgccc cgcaggatcg tcggcatcgg cggatctggc cggtaccccg
1021 cggtgaataa aatcattctg taaccttcat cacggttggt tttaggtatc cgcccctttc
1081 gtcctgaccc cgtccccggc gcgcgggagc ccgcgggttg cggtagacag gggagacgtg
1141 gacaccatga ggacaacggt catcgcagca agcgcattac tccttctcgc cggatgcgcg
1201 gatggggccc gggaggagac cgccggtgca ccgccgggtg agtcctccgg gggcatccgg
1261 gaggaggggg cggaggcgtc gacaagcatc accgacgtct acatcgccct cggggattcc
1321 tatgcggcga tgggcgggcg ggatcagccg ttacggggtg agccgttctg cctgcgctcg
1381 tccggtaatt acccggaact cctccacgca gaggtcaccg atctcacctg ccaggggggcg
1441 gtgaccgggg atctgctcga acccaggacg ctgggggagc gcacgctgcc ggcgcaggtg
1501 gatgcgctga cggaggacac caccctggtc accctctcca tcgggggcaa tgacctcgga
1561 ttcggggagg tggcgggatg catccgggaa cggatcgccg gggagaacgc tgatgattgc
1621 gtggacctgc tgggggaaac catcggggag cagctcgatc agcttccccc gcagctggac
1681 cgcgtgcacg aggctatccg ggaccgcgcc ggggacgcgc aggttgtggt caccggttac
1741 ctgccgctcg tgtctgccgg ggactgcccc gaactggggg atgtctccga ggcggatcgt
1801 cgttgggcgg ttgagctgac cgggcagatc aacgagaccg tgcgcgaggc ggccgaacga
1861 cacgatgccc tctttgtcct gcccgacgat gccgatgagc acaccagttg tgcacccccca
1921 cagcagcgct gggcggatat ccagggccaa cagaccgatg cctatccgct gcacccgacc
1981 tccgccggcc atgaggcgat ggccgccgcc gtccgggacg cgctgggcct ggaaccggtc
2041 cagccgtagc gccgggcgcg cgcttgtcga cgaccaaccc atgccaggct gcagtcacat
2101 ccgcacatag cgcgcgcggg cgatggagta cgcaccatag aggatgagcc cgatgccgac
2161 gatgatgagc agcacactgc cgaagggttg ttccccgagg gtgcgcagag ccgagtccag
2221 acctgcggcc tgctccggat catgggccca accggcgatg acgatcaaca cccccaggat
2281 cccgaaggcg ataccacggg cgacataacc ggctgttccg gtgatgatga tcgcggtccc
2341 gacctgccct gaccccgcac ccgcctccag atcctcccgg aaatcccggg tggccccctt
2401 ccagaggttg tagacacccg cccccagtac caccagcccg gcgaccacaa ccagcaccac
2461 accccagggt tgggatagga cggtggcggt gacatcggtg gcggtctccc catcggaggt
2521 gctgccgccc cgggcgaagg tggaggtggt caccgccagg gagaagtaga ccatggccat
2581 gaccgccccc ttggcccttt ccttgaggtc ctcgcccgcc agcagctggc tcaattgcca
2641 gagtcccagg gccgccaggg cgatgacggc aacccacagg aggaactgcc caccccggagc
2701 ctccgcgatg gtggccaggg cacctgaatt cgaggcctca tcacccgaac cgccggatcc
2761 agtggcgatg cgcaccgcga tccacccgat gaggatgtgc agtatgccca ggacaatgaa
2821 accacctctg gccagggtgg tcagcgcggg gtggtcctcg gcctggtcgg cagcccgttc
2881 gatcgtccgt ttcgcggatc tggtgtcgcc cttatccata gctcccattg aaccgccttg
2941 aggggtgggc ggccactgtc agggcggatt gtgatctgaa ctgtgatgtt ccatcaaccc
//

Novosphingobium\aromaticivorans\ GDSx 284 aa

SEQ ID No. 63

ZP_00094165

1 mgqvklfarr capvllalag lapaatvare aplaegaryv algssfaagp gvgpnapgsp
61 ercgrgtlny phllaealkl dlvdatcsga tthhvlgpwn evppqidsvn gdtrlvtlti
121 ggndvsfvgn ifaaacekma spdprcgkwr eiteeewqad eermrsivrq iharaplarv
181 vvvdyitvlp psgtcaamai spdrlaqsrs aakrlarita rvareegasl lkfshisrrh
241 hpcsakpwsn glsapaddgi pvhpnrlgha eaaaalvklv klmk
//

SEQ ID No. 64

1 tgccggaact caagcggcgt ctagccgaac tcatgcccga aagcgcgtgg cactatcccg
61 aagaccaggt ctcggacgcc agcgagcgcc tgatggccgc cgaaatcacg cgcgaacagc
121 tctaccgcca gctccacgac gagctgccct atgacagtac cgtacgtccc gagaagtacc
181 tccatcgcaa ggacggttcg atcgagatcc accagcagat cgtgattgcc cgcgagacac
241 agcgtccgat cgtgctgggc aagggtggcg cgaagatcaa ggcgatcgga gaggccgcac
301 gcaaggaact ttcgcaattg ctcgacacca aggtgcacct gttcctgcat gtgaaggtcg
361 acgagcgctg ggccgacgcc aaggaaatct acgaggaaat cggcctcgaa tgggtcaagt
421 gaagctcttc gcgcgccgct gcgccccagt acttctcgcc cttgccgggc tggctccggc
481 ggctacggtc gcgcgggaag caccgctggc cgaaggcgcg cgttacgttg cgctgggaag
541 ctccttcgcc gcaggtccgg gcgtggggcc caacgcgccc ggatcgcccg aacgctgcgg
601 ccggggcacg ctcaactacc cgcacctgct cgccgaggcg ctcaagctcg atctcgtcga
661 tgcgacctgc agcggcgcga cgacccacca cgtgctgggc ccctggaacg aggttccccc
721 tcagatcgac agcgtgaatg gcgacacccg cctcgtcacc ctgaccatcg gcggaaacga
781 tgtgtcgttc gtcggcaaca tcttcgccgc cgcttgcgag aagatggcgt cgcccgatcc
841 gcgctgcggc aagtggcggg agatcaccga ggaagagtgg caggccgacg aggagcggat
901 gcgctccatc gtacgccaga tccacgcccg cgcgcctctc gcccgggtgg tggtggtcga
961 ttacatcacg gtcctgccgc catcaggcac ttgcgctgcc atggcgattt cgccggaccg
1021 gctggcccag agccgcagcg ccgcgaaacg gcttgcccgg attaccgcac gggtcgcgcg
1081 agaagagggt gcatcgctgc tcaagttctc gcatatctcg cgccggcacc atccatgctc
1141 tgccaagccc tggagcaacg gcctttccgc cccggccgac gacggcatcc cggtccatcc
1201 gaaccggctc ggacatgctg aagcggcagc ggcgctggtc aagcttgtga aattgatgaa
1261 gtagctactg cactgatttc aaatagtatt gcctgtcagc tttccagccc ggattgttgc
1321 agcgcaacag aaacttgtcc gtaatggatt gatggtttat gtcgctcgca aattgccgtc
1381 gaagggaacg ggcgcgtcgc tcgttaacgt cctgggtgca gcagtgacgg agcgcgtgga
1441 tgagtgatac tggcggtgtc atcggtgtac gcgccgccat tcccatgcct gtacgcgccg
//

S.coelicolor\ GDSx 268 aa

SEQ ID No. 65

NP_625998.

1 mrrfrlvgfl sslvlaagaa ltgaataqaa qpaaadgyva lgdsyssgvg agsyissgd
61 ckrstkahpy lwaaahspst fdftacsgar tgdvlsgqlg plssgtglvs isiggndagf
121 adtmttcvlq sessclsria taeayvdstl pgkldgvysa isdkapnahv vvigyprfyk
181 lgttciglse tkrtainkas dhlntvlaqr aaahgftfgd vrttftghel csgspwlhsv
241 nwlnigesyh ptaagqsggy lpvlngaa
//

SEQ ID No. 66

1 cccggcggcc cgtgcaggag cagcagccgg cccgcgatgt cctcgggcgt cgtcttcatc
61 aggccgtcca tcgcgtcggc gaccggcgcc gtgtagttgg cccggacctc gtcccaggtg
121 cccgcggcga tctggcgggt ggtgcggtgc gggccgcgcc gaggggagac gtaccagaag
181 cccatcgtca cgttctccgg ctgcggttcg ggctcgtccg ccgctccgtc cgtcgcctcg
241 ccgagcacct tctcggcgag gtcggcgctg gtcgccgtca ccgtgacgtc ggcgccccgg
301 ctccagcgcg agatcagcag cgtccagccg tcgccctccg ccagcgtcgc gctgcggtcg
361 tcgtcgcggg cgatccgcag cacgcgcgcg ccgggcggca gcagcgtggc gccggaccgt
421 acgcggtcga tgttcgccgc gtgcgagtac ggctgctcac ccgtggcgaa acggccgagg
481 aacagcgcgt cgacgacgtc ggacggggag tcgctgtcgt ccacgttgag ccggatcggc
541 agggcttcgt gcgggttcac ggacatgtcg ccatgatcgg gcacccggcc gccgcgtgca
601 cccgctttcc cgggcacgca cgacaggggc tttctcgccg tcttccgtcc gaacttgaac

661 gagtgtcagc catttcttgg catggacact tccagtcaac gcgcgtagct gctaccacgg
721 ttgtggcagc aatcctgcta agggaggttc catgagacgt ttccgacttg tcggcttcct
781 gagttcgctc gtcctcgccg ccggcgccgc cctcaccggg gcagcgaccg cccaggcggc
841 ccaacccgcc gccgccgacg gctatgtggc cctcggcgac tcctactcct ccggggtcgg
901 agcgggcagc tacatcagct cgagcggcga ctgcaagcgc agcacgaagg cccatcccta
961 cctgtgggcg gccgcccact cgccctccac gttcgacttc accgcctgtt ccggcgcccg
1021 tacgggtgat gttctctccg gacagctcgg cccgctcagc tccggcaccg gcctcgtctc
1081 gatcagcatc ggcggcaacg acgccggttt cgccgacacc atgacgacct gtgtgctcca
1141 gtccgagagc tcctgcctgt cgcggatcgc caccgccgag gcgtacgtcg actcgacgct
1201 gcccggcaag ctcgacggcg tctactcggc aatcagcgac aaggcgccga acgcccacgt
1261 cgtcgtcatc ggctacccgc gcttctacaa gctcggcacc acctgcatcg gcctgtccga
1321 gaccaagcgg acggcgatca acaaggcctc cgaccacctc aacaccgtcc tcgcccagcg
1381 cgccgccgcc cacggcttca ccttcggcga cgtacgcacc accttcaccg gccacgagct
1441 gtgctccggc agcccctggc tgcacagcgt caactggctg aacatcggcg agtcgtacca
1501 ccccaccgcg gccggccagt ccggtggcta cctgccggtc ctcaacggcg ccgcctgacc
1561 tcaggcggaa ggagaagaag aaggagcgga gggagacgag gagtgggagg ccccgcccga
1621 cggggtcccc gtccccgtct ccgtctccgt cccggtcccg caagtcaccg agaacgccac
1681 cgcgtcggac gtggcccgca ccggactccg cacctccacg cgcacggcac tctcgaacgc
1741 gccggtgtcg tcgtgcgtcg tcaccaccac gccgtcctgg cgcgagcgct cgccgcccga
1801 cgggaaggac agcgtccgcc accccggatc ggagaccgac ccgtccgcgg tcacccaccg
1861 gtagccgacc tccgcgggca gccgcccgac cgtgaacgtc gccgtgaacg cgggtgcccg
1921 gtcgtgcggc ggcggacagg cccccgagta gtgggtgcgc gagcccacca cggtcacctc
1981 caccgactgc gctgcggggc
//

S.avermitilis\ GDSx 269 aa

SEQ ID No. 67

NP_827753.
1 mrrsritayv tslllavgca ltgaataqas paaaatgyva lgdsyssgvg agsylsssgd
61 ckrsskaypy lwqaahspss fsfmacsgar tgdvlanqlg tlnsstglvs ltiggndagf
121 sdvmttcvlq sdsaclsrin takayvdstl pgqldsvyta istkapsahv avlgyprfyk
181 lggsclagls etkrsainda adylnsaiak raadhgftfg dvkstftghe icsssțwlhs
241 ldllnigqsy hptaagqsgg ylpvmnsva
//

SEQ ID No. 68

1 ccaccgccgg gtcggcggcg agtctcctgg cctcggtcgc ggagaggttg gccgtgtagc
61 cgttcagcgc ggcgccgaac gtcttcttca ccgtgccgcc gtactcgttg atcaggccct
121 tgcccttgct cgacgcggcc ttgaagccgg tgcccttctt gagcgtgacg atgtagctgc
181 ccttgatcgc ggtgggggag ccggcggcga gcaccgtgcc ctcggccggg gtggcctggg
241 cgggcagtgc ggtgaatccg cccacgaggg cgccggtcgc cacggcggtt atcgcggcga
301 tccggatctt cttgctacgc agctgtgcca tacgagggag tcctcctctg ggcagcggcg
361 cgcctgggtg gggcgcacgg ctgtgggggg tgcgcgcgtc atcacgcaca cggccctgga
421 gcgtcgtgtt ccgccctggg ttgagtaaag cctcggccat ctacgggggt ggctcaaggg
481 agttgagacc ctgtcatgag tctgacatga gcacgcaatc aacggggccg tgagcacccc
541 ggggcgaccc cggaaagtgc cgagaagtct tggcatggac acttcctgtc aacacgcgta
601 gctggtacga cggttacggc agagatcctg ctaaagggag gttccatgag acgttcccga
661 attacggcat acgtgacctc actcctcctc gccgtcggct gcgccctcac cggggcagcg
721 acggcgcagg cgtccccagc cgccgcggcc acgggctatg tggccctcgg cgactcgtac
781 tcgtccggtg tcggcgccgg cagctacctc agctccagcg gcgactgcaa gcgcagttcg
841 aaggcctatc cgtacctctg gcaggccgcg cattcaccct cgtcgttcag tttcatggct
901 tgctcgggcg ctcgtacggg tgatgtcctg gccaatcagc tcggcaccct gaactcgtcc

961 accggcctgg tctccctcac catcggaggc aacgacgcgg gcttctccga cgtcatgacg
1021 acctgtgtgc tccagtccga cagcgcctgc ctctcccgca tcaacacggc gaaggcgtac
1081 gtcgactcca ccctgcccgg ccaactcgac agcgtgtaca cggcgatcag cacgaaggcc
1141 ccgtcggccc atgtggccgt gctgggctac ccccgcttct acaaactggg cggctcctgc
1201 ctcgcgggcc tctcggagac caagcggtcc gccatcaacg acgcggccga ctatctgaac
1261 agcgccatcg ccaagcgcgc cgccgaccac ggcttcacct tcggcgacgt caagagcacc
1321 ttcaccggcc atgagatctg ctccagcagc acctggctgc acagtctcga cctgctgaac
1381 atcggccagt cctaccaccc gaccgcggcc ggccagtccg gcggctatct gccggtcatg
1441 aacagcgtgg cctgagctcc cacggcctga atttttaagg cctgaatttt taaggcgaag
1501 gtgaaccgga agcggaggcc ccgtccgtcg ggtctccgt cgcacaggtc accgagaacg
1561 gcacggagtt ggacgtcgtg cgcaccgggt cgcgcacctc gacggcgatc tcgttcgaga
1621 tcgttccgct cgtgtcgtac gtggtgacga acacctgctt ctgctgggtc tttccgccgc
1681 tcgccgggaa ggacagcgtc ttccagcccg gatccgggac ctcgcccttc ttggtcaccc
1741 agcggtactc cacctcgacc ggcacccggc ccaccgtgaa ggtcgccgtg aacgtgggcg
1801 cctgggcggt gggcggcggg caggcaccgg agtagtcggt gtgcacgccg gtgaccgtca
1861 ccttcacgga ctgggccggc ggggtcgtcg taccgccgcc gccaccgccg cctcccggag
1921 tggagcccga gctgtggtcg cccccgccgt cggcgttgtc gtcctcgggg gttttcgaac
//

*Streptomyces* diglyceride:glycerol acyltransferase

SEQ ID No. 69

ACAGGCCGATGCACGGAACCGTACCTTTCCGCAGTGAAGCGCTCTCCCCCCATCGTTCGC
CGGGACTTCATCCGCGATTTTGGCATGAACACTTCCTTCAACGCGCGTAGCTTGCTACAA
GTGCGGCAGCAGACCCGCTCGTTGGAGGCTCAGTGAGATTGACCCGATCCCTGTCGGCCG
CATCCGTCATCGTCTTCGCCCTGCTGCTCGCGCTGCTGGGCATCAGCCCGGCCCAGGCAG
CCGGCCCGGCCTATGTGGCCCTGGGGGATTCCTATTCCTCGGGCAACGGCGCCGGAAGTT
ACATCGATTCGAGCGGTGACTGTCACCGCAGCAACAACGCGTACCCCGCCCGCTGGGCGG
CGGCCAACGCACCGTCCTCCTTCACCTTCGCGGCCTGCTCGGGAGCGGTGACCACGGATG
TGATCAACAATCAGCTGGGCGCCCTCAACGCGTCCACCGGCCTGGTGAGCATCACCATCG
GCGGCAATGACGCGGGCTTCGCGGACGCGATGACCACCTGCGTCACCAGCTCGGACAGCA
CCTGCCTCAACCGGCTGGCCACCGCCACCAACTACATCAACACCACCCTGCTCGCCCGGC
TCGACGCGGTCTACAGCCAGATCAAGGCCCGTGCCCCCAACGCCCGCGTGGTCGTCCTCG
GCTACCCGCGCATGTACCTGGCCTCGAACCCCTGGTACTGCCTGGGCCTGAGCAACACCA
AGCGCGCGGCCATCAACACCACCGCCGACACCCTCAACTCGGTGATCTCCTCCCGGGCCA
CCGCCCACGGATTCCGATTCGGCGATGTCCGCCCGACCTTCAACAACCACGAACTGTTCT
TCGGCAACGACTGGCTGCACTCACTCACCCTGCCGGTGTGGGAGTCGTACCACCCCACCA
GCACGGGCCATCAGAGCGGCTATCTGCCGGTCCTCAACGCCAACAGCTCGACCTGATCAA
CGCACGGCCGTGCCCGCCCCGCGCGTCACGCTCGGCGCGGGCGCCGCAGCGCGTTGATCA
GCCCACAGTGCCGGTGACGGTCCCACCGTCACGGTCGAGGGTGTACGTCACGGTGGCGCC
GCTCCAGAAGTGGAACGTCAGCAGGACCGTGGAGCCGTCCCTGACCTCGTCGAAGAACTC
CGGGGTCAGCGTGATCACCCCTCCCCCGTAGCCGGGGGCGAAGGCGGCGCCGAACTCCTT
GTAGGACGTCCAGTCGTGCGGCCCGGCGTTGCCACCGTCCGCGTAGACCGCTTCCATGGT
CGCCAGCCGGTCCCCGCGGAACTCGGTGGGGATGTCCGTGCCCAAGGTGGTCCCGGTGGT
GTCCGAGAGCACCGGGGGCTCGTACCGGATGATGTGCAGATCCAAAGAATT

*Streptomyces* diglyceride:glycerol acyltransferase

SEQ ID No. 70

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

IDENTIFICATION OF A DIGLYCERIDE:GLYCEROL ACYLTRANSFERASE ACCORDING TO THE PRESENT INVENTION

Assay for enzymatic reduction of diglyceride in palm oil.

1 gram of palm oil containing 7% diglyceride is scaled in a glass with lid. 50 mg glycerol and 10 μl enzyme solution is added. The reaction mixture is agitated with a magnetic stirrer in a heating chamber at 40°C for 20 hours. The enzyme reaction is stopped by heating to 100°C for 10 minutes. A reference sample added 10μl water instead of enzyme solution is treated in the same way. The samples are analysed by GLC according to standard procedures (see hereinbelow) and the amount of fatty acids, monoglyceride and diglyceride are calculated.

*Calculation:*

From the results of the GLC analyses the increase in free fatty acids and the decrease in diglycerides can be calculated:

$$\Delta\ \%\ \text{fatty acid} = \%\ \text{Fatty acid(enzyme)} - \%\ \text{fatty acid(control)};$$

Mv Fa = average molecular weight of the fatty acids;

$$\Delta\ \%\ \text{diglyceride} = \%\ \text{Diglyceride(control)} - \%\ \text{Diglyceride(enzyme)};$$

Mv Di= average molecular weight of diglyceride;
The transferase activity is calculated as a percentage of the total enzymatic activity:

$$\%\ \text{transferase activity} = \;=\frac{((\Delta\ \%\ \text{Di}/(\text{Mv Di}) - \Delta\ \%\ \text{Fa}/(\text{Mv FA})) \times 100}{\Delta\ \%\ \text{Di}/(\text{Mv Di})}$$

GLC analysis

Perkin Elmer Autosystem 9000 Capillary Gas Chromatograph equipped with WCOT fused silica column 12.5 m x 0.25 mm ID x 0.1 μ film thickness 5% phenyl-methyl-silicone (CP Sil 8 CB from Chrompack).

Carrier gas: Helium.
Injector. PSSI cold split injection (initial temp 50°C heated to 385°C), volume 1.0μl
Detector FID: 395°C

| Oven program: | 1 | 2 | 3 |
|---|---|---|---|
| Oven temperature, °C. | 90 | 280 | 350 |
| Isothermal, time, min. | 1 | 0 | 10 |
| Temperature rate, °C/min. | 15 | 4 | |

Sample preparation: 30 mg of sample was dissolved in 9 ml Heptane:Pyridin, 2:1 containing internal standard heptadecane, 0.5 mg/ml. 300μl sample solution was transferred to a crimp vial, 300 μl MSTFA (N-Methyl-N-trimethylsilyltrifluoraceamid) was added and reacted for 20 minutes at 60°C.

ISOLATED

In one aspect, preferably the polypeptide or protein for use in the present invention is in an isolated form. The term "isolated" means that the sequence is at least substantially free from at least one other component with which the

sequence is naturally associated in nature and as found in nature.

PURIFIED

In one aspect, preferably the polypeptide or protein for use in the present invention is in a purified form. The term "purified" means that the sequence is in a relatively pure state - e.g. at least about 51% pure, or at least about 75%, or at least about 80%, or at least about 90% pure, or at least about 95% pure or at least about 98% pure.

CLONING A NUCLEOTIDE SEQUENCE ENCODING A POLYPEPTIDE ACCORDING TO THE PRESENT INVENTION

A nucleotide sequence encoding either a polypeptide which has the specific properties as defined herein or a polypeptide which is suitable for modification may be isolated from any cell or organism producing said polypeptide. Various methods are well known within the art for the isolation of nucleotide sequences.

For example, a genomic DNA and/or cDNA library may be constructed using chromosomal DNA or messenger RNA from the organism producing the polypeptide: If the amino acid sequence of the polypeptide is known, labelled oligonucleotide probes may be synthesised and used to identify polypeptide-encoding clones from the genomic library prepared from the organism. Alternatively, a labelled oligonucleotide probe containing sequences homologous to another known polypeptide gene could be used to identify polypeptide-encoding clones. In the latter case, hybridisation and washing conditions of lower stringency are used.

Alternatively, polypeptide-encoding clones could be identified by inserting fragments of genomic DNA into an expression vector, such as a plasmid, transforming enzyme-negative bacteria with the resulting genomic DNA library, and then plating the transformed bacteria onto agar containing an enzyme inhibited by the polypeptide, thereby allowing clones expressing the polypeptide to be identified.

In a yet further alternative, the nucleotide sequence encoding the polypeptide may be prepared synthetically by established standard methods, e.g. the phosphoroamidite method described by Beucage S.L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al (Science (1988) 239, pp 487-491).

NUCLEOTIDE SEQUENCES

The present invention also encompasses the use of nucleotide sequences encoding polypeptides having the specific properties as defined herein. The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be doublestranded or single-stranded whether representing the sense or antisense strand.

The term "nucleotide sequence" includes genomic DNA, cDNA, synthetic DNA, and RNA. Preferably it means DNA, more preferably cDNA for the coding sequence.

In a preferred embodiment, the nucleotide sequence *per se* encoding a polypeptide having the specific properties as defined herein does not cover the native nucleotide sequence in its natural environment when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. Thus, the polypeptide for use in present invention can be expressed by a nucleotide sequence in its native organism but wherein the nucleotide sequence is not under the control of the promoter with which it is naturally associated within that organism.

Preferably the polypeptide is not a native polypeptide. In this regard, the term "native polypeptide" means an entire polypeptide that is in its native environment and when it has been expressed by its native nucleotide sequence.

Typically, the nucleotide sequence encoding polypeptides having the specific properties as defined herein is prepared using recombinant DNA techniques (i.e. recombinant DNA). However, in an alternative embodiment of the invention, the nucleotide sequence could be synthesised, in whole or in part, using chemical methods well known in the art (see Caruthers MH et al (1980) Nuc Acids Res Symp Ser 215-23 and Horn T et al (1980) Nuc Acids Res Symp Ser 225-232).

MOLECULAR EVOLUTION

Once an enzyme-encoding nucleotide sequence has been isolated, or a putative enzyme-encoding nucleotide sequence has been identified, it may be desirable to modify the selected nucleotide sequence, for example it may be desirable to mutate the sequence in order to prepare an enzyme in accordance with the present invention.

Mutations may be introduced using synthetic oligonucleotides. These oligonucleotides contain nucleotide sequences flanking the desired mutation sites.

A suitable method is disclosed in Morinaga et al (Biotechnology (1984) 2, p646-649). Another method of introducing mutations into enzyme-encoding nucleotide sequences is described in Nelson and Long (Analytical Biochemistry (1989), 180, p 147-151).

Instead of site directed mutagenesis, such as described above, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which can also be combined with the use of mutagenic DNA analogues such as those described in EP 0 866 796. Error prone PCR technologies are suitable for the production of variants of lipid acyl transferases with preferred characteristics. WO0206457 refers to molecular evolution of lipases.

A third method to obtain novel sequences is to fragment non-identical nucleotide sequences, either by using any number of restriction enzymes or an enzyme such as Dnase I, and reassembling full nucleotide sequences coding for functional proteins. Alternatively one can use one or multiple non-identical nucleotide sequences and introduce mutations during the reassembly of the full nucleotide sequence. DNA shuffling and family shuffling technologies are suitable for the production of variants of lipid acyl transferases with preferred characteristics. Suitable methods for performing 'shuffling' can be found in EP0 752 008, EP1 138 763, EP1 103 606. Shuffling can also be combined with other forms of DNA mutagenesis as described in US 6,180,406 and WO 01/34835.

Thus, it is possible to produce numerous site directed or random mutations into a nucleotide sequence, either *in vivo* or *in vitro,* and to subsequently screen for improved functionality of the encoded polypeptide by various means. Using in silico and exo mediated recombination methods (see WO 00/58517, US 6,344,328, US 6,361,974), for example, molecular evolution can be performed where the variant produced retains very low homology to known enzymes or proteins. Such variants thereby obtained may have significant structural analogy to known transferase enzymes, but have very low amino acid sequence homology.

As a non-limiting example, In addition, mutations or natural variants of a polynucleotide sequence can be recombined with either the wild type or other mutations or natural variants to produce new variants. Such new variants can also be screened for improved functionality of the encoded polypeptide.

The application of the above-mentioned and similar molecular evolution methods allows the identification and selection of variants of the enzymes for use in the present invention which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate

As will be apparent to a person skilled in the art, using molecular evolution tools an enzyme may be altered to improve the functionality of the enzyme.

Suitably, the lipid acyltransferase used in the invention may be a variant, i.e. may contain at least one amino acid substitution, deletion or addition, when compared to a parental enzyme. Variant enzymes retain at least 20%, 30%, 40%, 50 %, 60%, 70%, 80%, 90%, 95%, 97%, 99% homology with the parent enzyme. Suitable parent enzymes may include any enzyme with esterase or lipase activity. Preferably, the parent enzyme aligns to the pfam00657 consensus sequence.

In a preferable embodiment a variant lipid acyltransferase enzyme retains or incorporates at least one or more of the pfam00657 consensus sequence amino acid residues found in the GDSx, GANDY and HPT blocks.

Enzymes, such as lipases with no or low lipid acyltransferase activity in an aqueous environment may be mutated using molecular evolution tools to introduce or enhance the transferase activity, thereby producing a lipid acyltransferase enzyme with significant transferase activity suitable for use in the compositions and methods of the present invention.

Suitably, the lipid acyltransferase for use in the invention may be a variant with enhanced enzyme activity on polar lipids, preferably phospholipids and/or glycolipids when compared to the parent enzyme. Preferably, such variants also have low or no activity on lyso polar lipids. The enhanced activity on polar lipids, phospholipids and/or glycolipids may be the result of hydrolysis and/or transferase activity or a combination of both.

Variant lipid acyltransferases for use in the invention may have decreased activity on triglycerides, and/or

monoglycerides and/or diglycerides compared with the parent enzyme.

Suitably the variant enzyme may have no activity on triglycerides and/or monoglycerides and/or diglycerides.

Alternatively, the variant enzyme for use in the invention may have increased activity on triglycerides, and/or may also have increased activity on one or more of the following, polar lipids, phospholipids, lecithin, phosphatidylcholine, glycolipids, digalactosyl monoglyceride, monogalactosyl monoglyceride.

Variants of lipid acyltransferases are known, and one or more of such variants may be suitable for use in the methods and uses according to the present invention and/or in the enzyme compositions. By way of example only, variants of lipid acyltransferases are described in the following references may be used in accordance with the present invention: Hilton & Buckley J Biol. Chem. 1991 Jan 15: 266 (2): 997-1000; Robertson et al J. Biol. Chem. 1994 Jan 21; 269(3):2146-50; Brumlik et al J. Bacteriol 1996 Apr; 178 (7): 2060-4; Peelman et al Protein Sci. 1998 Mar; 7(3):587-99.

AMINO ACID SEQUENCES

As used herein, the term "amino acid sequence" is synonymous with the term "polypeptide" and/or the term "protein". In some instances, the term "amino acid sequence" is synonymous with the term "peptide".

The amino acid sequence may be prepared/isolated from a suitable source, or it may be made synthetically or it may be prepared by use of recombinant DNA techniques.

Suitably, the amino acid sequences may be obtained from the isolated polypeptides taught herein by standard techniques.

One suitable method for determining amino acid sequences from isolated polypeptides is as follows:

Purified polypeptide may be freeze-dried and 100 μg of the freeze-dried material may be dissolved in 50 μl of a mixture of 8 M urea and 0.4 M ammonium hydrogen carbonate, pH 8.4. The dissolved protein may be denatured and reduced for 15 minutes at 50°C following overlay with nitrogen and addition of 5 μl of 45 mM dithiothreitol. After cooling to room temperature, 5 μl of 100 mM iodoacetamide may be added for the cysteine residues to be derivatized for 15 minutes at room temperature in the dark under nitrogen.

135 μl of water and 5 μg of endoproteinase Lys-C in 5 μl of water may be added to the above reaction mixture and the digestion may be carried out at 37°C under nitrogen for 24 hours.

The resulting peptides may be separated by reverse phase HPLC on a VYDAC C18 column (0.46x15cm;10μm; The Separation Group; California, USA) using solvent A: 0.1% TFA in water and solvent B: 0.1% TFA in acetonitrile. Selected peptides may be re-chromatographed on a Develosil C18 column using the same solvent system, prior to N-terminal sequencing. Sequencing may be done using an Applied Biosystems 476A sequencer using pulsed liquid fast cycles according to the manufacturer's instructions (Applied Biosystems, California, USA).

SEQUENCE IDENTITY OR SEQUENCE HOMOLOGY

The present invention also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically; the homologues will comprise the same sequences that code for the active sites etc. as the subject sequence. Although homology can also be considered in terms of similarity. (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence,

one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (Devereux et al 1984 Nuc. Acids Research 12 p387). Examples of other software that can perform sequence comparisons include, but are not limited to, the BLAST package (see Ausubel et al 1999 Short Protocols in Molecular Biology, 4th Ed - Chapter 18), FASTA (Altschul et al 1990 J. Mol. Biol. 403-410) and the GENEWORKS suite of comparison tools. Both BLAST and FASTA are available for offline and online searching (see Ausubel *et al* 1999, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |

(continued)

| | | |
|---|---|---|
| AROMATIC | | H F W Y |

The method of the present invention also encompasses homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

Nucleotide sequences for use in the present invention or encoding a polypeptide having the specific properties defined herein may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences.

The present invention also encompasses the use of nucleotide sequences that are complementary to the sequences discussed herein, or any derivative, fragment or derivative thereof If the sequence is complementary to a fragment thereof then that sequence can be used as a probe to identify similar coding sequences in other organisms etc.

Polynucleotides which are not 100% homologous to the sequences of the present invention but fall within the scope of the method of the invention can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction polypeptide recognition sites, or to alter the property or function of the polypeptides encoded by the polynucleotides.

Polynucleotides (nucleotide sequences) for use in the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.

Polynucleotides such as DNA polynucleotides and probes may be produced recombinantly, synthetically, or by any means available to those of skill in the art. They may also be cloned by standard techniques.

In general, primers will be produced by synthetic means, involving a stepwise manufacture of the desired

nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

HYBRIDISATION

The present use also encompasses sequences that are complementary to the sequences of the present invention or sequences that are capable of hybridising either to the sequences for use in the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the subject sequences discussed herein, or any derivative, fragment or derivative thereof.

The present invention also encompasses the use of sequences that are complementary to sequences that are capable of hybridising to the nucleotide sequences discussed herein.

Hybridisation conditions are based on the melting temperature (Tm) of the nucleotide binding complex, as taught in Berger and Kimmel (1987, Guide to Molecular Cloning Techniques, Methods in Enzymology, Vol. 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridisation can be used to identify or detect identical nucleotide sequences while an intermediate (or low) stringency hybridisation can be used to identify or detect similar or related polynucleotide sequences.

The present invention encompasses the use of sequences that are complementary to sequences that are capable of hybridising under high stringency conditions or intermediate stringency conditions to nucleotide sequences encoding polypeptides having the specific properties as defined herein.

More preferably, the present invention encompasses sequences that are complementary to sequences that are capable of hybridising under high stringent conditions (e.g. 65°C and 0.1xSSC {1xSSC =0.15 M NaCl, 0.015 M Na-citrate pH 7.0}) to nucleotide sequences encoding polypeptides having the specific properties as defined herein.

The present invention also relates to the use of nucleotide sequences that can hybridise to the nucleotide sequences discussed herein (including complementary sequences of those discussed herein).

The present invention also relates to the use of nucleotide sequences that are complementary to sequences that can hybridise to the nucleotide sequences discussed herein (including complementary sequences of those discussed herein).

Also included within the scope of the present invention is the use of polynucleotide sequences that are capable of hybridising to the nucleotide sequences discussed herein under conditions of intermediate to maximal stringency.

In a preferred aspect, the present invention covers the use of nucleotide sequences that can hybridise to the nucleotide sequences discussed herein, or the complement thereof, under stringent conditions (e.g. 50°C and 0.2xSSC).

In a more preferred aspect, the present invention covers the use of nucleotide sequences that can hybridise to the nucleotide sequences discussed herein, or the complement thereof, under high stringent conditions (e.g. 65°C and 0.1xSSC).

EXPRESSION OF POLYPEPTIDES

A nucleotide sequence for use in the present invention or for encoding a polypeptide having the specific properties as defined herein can be incorporated into a recombinant replicable vector. The vector may be used to replicate and express the nucleotide sequence, in polypeptide form, in and/or from a compatible host cell. Expression may be controlled using control sequences which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters

described above.

The polypeptide produced by a host recombinant cell by expression of the nucleotide sequence may be secreted or may be contained intracellularly depending on the sequence and/or the vector used. The coding sequences can be designed with signal sequences which direct secretion of the substance coding sequences through a particular prokaryotic or eukaryotic cell membrane.

EXPRESSION VECTOR

The term "expression vector" means a construct capable of *in vivo* or *in vitro* expression,

Preferably, the expression vector is incorporated in the genome of the organism. The term "incorporated" preferably covers stable incorporation into the genome.

The nucleotide sequence for use in the present invention or coding for a polypeptide having the specific properties as defined herein may be present in a vector, in which the nucleotide sequence is operably linked to regulatory sequences such that the regulatory sequences are capable of providing the expression of the nucleotide sequence by a suitable host organism, i.e. the vector is an expression vector.

The vectors for use in the present invention may be transformed into a suitable host cell as described below to provide for expression of a polypeptide having the specific properties as defined herein.

The choice of vector, e.g. plasmid, cosmid, virus or phage vector, will often depend on the host cell into which it is to be introduced.

The vectors may contain one or more selectable marker genes - such as a gene which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect or transform a host cell.

Further disclosed is a method of making nucleotide sequences for use in the present invention or nucleotide sequences encoding polypeptides having the specific properties as defined herein by introducing a nucleotide sequence into a replicable vector, introducing the vector into a compatible host cell, and growing the host cell under conditions which bring about replication of the vector.

The vector may further comprise a nucleotide sequence enabling the vector to replicate in the host cell in question. Examples of such sequences are the origins of replication of plasmids pUC19, pACYC177, pUB110, pE194, pAMB1 and pIJ702.

REGULATORY SEQUENCES

In some applications, a nucleotide sequence for use in the present invention or a nucleotide sequence encoding a polypeptide having the specific properties as defined herein may be operably linked to a regulatory sequence which is capable of providing for the expression of the nucleotide sequence, such as by the chosen host cell. By way of example, the present invention covers the use of a vector comprising the nucleotide sequence of the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under conditions compatible with the control sequences.

The term "regulatory sequences" includes promoters and enhancers and other expression regulation signals.

The term "promoter" is used in the normal sense of the art, e.g. an RNA polymerase binding site.

Enhanced expression of the nucleotide sequence encoding the enzyme having the specific properties as defined herein may also be achieved by the selection of heterologous regulatory regions, e.g. promoter, secretion leader and terminator regions.

Preferably, the nucleotide sequence for use in the present invention may be operably linked to at least a promoter.

Examples of suitable promoters for directing the transcription of the nucleotide sequence in a bacterial, fungal or yeast host are well known in the art.

CONSTRUCTS

The term "construct" - which is synonymous with terms such as "conjugate", "cassette" and "hybrid" - includes a nucleotide sequence encoding a polypeptide having the specific properties as defined herein for use according to the present invention directly or indirectly attached to a promoter. An example of an indirect attachment is the provision of a suitable spacer group such as an intron sequence, such as the Sh1-intron or the ADH intron, intermediate the promoter and the nucleotide sequence of the present invention. The same is true for the term "fused" in relation to the present

invention which includes direct or indirect attachment. In some cases, the terms do not cover the natural combination of the nucleotide sequence coding for the protein ordinarily associated with the wild type gene promoter and when they are both in their natural environment.

The construct may even contain or express a marker which allows for the selection of the genetic construct.

For some applications, preferably the construct comprises at least a nucleotide sequence of the present invention or a nucleotide sequence encoding a polypeptide having the specific properties as defined herein operably linked to a promoter.

HOST CELLS

The term "host cell" - in: relation to the present invention includes any cell that comprises either a nucleotide sequence encoding a polypeptide having the specific properties as defined herein or an expression vector as described above and which is used in the recombinant production of a polypeptide having the specific properties as defined herein.

Thus, a further embodiment of the present invention provides the use of host cells transformed or transfected with a nucleotide sequence of the present invention or a nucleotide sequence that expresses a polypeptide having the specific properties as defined herein. The cells will be chosen to be compatible with the said vector and may for example be prokaryotic (for example bacterial), fungal, yeast or plant cells. Preferably, the host cells are not human cells.

Examples of suitable bacterial host organisms are gram negative bacterium or gram positive bacteria.

Depending on the nature of the nucleotide sequence encoding a polypeptide having the specific properties as defined herein, and/or the desirability for further processing of the expressed protein, eukaryotic hosts such as yeasts or other fungi may be preferred.

In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

The use of suitable host cells, such as yeast, fungal and plant host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

The host cell may be a protease deficient or protease minus strain.

ORGANISM

The term "organism" in relation to the present invention includes any organism that could comprise a nucleotide sequence according to the present invention or a nucleotide sequence encoding for a polypeptide having the specific properties as defined herein and/or products obtained therefrom.

Suitable organisms may include a prokaryote, fungus, yeast or a plant.

The term "transgenic organism" in relation to the present invention includes any organism that comprises a nucleotide sequence coding for a polypeptide having the specific properties as defined herein and/or the products obtained therefrom, and/or wherein a promoter can allow expression of the nucleotide sequence coding for a polypeptide having the specific properties as defined herein within the organism. Preferably the nucleotide sequence is incorporated in the genome of the organism.

The term "transgenic organism" does not cover native nucleotide coding sequences in their natural environment when they are under the control of their native promoter which is also in its natural environment.

Therefore, the transgenic organism of the present invention includes an organism comprising any one of, or combinations of, a nucleotide sequence coding for a polypeptide having the specific properties as defined herein, constructs as defined herein, vectors as defined herein, plasmids as defined herein, cells as defined herein, or the products thereof For example the transgenic organism can also comprise a nucleotide sequence coding for a polypeptide having the specific properties as defined herein under the control of a heterologous promoter.

TRANSFORMATION OF HOST CELLS/ORGANISM

As indicated earlier, the host organism can be a prokaryotic or a eukaryotic organism. Examples of suitable prokaryotic hosts include E. *coli* and *Bacillus subtilis.*

Teachings on the transformation of prokaryotic hosts is well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.

In another embodiment the transgenic organism can be a yeast.

Filamentous fungi cells may be transformed using various methods known in the art - such as a process

involving protoplast formation and transformation of the protoplasts followed by regeneration of the cell wall in a manner known. The use of *Aspergillus* as a host microorganism is described in EP 0 238 023.

Another host organism can be a plant. A review of the general techniques used for transforming plants may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

General teachings on the transformation of fungi, yeasts and plants are presented in following sections.

TRANSFORMED FUNGUS

A host organism may be a fungus - such as a filamentous fungus. Examples of suitable such hosts include any member belonging to the genera Thermomyces, Acremonium, Aspergillus, Penicillium, Mucor, Neurospora, Trichoderma and the like.

Teachings on transforming filamentous fungi are reviewed in US-A-5741665 which states that standard techniques for transformation of filamentous fungi and culturing the fungi are well known in the art. An extensive review of techniques as applied to *N. crassa* is found, for example in Davis and de Serres, Methods Enzymol (1971) 17A: 79-143.

Further teachings on transforming filamentous fungi are reviewed in US-A-5674707.

In one aspect, the host organism can be of the genus *Aspergillus,* such as *Aspergillus niger.*

A transgenic *Aspergillus* according to the present invention can also be prepared by following, for example, the teachings of Turner G. 1994 (Vectors for genetic manipulation. In: Martinelli S.D., Kinghorn J.R. (Editors) Aspergillus: 50 years on. Progress in industrial microbiology vol 29. Elsevier Amsterdam 1994. pp. 641-666).

Gene expression in filamentous fungi has been reviewed in Punt et al. (2002) Trends Biotechnol 2002 May; 20(5):200-6, Archer & Peberdy Crit Rev Biotechnol (1997) 17(4):273-306.

TRANSFORMED YEAST

In another embodiment, the transgenic organism can be a yeast.

A review of the principles of heterologous gene expression in yeast are provided in, for example, Methods Mol Biol (1995), 49:341-54, and Curr Opin Biotechnol (1997) Oct;8(5):554-60

In this regard, yeast - such as the species *Saccharomyces cerevisi or Pichia pastoris* (see FEMS Microbiol Rev (2000 24(1):45-66), may be used as a vehicle for heterologous gene expression.

A review of the principles of heterologous gene expression in *Saccharomyces cerevisiae* and secretion of gene products is given by E Hinchcliffe E Kenny (1993, "Yeast as a vehicle for the expression, of heterologous genes", Yeasts, Vol 5, Anthony H Rose and J Stuart Harrison, eds, 2nd edition, Academic Press. Ltd.).

For the transformation of yeast, several transformation protocols have been developed. For example, a transgenic Saccharomyces according to the present invention can be prepared by following the teachings of Hinnen et al., (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells may be selected using various selective markers - such as auxotrophic markers dominant antibiotic resistance markers.

A suitable yeast host organism can be selected from the biotechnologically relevant yeasts species such as, but not limited to, yeast species selected from *Pichia* spp., *Hansenula* spp., *Kluyveromyces, Yarrowinia* spp., *Saccharomyces* spp., including *S. cerevisiae,* or *Schizosaccharomyce* spp. including *Schizosaccharomyce pombe.*

A strain of the methylotrophic yeast species *Pichia pastoris* may be used as the host organism.

In one embodiment, the host organism may be a *Hansenula* species, such as *H. polymorpha* (as described in WO01/39544).

TRANSFORMED PLANTS/PLANT CELLS

A host organism suitable for the present invention may be a plant. A review of the general techniques may be found in articles by Potrykus (Annu Rev Plant Physiol Plant Mol Biol [1991] 42:205-225) and Christou (Agro-Food-Industry Hi-Tech March/April 1994 17-27), or in WO01/16308. The transgenic plant may produce enhanced levels of phytosterol esters and phytostanol esters, for example.

Therefore the present invention also relates to a method for the production of a transgenic plant with enhanced levels of phytosterol esters and phytostanol esters, comprising the steps of transforming a plant cell with a lipid acyltransferase as defined herein (in particular with an expression vector or construct comprising a lipid acyltransferase as defined herein), and growing a plant from the transformed plant cell.

SECRETION

Often, it is desirable for the polypeptide to be secreted from the expression host into the culture medium from where the enzyme may be more easily recovered. According to the present invention, the secretion leader sequence may be selected on the basis of the desired expression host. Hybrid signal sequences may also be used with the context of the present invention.

Typical examples of heterologous secretion leader sequences are those originating from the fungal amyloglucosidase (AG) gene (*gla*A - both 18 and 24 amino acid versions e.g. from *Aspergillus*), the a-factor gene (yeasts e.g. *Saccharomyces, Kluyveromyces* and *Hansenula*) or the α-amylase gene (*Bacillus*).

DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS).

A wide variety of labels and conjugation techniques are known by those skilled in the art and can be used in various nucleic and amino acid assays.

A number of companies such as Pharmacia Biotech (Piscataway, NJ), Promega (Madison, WI), and US Biochemical Corp (Cleveland, OH) supply commercial kits and protocols for these procedures.

Suitable reporter molecules or labels include those ; radionuclides; - enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241.

Also, recombinant immunoglobulins may be produced as shown in US-A-4,816,567.

FUSION PROTEINS

A polypeptide having the specific properties as defined herein may be produced as a fusion protein, for example to aid in extraction and purification thereof. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the activity of the protein sequence.

Gene fusion expression systems in *E. coli* have been reviewed in Curr. Opin. Biotechnol. (1995) 6(5):501-6.

In another embodiment of the invention, the amino acid sequence of a polypeptide having the specific properties as defined herein may be ligated to a heterologous sequence to encode a fusion protein. For example, for screening of peptide libraries for agents capable of affecting the substance activity, it may be useful to encode a chimeric substance expressing a heterologous epitope that is recognized by a commercially available antibody.

The invention will now be described, by way of example only, with reference to the following Figures and Examples.

Figure 1 shows a pfam00657 consensus sequence from database version 6 (SEQ. ID No. 1);

Figure 2 shows an amino acid sequence (SEQ ID No. 2) obtained from the organism *Aeromonas hydrophila* (P10480; GI:121051);

Figure 3 shows an amino acid sequence (SEQ ID No. 3) obtained from the organism *Aeromonas salmonicida* (AAG098404; GI:9964017);

Figure 4 shows an amino acid sequence (SEQ ID No. 4) obtained from the organism *Streptomyces coelicolor* A3 (2) (Genbank accession number NP_631558);

Figure 5 shows an amino acid sequence (SEQ ID No. 5) obtained from the organism *Streptomyces coelicolor* A3 (2) (Genbank accession number: CAC42140);

Figure 6 shows an amino acid sequence (SEQ ID No. 6) obtained from the organism *Saccharomyces cerevisiae* (Genbank accession number P41734);

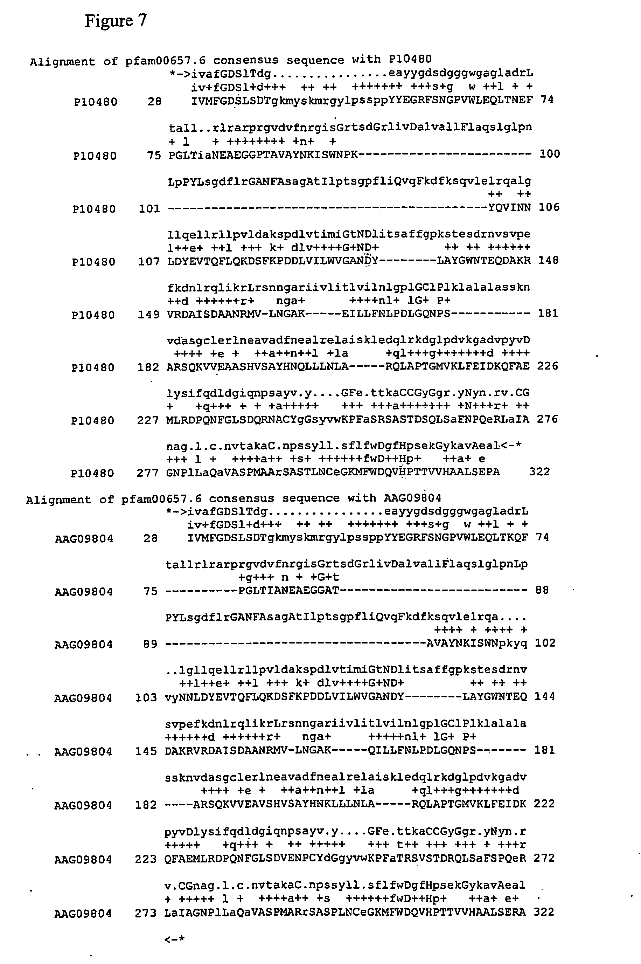

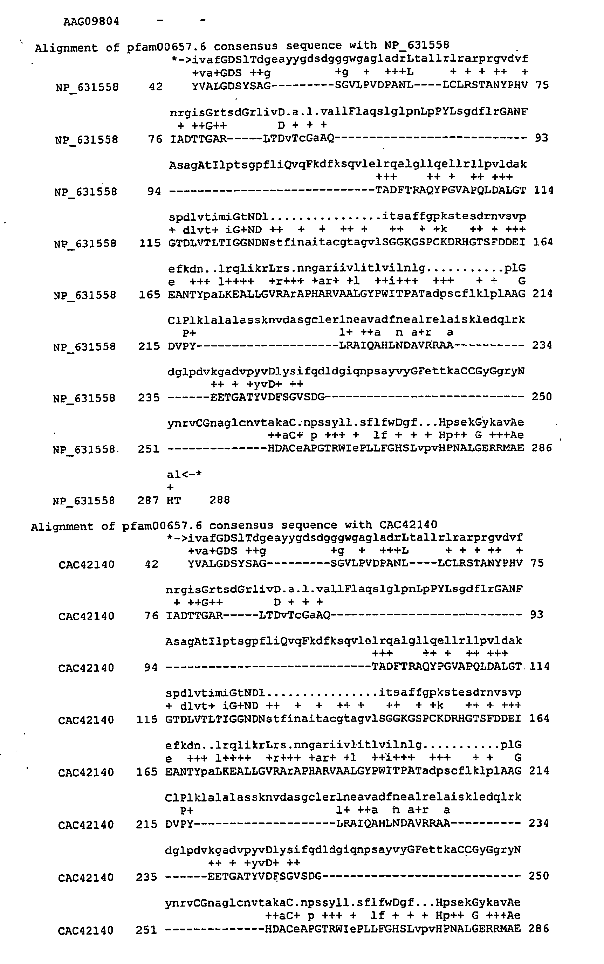

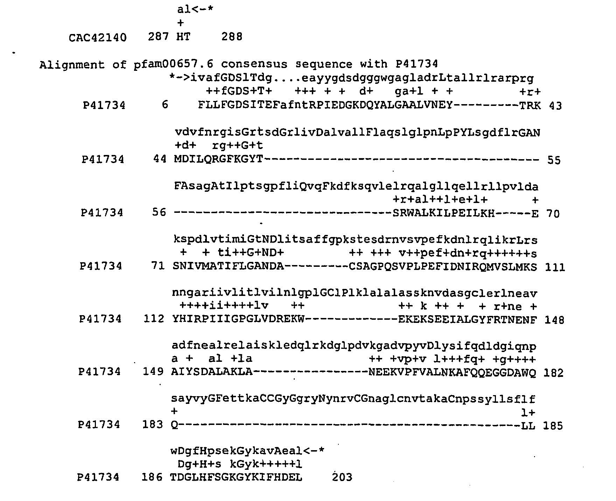

Figure 7 shows an alignment of selected sequences to pfam00657 consensus sequence;

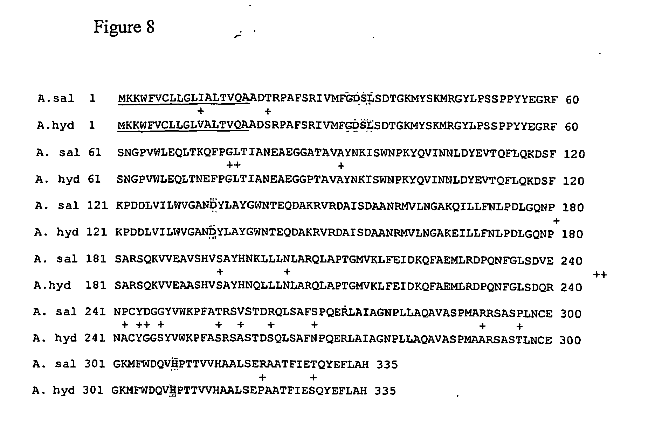

Figure 8 shows a pairwise alignment of SEQ ID No. 3 with SEQ ID No. 2 showing 93% amino acid sequence identity. The signal sequence is underlined. + denotes differences. The GDSX motif containing the active site serine 16, and the active sites aspartic acid 116 and histidine 291 are highlighted (see shaded regions). Numbers after the amino acid is minus the signal sequence;

Figure 9 shows a nucleotide sequence (SEQ ID No. 7) encoding a lipid acyl transferase obtained from the organism *Aeromonas hydrophila;*

Figure 10 shows a nucleotide sequence (SEQ ID No. 8) encoding a lipid acyl transferase obtained from the organism *Aeromonas salmonicida;*

Figure 11 shows a nucleotide sequence (SEQ ID No. 9) encoding a lipid acyl transferase obtained from the organism *Streptomyces coelicolor* A3(2) (Genbank accession number NC_003888.1:8327480..8328367);

Figure 12 shows a nucleotide sequence (SEQ ID No. 10) encoding a lipid acyl transferase obtained from the organism *Streptomyces coelicolor* A3(2) (Genbank accession number AL939131.1:265480..266367);

Figure 13 shows a nucleotide sequence (SEQ ID No. 11) encoding a lipid acyl transferase obtained from the organism *Saccharomyces cerevisiae* (Genbank accession number Z75034);

Figure 14 shows an amino acid sequence (SEQ ID No. 12) obtained from the organism *Ralstonia* (Genbank accession number: AL646052);

Figure 15 shows a nucleotide sequence (SEQ ID No. 13) encoding a lipid acyl transferase obtained from the organism *Ralstonia*;

Figure 16 shows SEQ ID No. 20. Scoe1 NCBI protein accession code CAB39707.1 GI:4539178 conserved hypothetical protein [Streptomyces coelicolor A3(2)];

Figure 17 shows a nucleotide sequence shown as SEQ ID No. 21 encoding NCBI protein accession code CAB39707.1 GI:4539178 conserved hypothetical protein [Streptomyces coelicolor A3 (2)];

Figure 18 shows an amino acid shown as SEQ ID No.22. Scoe2 NCBI protein accession code CAC01477.1 GI: 9716139 conserved hypothetical protein [Streptomyces coelicolor A3(2)];

Figure 19 shows a nucleotide sequence shown as SEQ ID No. 23 encoding Scoe2 NCBI protein accession code CAC01477.1 GI:9716139 conserved hypothetical protein [Streptomyces coelicolor A3(2)];

Figure 20 shows an amino acid sequence (SEQ ID No.24) Scoe3 NCBI protein accession code CAB88833.1 GI: 7635996 putative secreted protein. [Streptomyces coelicolor A3(2)];

Figure 21 shows a nucleotide sequence shown as SEQ ID No. 25 encoding Scoe3 NCBI protein accession code CAB88833.1 GI:7635996 putative secreted protein. [Streptomyces coelicolor A3(2)];

Figure 22 shows an amino acid sequence (SEQ ID No.26) Scoe4 NCBI protein accession code CAB89450.1 GI: 7672261 putative secreted protein. [Streptomyces coelicolor A3(2)];

Figure 23 shows an nucleotide sequence shown as SEQ ID No. 27 encoding Scoe4 NCBI protein accession code CAB89450.1 GI:7672261 putative secreted protein. [Streptomyces coelicolor A3(2)];

Figure 24 shows an amino acid sequence (SEQ ID No.28) Scoe5 NCBI protein accession code CAB62724.1 GI: 6562793 putative lipoprotein [Streptomyces coelicolor A3(2)];

Figure 25 shows a nucleotide sequence shown as SEQ ID No. 29, encoding Scoe5 NCBI protein accession code CAB62724.1 GI:6562 793 putative lipoprotein [Streptomyces coelicolor A3(2)];

Figure 26 shows an amino acid sequence (SEQ ID No.30) Srim1 NCBI protein accession code AAK84028.1 GI: 15082088 GDSL-lipase [Streptomyces rimosus];

Figure 27 shows a nucleotide sequence shown as SEQ ID No. 31 encoding Srim1 NCBI protein accession code AAK84028.1 GI:15082088 GDSL-lipase [Streptomyces rimosus];

Figure 28 shows an amino acid sequence (SEQ ID No.32) A lipid acyl transferase from *Aeromonas hydrophila* (ATCC #7965);

Figure 29 shows a nucleotide sequence (SEQ ID No. 33) encoding a lipid acyltransferase from *Aeromonas hydrophila* (ATCC #7965);

Figure 30 shows an amino acid sequence (SEQ ID No.34) of a lipid acyltransferase from *Aeromonas salmonicida* subsp. *Salmonicida* (ATCC#14174);

Figure 31 shows a nucleotide sequence (SEQ ID No 35) encoding a lipid acyltransferase from *Aeromonas salmonicida* subsp. *Salmonicida* (ATCC#14174);

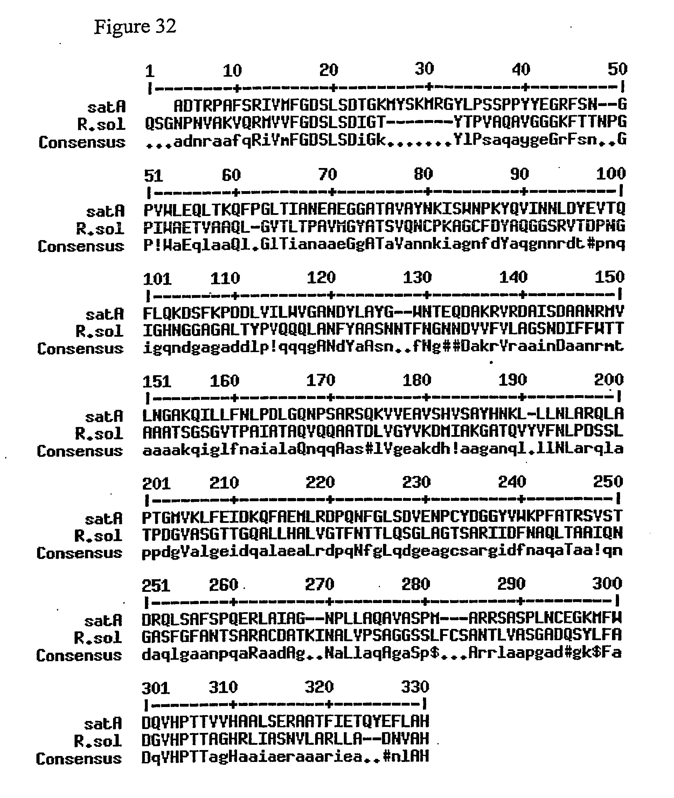

Figure 32 shows that homologues of the *Aeromonas* genes can be identified using the basic local alignment search tool service at the National Center for Biotechnology Information, NIH, MD, USA and the completed genome databases. The GDSX motif was used in the database search and a number of sequences/genes potentially encoding enzymes with lipolytic activity were identified. Genes were identified from the genus Streptomyces, Xanthomonas and Ralstonia. As an example below, the Ralstonia solanacearum was aligned to the Aeromonas salmonicida (satA) gene. Pairwise alignment showed 23% identity. The active site serine is present at the amino terminus and the catalytic residues histidine and aspartic acid can be identified;

Figure 33 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The symbol indicates a residue without a corresponding residue in the Pfam consensus. The sequences are the amino acid sequences listed in Figures 16, 18, 20, 22, 24, 26, 28 and 30.

Figure 34 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The symbol indicates a residue without a corresponding residue in the Pfam consensus. The sequences are the amino acid sequences listed in Figures 2, 16, 18, 20, 26, 28 and 30. All these proteins were found to be active against lipid substrates.

Figure 35 shows a expression vector pet12-AsalGCAT= pSM containing the C-terminal His-tagged *Aeromonas salmonicida* lipid acyltransferase gene;

Figure 36 shows the results of testing cell extracts in a NEFA Kit Assay, which depicts the activity of a recombinant, *A. salmonicida* lipid acyltransferase, towards lecithin. The wells from left to right indicate: a positive control, a negative control (i.e. extracts from empty plasmid) and samples collected after 0, 1, 2 and 3 hours cultivation after IPTG induction;

Figure 37 shows growth optimisation of BL21 (DE3)pLysS harboring the expression vector petl2-AsaIGCAT= pSM showing cultivation at 30 °C resulted in the production of enzyme with high activity towards lecithin. Cell extracts were tested for phospholipase activity using the NEFA kit assay. Wells from left to right: positive control; negative control; 20°C; 30°C;

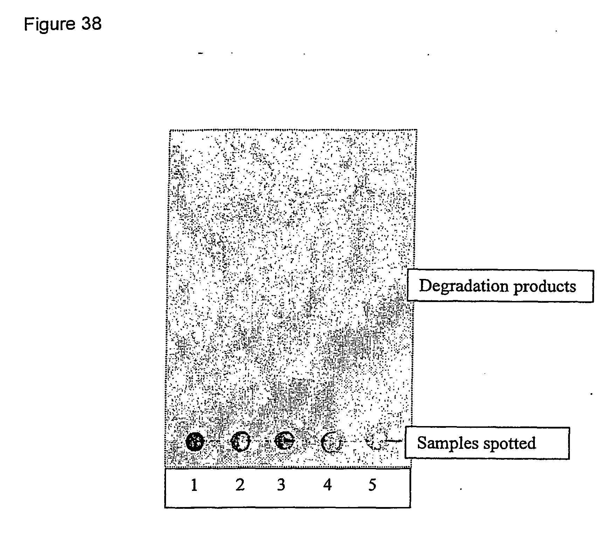

Figure 38 shows crude cell extracts from BL21(DE3)pLysS expressing active lipid acyltransferase incubated with the substrate lecithin and reaction mixture was analyzed using thin layer chromatography showing the presence of degradation products. Lanes: 1. No enzyme; 2. +A.sal-10ul 37°C; 3. + A. sal -20ul 37°C; 4. + A.sal - 10ul 24°C; 5. +-A. sal -20u 24°C;

Figure 39 shows partial purification of the *Aeromonas salmonicida* Acyl Transferase showing the phospholipase activity associated with purified His-tag protein. SE = Sonicated extracts, His = Purified with Ni-NTA spin-kit from Qiagen;

Figure 40 shows the expression vector pet12-A.h. GCAT=pSMa containing the C-terminal His-tagged *Aeromonas hydrophila* Glycerolipid Acyl Transferase (GCAT) gene was used to transform *E.coli* strain BL21(DE3)pLysS;

Figure 41 shows the activity of the crude extracts (5 & 10ul) containing the recombinant *Aeromonas hydrophila* GCAT enzyme was tested towards lecithin using Non-Esterified Fatty Acid (NEFA) kit (Roche, Switzerland), showing the presence of active enzyme towards the phospholipid, lecithin;

Figure 42 shows growth optimisation of BL21(DE3)pLysS harboring the expression vector pet12-AsalGCAT= pSM showing cultivation at 30 °C resulted in the production of enzyme with high activity towards lecithin. Cell extracts were tested for phospholipase activity using the NEFA kit assay;

Figure 43 shows the partial purification of the *Aeromonas hydrophila & A. salmonicida* Acyl Transferases showing the phospholipase activity associated with purified His-tag protein. SE = Sonicated extracts, His = Purified with Ni-NTA spin-kit from Qiagen);

Figure 44 shows the expression of the *Aeromonas* genes in *Bacillus subtilis* 163 showing the production of secreted enzyme with activity towards both lecithin and DGDG. pUB-AH= construct containing the *A. hydrophila* gene and pUB-AS, construct with the *A. salmonicida* gene, Culture filtrate was incubated with the substrates for 60 minutes.

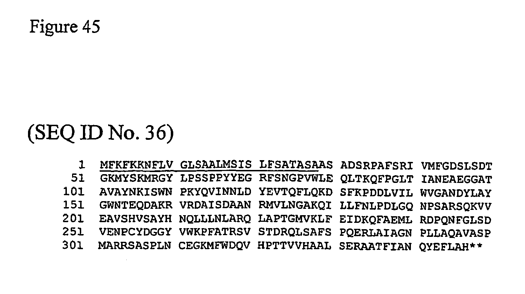

Figure 45 shows an amino acid sequence (SEQ ID No. 36) of the fusion construct used for mutagenesis of the *Aeromonas hydrophila* lipid acyltransferase gene in Example 17. The underlined amino acids is a xylanase signal peptide;

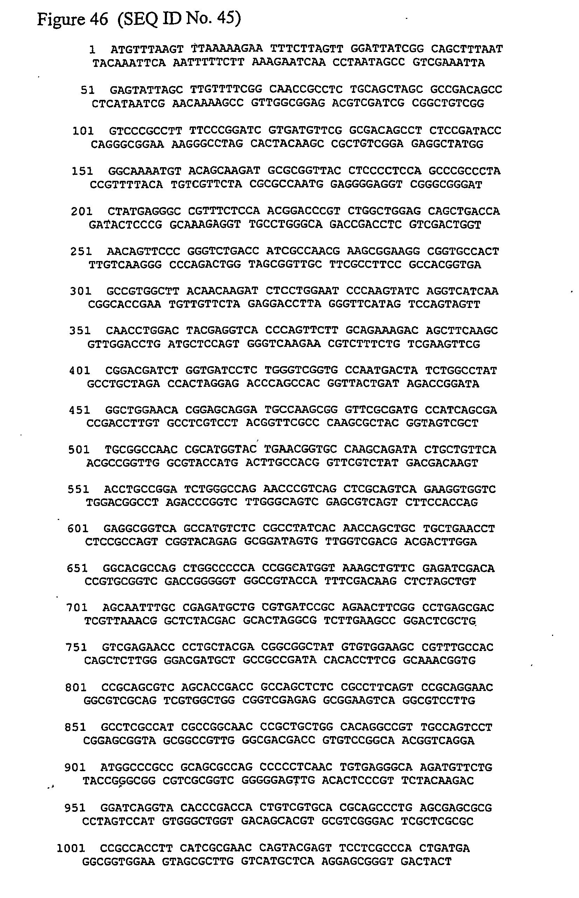

Figure 46 shows a nucleotide sequence (SEQ ID No. 45) encoding an enzyme from *Aeromonas hydrophila* including a xylanase signal peptide;

Figure 47 shows the result of the HPTLC analysis in Experiment I;

Figure 48 shows the result of the HPTLC analysis in Experiment II;

Figure 49 shows a calibration curve for monoglyceride standard solutions;

Figure 50 shows a calibration curve for diglyceride standard solutions.

Figure 51 shows a nucleotide sequence encoding a lipid acyltransferase enzyme according to the present invention from *Streptomyces* (SEQ ID No. 54);

Figure 52 shows a polypeptide sequence of a lipid acyltransferase enzyme according to the present invention from *Streptomyces* (SEQ ID No. 55);

## EXAMPLES

For the avoidance of doubt, the following abbreviations may be used herein:

MONO = monoglyceride
MAG = monoacylglycerol = monoglyceride
MAG and MONO are interchangeable herein.
DAG = diacylglycerol
FFA = free fatty acid

## EXAMPLE 1: The cloning, sequencing and heterologous expression of a transferase from *Aeromonas salmonicida* subsp. *Salmonicida*

Strains used:

*Aeromonas salmonicida subsp. Salmonicida* (ATCC 14174) was obtained from ATCC and grown overnight at 30°C in Luria-Bertani medium (LB). The cells were centrifuged and genomic DNA was isolated using the procedures for genomic DNA isolation from Qiagen Ltd. Genomic DNA buffer set (cat.19060), protease K (cat. 19131) and RNAse A (cat. 19101) were all obtained from Qiagen Ltd. (Boundary court Gatwick Court, West Sussex, RH10 2AX).

Host bacterial strain BL21 (DE3)pLysS (Novagen) was used for production of the recombinant *Aeromonas* enzymes. Competent cells of BL21(DE3)pLysS were used as host for transformation with the expression vector **pet12-AsalGCAT=pSM.**

Transformants containing the appropriate plasmid were grown at 37 °C in LB agar medium containing 100-ug ampicillin/ml.

### Construction of expression vector pet12-AsalGCAT- pSM:

For all DNA amplifications of the transferase genes from *Aeromonas,* genomic DNA (0.2-1 ul) was used as template and *pfu* DNA polymerase (2.5 units) was used with 10ul of 10x pfu buffer, 1ul each primer (50pmol/ul), 200 uMdNTP in a total reaction volume of 100ul. PCR reactions were performed in a programmable thermal cycler using the following conditions: 95 °C for 30 seconds, 30 cycles of 95 °C for 30 seconds, 60 °C for 1 minute and 68 °C for 2 minutes. An additional extension of 5 minutes at 72 °C was applied.

The PCR amplification of the transferase gene from *A. salmonicida* was carried in 2 separate PCR reactions. PCR reaction 1 was performed using primer pairs, as 1 USNEW(5'AGCATATGAAAA AATGGTTTGT TTGTTTATTG GGG 3' [SEQ ID No. 56]) and asls950new (5' GTG ATG GTG GGC GAG GAA CTC GTA CTG3' [SEQ ID No. 37]). A second PCR reaction was performed to incorporate a C-terminal Histidine tag using the PCR product from the first reaction and the primers: as1USNEW(5'AGCATATGAAAA AATGGTTTGT TTGTTTATTG GGG 3' [SEQ ID No. 38]) and AHLS1001(5'TTGGATCC GAATTCAT CAATG GTG ATG GTG ATG GTG GGC3' [SEQ ID No. 39]). The PCR product from the second reaction was purified and digested with restriction enzymes Nde1 and BamHI. 2 ug of pET 12a vector DNA was also digested with restriction enzymes Nde1 and BamHI and treated with phosphatase. The restriction enzyme-treated pet12a and PCR product from reaction 2 were purified and ligated using the Rapid Ligation Kit (Roche, Switzerland). The ligation mix was used to transform *E. coli* TOP10 cells. Transformants were plated on LB agar medium containing 1.00ug/ml ampicillin.

The T7 promoter primer (5'TAATACGACTCACTATAG3' [SEQ ID No. 40]) and the T7 terminator primer (5'CTAGTTATTGCTCAGCGG3' [SEQ ID No. 41]) were used to verify the sequences and the orientation of the cloned transferase genes in pET12a vector. DNA sequencing was performed using ABI Prism® BigDye™ Terminators Cycle sequencing kit with 500ng plasmid DNA as template and 3.2pmol T7 promoter and terminator primers.

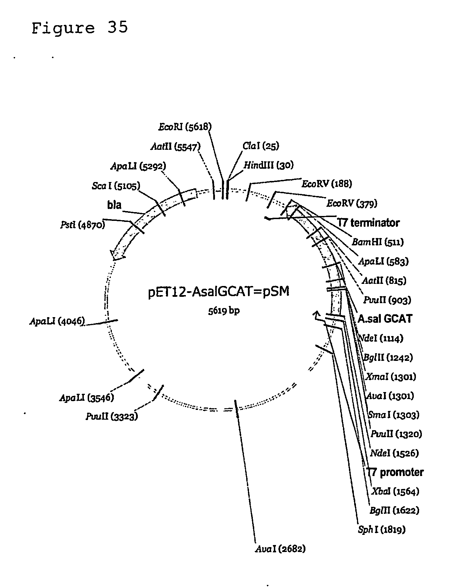

The construct shown in Figure 35 was used to transform competent bacterial host strain BL21(DE3)pLysS (Novagen) and ampicillin resistant transformants were picked and used for expression analysis:

Expression of the recombinant *Aeromonas salmonicida* lipid acyltransferase

Quantification of enzyme activity towards lecithin was determined on cell extracts using Non-Esterified Fatty Acid (NEFA) kit (Roche, Switzerland).

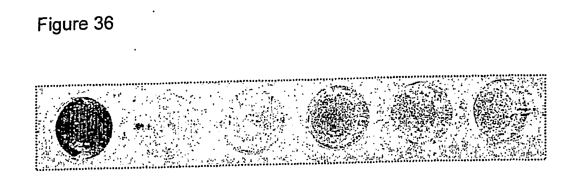

In Figure 36, BL21(DE3)pLysS harboring the expression vector pet12-AsalGCAT= pSM was grown in LB medium + 100ug/ml ampicillin and incubated with shaking at 37°C until $OD_{600}$ = 0.6 to 1.0 is reached. The cultures are then induced using IPTG (0.4mM) and incubation was continued for the next 3 hours. Samples where taken at 0 hour, 1, 2, and 3 hours after IPTG induction. Enzyme Activity was tested using the NEFA kit and lecithin as substrate.

Growth Optimisation for the production of more active enzymes

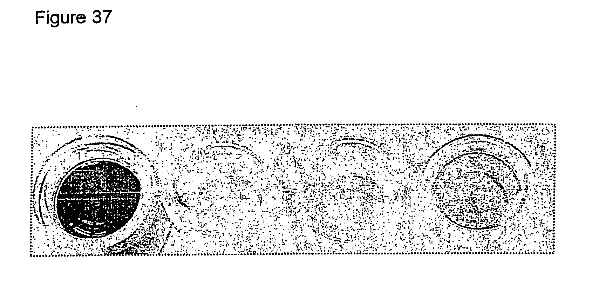

BL21(DE3)pLysS harboring, the expression vector pet12-AsalGCAT= pSM was grown in LB medium + 100ug/ml ampicillin and incubated with shaking at different growth temperatures (37°C, 30 °C, & 20 °C). The optimal condition for the production of active lipid acyltransferase enzyme was when cultures are grown at 30°C as shown in Figure 37.

Partial purification of recombinant *Aeromonas salmonicida* transferase

Strain BL21(DE3)pLysS harboring the expression vector pet12-AsalGCAT=pSM was grown at 37°C & crude

cell extracts were prepared by sonication. The recombinant enzyme was further purified from the sonicated crude cell extracts using the Ni-NTA spin kit from Qiagen. Phospholipase activity using the NEFA kit & Lecithin as substrate. Crude cell extracts from BL21 (DE3)pLysS expressing active transferase incubated with the substrate lecithin and reaction mixture was analysed using thin layer chromatography showing the presence of degradation products (see Figure 3 8).

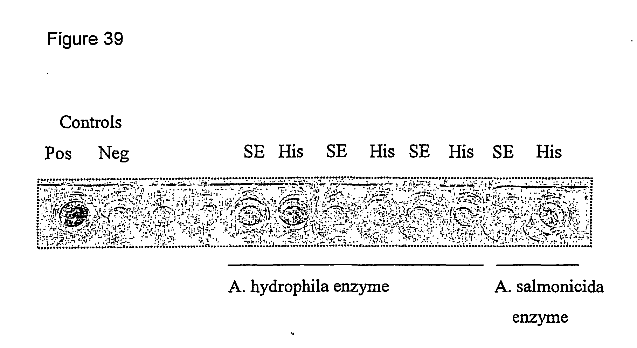

Partial Purification of recombinant *Aeromonas salmonicidae* transferase. Strain BL21(DE3)pLysS harbouring the expression vector pet12-AsalGCAT=pSM was grown at 37°C and crude cell extracts were prepared by sonication. The recombinant enzyme ware further purified from the sonicated crude cell extract using the Ni-NTA spin kit from Qiagen. Phospholipase activity using the NEFA kit and lecithin as substrate was tested (see Figure 39).

## EXAMPLE 2 Cloning and Expression of *Aeromonas hydrophila* transferase in *E. coli*

*Aeromonas hydrophila* (ATCC # 7965) was obtained from ATCC and grown overnight at 30°C in Luria-Bertani medium (LB). The cells were centrifuged and genomic DNA was isolated using the procedures for genomic DNA isolation from Qiagen Ltd. Genomic DNA buffer set (cat.19060), protease K (cat. 19131) and RNAse A (cat. 19101) were all obtained from Qiagen Ltd. (Boundary court Gatwick Court, West Sussex, RH10 2AX).

Host bacterial strain BL21(DE3)pLysS (Novagen) was used for production of the recombinant *Aeromonas* enzymes. Competent cells of BL21(DE3)pLysS were used as host for transformation with the expression vector pet12a-A.h.GCAT=pSMa. Transformants containing the appropriate plasmid were grown at 37 °C in LB agar medium containing 100-ug ampicillin/ml.

### Construction of expression vector pet12a-A.h.GCAT- pSMa:

For all DNA amplifications of the transferase gene from *Aeromonas*, genomic DNA (0.2-1 ul) was used as template and *pfu* DNA polymerase (2.5 units) was used with 10ul of 10x pfu buffer, 1ul each primer (50pmol/ul), 200 uMdNTP in a total reaction volume of 100ul. PCR reactions were performed in a programmable thermal cycler using the following conditions: 95 °C for 30 seconds, 30 cycles of 95 °C for 30 seconds, 60 °C for 1 minute and 68 °C for 2 minutes. An additional extension of 5 minutes at 72°C was applied.

The PCR amplification of the transferase gene from *A. hydrophila* (ATCC # 7965) was carried out in 2 separate PCR reactions.

PCR reaction 1 was performed using primer pairs, AHUS1 (5'GTCATATGAAAAAATGGTTTGTGTGTTTATTGGGATTGGTC3', SEQ ID No. 42) and ahls950 (5'ATGGTGATGGTGGGCGAGGAACTCGTACTG3', SEQ ID No. 43).

A second PCR reaction was performed to incorporate a C-terminal Histidine tag using the PCR product from the first reaction and the primer pairs:

AHUS1 (5'GTCATATGAAAAAATGGTTTGTGTGTTTATTGGGATTOGTC3' SEQ ID No. 44,) and
AHLS 1001 (5'TTGGATCCGAATTCATCAATGGTGATGGTGATGGTGGGC3' SEQ ID No. 57).

The PCR product from the second reaction was purified and digested with restriction enzymes Nde1 and BamHI. 2 ug of pET 12a vector DNA was also digested with restriction enzymes Nde1 and BamHI and treated with phosphatase. The restriction enzyme-treated pet12a and PCR product from reaction 2 were purified and ligated using the Rapid Ligation Kit (Roche, Switzerland). The ligation mix was used to transform *E. coli* TOP 10 cells. Transformants were plated on LB agar medium containing 100ug/ml ampicillin.

The T7 promoter primer (5'TAATACGACTCACTATAG3') and the T7 terminator primer (5'CTAGTTATTGCTCAGCGG3') were used to verify the sequences and the orientation of the cloned GCAT genes in pET12a vector. DNA sequencing was performed using ABI Prism® BigDye™ Terminators Cycle sequencing kit with 500ng plasmid DNA as template and 3.2pmol T7 promoter and terminator primers.

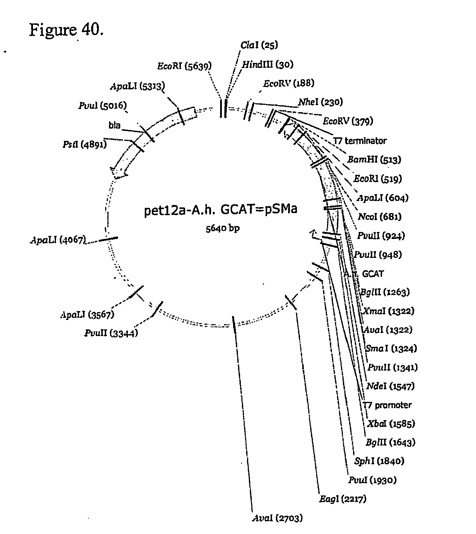

The construct shown in Figure 40 was used to transform competent bacterial host strain BL21 (DE3)pLysS (Novagen) and ampicillin resistant transformants were picked and used for expression analysis.

### Expression of the *Aeromonas hydrophila* transferase in BL21(DE3)pLysS

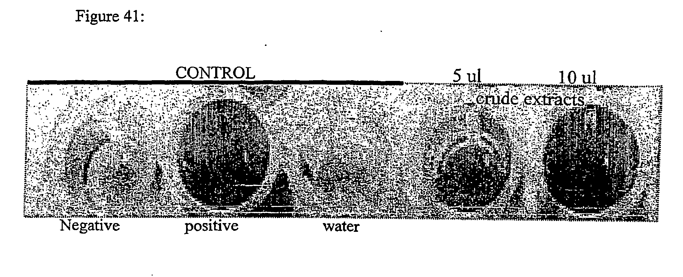

The *E. coli* strain BL21(DE3)pLysS harboring the expression vector pet12a-A.h.GCAT= pSMa was grown in LB medium + 100ug/ml ampicillin and incubated with shaking at 37°C until $OD_{600}$= 0.6 to1.0 is reached. The cultures are then induced using IPTG (0.4mM) and incubation was continued for the next 3 hours. Samples where taken at 0hour, 1, 2, and 3 hours after IPTG induction. Enzyme Activity was tested using the NEFA kit and lecithin as substrate (Figure 41).

**Growth Optimisation for the production of more active enzymes**

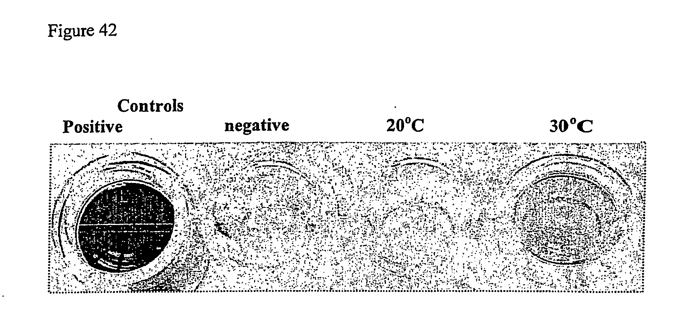

BL21(DE3)pLysS harboring the expression vector pet12a-A.h.GCAT= pSMa was grown in LB medium + 100ug/ml ampicillin and incubated with shaking at different growth temperatures (37°C, 30 °C, & 20 °C). The optimal condition for the production of active GCAT enzyme was when cultures are grown at 30°C as shown in Figure 42.

**Partial purification of recombinant *A.hydrophila* transferase (GCAT)**

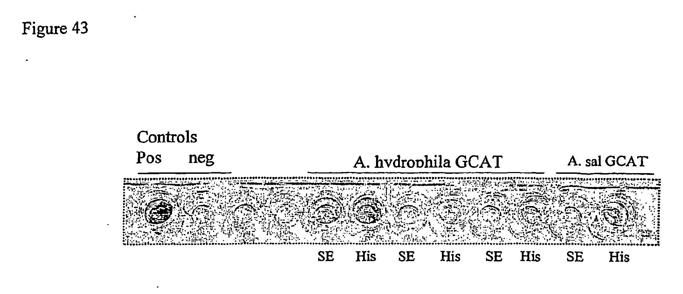

Strain BL21(DE3)pLysS harboring the expression vector pet12a-A.h.GCAT=pSMa was grown at 37°C & crude cell extracts were prepared by sonication. The recombinant enzyme was further purified from the sonicated crude cell extracts using the Ni-NTA spin kit from Qiagen. Phospholipase activity assay using the NEFA kit & Lecithin as substrate. (Figure 43).

**EXAMPLE 3: Expression of *Aeromonas* transferases in *Bacillus subtilis* 163**

**Plasmid Construction**

Two different *Bacillus subtilis* expression vectors (pUB 110 & pBE5) were used for the heterologous expression of the *Aeromonas* genes in *Bacillus subtilis.* The pUB110 vector contains the alpha amylase promoter while the pBE vector has the P32 promoter as the regulatory region for the expression of the fused *Aeromonas* genes. In pUB110, the first amino acid of the mature GCAT genes of *Aeromonas* were fused in frame with the last amino acid of the xylanase signal peptide sequence from *Bacillus subtilis* via the restriction site Nhe1, creating an additional 2 amino acids in front of the mature proteins. pBE5 contains the cgtase signal sequence fusion at the Nco1 site for secretion of the recombinant proteins into the culture filtrate.
PCR reactions were carried out to obtain the *Aeromonas* genes fuse in frame to the signal sequences of the pUB 110 and the pBE5 vectors. PCRs were performed using the following primer pairs for *A. hydrophila* gene:

PCR reaction 1: usAHncol (5'ATGCCATGGCCGACAGCCGTCCCGCC3', SEQ ID No. 46) and 1sAH (5'TTGGATCCGAATTCATCAATGGTGATG3', SEQ ID No. 47)

PCR reaction 2: US-Ahnhel (5'TTGCTAGCGCCGACAGCCGTCCCGCC3', SEQ ID No. 48.) and lsAH (5'TTGGATCCGAATTCATCAATGGTGATG3, SEQ ID No. 49)
PCRs were performed using the following primer pairs for *A. salmonicida* gene:

PCR reaction 3: US-Asncol (5'TTGCCATGGCCGACACTCGCCCCGCC3', SEQ ID No. 50) and lsAH (5'TTGGATCCGAATTCATCAATGGTGATG3', SEQ ID No. 51)

PCR reaction 4: US-ASnhel (5'TTGCTAGCGCCGACACTCGCCCCGCC3', SEQ ID No. 52) and lsAH (5'TTGGATCCGAATTCATCAATGGTGATG3', SEQ ID No. 53).

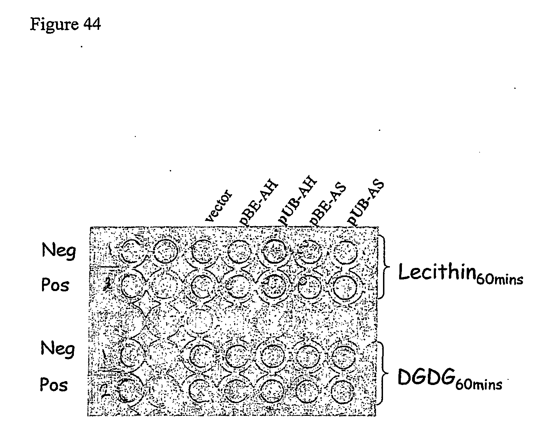

All the PCR products were cloned into PCR blunt II (TOPO vector) and sequenced with reverse & forward sequencing primers.
Clones from PCR reactions 1 & 3 were cut with Nco1 & Bam HI and used as inserts for ligation to the pBE5 vector cut with Nco1/BamH1/phosphatase. Clones from PCR reactions 2 & 4 were cut with Nhe1 & Bam H1 and used as inserts for ligation to the pUB vector that was cut with Nhe1/BamH1/phosphatase.
Expression of the *Aeromonas* transferase genes in *Bacillus subtilis* and characterization of the enzyme activity.
The acyl transferases from the two *Aeromonas* species have been successfully expressed in *E. coli* (results above). The *Bacillus* pUB110 & pBE5 gene fusion constructs were used to transform *Bacillus subtilis* and transformants were selected by plating on kanamycin plates. The kanamycin resistant transformants isolated and grown in 2xYT are capable of heterologous expression of the *Aeromonas* genes in *Bacillus.* The culture filtrates have digalactosyldiacylglycerol (DGDG) galactolipase activity, in addition to having both acyl transferase and phospholipase activities. The activity towards digalactosyldiacylglycerol (DGDG) was measured after 60 minutes of incubation of culture supernatant with the substrate, DGDG from wheat flour (obtainable form Sigma) as well as the activity towards lecithin as shown in Figure 44. *Bacillus* produced the enzyme after overnight (20-24 hours) to 48 hours of cultivation in the culture medium as a secreted protein. In some instances, the expression of the *Aeromonas* genes has been shown to interfere with cell viability and growth in *Bacillus & E. coli,* it is therefore necessary to carefully select expression strains and optimise the growth conditions to ensure expression. For example, several *Bacillus* host strains (B.s 163, DB104 and OS 21) were transformed with the expression vectors for growth comparison. B.s163 is transformable with the 2 *Aeromonas* genes

and is capable of expressing active protein. DB 104 is transformable with all the constructs but is only able to express *A. salmonicida* transferase.

**EXAMPLE 4: Fermentation and Purification of *Aeromonas* lipid acyltransferases produced in *E.coli***

***E.coli* Fermentations:**

Microorganisms

Two strains of *Eschericia coli, one* containing an *Aeromonas hydrophila* (Example 2) lipid acyltransferase and two containing *Aeromonas salmonicida* lipid acyltransferases, (Example 1) were used in this study.

The *E. coli* strain containing the *A. hydrophila* gene was named DIDK0124 , and the *E. coli* strain containing the *A. salmonicida* gene was named DIDK0125. The fermentation with DIDK0124 was named HYDRO0303 and the fermentation with DIDK0125 was named SAL0302. The purified protein from HYDRO025 was named REF#138. The purified protein from HYDRO0303 was named REF#135.

Growth media and culture conditions

**LB-agar**

The LB agar plates used for maintaining the strains contained: 10 g/L tryptone, 5 g/L yeast extract, 5 g/L NaCl, 15 g/L agar, 100 mg/L ampicillin and 35 mg/L chloramphenicol. The agar plates were incubated at 30°C.

**LB shake flask**

The LB medium (50 mL pr shake flask) used for production of inoculum material for the bioreactor cultivations contained: 10 g/L tryptone, 5 g/L yeast extract, 5 g/L NaCl, 100 mg/L ampicillin and 35 mg/L chloramphenicol. The shake flasks were inoculated from the LB agar plates, and incubated at 30°C and 200 rpm.

**Bioreactor cultivation**

The bioreactor cultivations were carried out in 6 L in-house built bioreactors filled with 4 L medium containing: 10 g/L tryptone, 5 g/L yeast extract, 5 g/L NaCl, 8 g/L $KH_2PO_4$, 0.9 g/L $MgSO_4$,$7H_2O$, 40 g/L glucose monohydrate, 0.4 mL/ ADD APT® Foamstop Sin 260 (ADD APT Chemicals AG, Helmond, The Netherlands), 10 mg/L $(NH_4)_2Fe(SO_4)_2 \cdot 6H_2O$, 0.7 mg/L $CuSO_4 \cdot 5H_2O$, 3 mg/L $ZnSO_4 \cdot 7H_2O$, 3 mg/L $MnSO_4 \cdot H_2O$, 10 mg/L EDTA, 0.1 mg/L $NiSO_4 \cdot 6H_2O$, 0.1 mg/L $CoCl_2$, 0.1 mg/L $H_3BO_4$, 0.1 mg/L KI, 0.1 mg/L $Na_2MoO_4 \cdot 2H_2O$, 1 g/L ampicillin and 35 mg/L chloramphenicol.

The bioreactors were inoculated with an amount of LB culture ensuring end of growth after approximately 20 hours of cultivation (calculated from the maximum specific growth rate of 0.6 $h^{-1}$, the $OD_{600}$ of the LB shake flask and the final $OD_{600}$ in the bioreactor of approximately 20).

SAL0302 was inoculated with 10 mL of LB culture, and HYDRO0303 was inoculated with 4 mL of LB culture.

The bioreactors were operated at the following conditions: temperature 30°C, stirring 800-1000 rpm (depending on experiment), aeration 5 L/min, pH /6.9, pH control 8.75% (w/v) $NH_3$-water and 2 M $H_2SO_4$. Induction was achieved by addition of isopropyl β-D-thiogalactoside to a final concentration of 0.6 mM, when 0.4 moles (HYDRO0303) and 0.7 moles $CO_2$ was produced respectively.

Harvest

The following procedure was used for harvest and homogenisation of the biomass:

1) The fermentation broth from the fermentations was centrifuged at 5000 × g and 4°C for 10 minutes, and the supernatant was discharged. The biomass was stored at -20°C until use. The biomass was thawed and resuspended in 500 mL of 20 mM $NaH_2PO_4$, pH 7.4, 500 mM NaCl, 10 mM Imidazole and Complete (EDTA-free) protease inhibitor (Roche, Germany).
2) The suspended biomass was homogenized at 2 kbar and 4°C in a cell disrupter from Constant Systems Ltd (Warwick, UK).
3) The cell debris was removed by centrifugation at 10.000 × g and 4°C for 30 minutes followed by collection of the supernatant.
4) The supernatant was clarified further by centrifugation at 13.700× g and 4°C for 60 minutes, followed by collection

of the supernatant.

5) The supernatant was filtered through 0.2 μm Vacu Cap filters (Pall Life Sciences, UK) and the filtrate was collected for immediate chromatographic purification.

Chromatographic purification of the Transferases

A column (2.5 x 10 cm) was packed with 50 ml of Chelating Sepharose ff. gel and charged with Ni-sulphate (according to the method described by manufacturer, Amersham Biosciences). The column was equilibrated with 200 ml of 20 mM $NaH_2PO_4$, pH 7.4, 500 mM NaCl, 10 mM Imidazole. 400 ml of crude was applied to the column at a flow rate of 5 ml/min. The column was then washed with 20 mM $NaH_2PO_4$, pH 7.4, 500 mM NaCl, 10 mM Imidazole until the $UV_{280}$ reached the base line. The GCAT was then eluted with 40 ml of 20 mM $NaH_2PO_4$, pH 7.4, 500 mM NaCl and 500 mM Imidazole.

**EXAMPLE 5: Fermentation and Purification of *Aeromona*s lipid acyltransferases produced in *Bacillus subtilis.***

Fermentations

BAC0318-19, BAC0323-24

Microorganism

The microorganisms used in this study originate from transformation of a *Bacillus subtilis* host strain, #163 with a plasmid containing the gene encoding the *Aeromonas salmonicida* transferase inserted in the vector pUB110OIS. The expression of the gene is controlled by an alpha-amylase promoter, and the secretion of the transferase is mediated by the *B. subtilis* xylanase signal sequence (Example 3). The strains were named DIDK0138 (fermentation BAC0318-19) and DIDK0153 (fermentation BAC0323-24).

Growth media and culture conditions

**Pre culture medium**

A shake flask (500 mL total volume, with baffles) was added 100 mL of a medium containing:

| | |
|---|---|
| NaCl | 5 g/L |
| $K_2HPO_4$ | 10 g/L |
| Soy flour | 20 g/L |
| Yeast extract, BioSpringer 106 | 20 g/L |
| Antifoam, SIN260 | 5 mL/L |

pH was adjusted to 7.0 before autoclaving

After autoclaving 6 mL 50% (w/w) Nutriose were added pr flask. Kanamycin was added at a concentration of 50 mg/L after autoclaving.

**Inoculation**

A pre culture shake flask was inoculated with frozen culture directly from a 25% (w/v) glycerol stock. The shake flask was incubated at 33°C and 175 rpm for approximately 16 hours, whereupon 50 mL was used to inoculate the fermentor.

*Fermentations*

The fermentations were carried out in 6 L in house built fermentors.

The batch medium (3 L) contained:

| | |
|---|---|
| Corn steep liquor (50% dw) | 40 g/L |
| Yeast extract BioSpringer 153 (50% dw) | 10 g/L |

(continued)

| | |
|---|---|
| NaCl | 5 g/L |
| $CaCl_2$, $2H_2O$ | 0.25 g/L |
| $Mn(NO_3)_2$, $H_2O$ | 0.2 g/L |
| Antifoam SIN260 | 1 mL/L |
| Kanamycin (filter sterilised to the fermentor after autoclaving | 50 mg/L |

The feed contained:

| | | |
|---|---|---|
| Glucose monohydrate | 540 g/kg | |
| $MgSO_4$, $7H_2O$ | 4.8 g/kg | |
| Antofoam SIN260 | 4 mL/kg | |
| Yeast extract, BioSpringer 153 (50% dw) (autoclaved separately) | 150 | g/kg |

The feed in fermentation BAC0318 and BAC0323 was started based on the accumulated $CO_2$, according to the equations below:

$$\text{Feed}-\text{flow}[g/h]=0, \text{AcCO}_2 < 0.15$$

$$\text{Feed}-\text{flow}[g/h]=2.85+t\cdot 1.54, \text{AcCO}_2 \geq 0.15 \text{ and } t<12$$

$$\text{Feed}-\text{flow}[g/h]=21.3, t>12$$

t: time (hours) from the point when the accumulated $CO_2$ ($AcCO_2$) reached 0.15 moles.

The feed in fermentation BAC0319 and BAC0324 was started based on the accumulated $CO_2$, according to the equations below:

$$\text{Feed}-\text{flow}[g/h]=0, \text{AcCO}_2 < 0.15$$

$$\text{Feed}-\text{flow}[g/h]=2.0+t\cdot 1.08, \text{AcCO}_2 \geq 0.15 \text{ and } t<12$$

$$\text{Feed}-\text{flow}[g/h]=15, t>12$$

t: time (hours) from the point when the accumulated $CO_2$ ($AcCO_2$) reached 0.15 moles.

The pH was controlled at 7.0 by adding 12.5% (w/v) $NH_3$-water or 2M phosphoric acid.

The aeration was 3 L/min corresponding to 1 vvm.

The temperature was 33°C.

The fermentor was equipped with two 8 cm Ø Rushton impellers placed with a distance of 10 cm.

Harvest

The biomass was removed by centrifugation at 16,000× g for 10 minutes at room temperature. The supernatant was filter sterilized, and the filtrate was used for purification and application tests.

**EXAMPLE 6: Enzymatic removal of DAGs in palm oil catalysed by a lipid acyltransferase from *Aeromonas salmonicidae.***

SUMMARY

The enzymatic glycerolysis experiments were initiated by the addition of the glycerol/transferase solution to the reactor containing palm oil and glycerol/water in varying concentrations. All the reactions were conducted at 43°C, using magnetic stirring for 24 hours. After reaction, the samples were analyzed using HPTLC, and in some experiments the result was confirmed by GC analysis. The conducted trials showed that there was a good correlation between the water concentration and levels of DAGs and MAGs, respectively: the lowest water concentrations tested gave the highest MAG level and the lowest DAG level.

Based on the trials, it can be concluded that it was possible to reduce the amount of DAGs in palm oil by a transferase-catalysed reaction where the enzyme used DAGs as donor molecules and glycerol as acceptor molecule - in a glycerolysis reaction in which monoglycerides were synthesized. This was in contrast to the glycerolysis reaction with conventional triglyceride hydrolyzing lipases where the amount of DAG increases.

Furthermore, it can be concluded that the water concentration has a significant impact on the synthesized yield of monoglycerides and amount of DAG. The following correlation was found: Low water concentration (<1%) and 5% glycerol gives increased MAG yield and decreased concentration of DAGs. The obtained results also show that primarily 1,2 isomer of diglyceride tend to be reduced.

It is known that diglycerides, especially 1,2 isomers delay the crystallization of fat. This effect causes post-crystallization of fat products like margarine and shortenings, which favors formation of large crystals (sandiness). In certain fat blends it was observed that diglycerides improve the stability of β'crystals. A reduction but not a complete removal of diglycerides in palm oil would therefore be preferable. It can thus be concluded that a reduction of diglyceride will result in a better and more uniform oil quality; a fact, which must not be underestimated.

INTRODUCTION

Problems concerning diglycerides depend to some extent on type of product, e.g. whether it is in margarine and shortenings. Thus, depending on the product the presence of diglycerides both have negative and positive effects. For margarine production, only fat product of a required crystal structure can be used in order to obtain proper consistency and plasticity. The β'-crystal form is the most required structure, showing small crystals and a high ability for maintaining the liquid fraction. The most stable crystal form (β-form) is undesirable, as having big crystals causes grained structure of the margarine. According to Hernquist & Anjou (1993) and Wahnelt *et al.* (1991) the presence of diglycerides in fat retard the transition from β' to β-crystal. But as diglycerides retard the transition from β' to β, they also retard the transition from α form to β' form (Walnet *et al.,* 1991). For this reason it can be defined that the presence of diglycerides retards the whole crystallization process. Slow crystallization has a crucial impact on margarine application. The effect of retarding crystallization in margarine blends containing a high portion of palm oil is that after conventional processing the product may be somewhat soft, causing packing difficulties, and subsequent crystallization may lead to a firmer texture than desired (Berger, 1990). In spite of advantages and disadvantages, it may be important to find the right balance between reduced and total removal of diglycerides.

It has surprisingly been found that it is possible to reduce the amount of diglyceride in palm oil by enzymatic glycerolysis of palm oil using a lipid acyltransferase, for example the GDSx lipid acyl transferase from *Aeromonas salmonicida.* Without wishing to be bound by theory, in this process the enzyme is added to palm oil together with small amount of glycerol and the enzyme catalyses the following reaction:

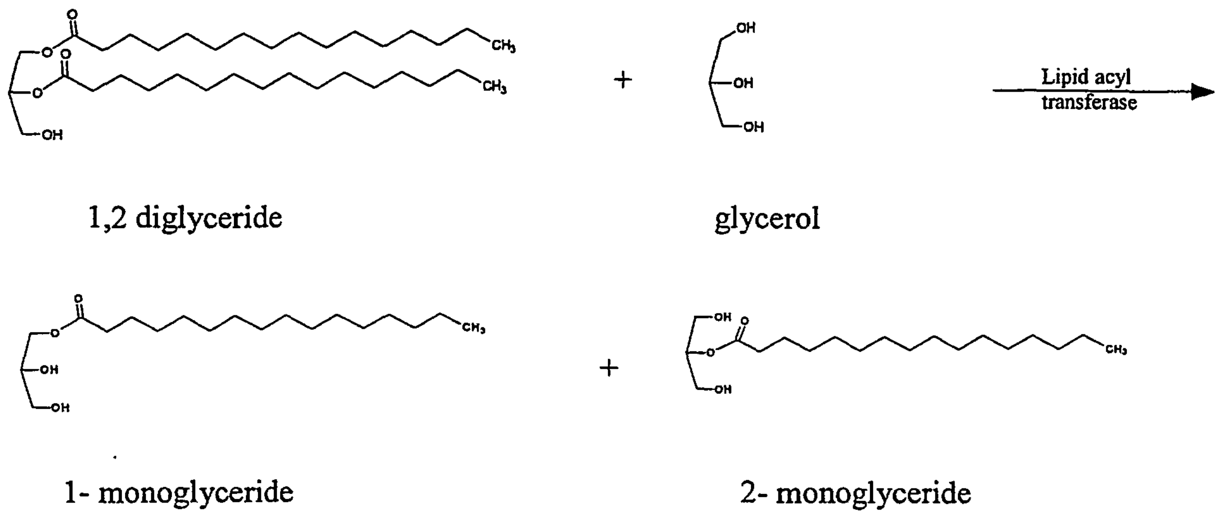


1,2 diglyceride

glycerol

1- monoglyceride

2- monoglyceride

After the reaction the surplus glycerol can, if deemed necessary, easily be separated from the reaction mixture by centrifugation or other processes.

The advantage of this process is that the amount of diglyceride (preferably 1,2 diglyceride) is reduced by a glycerolysis reaction catalysed by an enzyme which is specific for diglyceride without using the triglyceride as donor for the transferase reaction.

The reaction products monoglycerides do not have to be removed from the reaction mixture, but can advantageously be used as an efficient emulsifier for the production of food products like margarine and shortening. The process thus solves two problems. First of all the amount of diglyceride is reduced and preferable 1,2 diglyceride is removed, which has a negative impact on the crystallisation properties of the triglycerides. Secondly the reaction product monoglyceride can be used as an efficient emulsifier in the production of food products like margarine and shortening.

In one embodiment, it is preferably to remove the monoglycerides produced (if any) by the transferase reaction.

Suitably, if the transferase reaction has been carried out in the crude palm oil, the monoglyceride and/or glycerol and/or residues of water (if any) may be removed by a deodorisation process during the edible oil refining process.

Another very interesting advantage of the use of the lipid acyl transferase is that this enzyme is less dependent of the water content in the reaction mixture and because this enzyme is a transferase low or no hydrolytic activity takes place which means that the amount of free fatty acids do not increase significantly.

It is well known the water content in glycerolysis reactions are very important when lipolytic enzymes like lipases are use in this process (Kristensen, 2004). Even small amount of water, which is necessary for most lipolytic reactions will cause the formation of significant amount of free fatty acids. This problem can be overcome by using the lipid acyl transferase in the glycerolysis reaction.

Another aspect is that lipases known in the art to catalyse glycerolysis reaction, mainly uses triglyceride as donor during formation of diglyceride instead of reducing the amount of diglyceride.

Materials:

| | |
|---|---|
| Palm oil: | Palmotex, Aarhus United, Denmark |
| Glycerol: | J. T. Baker, (7044) |
| DIMODAN® P: | Danisco A/S, Denmark |
| Enzyme: | Lipid acyltransferase GCAT from *Aeromonas salmonicida* expressed in *B.subtilis* and fermented in lab scale (Transferase #196). |

Methods

Lyophilization of enzyme

The enzymes were desalted (PD-10 Desalting columns, Amersham Biosciences) before lyophilization. The

desalted enzyme were mixed with glycerol (Enzyme:Glycerol ratio 3,5:1). The sample was lyophilised and added 10% water. The sample contained approximately 20 U (phospholipase units) per gram.

**Determination of phospholipase activity (phospholipase activity assay):**

Substrate

0.6% L-α Phosphatidylcholine 95% Plant (Avanti #441601), 0.4% Triton-X 100 (Sigma X-100) and 5 mM $CaCl_2$ was dissolved in 0.05M HEPES buffer pH 7.

Assay procedure:

400 μL substrate was added to an 1.5 mL Eppendorf tube and placed in an Eppendorf Thermomixer at 37°C for 5 minutes. At time t= 0 min, 50 μL enzyme solution was added. Also a blank with water instead of enzyme was analyzed. The sample was mixed at 10*100 rpm in an Eppendorf Thermomixer at 37°C for 10 minutes. At time t=10 min the Eppendorf tube was placed in another thermomixer at 99°C for 10 minutes to stop the reaction.

Free fatty acid in the samples was analyzed by using the NEFA C kit from WAKO GmbH.

Enzyme activity PLU-7 at pH 7 was calculated as micromole fatty acid produced per minute under assay conditions

Enzyme reaction

Palm oil was reacted with the glycerol-enzyme solution according to the following recipes in Table 1 and Table 2

Table 1: Experiment I

| Enz. no. | Transferase #196 | | | |
|---|---|---|---|---|
| Sample no. | 1 | 2 | 3 | 4 |
| Gl(%) | 5 | 5 | 5 | 10 |
| Water (%) | 0 | 1 | 5 | 1 |
| Oil phase (%) | 95 | 94 | 90 | 89 |
| Water in the reaction mixture | 0.5 | 1.5 | 5.5 | 1.5 |

Table 2: Experiment II

| Enz. No. | Transferase #196 | | | | | |
|---|---|---|---|---|---|---|
| Sample no. | 1 | 2 | 3 | 4 | 5 | 6 |
| Gl(%) | 5 | 5 | 5 | 10 | 10 | 10 |
| Water (%) | 1 | 5 | 7.5 | 1 | 5 | 7.5 |
| Oil phase (%) | 94 | 90 | 87,5 | 89 | 85 | 82,5 |
| Water in the reaction mixture | 1 | 5 | 7.5. | 1 | 5 | 7.5 |

The palm oil was scaled in a 20 ml Wheaton glass and the glycerol/enzyme and optional water was added. The sample was placed in heating block (Multitherm HP 15 Stheating block heated with differential stirring (15 wells) controlled by a VARIOMAG - Thermomodul 40 ST thermostat) and reacted under the following conditions:

| | |
|---|---|
| Reaction temperature : | 43°C |
| Magnetic Stirring : | 650 rpm |
| Reaction time : | 20 hours |

The enzyme in the reaction mixture was inactivated at 97.5°C in 10 min. After reaction the sample were homogenized (Ultra Turrax) for 20 sec. and a homogeneous sample was taken out for further analysis.

HPTLC

Applicator: LINOMAT 5, CAMAG applicator.
HPTLC plate: 10 x 10 cm (Merck no. 1.05633)

The plate was activated before use by drying in an oven at 180°C for 20-30 minutes.

Application: 2,0μl of a 2,0% solution of reacted palm oil dissolved in Chloroform:Methanol (2:1) was applied to the HPTLC plate using LINOMAT 5 applicator.

Running-buffer: P-ether:MTBE:Acetic acid (50:50:1)
Application/Elution time: 8 minutes.
Developing fluid: 6% Cupriacetate in 16% $H_3PO_4$

After elution the plate was dried in an oven at 180°C for 10 minutes, cooled and immersed in the developing fluid and then dried additional in 20 minutes at 180°C. The plate was evaluated visually and scanned (ScanWizard 5) directly.

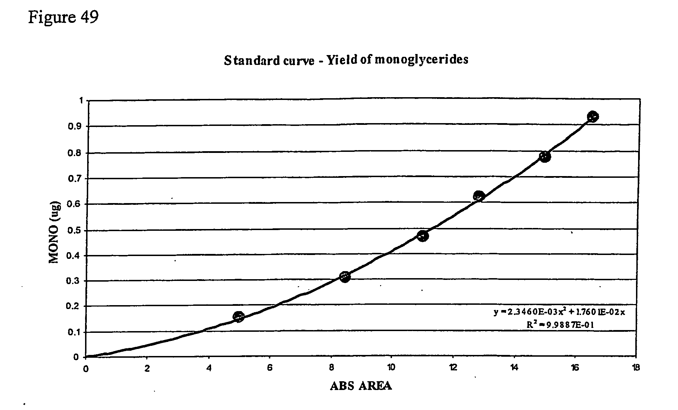

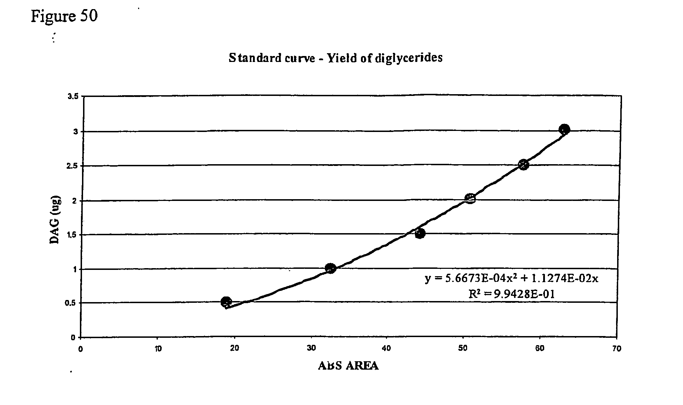

In Experiment II the components are quantified by Adobe photoshop 6,0 and the amount of MAG and DAG was calculated from calibrations curves of DAG and MAG standard solutions (see Figures 49 & 50).

Gas chromatography

Perkin Elmer 8420 Capillary Gas Chromatography equipped with WCOT fused silica column 12.5 m x 0,25 mm ID x 0,1μm 5%phenyl-methyl-silicone (CP Sil 8 CB from Crompack).

Carrier: Helium.
Injection: 1.5 μl with split.
Detector: FID. 385 °C.

| Oven program : | 1 | 2 | 3 | 4 |
|---|---|---|---|---|
| Oven temperature, °C : | 80 | 200 | 240 | 360 |
| Isothermal, time, min. : | 2 | 0 | 0 | 10 |
| Temperature rate, °C. /min. : | 20 | 10 | 12 | |

Sample preparation: 50 mg lipid was dissolved in 12ml heptane:pyridine (2:1) containing an internal standard heptadecane, 2 mg/ml. 500 μl of the sample was transferred to a crimp vial. 100 μl MSTFA (N-Methyl-N-trimethylsilyltrifluoracetamid) was added and the reaction was incubated for 15 minutes at 60 °C.

Calculation: Response factors for mono-di-triglycerides, free fatty acids were determined from reference mixtures of these components. Based on these response factors the lipids in the samples were calculated.

Results

Experiment I:

The aim of this experiment was to examine the impact of a diglyceride:glycerol acyltransferase from *Aeromonas salmonicida* on DAGs in palm oil by glycerolysis reaction. It is known that a GCAT is able to transfer fatty acid from lecithin the cholesterol during formation of cholesterolester and lysolecithin. In this study we have investigated the possibility of the enzyme to use DAGs as donor and glycerol as acceptor in order to reduce the amount of DAGs in palm oil and produce monoglyceride.

It is well known in the literature to use enzyme like lipases to catalyze glycerolysis reactions. In these reactions triglycerides is the main substrate and mono-and diglycerides are the reactions products. In these processes the amount of diglycerides increases significantly and the amount of diglycerides produced is on level or high than the amount of monoglyceride produced (Kristensen, 2004).

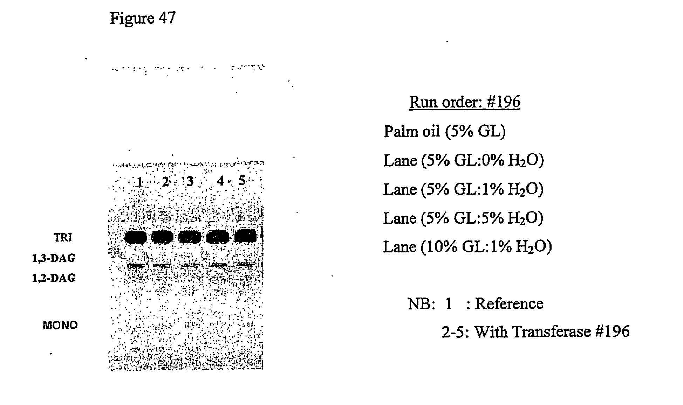

Four different sample compositions were tested and compared to a reference of palm oil mixed with 5% glycerol but without enzyme and treated in the same way as the samples. Figure 47 shows the result of the HPTLC analysis of

the sample from table 1.

The results obtained from the HPTLC analysis shows that the amount of DAGs varies according to the reaction conditions (referring to ratio between GL:$H_2O$). Equal to all the reactions was that the 1,3-isomers of diglyceride were in higher portion than the 1,2-(2,3-) isomers of diglyceride. This observation can be confirmed by theory, which says that the ratio of 1,3-isomer to 1,2- (2,3-) isomer in crude palm oil is 7:3 (Siew & Ng, 1999; Timms, 2004). Furthermore, analyzing the result, the HPTLC plate shows that the transferease successfully reduces the amount of DAGs. The reduction can to some extent correlate to the amount of synthesized MONO.

If we compare the 1,2 isomer with the 1,3 isomer DAGs in figure 1 it appears that the 1,2 DAGs are primarily reduced by the enzyme-catalyzed reaction. This is in agreement with the transferease has a preferred specificity for fatty acids in sn2-position.

In experiments conducted different water concentrations in range of 0.5-5.5% were used. It appears from the result that the water concentration has a significant impact on the synthesized amount of MONO combined to the reduced amount of DAGs. In this instance transferase #196 shows that if the system contains low water concentration increased concentration of MONO is formed and corresponding decreased amount of DAG is observed. The experiment also investigates whether the amount of glycerol has an effect on the equilibrium in the system (Lane 5: 10% GL:1% $H_2O$). Transferease #196 shows that higher concentration of glycerol - also meaning double dosage of enzyme activity - dose not result in higher MONO concentration.

Preferably the present invention is carried out in a low water environment i.e. less than about 1%.

One of the advantages working with transfereases instead of lipases is that the synthesis of MONO dose not correlates to excessive increased in the concentration of FFA. This fact is confirmed in present experiment. Figure 1 shows that none of the reactions shows clear band of FFA (FFA band was expected to be visible between 1,3 DAGs and TAGs).

From this experiment it can be summarized that the optimal concentration of glycerol and water for Transferase #196 is as following:

Transferase #196: 5%GL:0.5% $H_2O$

The sample procedure in HPTLC analysis did not include specific weighing, because of that it was not possible to calculate the concentrations of the different components in the reaction mixtures. Because of that the observation is solely based on visual evaluation.

Result GC

Based on the HPTLC results sample No. 2 was selected for GC analysis. To find out whether the visual evaluation of the HPTLC plates could be confirmed by quantitative analyses of DAG. Before analysis glycerol was removed from the sample by centrifugation and only the lipid phase was exposed to GC analysis The GC results are presented in Table 3 below.

Table 3: Result of GC on selected reaction in Experiment I

| | Ref. | #196 |
|---|---|---|
| Sample No. | 1 | 2 |
| Gl (%)* | 5 | 5 |
| Water(%) | 0 | 0 |
| Oil phase (%) | 95 | 95 |
| GC results | | |
| MONO | <0,01 | 0.34 |
| DAG | 6.21 | 5.77 |
| * Glycerol contains 10% water | | |

Analyzing the result of the GC study it was found that the selected sample differ from the reference in higher MONO content and lower DAG yield. This observation supports the obtained results in the HPTLC analysis.

Conclusion: Experiment I

Concerning content of monoglycerides and diglycerides in the product of glycerolysis, the results of HPTLC analysis indicated that there is a correlation between the concentration of water and the amounts of MONO and DAGs, respectively: The lower concentration of water, the lower DAGs and higher MONO.

GC analysis confirmed degree of reduction in DAG. In this experiment the reduced amount of DAGs counts for 7.1% of the total amount of diglycerides palm oil (Table 4).

Table 4: Degree of reduction of DAGs

| | |
|---|---|
| Enz. No. | #196 |
| Sample no. | 2 |
| Reduction of DAGs (%) | 7,1 |

Experiment II:

The aim of this part was to continue the optimization of DAG reduction in palm oil and analyze the transferase-catalysed glycerolysis using Transferease #196. The primarily purpose is to reach a well-balanced reaction in which the amount of DAG has been reduced in time with increase concentration of MONO.

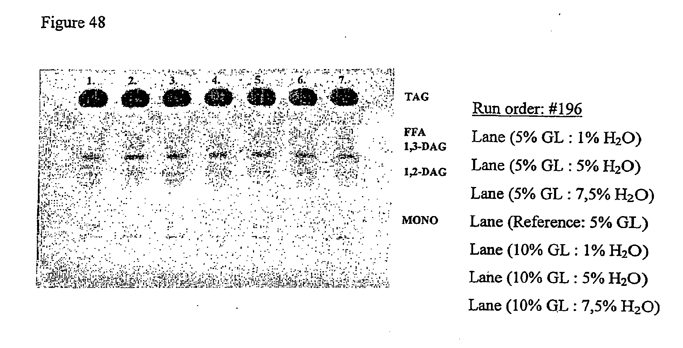

In this part of the experimental work six different sample compositions were tested and compared to a reference of palm oil mixed with 5% glycerol (Table 2). The reference was exposed to the same heating profile as the enzyme reactions, which makes it possible to observe and determine the degree of thermal degradation during reduction. Figure 48 shows the result of the HPTLC analysis.

It appears from the results in figure 48 that especially the amount of MAG is varying. The HPTLC analysis shows that in samples containing 5% glycerol (Lane: 1-3) there is continuous relation between water concentration and MAG yield: Decreased amount of water in the reaction mixture is followed by increased MAG yield. The same tendency is observed in the reactions containing 10% glycerol (Lane: 5-7).

According to the amount of diglycerides, it was not possible to differentiate the samples solely based on visual evaluation of the HPTLC plate. Therefore, to be able to distinguish between the samples, components were quantified by analyzing standard materials with known composition of MAG and DAG. The HPTLC plates were scanned and handle by Adobe Photoshop 6.0 and further calculated by help from a constructed calculation macro (Microsoft Excel 2000). The quantitative analysis of the components analyzed by HPTLC is presented in Table 5 below.

Table 5:

| Enz. No. | Transferase #196 | | | | | | |
|---|---|---|---|---|---|---|---|
| Run order: HPTLC | 1 | 2 | 3 | 4 | 5 | 6 | 7 |
| MONO (%) | 0,38 | 0.01 | Nm*. | 0.01 | 0.15 | 0.07 | 0.03 |
| DAG (%) | 7.24 | 8.13 | Nm*. | 8.35 | 8.26 | 7.88 | 7.62 |
| Reduction of DAG (%) | 13,29 | 2,63 | - | - | 1,08 | 5,63 | 8,74 |
| Nm* Not measured<br>- not calculated. | | | | | | | |

The investigation supports the visual evaluation and gives a fine picture of the varying level of diglycerides. It is seen from the results that the highest degree of reduction in the amount of DAGs was achieved in sample no. 1 (5% GL:1.5% $H_2O$). Further it is observed that this sample also contains the highest amount of MAG.

Conclusion: Experiment II

Experiment II confirmed the observations that a lipid acyltransferase GCAT from *Aeromonas salmonicida* was able to reduce the amount of diglycerides in palm oil. For the experiments with 5% glycerol a correlation was found between the concentration of water in the reaction mixture and the amounts of MAG and DAGs, respectively: The lower concentration of water, the lower DAGs and higher MAG.

HPTLC quantified the degree of reduction (see Table 5). It appears from the results, that the reduced amount of DAGs counts for 13,29% of the total amount of diglycerides in palm oil and the amount of synthesized MONO raised

from 0,01% (ref.) to 0.38% (sample No. 1).

From this experiment it can be summarized that the optimal concentration of glycerol and water for Transferase #196 is as following:

Transferase #196: 5%GL:1.5% $H_2O$

Comparing these observations with the result gained in Experiment I, it is confirmed that good consistency in the results is obtained. The optimal concentration of water is presumably between 0-1% $H_2O$ depending on enzyme activity and content of glycerol.

Based on the two experiments conducted, it can be concluded that it is possible to reduce the amount of DAGs in palm oil by a transferase-catalysed reaction, where the enzyme uses DAGs as donor molecules and glycerol as acceptor molecule, in a glycerolysis reaction in which monoglycerides is synthesized. This is in strong contrast to the glycerolysis reaction with conventional triglycerides hydrolyzing lipases where the amount of DAG increases due to the partial hydrolysis activity of the triglycerides hydrolyzing lipase.

Based on the fact that the structure of diglycerides is a mixture 1,2- and 1,3-isomers and that the transferase is specific to the sn2-position, even a small reduction in the amount of diglyceride will have a huge physical impact on palm-based products.

**EXAMPLE 7: <u>Immobilisation of a diacylglycerol:glycerol transferase (an lipid acyltransferase) from *Aeromonas salmonicida.*</u>**

The diacylglycerol:glycerol transferase is immobilised on Celite by acetone precipitation. 10 ml enzyme solution in 20 mM TEA buffer pH 7 is agitated slowly with 0,1 gram Celite 535 (from Fluka) for 2 hours at room temperature.

50ml cool acetone is added during continued agitation.

The precipitate is isolated by centrifugation 5000 g for 1 minute.

The precipitate is washed 2 times with 20ml cold acetone.

The Celite is tried at ambient temperature for about 1 hour

The immobilised transferase is tested in palm oil (see table below):

**Table**

| | % |
|---|---|
| Palm oil | 92.5 |
| Glycerol | 5 |
| Immobilised diacylglycerol:glycerol On Celite, #178,45 U/g | 2.0 |
| Water | 0.5 |

Palm oil and glycerol is heated to 42°C. The immobilised transferase was added.

The transferase reaction continued at 42°C during gentle agitation with a magnetic stirrer. Samples are taken out for analyses after ½, 1, 3, 6 and 24 hours and analysed by HPTLC. The reaction is stopped after 24 hours reaction time and the immobilised enzyme was filtered off.

The HPTLC analysis clearly shows the effect of the immobilised diacylglycerol:glycerol from *A. salmonicida* by the formation of monoglyceride and reduction of diglyceride in palm oil.

REFERENCES

Amano Enzyme Inc. (2004). http://www.amano-enzyme.co.jp/english/productuse/oil_fat.html. Dato: 21.06.04

Fødevareministeriet (2003). Bekendtgørelse om indhold af transfedtsyrer i olier og fedtstoffer. Bekendtgørelse nr. 160 af 11/03/2003

Directive 2000/36/EC. http://europa.eu.int/scadplus/leg/en/lvb/121122b.htm. Dato: 16.06.04

Karlshamns, (2004). Press information. http://www.karlshamns.com/investor/press information-show.asp?ID=319. Dato: 16.06.04

Berger, K. G. (1990). Recent developments in palm oil. In Oleagineux 45:437-443

Drozdowski, B. (1994). General characteristics of eatable fats. WNT, Warszawa, Poland, pp. 240-243 (In polish)

Hernquist, L. & Anjou, K. (1983). Diglycerides as a stabilizer of the β'-crystal Form in Margarines and Fats. In Fette Seifen Anstrichmittel. 2:64-66

Hernquist, L. Herslof, B. Larsson, K. & Podlaha, O (1981). Polymorphism of rapeseed oil with low content of erucic acid and possibilities to stabilize the β' crystal form in fats. In Journal of Science and Food Agriculture. 32:1197-1202

Jacobsberg, B. & Oh, C.H. (1976). Studies in Palm Oil Crystallisation. In Journal of the American Oil Chemist Society. 53:609-616

Kristensen, A. C. J. (2004). Preparation of margarine and spreads by enzyme-generated emulsifiers. Master thesis, The Royal Veterinary and Agricultural University, Frederiksberg, Copenhagen

McNeill, G. P. & Berger, R. G. (1993). Enzymatic glycerolysis of palm oil fractions and palm oil based model mixture: Relationship between fatty acid composition and monoglyceride yield. In Food Biotechnology 7:75-87

Okiy, D. A. (1977). Partial glycerides and palm oil Crystallisation. In Journal of Science and Food Agriculture. 28:955

Okiy, D. A. (1978). Interaction of triglycerides and diglycerides of palm oil. In Oleagineux. 33:625-628

Okiy, D. A., Wright, W. B., Berger, K. G. & Morton, I. D. (1978). The physical properties of modified palm oil. In Journal of Science of Food and Agriculture. 29:625-628

Walnett, S. V., Meusel, D. & Tülsner, M. (1991). Zur kenntnis des diglyceride influsses auf das kristallisationsverhalten von Fetten. In Fat Science Technology. 4:117-121

Siew, N. L. (2001). Understanding the Interactions of Diacylglycerols with oil for better Product performance. Paper presented at the 2001 PIPOC International Palm Oil Congress - Chemistry and Technology Conference. 20-23 August 2001, Kuala Lumpur, Malaysia

Siew, N. L. & Ng, W. L. (2000). Differential scanning thermograms of palm oil triglycerides in the presence of diglycerides. In Journal of Oil Palm Research. 12:1-7 Siew, N. L. & Ng, W. L. (1999). Influence of diglycerides on crystalisation of palm oil. In Journal of Science of Food and Agriculture. 79:722-726

Sonntag, N. O. V. (1982a). Fat splitting, esterification and interesterification. Bailey's Industrial Oils and Fat products, Vol. 2, 4th edn. John Wiley and Sons, New york pp. 97-173

Sonntag, N. O. V. (1982b). Glycerolysis of Fats and methyl esters - status, review and critique. In Journal of American Oil Chemist Society . 59: 795A-802A

Timms, R. (2004). Oral presentation (Lecture: Trends & delvlopment) at Danisco A/S, Brabrand, Denmark.

**BUDAPEST TREATY ON THE INTERNATIONAL RECOGNITION OF THE DEPOSIT OF MICROORGANISMS FOR THE PURPOSES OF PATENT PROCEDURE**

Danisco A/S
Langebrogade 1
DK-1001 Copenhagen
Denmark

NAME AND ADDRESS OF DEPOSITOR

**INTERNATIONAL FORM**

**RECEIPT IN THE CASE OF AN ORIGINAL DEPOSIT issued pursuant to Rule 7.1 by the INTERNATIONAL DEPOSITARY AUTHORITY identified at the bottom of this page**

I. IDENTIFICATION OF THE MICROORGANISM

| Identification reference given by the DEPOSITOR: | Accession number given by the INTERNATIONAL DEPOSITARY AUTHORITY: |
|---|---|
| *Escherichia coli* TOP10pPet12aAhydro | NCIMB 41204 |

II. SCIENTIFIC DESCRIPTION AND/OR PROPOSED TAXONOMIC DESIGNATION

The microorganism identified under I above was accompanied by:

[ ] a scientific description

[X] a proposed taxonomic designation

(Mark with a cross where applicable)

III. RECEIPT AND ACCEPTANCE

This International Depositary Authority accepts the microorganism identified under I above, which was received by it on 22 December 2003 (date of the original deposit)[1]

IV. RECEIPT OF REQUEST FOR CONVERSION

The microorganism identified under I above was received by this International Depositary Authority on (date of the original deposit) and a request to convert the original deposit to a deposit under the Budapest Treaty was received by it on (date of receipt of request for conversion)

V. INTERNATIONAL DEPOSITARY AUTHORITY

| | |
|---|---|
| Name: NCIMB Ltd., | Signature(s) of person(s) having the power to represent the International Depositary Authority or of authorised official(s): |
| Address: 23 St Machar Drive<br>Aberdeen<br>AB24 3RY<br>Scotland, UK. | Date: 9 January 2004 |

[1] Where Rule 6/4(d) applies, such date is the date on which the status of International Depositary Authority was acquired.

Form BP/4 (sole page)

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

Danisco A/S
Langebrogade 1
DK-1001 Copenhagen
Denmark

VIABILITY STATEMENT
issued pursuant to Rule 10.2 by the INTERNATIONAL DEPOSITARY AUTHORITY identified on the following page

NAME AND ADDRESS OF THE PARTY TO WHOM THE VIABILITY STATEMENT IS ISSUED

| I. DEPOSITOR | II. IDENTIFICATION OF THE MICROORGANISM |
|---|---|
| Name: AS ABOVE<br>Address: | Accession number given by the INTERNATIONAL DEPOSITARY AUTHORITY: NCIMB 41204<br>Date of the deposit or of the transfer[1]:<br>22 December 2003 |

III. VIABILITY STATEMENT

The viability of the microorganism identified under II above was tested on 22 December 2003 [2]. On that date, the said microorganism was:

[X] viable [3]

[ ] no longer viable [3]

1 Indicate the date of the original deposit or, where a new deposit or a transfer has been made, the most recent relevant date (date of the new deposit or date of the transfer).

2 In the cases referred to in Rule 10.2(a)(ii) and (iii), refer to the most recent viability test.

3 Mark with a cross the applicable box.

Form BP/9 (first page)

| IV. CONDITIONS UNDER WHICH THE VIABILITY TEST HAS BEEN PERFORMED[4] | |
|---|---|
| | |

| V. INTERNATIONAL DEPOSITARY AUTHORITY | |
|---|---|
| Name: NCIMB Ltd.,<br>Address: 23 St Machar Drive<br>Aberdeen<br>AB24 3RY<br>Scotland | Signature(s) of person(s) having the power to represent the International Depositary Authority or of authorised official(s):<br>Date: 9 January 2004 |

4 Fill in if the information has been requested and if the results of the test were negative.

**BUDAPEST TREATY ON THE INTERNATIONAL RECOGNITION OF THE DEPOSIT OF MICROORGANISMS FOR THE PURPOSES OF PATENT PROCEDURE**

Danisco A/S
Langebrogade 1
DK-1001 Copenhagen
Denmark

NAME AND ADDRESS OF DEPOSITOR

**INTERNATIONAL FORM**

**RECEIPT IN THE CASE OF AN ORIGINAL DEPOSIT**
**issued pursuant to Rule 7.1 by the**
**INTERNATIONAL DEPOSITARY AUTHORITY**
**identified at the bottom of this page**

I. IDENTIFICATION OF THE MICROORGANISM

| Identification reference given by the DEPOSITOR: | Accession number given by the INTERNATIONAL DEPOSITARY AUTHORITY: |
|---|---|
| *Escherichia coli* TOP10pPet12aAsalmo | NCIMB 41205 |

II. SCIENTIFIC DESCRIPTION AND/OR PROPOSED TAXONOMIC DESIGNATION

The microorganism identified under I above was accompanied by:

[ ] a scientific description

[X] a proposed taxonomic designation

(Mark with a cross where applicable)

III. RECEIPT AND ACCEPTANCE

This International Depositary Authority accepts the microorganism identified under I above, which was received by it on 22 December 2003 (date of the original deposit)[1]

IV. RECEIPT OF REQUEST FOR CONVERSION

The microorganism identified under I above was received by this International Depositary Authority on (date of the original deposit) and a request to convert the original deposit to a deposit under the Budapest Treaty was received by it on (date of receipt of request for conversion)

V. INTERNATIONAL DEPOSITARY AUTHORITY

Name: NCIMB Ltd.,

Address: 23 St Machar Drive
Aberdeen
AB24 3RY
Scotland, UK.

Signature(s) of person(s) having the power to represent the International Depositary Authority or of authorised official(s):

Terence Dando

Date: 9 January 2004

[1] Where Rule 6/4(d) applies, such date is the date on which the status of International Depositary Authority was acquired.

Form BP/4 (sole page)

BUDAPEST TREATY ON THE INTERNATIONAL RECOGNITION OF THE DEPOSIT OF MICROORGANISMS FOR THE PURPOSES OF PATENT PROCEDURE

| | |
|---|---|
| Danisco A/S<br>Langebrogade 1<br>DK-1001 Copenhagen<br>Denmark | INTERNATIONAL FORM<br><br>**VIABILITY STATEMENT**<br>**issued pursuant to Rule 10.2 by the INTERNATIONAL DEPOSITARY AUTHORITY identified on the following page** |
| NAME AND ADDRESS OF THE PARTY TO WHOM THE VIABILITY STATEMENT IS ISSUED | |

| I. DEPOSITOR | II. IDENTIFICATION OF THE MICROORGANISM |
|---|---|
| Name: AS ABOVE<br>Address: | Accession number given by the INTERNATIONAL DEPOSITARY AUTHORITY:<br>NCIMB 41205<br>Date of the deposit or of the transfer[1]:<br>22 December 2003 |

| III. VIABILITY STATEMENT |
|---|
| The viability of the microorganism identified under II above was tested on 22 December 2003 [2]. On that date, the said microorganism was: |
| [X] viable [3] |
| [ ] no longer viable [3] |

[1] Indicate the date of the original deposit or, where a new deposit or a transfer has been made, the most recent relevant date (date of the new deposit or date of the transfer).

[2] In the cases referred to in Rule 10.2(a)(ii) and (iii), refer to the most recent viability test.

[3] Mark with a cross the applicable box.

Form BP/9 (first page)

| IV. CONDITIONS UNDER WHICH THE VIABILITY TEST HAS BEEN PERFORMED[4] | |
|---|---|
| | |

| V. INTERNATIONAL DEPOSITARY AUTHORITY | |
|---|---|
| Name: NCIMB Ltd.,<br>Address: 23 St Machar Drive<br>Aberdeen<br>AB24 3RY<br>Scotland | Signature(s) of person(s) having the power to represent the International Depositary Authority or of authorised official(s):<br>Date: 9 January 2004 |

[4] Fill in if the information has been requested and if the results of the test were negative.

Form BP/9 (second and last page)

## Claims

1. A method of reducing and/or removing diglyceride from an edible oil, comprising a) admixing an edible oil with an acyl acceptor substrate and a fatty-acid CoA independent diglyceride:glycerol acyltransferase, wherein the fatty-acid CoA independent diglyceride:glycezol acyltransferase is characterized as an enzyme which in an edible oil transfers an acyl group from a diglyceride to glycerol, and wherein the fatty-acid CoA independent diglyceride: glycerol acyltransferase comprises the amino acid sequence motif GDSX, wherein X is one or more of the following amino acid residues L, A, V, I, F, Y, H, Q, T, N, M or S.

2. A method according to claim 1, wherein the diglyceride is a 1,2-diglyceride.

3. A method according to claim 1 or claim 2 wherein the fatty-acid CoA independent diglyceride:glycezol acyltransferase enzyme comprises H-309 or comprises a histidine residue at a position corresponding to His-309 in the amino acid sequence of the *Aeromonas hydrophila* lipolytic enzyme shown as SEQ ID No. 2 or SEQ ID No. 32.

4. A method according to any one of the preceding claims wherein the fatty-acid CoA independent diglyceride:glycerol acyltransferase is obtainable from an organism from one or more of the following genera: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas and Candida.*

5. A method according to any one of the preceding claims wherein the fatty-acid CoA independent diglyceride:glycerol acyltransferase comprises one or more of the following amino acid sequences: (i) the amino acid sequence shown as SEQ ID No. 2; (ii) the amino acid sequence shown as SEQ ID No. 3; (iii) the amino acid sequence shown as SEQ ID No. 4; (iv) the amino acid sequence shown as SED ID No. 5; (v) the amino acid sequence shown as SEQ ID No. 6; (vi) the amino acid sequence shown as SEQ ID No. 12, (vii) the amino acid sequence shown as SEQ ID No. 20, (viii) the amino acid sequence shown as SEQ ID No. 22, (ix) the amino acid sequence shown as SEQ ID No. 24, (x) the amino acid sequence shown as SEQ ID No. 26, (xi) the amino acid sequence shown as SEQ ID No. 28, (xii) the amino acid sequence shown as SEQ ID No. 30, (xiii) the amino acid sequence shown as SEQ ID No. 32, (xiv) the amino acid sequence shown as SEQ ID No. 34, (xv) the amino acid sequence shown as SEQ ID no. 55; (xvi) the amino acid sequence shown as SEQ ID No. 58; (xvii) the amino acid sequence shown as SEQ ID No 60; *(xviii)* the amino acid sequence shown as SEQ ID No. 61; (xix) the amino acid sequence shown as SEQ ID No. 63; (xx) the amino acid sequence shown as SEQ ID No. 65, (xxi) the amino acid sequence shown as SEQ ID No. 67; (xxii) the amino acid sequence shown as SEQ ID No. 70 or (xxiii) an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32. SEQ ID No. 34, SEQ ID No. 55, SEQ ID No. 58, SEQ ID No. 60, SEQ ID No. 61, SEQ ID No. 63, SEQ ID No. 65, SEQ ID No. 67 or SEQ ID No. 70.

6. A method according to any one of the preceding claims wherein the fatty-acid CoA independent diglyceride:glycerol acyltransferase comprises an amino acid sequence encoded by one or more of the following nucleotide sequences:

(a) the nucleotide sequence shown as SEQ ID No. 7;
(b) the nucleotide sequence shown as SEQ ID No. 8;
(c) the nucleotide sequence shown as SEQ ID No. 9;
(d) the nucleotide sequence shown as SEQ ID No. 10;
(e) the nucleotide sequence shown as SEQ ID No. 11;
(f) the nucleotide sequence shown as SEQ ID No. 13;
(g) the nucleotide sequence shown as SEQ ID No. 21;
(h) the nucleotide sequence shown as SEQ ID No. 23;
(i) the nucleotide sequence shown as SEQ ID No. 25;
(j) the nucleotide sequence shown as SEQ ID No. 27;
(k) the nucleotide sequence shown as SEQ ID No. 29;
(l) the nucleotide sequence shown as SEQ ID No. 31;
(m)the nucleotide sequence shown as SEQ ID No. 33;
(n) the nucleotide sequence shown as SEQ ID No. 35;
(o) the nucleotide sequence shown as SEQ ID No. 54;
(p) the nucleotide sequence shown as SEQ ID No. 59;
(q) the nucleotide sequence shown as SEQ ID No. 62;
(r) the nucleotide sequence shown as SEQ ID No. 64;
(s) the nucleotide sequence shown as SEQ ID No. 66;
(t) the nucleotide sequence shown as SEQ ID No. 68
(u) the nucleotide sequence shown as SEQ ID No. 69 or
(v) a nucleotide sequence which has 75% or more identity with any one of the sequences shown as SEQ DJ No. 7, SEQ ID No. 8, SEQ ID No. 9, SEQ ID No. 10, SEQ ID No, 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33, SEQ ID No. 35, SEQ ID No- 54, SEQ ID No. 59, SEQ ID No. 62, SEQ ID No. 64, SEQ ID No. 66, SEQ ID No. 68 or SEQ ID No. 69.

7. A method according to any one of the preceding claims comprising admixing the treated edible oil with one or more

food constituents to formulate a foodstuff.

**8.** Use of a fatty-acid CoA independent diglyceride:glycerol acyltransferase characterized as an enzyme which in an edible oil transfers an acyl group from a diglyceride to glycerol, in the manufacture of an edible oil, for reducing and/or removing (preferably selectively reducing and/or removing) diglyceride from said edible oil wherein the fatty-acid CoA independent diglyceride:glycerol acyltransferase comprises the amino acid sequence motif GDSX, wherein X is one or more of the following amino acid residues L, A, V, I, F, Y, H, Q, T, N, M or S.

**9.** Use of a fatty-acid CoA independent diglycearide:glycerol acyltransferase characterized as an enzyme which in an edible oil transfers an acyl group from a diglyceride to glycerol, in the manufacture of a foodstuff comprising an edible oil for improving the crystallization properties of said foodstuff, wherein the fatty-acid CoA independent diglyceride:glycerol acyltransferase comprises the amino acid sequence motif GDSX, wherein X is one or more of the following amino acid residues L; A, V, I, F, Y, H, Q, T, N, M or S.

**10.** Use according to claim 8 or claim 9 wherein the amount of diglyceride in the edible oil is reduced.

**11.** Use according any one of claims 8 to 10, wherein the diglyceride is a 1,2-diglyceride.

**12.** Use according to any one of claims 8-11 wherein the fatty-acid CoA independent diglyceride:glycerol acyltransferase enzyme comprises H-309 or comprises a histidine residue at a position corresponding to His-309 in the amino acid sequence of the *Aeromonas hydrophila* lipolytic enzyme shown as SEQ ID No. 2 or SEQ ID No. 32.

**13.** Use according to any one of claims 8-12 wherein the fatty-acid CoA independent diglyceride:glycerol acyltransferase is obtainable from an organism from one or more of the following genera: *Aeromonas, Streptomyces, Saccharomyces, Lactococcus, Mycobacterium, Streptococcus, Lactobacillus, Desulfitobacterium*, *Bacillus, Campylobacter, Vibrionaceae, Xylella, Sulfolobus, Aspergillus, Schizosaccharomyces, Listeria, Neisseria, Mesorhizobium, Ralstonia, Xanthomonas and Candida.*

**14.** Use according to any one of claims 8-13 wherein the fatty-acid CoA independent diglyceride:glycerol acyltransferase comprises one or more of the following amino acid sequences: (i) the amino acid sequence shown as SEQ ID No. 2; (ii) the amino acid sequence shown as SEQ ID No. 3; (iii) the amino acid sequence shown as SEQ ID No. 4; (iv) the amino acid sequence shown as SED ID No. 5; (v) the amino acid sequence shown as SEQ ID No. 6; (vi) the amino acid sequence shown as SEQ ID No. 12, (vii) the amino acid sequence shown as SEQ ID No. 20, (viii) the amino acid sequence shown as SEQ ID No. 22, (ix) the amino acid sequence shown as SEQ ID No. 24, (x) the amino acid sequence shown as SEQ ID No. 26, (xi) the amino acid sequence shown as SEQ ID No. 28, (xii) the amino acid sequence shown as SEQ ID No. 30, (xiii) the amino acid sequence shown as SEQ ID No. 32, (xiv) the amino acid sequence shown as SEQ ID No. 34, (xv) the amino acid sequence shown as SEQ ID No. 55; (xvi) the amino acid sequence shown as SEQ ID No. 58; *(xvii)* the amino acid sequence shown as SEQ ID No. 60; (xviii) the amino acid sequence shown as SEQ ID No. 61; (xix) the amino acid sequence shown as SEQ ID No. 63; (xx) the amino acid sequence shown as SEQ ID No. 65; (xxi) the amino acid sequence shown as SEQ ID No. 67; (xxii) the amino acid sequence shown as SEQ ID No. 70 or (xxiii) an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 20, SEQ ID No. 22, SEQ ID No. 24, SEQ ID No. 26, SEQ ID No. 28, SEQ ID No. 30, SEQ ID No. 32, SEQ ID No. 34, SEQ ID No. 55, SEQ ID No. 58, SEQ ID No. 60, SEQ ID No. 61, SEQ ID No. 63, SEQ ID No. 65, SEQ ID No. 67 or SEQ ID No. 70.

**15.** Use according to any one of claims 8-14 wherein the fatty-acid CoA independent diglyceride:glycerol acyltransferase comprises an amino acid sequence encoded by one or more of the following nucleotide sequences:

(a) the nucleotide sequence shown as SEQ ID No. 7;
(b) the nucleotide sequence shown as SEQ ID No. 8;
(c) the nucleotide sequence shown as SEQ ID No. 9;
(d) the nucleotide sequence shown as SEQ ID No. 10;
(e) the nucleotide sequence shown as SEQ ID No. 11;
(f) the nucleotide sequence shown as SEQ ID No, 13;
(g) the nucleotide sequence shown as SEQ ID No. 21;
(h) the nucleotide sequence shown as SEQ ID No. 23;
(i) the nucleotide sequence shown as SEQ ID No. 25;

(j) the nucleotide sequence shown as SEQ ID No. 27;
(k) the nucleotide sequence shown as SEQ ID No. 29;
(l) the nucleotide sequence shown as SEQ ID No. 31;
(m)the nucleotide sequence shown as SEQ ID No. 33;
(n) the nucleotide sequence shown as SEQ ID No. 35;
(o) the nucleotide sequence shown as SEQ ID No. 54;
(p) the nucleotide sequence shown as SEQ ID No. 59;
(q) the nucleotide sequence shown as SEQ ID No. 62;
(r) the nucleotide sequence shown as SEQ ID No. 64;
(s) the nucleotide sequence shown as SEQ ID No. 66;
(t) the nucleotide sequence shown as SEQ ID No. 68
(u) the nucleotide sequence shown as SEQ ID No. 69 or
(v) a nucleotide sequence which has 75% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 21, SEQ ID No. 23, SEQ ID No. 25, SEQ ID No. 27, SEQ ID No. 29, SEQ ID No. 31, SEQ ID No. 33, SEQ ID No. 35, SEQ ID No. 54, SEQ ID No. 59, SEQ ID No. 62, SEQ ID No. 64, SEQ ID No. 66, SEQ ID No. 68 or SEQ ID No. 69.

**16.** Use according to any one of claims 8-15 wherein the fatty-acid CoA independent diglyceride:glycerol acyltransferase is used in combination with a crystallization inhibitor.

**Patentansprüche**

**1.** Verfahren zum Reduzieren und/oder Entfernen von Diglycerid aus einem Speiseöl, welches folgendes umfaßt: a) Mischen eines Speiseöls mit einem Acylakzeptorsubstrat und einer Fettsäure-CoA-unabhängigen Diglycerid:Glycerol-Acyltransferase, wobei die Fettsäure-CoA-unabhängige Diglycerid:Glycerol-Acyltransferase als ein Enzym gekennzeichnet ist, welches in einem Speiseöl eine Acylgruppe von einem Diglycerid zu Glycerol überträgt, und wobei die Fettsäure-CoA-unabhängige Diglycerid:Glycerol-Acyltransferase das Aminosäuresequenzmotiv GDSX umfaßt, wobei X einer oder mehrere der Aminosäurereste L, A, V, I, F, Y, H, Q, T, N, M oder S ist.

**2.** Verfahren nach Anspruch 1, wobei das Diglycerid ein 1,2-Diglycerid ist.

**3.** Verfahren nach Anspruch 1 oder Anspruch 2, wobei das Fettsäure-CoA-unabhängige Diglycerid:Glycerol-Acyltransferaseenzym H-309 umfaßt oder einen Histidinrest an einer Position umfaßt, die His-309 in der als SEQ ID NO: 2 oder SEQ ID NO: 32 gezeigten Aminosäuresequenz des lipolytischen Enzyms von *Aeromonas hydrophila* entspricht.

**4.** Verfahren nach einem der vorangegangenen Ansprüche, wobei die Fettsäure-CoA-unabhängige Diglycerid:Glycerol-Acyltransferase aus einem Organismus einer oder mehrerer der folgenden Gattungen erhalten werden kann: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* und *Candida.*

**5.** Verfahren nach einem der vorangegangenen Ansprüche, wobei die Fettsäure-CoA-unabhängige Diglycerid:Glycerol-Acyltransferase eine oder mehrere der folgenden Aminosäuresequenzen umfaßt: (i) die Aminosäuresequenz, die als SEQ ID NO: 2 gezeigt ist, (ii) die Aminosäuresequenz, die als SEQ ID NO: 3 gezeigt ist, (iii) die Aminosäuresequenz, die als SEQ ID NO: 4 gezeigt ist, (iv) die Aminosäuresequenz, die als SEQ ID NO: 5 gezeigt ist, (v) die Aminosäuresequenz, die als SEQ ID NO: 6 gezeigt ist, (vi) die Aminosäuresequenz, die als SEQ ID NO: 12 gezeigt ist, (vii) die Aminosäuresequenz, die als SEQ ID NO: 20 gezeigt ist, (viii) die Aminosäuresequenz, die als SEQ ID NO: 22 gezeigt ist, (ix) die Aminosäuresequenz, die als SEQ ID NO: 24 gezeigt ist, (x) die Aminosäuresequenz, die als SEQ ID NO: 26 gezeigt ist, (xi) die Aminosäuresequenz, die als SEQ ID NO: 28 gezeigt ist, (xii) die Aminosäuresequenz, die als SEQ ID NO: 30 gezeigt ist, (xiii) die Aminosäuresequenz, die als SEQ ID NO: 32 gezeigt ist, (xiv) die Aminosäuresequenz, die als SEQ ID NO: 34 gezeigt ist, (xv) die Aminosäuresequenz, die als SEQ ID NO: 55 gezeigt ist, (xvi) die Aminosäuresequenz, die als SEQ ID NO: 58 gezeigt ist, (xvii) die Aminosäuresequenz, die als SEQ ID NO: 60 gezeigt ist, (xviii) die Aminosäuresequenz, die als SEQ ID NO: 61 gezeigt ist, (xix) die Aminosäuresequenz, die als SEQ ID NO: 63 gezeigt ist, (xx) die Aminosäuresequenz, die als SEQ ID NO: 65 gezeigt ist, (xxi) die Aminosäuresequenz, die als SEQ ID NO: 67 gezeigt ist, (xxii) die Aminosäuresequenz, die als SEQ ID NO: 70 gezeigt ist, oder (xxiii) eine Aminosäuresequenz, die eine 75%-ige oder größere Identität zu irgendeiner der Sequenzen hat, die gezeigt sind als SEQ ID NO: 2, SEQ ID NO: 3, SEQ ID NO: 4, SEQ ID NO: 5, SEQ ID NO: 6, SEQ

ID NO: 12, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 55, SEQ ID NO: 58, SEQ ID NO: 60, SEQ ID NO: 61, SEQ ID NO: 63, SEQ ID NO: 65, SEQ ID NO: 67 oder SEQ ID NO: 70.

**6.** Verfahren nach einem der vorangegangenen Ansprüche, wobei die Fettsäure-CoA-unabhängige Diglycerid:Glycerol-Acyltransferase eine Aminosäuresequenz umfaßt, die durch eine oder mehrere der folgenden Nukleotidsequenzen codiert wird:

(a) die als SEQ ID NO: 7 gezeigte Nukleotidsequenz,
(b) die als SEQ ID NO: 8 gezeigte Nukleotidsequenz,
(c) die als SEQ ID NO: 9 gezeigte Nukleotidsequenz,
(d) die als SEQ ID NO: 10 gezeigte Nukleotidsequenz,
(e) die als SEQ ID NO: 11 gezeigte Nukleotidsequenz,
(f) die als SEQ ID NO: 13 gezeigte Nukleotidsequenz,
(g) die als SEQ ID NO: 21 gezeigte Nukleotidsequenz,
(h) die als SEQ ID NO: 23 gezeigte Nukleotidsequenz,
(i) die als SEQ ID NO: 25 gezeigte Nukleotidsequenz,
(j) die als SEQ ID NO: 27 gezeigte Nukleotidsequenz,
(k) die als SEQ ID NO: 29 gezeigte Nukleotidsequenz,
(l) die als SEQ ID NO: 31 gezeigte Nukleotidsequenz,
(m) die als SEQ ID NO: 33 gezeigte Nukleotidsequenz,
(n) die als SEQ ID NO: 35 gezeigte Nukleotidsequenz,
(o) die als SEQ ID NO: 54 gezeigte Nukleotidsequenz,
(p) die als SEQ ID NO: 59 gezeigte Nukleotidsequenz,
(q) die als SEQ ID NO: 62 gezeigte Nukleotidsequenz,
(r) die als SEQ ID NO: 64 gezeigte Nukleotidsequenz,
(s) die als SEQ ID NO: 66 gezeigte Nukleotidsequenz,
(t) die als SEQ ID NO: 68 gezeigte Nukleotidsequenz,
(u) die als SEQ ID NO: 69 gezeigte Nukleotidsequenz oder
(v) eine Nukleotidsequenz, die eine 75%-ige oder größere Identität zu einer der Sequenzen hat, die gezeigt sind als SEQ ID NO: 7, SEQ ID NO: 8, SEQ ID NO: 9, SEQ ID NO: 10, SEQ ID NO: 11, SEQ ID NO: 13, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 54, SEQ ID NO: 59, SEQ ID NO: 62, SEQ ID NO: 64, SEQ ID NO: 66, SEQ ID NO: 68 oder SEQ ID NO: 69.

**7.** Verfahren nach einem der vorangegangenen Ansprüche, welches das Mischen des behandelten Speiseöls mit einem oder mehreren Nahrungsmittelbestandteilen zum Formulieren eines Nahrungsmittels umfaßt.

**8.** Verwendung einer Fettsäure-CoA-unabhängigen Diglycerid:Glycerol-Acyltransferase, die als ein Enzym gekennzeichnet ist, welches in einem Speiseöl eine Acylgruppe von einem Diglycerid zu Glycerol überträgt, bei der Herstellung eines Speiseöls zum Reduzieren und/oder Entfernen (vorzugsweise zum wahlweisen Reduzieren und/oder Entfernen) von Diglycerid aus dem Speiseöl, wobei die Fettsäure-CoA-unabhängige Diglycerid:Glycerol-Acyltransferase das Aminosäuresequenzmotiv GDSX umfaßt, wobei X einer oder mehrere der Aminosäurereste L, A, V, I, F, Y, H, Q, T, N, M oder S ist.

**9.** Verwendung einer Fettsäure-CoA-unabhängigen Diglycerid:Glycerol-Acyltransferase, die als ein Enzym gekennzeichnet ist, welches in einem Speiseöl eine Acylgruppe von einem Diglycerid zu Glycerol überträgt, bei der Herstellung eines Nahrungsmittels, welches ein Speiseöl umfaßt, um die Kristallisationseigenschaften des Nahrungsmittels zu verbessern, wobei die Fettsäure-CoA-unabhängige Diglycerid:Glycerol-Acyltransferase das Aminosäuresequenzmotiv GDSX umfaßt, wobei X einer oder mehrere der Aminosäurereste L, A, V, I, F, Y, H, Q, T, N, M oder S ist.

**10.** Verwendung nach Anspruch 8 oder Anspruch 9, wobei die Menge an Diglycerid in dem Speiseöl reduziert ist.

**11.** Verwendung nach einem der Ansprüche 8 bis 10, wobei das Diglycerid ein 1,2-Diglycerid ist.

**12.** Verwendung nach einem der Ansprüche 8 bis 11, wobei das Fettsäure-CoA-unabhängige Diglycerid:Glycerol-Acyltransferaseenzym H-309 umfaßt oder einen Histidinrest an einer Position umfaßt, die His-309 in der als SEQ

ID NO: 2 oder SEQ ID NO: 32 gezeigten Aminosäuresequenz des lipolytischen Enzyms von *Aeromonas hydrophila* entspricht.

**13.** Verwendung nach einem der Ansprüche 8 bis 12, wobei die Fettsäure-CoA-unabhängige Diglycerid:Glycerol-Acyltransferase aus einem Organismus einer oder mehrerer der folgenden Gattungen erhalten werden kann: *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* und *Candida.*

**14.** Verwendung nach einem der Ansprüche 8 bis 13, wobei die Fettsäure-CoA-unabhängige Diglycerid:Glycerol-Acyltransferase eine oder mehrere der folgenden Aminosäuresequenzen umfaßt: (i) die Aminosäuresequenz, die als SEQ ID NO: 2 gezeigt ist, (ii) die Aminosäuresequenz, die als SEQ ID NO: 3 gezeigt ist, (iii) die Aminosäuresequenz, die als SEQ ID NO: 4 gezeigt ist, (iv) die Aminosäuresequenz, die als SEQ ID NO: 5 gezeigt ist, (v) die Aminosäuresequenz, die als SEQ ID NO: 6 gezeigt ist, (vi) die Aminosäuresequenz, die als SEQ ID NO: 12 gezeigt ist, (vii) die Aminosäuresequenz, die als SEQ ID NO: 20 gezeigt ist, (viii) die Aminosäuresequenz, die als SEQ ID NO: 22 gezeigt ist, (ix) die Aminosäuresequenz, die als SEQ ID NO: 24 gezeigt ist, (x) die Aminosäuresequenz, die als SEQ ID NO: 26 gezeigt ist, (xi) die Aminosäuresequenz, die als SEQ ID NO: 28 gezeigt ist, (xii) die Aminosäuresequenz, die als SEQ ID NO: 30 gezeigt ist, (xiii) die Aminosäuresequenz, die als SEQ ID NO: 32 gezeigt ist, (xiv) die Aminosäuresequenz, die als SEQ ID NO: 34 gezeigt ist, (xv) die Aminosäuresequenz, die als SEQ ID NO: 55 gezeigt ist, (xvi) die Aminosäuresequenz, die als SEQ ID NO: 58 gezeigt ist, (xvii) die Aminosäuresequenz, die als SEQ ID NO: 60 gezeigt ist, (xviii) die Aminosäuresequenz, die als SEQ ID NO: 61 gezeigt ist, (xix) die Aminosäuresequenz, die als SEQ ID NO: 63 gezeigt ist, (xx) die Aminosäuresequenz, die als SEQ ID NO: 65 gezeigt ist, (xxi) die Aminosäuresequenz, die als SEQ ID NO: 67 gezeigt ist, (xxii) die Aminosäuresequenz, die als SEQ ID NO: 70 gezeigt ist, oder (xxiii) eine Aminosäuresequenz, die eine 75%-ige oder größere Identität zu irgendeiner der Sequenzen hat, die gezeigt sind als SEQ ID NO: 2, SEQ ID NO: 3, SEQ ID NO: 4, SEQ ID NO: 5, SEQ ID NO: 6, SEQ ID NO: 12, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 55, SEQ ID NO: 58, SEQ ID NO: 60, SEQ ID NO: 61, SEQ ID NO: 63, SEQ ID NO: 65, SEQ ID NO: 67 oder SEQ ID NO: 70.

**15.** Verwendung nach einem der Ansprüche 8 bis 14, wobei die Fettsäure-CoA-unabhängige Diglycerid:Glycerol-Acyltransferase eine Aminosäuresequenz umfaßt, die durch eine oder mehrere der folgenden Nukleotidsequenzen codiert wird:

(a) die als SEQ ID NO: 7 gezeigte Nukleotidsequenz,
(b) die als SEQ ID NO: 8 gezeigte Nukleotidsequenz,
(c) die als SEQ ID NO: 9 gezeigte Nukleotidsequenz,
(d) die als SEQ ID NO: 10 gezeigte Nukleotidsequenz,
(e) die als SEQ ID NO: 11 gezeigte Nukleotidsequenz,
(f) die als SEQ ID NO: 13 gezeigte Nukleotidsequenz,
(g) die als SEQ ID NO: 21 gezeigte Nukleotidsequenz,
(h) die als SEQ ID NO: 23 gezeigte Nukleotidsequenz,
(i) die als SEQ ID NO: 25 gezeigte Nukleotidsequenz,
(j) die als SEQ ID NO: 27 gezeigte Nukleotidsequenz,
(k) die als SEQ ID NO: 29 gezeigte Nukleotidsequenz,
(l) die als SEQ ID NO: 31 gezeigte Nukleotidsequenz,
(m) die als SEQ ID NO: 33 gezeigte Nukleotidsequenz,
(n) die als SEQ ID NO: 35 gezeigte Nukleotidsequenz,
(o) die als SEQ ID NO: 54 gezeigte Nukleotidsequenz,
(p) die als SEQ ID NO: 59 gezeigte Nukleotidsequenz,
(q) die als SEQ ID NO: 62 gezeigte Nukleotidsequenz,
(r) die als SEQ ID NO: 64 gezeigte Nukleotidsequenz,
(s) die als SEQ ID NO: 66 gezeigte Nukleotidsequenz,
(t) die als SEQ ID NO: 68 gezeigte Nukleotidsequenz,
(u) die als SEQ ID NO: 69 gezeigte Nukleotidsequenz oder
(v) eine Nukleotidsequenz, die eine 75%-ige oder größere Identität zu einer der Sequenzen hat, die gezeigt sind als SEQ ID NO: 7, SEQ ID NO: 8, SEQ ID NO: 9, SEQ ID NO: 10, SEQ ID NO: 11, SEQ ID NO: 13, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 54, SEQ ID NO: 59, SEQ ID NO: 62, SEQ ID NO: 64, SEQ ID NO: 66, SEQ ID

NO: 68 oder SEQ ID NO: 69.

**16.** Verwendung nach einem der Ansprüche 8 bis 15, wobei die Fettsäure-CoA-unabhängige Diglycerid:Glycerol-Acyltransferase in Kombination mit einem Kristallisationsinhibitor verwendet wird.

**Revendications**

**1.** Procédé pour réduire et/ou éliminer le diglycéride d'une huile comestible, comprenant a) le mélange d'une huile comestible avec un substrat accepteur d'acyle et une diglycéride:glycérol-acyltransférase indépendante d'acide gras-CoA, dans lequel la diglycéride:glycérol-acyltransférase indépendante d'acide gras-CoA est caractérisée comme une enzyme qui, dans une huile comestible, transfère un groupe acyle d'un diglycéride au glycérol, et dans lequel la diglycéridè:glycérol-acyltransférase indépendante d'acide gras-CoA comprend le motif de séquence d'acides aminés GDSX, où X est un ou plusieurs des résidus d'acides aminés suivants : L, A, V, I, F, Y, H, Q, T, N, M ou S.

**2.** Procédé selon la revendication 1, dans lequel le diglycéride est un 1,2-diglycéride.

**3.** Procédé selon la revendication 1 ou la revendication 2, dans lequel l'enzyme diglycéride:glycérol-acyltransférase indépendante d'acide gras-CoA comprend H-309 ou comprend un résidu d'histidine à une position correspondant à His-309 dans la séquence d'acides aminés de l'enzyme lipolytique de *Aeromonas hydrophila* représentée par SEQ ID N° 2 ou SEQ ID N° 32.

**4.** Procédé selon l'une quelconque des revendications précédentes, dans lequel la diglycéride:glycérol-acyltransférase indépendante d'acide gras-CoA peut être obtenue à partir d'un organisme appartenant à un ou plusieurs des genres suivants : *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* et *Candida.*

**5.** Procédé selon l'une quelconque des revendications précédentes, dans lequel la diglycéride:glycérol-acyltransférase indépendante d'acide gras-CoA comprend une ou plusieurs des séquences d'acides aminés suivantes : (i) la séquence d'acides aminés représentée par SEQ ID N° 2 ; (ii) la séquence d'acides aminés représentée par SEQ ID N° 3 ; (iii) la séquence d'acides aminés représentée par SEQ ID N° 4 ; (iv) la séquence d'acides aminés représentée par SEQ ID N° 5 ; (v) la séquence d'acides aminés représentée par SEQ ID N° 6 ; (vi) la séquence d'acides aminés représentée par SEQ ID N° 12 ; (vii) la séquence d'acides aminés représentée par SEQ ID N° 20 ; (viii) la séquence d'acides aminés représentée par SEQ ID N° 22 ; (ix) la séquence d'acides aminés représentée par SEQ ID N° 24 ; (x) la séquence d'acides aminés représentée par SEQ ID N° 26 ; (xi) la séquence d'acides aminés représentée par SEQ ID N° 28 ; (xii) la séquence d'acides aminés représentée par SEQ ID N° 30 ; (xiii) la séquence d'acides aminés représentée par SEQ ID N° 32 ; (xiv) la séquence d'acides aminés représentée par SEQ ID N° 34 ; (xv) la séquence d'acides aminés représentée par SEQ ID N° 55 ; (xvi) la séquence d'acides aminés représentée par SEQ ID N° 58 ; (xvii) la séquence d'acides aminés représentée par SEQ ID N° 60 ; (xviii) la séquence d'acides aminés représentée par SEQ ID N° 61 ; (xix) la séquence d'acides aminés représentée par SEQ ID N° 63 ; (xx) la séquence d'acides aminés représentée par SEQ ID N° 65 ; (xxi) la séquence d'acides aminés représentée par SEQ ID N° 67 ; (xxii) la séquence d'acides aminés représentée par SEQ ID N° 70 ou (xxiii) une séquence d'acides aminés qui a 75 % ou plus d'identité avec l'une quelconque des séquences représentées par SEQ ID N° 2, SEQ ID N° 3, SEQ ID N° 4, SEQ ID N° 5, SEQ ID N° 6, SEQ ID N° 12, SEQ ID N° 20, SEQ ID N° 22, SEQ ID N° 24, SEQ ID N° 26, SEQ ID N° 28, SEQ ID N° 30, SEQ ID N° 32, SEQ ID N° 34, SEQ ID N° 55, SEQ ID N° 58, SEQ ID N° 60, SEQ ID N° 61, SEQ ID N° 63, SEQ ID N° 65, SEQ ID N° 67 ou SEQ ID N° 70.

**6.** Procédé selon l'une quelconque des revendications précédentes, dans lequel la diglycéride:glycérol-acyltransférase indépendante d'acide gras-CoA comprend une séquence d'acides aminés codée par une ou plusieurs des séquences nucléotidiques suivantes :

(a) la séquence nucléotidique représentée par SEQ ID N° 7 ;
(b) la séquence nucléotidique représentée par SEQ ID N° 8 ;
(c) la séquence nucléotidique représentée par SEQ ID N° 9 ;
(d) la séquence nucléotidique représentée par SEQ ID N° 10 ;
(e) la séquence nucléotidique représentée par SEQ ID N° 11 ;
(f) la séquence nucléotidique représentée par SEQ ID N° 13 ;

(g) la séquence nucléotidique représentée par SEQ ID N° 21 ;
(h) la séquence nucléotidique représentée par SEQ ID N° 23 ;
(i) la séquence nucléotidique représentée par SEQ ID N° 25 ;
(j) la séquence nucléotidique représentée par SEQ ID N° 27 ;
(k) la séquence nucléotidique représentée par SEQ ID N° 29 ;
(l) la séquence nucléotidique représentée par SEQ ID N° 31 ;
(m) la séquence nucléotidique représentée par SEQ ID N° 33 ;
(n) la séquence nucléotidique représentée par SEQ ID N° 35 ;
(o) la séquence nucléotidique représentée par SEQ ID N° 54 ;
(p) la séquence nucléotidique représentée par SEQ ID N° 59 ;
(q) la séquence nucléotidique représentée par SEQ ID N° 62 ;
(r) la séquence nucléotidique représentée par SEQ ID N° 64 ;
(s) la séquence nucléotidique représentée par SEQ ID N° 66 ;
(t) la séquence nucléotidique représentée par SEQ ID N° 68 ;
(u) la séquence nucléotidique représentée par SEQ ID N° 69 ou
(v) une séquence nucléotidique qui a 75 % ou plus d'identité avec l'une quelconque des séquences représentées par SEQ ID N° 7, SEQ ID N° 8 ; SEQ ID N° 9, SEQ ID N° 10, SEQ ID N° 11, SEQ ID N° 13, SEQ ID N° 21, SEQ ID N° 23, SEQ ID N° 25 ; SEQ ID N° 27, SEQ ID N° 29, SEQ ID N° 31, SEQ ID N° 33, SEQ ID N° 35, SEQ ID N° 54, SEQ ID N° 59, SEQ ID N° 62, SEQ ID N° 64, SEQ ID N° 66, SEQ ID N° 68 ou SEQ ID N° 69.

**7.** Procédé selon l'une quelconque des revendications précédentes, comprenant le mélange de l'huile comestible traitée avec un ou plusieurs constituants alimentaires pour formuler un produit alimentaire.

**8.** Utilisation d'une diglycéride:glycérol-acyl-transférase indépendante d'acide gras-CoA, caractérisée comme étant une enzyme qui, dans une huile comestible, transfère un groupe acyle d'un diglycéride au glycérol, dans la fabrication d'une huile comestible, pour réduire et/ou éliminer (de préférence réduire et/ou éliminer sélectivement) le diglycéride de ladite huile comestible, dans laquelle la diglycéride:glycérol-acyltransférase indépendante d'acide gras-COA comprend le motif de séquence d'acides aminés GDSX, où X est un ou plusieurs des résidus d'acides aminés suivants : L, A, V, I, F, Y, H, Q, T, N, M ou S.

**9.** Utilisation d'une diglycéride:glycérol-acyl-transférase indépendante d'acide gras-CoA, caractérisée comme étant une enzyme qui, dans une huile comestible, transfère un groupe acyle d'un diglycéride au glycérol, dans la fabrication d'un produit alimentaire comprenant une huile comestible, pour améliorer les propriétés de cristallisation dudit produit alimentaire, dans laquelle la diglycéride:glycérol-acyltransférase indépendante d'acide gras-CoA comprend le motif de séquence d'acides aminés GDSX, où X est un ou plusieurs des résidus d'acides aminés suivants : L, A, V, I, F, Y, H, Q, T, N, M ou S.

**10.** Utilisation selon la revendication 8 ou la revendication 9, dans laquelle la quantité de diglycéride dans l'huile comestible est réduite.

**11.** Utilisation selon l'une quelconque des revendications 8 à 10, dans laquelle le diglycéride est un 1,2-diglycéride.

**12.** Utilisation selon l'une quelconque des revendications 8 à 11, dans laquelle l'enzyme diglycéride:glycérol-acyltransférase indépendante d'acide gras-CoA comprend H-309 ou comprend un résidu d'histidine à une position correspondant à His-309 dans la séquence d'acides aminés de l'enzyme lipolytique de *Aeromonas hydrophila* représentée par SEQ ID N° 2 ou SEQ ID N° 32.

**13.** Utilisation selon l'une quelconque des revendications 8 à 12, dans laquelle la diglycéride:glycérol-acyltransférase indépendante d'acide gras-CoA peut être obtenue à partir d'un organisme appartenant à un ou plusieurs des genres suivants : *Aeromonas, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* et *Candida.*

**14.** Utilisation selon l'une quelconque des revendications 8 à 13, dans laquelle la diglycéride:glycérol-acyltransférase indépendante d'acide gras-CoA comprend une ou plusieurs des séquences d'acides aminés suivantes : (i) la séquence d'acides aminés représentée par SEQ ID N° 2 ; (ii) la séquence d'acides aminés représentée par SEQ ID N° 3 ; (iii) la séquence d'acides aminés représentée par SEQ ID N° 4 ; (iv) la séquence d'acides aminés représentée par SEQ ID N° 5 ; (v) la séquence d'acides aminés représentée par SEQ ID N° 6 ; (vi) la séquence d'acides aminés

représentée par SEQ ID N° 12 ; (vii) la séquence d'acides aminés représentée par SEQ ID N° 20 ; (viii) la séquence d'acides aminés représentée par SEQ ID N° 22 ; (ix) la séquence d'acides aminés représentée par SEQ ID N° 24 ; (x) la séquence d'acides aminés représentée par SEQ ID N° 26 ; (xi) la séquence d'acides aminés représentée par SEQ ID N° 28 ; (xii) la séquence d'acides aminés représentée par SEQ ID N° 30 ; (xiii) la séquence d'acides aminés représentée par SEQ ID N° 32 ; (xiv) la séquence d'acides aminés représentée par SEQ ID N° 34 ; (xv) la séquence d'acides aminés représentée par SEQ ID N° 55 ; (xvi) la séquence d'acides aminés représentée par SEQ ID N° 58 ; (xvii) la séquence d'acides aminés représentée par SEQ ID N° 60 ; (xviii) la séquence d'acides aminés représentée par SEQ ID N° 61 ; (xix) la séquence d'acides aminés représentée par SEQ ID N° 63 ; (xx) la séquence d'acides aminés représentée par SEQ ID N° 65 ; (xxi) la séquence d'acides aminés représentée par SEQ ID N° 67 ; (xxii) la séquence d'acides aminés représentée par SEQ ID N° 70 ou (xxiii) une séquence d'acides aminés qui a 75 % ou plus d'identité avec l'une quelconque des séquences représentées par SEQ ID N° 2, SEQ ID N° 3, SEQ ID N° 4, SEQ ID N° 5, SEQ ID N° 6, SEQ ID N° 12, SEQ ID N° 20, SEQ ID N° 22, SEQ ID N° 24, SEQ ID N° 26, SEQ ID N° 28, SEQ ID N° 30, SEQ ID N° 32, SEQ ID N° 34, SEQ ID N° 55, SEQ ID N° 58, SEQ ID N° 60, SEQ ID N° 61, SEQ ID N° 63, SEQ ID N° 65, SEQ ID N° 67 ou SEQ ID N° 70.

**15.** Utilisation selon l'une quelconque des revendications 8 à 14, dans laquelle la diglycéride:glycérol-acyltransférase indépendante d'acide gras-CoA comprend une séquence d'acides aminés codée par une ou plusieurs des séquences nucléotidiques suivantes :

(a) la séquence nucléotidique représentée par SEQ ID N° 7 ;
(b) la séquence nucléotidique représentée par SEQ ID N° 8 ;
(c) la séquence nucléotidique représentée par SEQ ID N° 9 ;
(d) la séquence nucléotidique représentée par SEQ ID N° 10 ;
(e) la séquence nucléotidique représentée par SEQ ID N° 11 ;
(f) la séquence nucléotidique représentée par SEQ ID N° 13 ;
(g) la séquence nucléotidique représentée par SEQ ID N° 21 ;
(h) la séquence nucléotidique représentée par SEQ ID N° 23 ;
(i) la séquence nucléotidique représentée par SEQ ID N° 25 ;
(j) la séquence nucléotidique représentée par SEQ ID N° 27 ;
(k) la séquence nucléotidique représentée par SEQ ID N° 29 ;
(l) la séquence nucléotidique représentée par SEQ ID N° 31 ;
(m) la séquence nucléotidique représentée par SEQ ID N° 33 ;
(n) la séquence nucléotidique représentée par SEQ ID N° 35 ;
(o) la séquence nucléotidique représentée par SEQ ID N° 54 ;
(p) la séquence nucléotidique représentée par SEQ ID N° 59 ;
(q) la séquence nucléotidique représentée par SEQ ID N° 62 ;
(r) la séquence nucléotidique représentée par SEQ ID N° 64 ;
(s) la séquence nucléotidique représentée par SEQ ID N° 66 ;
(t) la séquence nucléotidique représentée par SEQ ID N° 68 ;
(u) la séquence nucléotidique représentée par SEQ ID N° 69 ou
(v) une séquence nucléotidique qui a 75 % ou plus d'identité avec l'une quelconque des séquences représentées par SEQ ID N° 7, SEQ ID N° 8 ; SEQ ID N° 9, SEQ ID N° 10, SEQ ID N° 11, SEQ ID N° 13, SEQ ID N° 21, SEQ ID N° 23, SEQ ID N° 25 ; SEQ ID N° 27, SEQ ID N° 29, SEQ ID N° 31, SEQ ID N° 33, SEQ ID N° 35, SEQ ID N° 54, SEQ ID N° 59, SEQ ID N° 62, SEQ ID N° 64, SEQ ID N° 66, SEQ ID N° 68 ou SEQ ID N° 69.

**16.** Utilisation selon l'une quelconque des revendications 8 à 15, dans laquelle la diglycéride:glycérol-acyltransférase indépendante d'acide gras-CoA est utilisée en association avec un inhibiteur de cristallisation.

Figure 1

SEQ ID No. 1

```
  1 ivafGDSlTd geayygdsdg ggwgagladr Ltallrlrar prgvdvfnrg isGrtsdGrl
 61 ivDalvallF laqslglpnL pPYLsgdflr GANFAsagAt Ilptsgpfli QvqFkdfksq
121 vlelrqalgl lqellrllpv ldakspdlvt imiGtNDlit saffgpkste sdrnvsvpef
181 kdnlrqlikr Lrsnngarii vlitlvilnl gplGClPlkl alalassknv dasgclerln
241 eavadfneal relaiskled qlrkdglpdv kgadvpyvDl ysifqdldgi qnpsayvyGF
301 ettkaCCGyG gryNynrvCG naglcnvtak aCnpssylls flfwDgfHps ekGykavAea
361 l
```

Figure 2

SEQ ID No. 2

```
  1 mkkwfvcllg lvaltvqaad srpafsrivm fgdslsdtgk myskmrgylp ssppyyegrf
 61 sngpvwleql tnefpgltia neaeggptav aynkiswnpk yqvinnldye vtqflqkdsf
121 kpddlvilwv gandylaygw nteqdakrvr daisdaanrm vlngakeill fnlpdlgqnp
181 sarsqkvvea ashvsayhnq lllnlarqla ptgmvklfei dkqfaemlrd pqnfglsdqr
241 nacyggsyvw kpfasrsast dsqlsafnpq erlaiagnpl laqavaspma arsastlnce
301 gkmfwdqvhp ttvvhaalse paatfiesqy eflah
```

Figure 3

SEQ ID No. 3

```
  1 mkkwfvcllg lialtvqaad trpafsrivm fgdslsdtgk myskmrgylp ssppyyegrf
 61 sngpvwleql tkqfpgltia neaeggatav aynkiswnpk yqvynnldye vtqflqkdsf
121 kpddlvilwv gandylaygw nteqdakrvr daisdaanrm vlngakqill fnlpdlgqnp
181 sarsqkvvea vshvsayhnk lllnlarqla ptgmvklfei dkqfaemlrd pqnfglsdve
241 npcydggyvw kpfatrsvst drqlsafspq erlaiagnpl laqavaspma rrsasplnce
301 gkmfwdqvhp ttvvhaalse raatfietqy eflahg
```

Figure 4

SEQ ID No. 4

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 5

SEQ ID No. 5

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 6

SEQ ID No. 6

```
  1 mdyekfllfg dsitefafnt rpiedgkdqy algaalvney trkmdilqrg fkgytsrwal
 61 kilpeilkhe snivmatifl gandacsagp qsvplpefid nirqmvslmk syhirpiiig
121 pglvdrekwe kekseeialg yfrtnenfai ysdalaklan eekvpfvaln kafqqeggda
181 wqqlltdglh fsgkgykifh dellkvietf ypqyhpknmq yklkdwrdvl ddgsnims
```

# Figure 7

```
Alignment of pfam00657.6 consensus sequence with P10480
                     *->ivafGDSlTdg................eayygdsdgggwgagladrL
                        iv+fGDSl+d+++  ++ ++  +++++++ +++s+g  w ++l + +
   P10480      28       IVMFGDSLSDTgkmyskmrgylpssppYYEGRFSNGPVWLEQLTNEF 74

                     tall..rlrarprgvdvfnrgisGrtsdGrlivDalvallFlaqslglpn
                     + l    + ++++++++ +n+  +
   P10480      75 PGLTiaNEAEGGPTAVAYNKISWNPK----------------------- 100

                     LpPYLsgdflrGANFAsagAtIlptsgpfliQvqFkdfksqvlelrqalg
                                                               ++  ++
   P10480     101 -------------------------------------------YQVINN 106

                     llqellrllpvldakspdlvtimiGtNDlitsaffgpkstesdrnvsvpe
                     l++e+ ++l +++ k+ dlv++++G+ND+          ++ ++ ++++++
   P10480     107 LDYEVTQFLQKDSFKPDDLVILWVGANDY--------LAYGWNTEQDAKR 148

                     fkdnlrqlikrLrsnngariivlitlvilnlgplGClPlklalalasskn
                     ++d ++++++r+    nga+      ++++nl+ lG+ P+
   P10480     149 VRDAISDAANRMV-LNGAK-----EILLFNLPDLGQNPS----------- 181

                     vdasgclerlneavadfnealrelaiskledqlrkdglpdvkgadvpyvD
                      ++++ +e +  ++a++n++l +la     +ql+++g+++++++d ++++
   P10480     182 ARSQKVVEAASHVSAYHNQLLLNLA-----RQLAPTGMVKLFEIDKQFAE 226

                     lysifqdldgiqnpsayv.y....GFe.ttkaCCGyGgr.yNyn.rv.CG
                     +   +q+++ + + +a+++++    +++ +++a+++++++ +N+++r+ ++
   P10480     227 MLRDPQNFGLSDQRNACYgGsyvwKPFaSRSASTDSQLSaENPQeRLaIA 276

                     nag.l.c.nvtakaC.npssyll.sflfwDgfHpsekGykavAeal<-*
                     +++ l +  ++++a++ +s+ ++++++fwD++Hp+   ++a+ e
   P10480     277 GNPlLaQaVASPMAArSASTLNCeGKMFWDQVHPTTVVHAALSEPA    322

Alignment of pfam00657.6 consensus sequence with AAG09804
                     *->ivafGDSlTdg................eayygdsdgggwgagladrL
                        iv+fGDSl+d+++  ++ ++  +++++++ +++s+g  w ++l + +
  AAG09804     28       IVMFGDSLSDTgkmyskmrgylpssppYYEGRFSNGPVWLEQLTKQF 74

                     tallrlrarprgvdvfnrgisGrtsdGrlivDalvallFlaqslglpnLp
                               +g+++ n + +G+t
  AAG09804     75 ----------PGLTIANEAEGGAT-------------------------- 88

                     PYLsgdflrGANFAsagAtIlptsgpfliQvqFkdfksqvlelrqa....
                                                         ++++ + ++++ +
  AAG09804     89 -------------------------------------AVAYNKISWNpkyq 102

                     ..lgllqellrllpvldakspdlvtimiGtNDlitsaffgpkstesdrnv
                       ++l++e+ ++l +++ k+ dlv++++G+ND+            ++ ++ ++
  AAG09804    103 vyNNLDYEVTQFLQKDSFKPDDLVILWVGANDY--------LAYGWNTEQ 144

                     svpefkdnlrqlikrLrsnngariivlitlvilnlgplGClPlklalala
                     ++++++d ++++++r+    nga+      +++++nl+ lG+ P+
  AAG09804    145 DAKRVRDAISDAANRMV-LNGAK-----QILLFNLPDLGQNPS------- 181

                     ssknvdasgclerlneavadfnealrelaiskledqlrkdglpdvkgadv
                         ++++ +e +  ++a++n++l +la     +ql+++g+++++++d
  AAG09804    182 ----ARSQKVVEAVSHVSAYHNKLLLNLA-----RQLAPTGMVKLFEIDK 222

                     pyvDlysifqdldgiqnpsayv.y....GFe.ttkaCCGyGgr.yNyn.r
                     +++++    +q+++ +  ++ +++++    +++ t++ +++ +++ + +++r
  AAG09804    223 QFAEMLRDPQNFGLSDVENPCYdGgyvwKPFaTRSVSTDRQLSaFSPQeR 272

                     v.CGnag.l.c.nvtakaC.npssyll.sflfwDgfHpsekGykavAeal
                     + +++++ l +  ++++a++ +s  ++++++fwD++Hp+   ++a+ e+
  AAG09804    273 LaIAGNPlLaQaVASPMARrSASPLNCeGKMFWDQVHPTTVVHAALSERA 322

                     <-*
```

```
AAG09804         -     -

Alignment of pfam00657.6 consensus sequence with NP_631558
                       *->ivafGDSlTdgeayygdsdgggwgagladrLtallrlrarprgvdvf
                          +va+GDS ++g          +g  +  +++L      + + + ++  +
NP_631558      42         YVALGDSYSAG---------SGVLPVDPANL----LCLRSTANYPHV 75

                       nrgisGrtsdGrlivD.a.l.vallFlaqslglpnLpPYLsgdflrGANF
                        + ++G++        D + + +
NP_631558      76      IADTTGAR-----LTDvTcGaAQ--------------------------- 93

                       AsagAtIlptsgpfliQvqFkdfksqvlelrqalgllqellrllpvldak
                                                      +++     ++ +  ++ +++
NP_631558      94      -----------------------------TADFTRAQYPGVAPQLDALGT 114

                       spdlvtimiGtNDl................itsaffgpkstesdrnvsvp
                       + dlvt+ iG+ND ++  +  +  ++ +    ++  + +k    ++ + +++
NP_631558     115      GTDLVTLTIGGNDNstfinaitacgtagvlSGGKGSPCKDRHGTSFDDEI 164

                       efkdn..lrqlikrLrs.nngariivlitlvilnlg...........plG
                       e  +++ l++++  +r+++ +ar+ +l  ++i+++  +++    + +   G
NP_631558     165      EANTYpaLKEALLGVRArAPHARVAALGYPWITPATadpscflklplAAG 214

                       ClPlklalalassknvdasgclerlneavadfnealrelaiskledqlrk
                        P+                        l+ ++a   n a+r   a
NP_631558     215      DVPY--------------------LRAIQAHLNDAVRRAA---------- 234

                       dglpdvkgadvpyvDlysifqdldgiqnpsayvyGFettkaCCGyGgryN
                             ++ + +yvD+ ++
NP_631558     235      ------EETGATYVDFSGVSDG---------------------------- 250

                       ynrvCGnaglcnvtakaC.npssyll.sflfwDgf...HpsekGykavAe
                                     ++aC+ p +++ +   lf + + + Hp++ G +++Ae
NP_631558     251      --------------HDACeAPGTRWIePLLFGHSLvpvHPNALGERRMAE 286

                       al<-*
                       +
NP_631558     287      HT    288

Alignment of pfam00657.6 consensus sequence with CAC42140
                       *->ivafGDSlTdgeayygdsdgggwgagladrLtallrlrarprgvdvf
                          +va+GDS ++g          +g  +  +++L      + + + ++  +
CAC42140       42         YVALGDSYSAG---------SGVLPVDPANL----LCLRSTANYPHV 75

                       nrgisGrtsdGrlivD.a.l.vallFlaqslglpnLpPYLsgdflrGANF
                        + ++G++        D + + +
CAC42140       76      IADTTGAR-----LTDvTcGaAQ--------------------------- 93

                       AsagAtIlptsgpfliQvqFkdfksqvlelrqalgllqellrllpvldak
                                                      +++     ++ +  ++ +++
CAC42140       94      -----------------------------TADFTRAQYPGVAPQLDALGT 114

                       spdlvtimiGtNDl................itsaffgpkstesdrnvsvp
                       + dlvt+ iG+ND ++  +  +  ++ +    ++  + +k    ++ + +++
CAC42140      115      GTDLVTLTIGGNDNstfinaitacgtagvlSGGKGSPCKDRHGTSFDDEI 164

                       efkdn..lrqlikrLrs.nngariivlitlvilnlg...........plG
                       e  +++ l++++  +r+++ +ar+ +l  ++i+++  +++    + +   G
CAC42140      165      EANTYpaLKEALLGVRArAPHARVAALGYPWITPATadpscflklplAAG 214

                       ClPlklalalassknvdasgclerlneavadfnealrelaiskledqlrk
                        P+                        l+ ++a   n a+r   a
CAC42140      215      DVPY--------------------LRAIQAHLNDAVRRAA---------- 234

                       dglpdvkgadvpyvDlysifqdldgiqnpsayvyGFettkaCCGyGgryN
                             ++ + +yvD+ ++
CAC42140      235      ------EETGATYVDFSGVSDG---------------------------- 250

                       ynrvCGnaglcnvtakaC.npssyll.sflfwDgf...HpsekGykavAe
                                     ++aC+ p +++ +   lf + + + Hp++ G +++Ae
CAC42140      251      --------------HDACeAPGTRWIePLLFGHSLvpvHPNALGERRMAE 286
```

```
                  al<-*
                  +
CAC42140     287  HT     288
```

Alignment of pfam00657.6 consensus sequence with P41734

```
                  *->ivafGDSlTdg....eayygdsdgggwgagladrLtallrlrarprg
                       ++fGDS+T+    +++ + +  d+   ga+l + +         +r+
   P41734      6     FLLFGDSITEFafntRPIEDGKDQYALGAALVNEY---------TRK 43

                  vdvfnrgisGrtsdGrlivDalvallFlaqslglpnLpPYLsgdflrGAN
                  +d+  rg++G+t
   P41734     44  MDILQRGFKGYT-------------------------------------- 55

                  FAsagAtIlptsgpfliQvqFkdfksqvlelrqalgllqellrllpvlda
                                                +r+al++l+e+l+      +
   P41734     56  ------------------------------SRWALKILPEILKH-----E 70

                  kspdlvtimiGtNDlitsaffgpkstesdrnvsvpefkdnlrqlikrLrs
                   +  + ti++G+ND+          ++ +++ v++pef+dn+rq++++++s
   P41734     71  SNIVMATIFLGANDA---------CSAGPQSVPLPEFIDNIRQMVSLMKS 111

                  nngariivlitlvilnlgplGClPlklalalassknvdasgclerlneav
                   ++++ii++++lv    ++             ++ k ++ +  + r+ne +
   P41734    112  YHIRPIIIGPGLVDREKW-------------EKEKSEEIALGYFRTNENF 148

                  adfnealrelaiskledqlrkdglpdvkgadvpyvDlysifqdldgiqnp
                  a +  al +la                    ++ +vp+v l+++fq+ +g++++
   P41734    149  AIYSDALAKLA-----------------NEEKVPFVALNKAFQQEGGDAWQ 182

                  sayvyGFettkaCCGyGgryNynrvCGnaglcnvtakaCnpssyllsflf
                  +                                                l+
   P41734    183  Q------------------------------------------------LL 185

                  wDgfHpsekGykavAeal<-*
                   Dg+H+s kGyk+++++l
   P41734    186  TDGLHFSGKGYKIFHDEL    203
```

Figure 8

```
A.sal  1    MKKWFVCLLGLIALTVQAADTRPAFSRIVMFGDSLSDTGKMYSKMRGYLPSSPPYYEGRF 60
                       +        +
A.hyd  1    MKKWFVCLLGLVALTVQAADSRPAFSRIVMFGDSLSDTGKMYSKMRGYLPSSPPYYEGRF 60

A. sal 61   SNGPVWLEQLTKQFPGLTIANEAEGGATAVAYNKISWNPKYQVINNLDYEVTQFLQKDSF 120
                       ++             +
A. hyd 61   SNGPVWLEQLTNEFPGLTIANEAEGGPTAVAYNKISWNPKYQVINNLDYEVTQFLQKDSF 120

A. sal 121  KPDDLVILWVGANDYLAYGWNTEQDAKRVRDAISDAANRMVLNGAKQILLFNLPDLGQNP 180
                                                                        +
A. hyd 121  KPDDLVILWVGANDYLAYGWNTEQDAKRVRDAISDAANRMVLNGAKEILLFNLPDLGQNP 180

A. sal 181  SARSQKVVEAVSHVSAYHNKLLLNLARQLAPTGMVKLFEIDKQFAEMLRDPQNFGLSDVE 240
                      +        +                                              ++
A.hyd  181  SARSQKVVEAASHVSAYHNQLLLNLARQLAPTGMVKLFEIDKQFAEMLRDPQNFGLSDQR 240

A. sal 241  NPCYDGGYVWKPFATRSVSTDRQLSAFSPQERLAIAGNPLLAQAVASPMARRSASPLNCE 300
             + ++ +       +  +   +     +                     +    +
A. hyd 241  NACYGGSYVWKPFASRSASTDSQLSAFNPQERLAIAGNPLLAQAVASPMAARSASTLNCE 300

A. sal 301  GKMFWDQVHPTTVVHAALSERAATFIETQYEFLAH 335
                                +      +
A. hyd 301  GKMFWDQVHPTTVVHAALSEPAATFIESQYEFLAH 335
```

Figure 9

```
  1  ATGAAAAAAT GGTTTGTGTG TTTATTGGGA TTGGTCGCGC TGACAGTTCA GGCAGCCGAC
 61  AGCCGTCCCG CCTTCTCCCG GATCGTGATG TTTGGCGACA GCCTCTCCGA TACCGGCAAG
121  ATGTACAGCA AGATGCGCGG TTACCTCCCC TCCAGCCCCC CCTACTATGA GGGCCGCTTC
181  TCCAACGGGC CCGTCTGGCT GGAGCAGCTG ACCAACGAGT TCCCGGGCCT GACCATAGCC
241  AACGAGGCGG AAGGCGGACC GACCGCCGTG GCTTACAACA AGATCTCCTG GAATCCCAAG
301  TATCAGGTCA TCAACAACCT GGACTACGAG GTCACCCAGT TCCTGCAAAA AGACAGCTTC
361  AAGCCGGACG ATCTGGTGAT CCTCTGGGTC GGCGCCAACG ACTATCTGGC CTATGGCTGG
421  AACACAGAGC AGGATGCCAA GCGGGTGCGC GACGCCATCA GCGATGCGGC CAACCGCATG
481  GTGCTGAACG GCGCCAAGGA GATACTGCTG TTCAACCTGC CGGATCTGGG CCAGAACCCC
541  TCGGCCCGCA GCCAGAAGGT GGTCGAGGCG GCCAGCCATG TCTCCGCCTA CCACAACCAG
601  CTGCTGCTGA ACCTGGCACG CCAGCTGGCT CCCACCGGCA TGGTGAAGCT GTTCGAGATC
661  GACAAGCAGT TTGCCGAGAT GCTGCGTGAT CCGCAGAACT TCGGCCTGAG CGACCAGAGG
721  AACGCCTGCT ACGGTGGCAG CTATGTATGG AAGCCGTTTG CCTCCCGCAG CGCCAGCACC
781  GACAGCCAGC TCTCCGCCTT CAACCCGCAG GAGCGCCTCG CCATCGCCGG CAACCCGCTG
841  CTGGCCCAGG CCGTCGCCAG CCCCATGGCT GCCCGCAGCG CCAGCACCCT CAACTGTGAG
901  GGCAAGATGT TCTGGGATCA GGTCCACCCC ACCACTGTCG TGCACGCCGC CCTGAGCGAG
961  CCCGCCGCCA CCTTCATCGA GAGCCAGTAC GAGTTCCTCG CCCAC
```

Figure 10

```
  1  ATGAAAAAAT GGTTTGTTTG TTTATTGGGG TTGATCGCGC TGACAGTTCA GGCAGCCGAC
 61  ACTCGCCCCG CCTTCTCCCG GATCGTGATG TTCGGCGACA GCCTCTCCGA TACCGGCAAA
121  ATGTACAGCA AGATGCGCGG TTACCTCCCC TCCAGCCCGC CCTACTATGA GGGCCGTTTC
181  TCCAACGGAC CCGTCTGGCT GGAGCAGCTG ACCAAGCAGT TCCCGGGTCT GACCATCGCC
241  AACGAAGCGG AAGGCGGTGC CACTGCCGTG GCTTACAACA AGATCTCCTG GAATCCCAAG
301  TATCAGGTCT ACAACAACCT GGACTACGAG GTCACCCAGT TCTTGCAGAA AGACAGCTTC
361  AAGCCGGACG ATCTGGTGAT CCTCTGGGTC GGTGCCAATG ACTATCTGGC ATATGGCTGG
421  AATACGGAGC AGGATGCCAA GCGAGTTCGC GATGCCATCA GCGATGCGGC CAACCGCATG
481  GTACTGAACG GTGCCAAGCA GATACTGCTG TTCAACCTGC CGGATCTGGG CCAGAACCCG
541  TCAGCCCGCA GTCAGAAGGT GGTCGAGGCG GTCAGCCATG TCTCCGCCTA TCACAACAAG
601  CTGCTGCTGA ACCTGGCACG CCAGCTGGCC CCCACCGGCA TGGTAAAGCT GTTCGAGATC
661  GACAAGCAAT TTGCCGAGAT GCTGCGTGAT CCGCAGAACT TCGGCCTGAG CGACGTCGAG
721  AACCCCTGCT ACGACGGCGG CTATGTGTGG AAGCCGTTTG CCACCCGCAG CGTCAGCACC
781  GACCGCCAGC TCTCCGCCTT CAGTCCGCAG GAACGCCTCG CCATCGCCGG CAACCCGCTG
841  CTGGCACAGG CCGTTGCCAG TCCTATGGCC CGCCGCAGCG CCAGCCCCCT CAACTGTGAG
901  GGCAAGATGT TCTGGGATCA GGTACACCCG ACCACTGTCG TGCACGCAGC CCTGAGCGAG
961  CGCGCCGCCA CCTTCATCGA GACCCAGTAC GAGTTCCTCG CCCACGGATG A
```

Figure 11

```
  1  ATGCCGAAGC CTGCCCTTCG CCGTGTCATG ACCGCGACAG TCGCCGCCGT CGGCACGCTC
 61  GCCCTCGGCC TCACCGACGC CACCGCCCAC GCCGCGCCCG CCCAGGCCAC TCCGACCCTG
121  GACTACGTCG CCCTCGGCGA CAGCTACAGC GCCGGCTCCG GCGTCCTGCC CGTCGACCCC
181  GCCAACCTGC TCTGTCTGCG CTCGACGGCC AACTACCCCC ACGTCATCGC GGACACGACG
241  GGCGCCCGCC TCACGGACGT CACCTGCGGC GCCGCGCAGA CCGCCGACTT CACGCGGGCC
301  CAGTACCCGG GCGTCGCACC CCAGTTGGAC GCGCTCGGCA CCGGCACGGA CCTGGTCACG
361  CTCACCATCG GCGGCAACGA CAACAGCACC TTCATCAACG CCATCACGGC CTGCGGCACG
421  GCGGGTGTCC TCAGCGGCGG CAAGGGCAGC CCCTGCAAGG ACAGGCACGG CACCTCCTTC
481  GACGACGAGA TCGAGGCCAA CACGTACCCC GCGCTCAAGG AGGCGCTGCT CGGCGTCCGC
541  GCCAGGGCTC CCCACGCCAG GGTGGCGGCT CTCGGCTACC CGTGGATCAC CCCGGCCACC
601  GCCGACCCGT CCTGCTTCCT GAAGCTCCCC CTCGCCGCCG GTGACGTGCC CTACCTGCGG
661  GCCATCCAGG CACACCTCAA CGACGCGGTC CGGCGGGCCG CCGAGGAGAC CGGAGCCACC
721  TACGTGGACT TCTCCGGGGT GTCCGACGGC CACGACGCCT GCGAGGCCCC CGGCACCCGC
781  TGGATCGAAC CGCTGCTCTT CGGGCACAGC CTCGTTCCCG TCCACCCCAA CGCCCTGGGC
841  GAGCGGCGCA TGGCCGAGCA CACGATGGAC GTCCTCGGCC TGGACTGA
```

Figure 12

```
  1  TCAGTCCAGG CCGAGGACGT CCATCGTGTG CTCGGCCATG CGCCGCTCGC CCAGGGCGTT
 61  GGGGTGGACG GGAACGAGGC TGTGCCCGAA GAGCAGCGGT TCGATCCAGC GGGTGCCGGG
121  GGCCTCGCAG GCGTCGTGGC CGTCGGACAC CCCGGAGAAG TCCACGTAGG TGGCTCCGGT
181  CTCCTCGGCG GCCCGCCGGA CCGCGTCGTT GAGGTGTGCC TGGATGGCCC GCAGGTAGGG
241  CACGTCACCG GCGGCGAGGG GGAGCTTCAG GAAGCAGGAC GGGTCGGCGG TGGCCGGGGT
301  GATCCACGGG TAGCCGAGAG CCGCCACCCT GGCGTGGGGA GCCCTGGCGC GGACGCCGAG
361  CAGCGCCTCC TTGAGCGCGG GGTACGTGTT GGCCTCGATC TCGTCGTCGA AGGAGGTGCC
421  GTGCCTGTCC TTGCAGGGGC TGCCCTTGCC GCCGCTGAGG ACACCCGCCG TGCCGCAGGC
481  CGTGATGGCG TTGATGAAGG TGCTGTTGTC GTTGCCGCCG ATGGTGAGCG TGACCAGGTC
541  CGTGCCGGTG CCGAGCGCGT CCAACTGGGG TGCGACGCCC GGGTACTGGG CCCGCGTGAA
601  GTCGGCGGTC TGCGCGGCGC CGCAGGTGAC GTCCGTGAGG CGGGCGCCCG TCGTGTCCGC
661  GATGACGTGG GGGTAGTTGG CCGTCGAGCG CAGACAGAGC AGGTTGGCGG GGTCGACGGG
721  CAGGACGCCG GAGCCGGCGC .TGTAGCTGTC GCCGAGGGCG ACGTAGTCCA GGGTCGGAGT
781  GGCCTGGGCG GGCGCGGCGT GGGCGGTGGC GTCGGTGAGG CCGAGGGCGA GCGTGCCGAC
841  GGCGGCGACT GTCGCGGTCA TGACACGGCG AAGGGCAGGC TTCGGCAT
```

Figure 13

```
  1  ATGGATTACG AGAAGTTTCT GTTATTTGGG GATTCCATTA CTGAATTTGC TTTTAATACT
 61  AGGCCCATTG AAGATGGCAA AGATCAGTAT GCTCTTGGAG CCGCATTAGT CAACGAATAT
121  ACGAGAAAAA TGGATATTCT TCAAAGAGGG TTCAAAGGGT ACACTTCTAG ATGGGCGTTG
181  AAAATACTTC CTGAGATTTT AAAGCATGAA TCCAATATTG TCATGGCCAC AATATTTTTG
241  GGTGCCAACG ATGCATGCTC AGCAGGTCCC CAAAGTGTCC CCCTCCCCGA ATTTATCGAT
301  AATATTCGTC AAATGGTATC TTTGATGAAG TCTTACCATA TCCGTCCTAT TATAATAGGA
361  CCGGGGCTAG TAGATAGAGA GAAGTGGGAA AAAGAAAAAT CTGAAGAAAT AGCTCTCGGA
421  TACTTCCGTA CCAACGAGAA CTTTGCCATT TATTCCGATG CCTTAGCAAA ACTAGCCAAT
481  GAGGAAAAAG TTCCCTTCGT GGCTTTGAAT AAGGCGTTTC AACAGGAAGG TGGTGATGCT
541  TGGCAACAAC TGCTAACAGA TGGACTGCAC TTTTCCGGAA AAGGGTACAA AATTTTTCAT
601  GACGAATTAT TGAAGGTCAT TGAGACATTC TACCCCCAAT ATCATCCCAA AAACATGCAG
661  TACAAACTGA AAGATTGGAG AGATGTGCTA GATGATGGAT CTAACATAAT GTCTTGA
```

Figure 14

(SEQ ID No. 12)

```
        10         20         30         40         50         60
         |          |          |          |          |          |
MNLRQWMGAA TAALALGLAA CGGGGTDQSG NPNVAKVQRM VVFGDSLSDI GTYTPVAQAV

        70         80         90        100        110        120
         |          |          |          |          |          |
GGGKFTTNPG PIWAETVAAQ LGVTLTPAVM GYATSVQNCP KAGCFDYAQG GSRVTDPNGI

       130        140        150        160        170        180
         |          |          |          |          |          |
GHNGGAGALT YPVQQQLANF YAASNNTFNG NNDVVFVLAG SNDIFFWTTA AATSGSGVTP

       190        200        210        220        230        240
         |          |          |          |          |          |
AIATAQVQQA ATDLVGYVKD MIAKGATQVY VFNLPDSSLT PDGVASGTTG QALLHALVGT

       250        260        270        280        290        300
         |          |          |          |          |          |
FNTTLQSGLA GTSARIIDFN AQLTAAIQNG ASFGFANTSA RACDATKINA LVPSAGGSSL

       310        320        330        340
         |          |          |          |
FCSANTLVAS GADQSYLFAD GVHPTTAGHR LIASNVLARL LADNVAH
```

Figure 15

(SEQ ID No. 13)

```
atgaacctgc gtcaatggat gggcgccgcc acggctgccc ttgccttggg cttggccgcg      60
tgcgggggcg gtgggaccga ccagagcggc aatcccaatg tcgccaaggt gcagcgcatg     120
gtggtgttcg gcgacagcct gagcgatatc ggcacctaca cccccgtcgc gcaggcggtg     180
ggcggcggca agttcaccac caacccgggc ccgatctggg ccgagaccgt ggccgcgcaa     240
ctgggcgtga cgctcacgcc ggcggtgatg ggctacgcca cctccgtgca gaattgcccc     300
aaggccggct gcttcgacta tgcgcagggc ggctcgcgcg tgaccgatcc gaacggcatc     360
ggccacaacg gcggcgcggg ggcgctgacc tacccggttc agcagcagct cgccaacttc     420
tacgcggcca gcaacaacac attcaacggc aataacgatg tcgtcttcgt gctggccggc     480
agcaacgaca ttttcttctg gaccactgcg gcggccacca gcggctccgg cgtgacgccc     540
gccattgcca cggcccaggt gcagcaggcc gcgacggacc tggtcggcta tgtcaaggac     600
atgatcgcca agggtgcgac gcaggtctac gtgttcaacc tgcccgacag cagcctgacg     660
ccggacggcg tggcaagcgg cacgaccggc caggcgctgc tgcacgcgct ggtgggcacg     720
ttcaacacga cgctgcaaag cgggctggcc ggcacctcgg cgcgcatcat cgacttcaac     780
gcacaactga ccgcggcgat ccagaatggc gcctcgttcg gcttcgccaa caccagcgcc     840
cgggcctgcg acgccaccaa gatcaatgcc ctggtgccga gcgccggcgg cagctcgctg     900
ttctgctcgg ccaacacgct ggtggcttcc ggtgcggacc agagctacct gttcgccgac     960
ggcgtgcacc cgaccacggc cggccatcgc ctgatcgcca gcaacgtgct ggcgcgcctg    1020
ctggcggata acgtcgcgca ctga                                           1044
```

Figure 16 (SEQ ID No. 20)

```
  1 migsyvavgd sftegvgdpg pdgafvgwad rlavlladrr pegdftytnl avrgrlldqi
 61 vaeqvprvvg lapdlvsfaa ggndiirpgt dpdevaerfe lavaaltaaa gtvlvttgfd
121 trgvpvlkhl rgkiatyngh vraiadrygc pvldlwslrs vqdrrawdad rlhlspeght
181 rvalragqal glrvpadpdq pwpplpprgt ldvrrddvhw areylvpwig rrlrgessgd
241 hvtakgtlsp daiktriaav a
```

Figure 17 (SEQ ID No. 21)

```
  1 gtgatcgggt cgtacgtggc ggtgggggac agcttcaccg agggcgtcgg cgaccccggc
 61 cccgacgggg cgttcgtcgg ctgggccgac cggctcgccg tactgctcgc ggaccggcgc
121 cccgagggcg acttcacgta cacgaacctc gccgtgcgcg gcaggctcct cgaccagatc
181 gtggcggaac aggtcccgcg ggtcgtcgga ctcgcgcccg acctcgtctc gttcgcggcg
241 ggcggcaacg acatcatccg gcccggcacc gatcccgacg aggtcgccga gcggttcgag
301 ctggcggtgg ccgcgctgac cgccgcggcc ggaaccgtcc tggtgaccac cgggttcgac
361 acccgggggg tgcccgtcct caagcacctg cgcggcaaga tcgccacgta caacgggcac
421 gtccgcgcca tcgccgaccg ctacggctgc ccggtgctcg acctgtggtc gctgcggagc
481 gtccaggacc gcagggcgtg ggacgccgac cggctgcacc tgtcgccgga ggggcacacc
541 cgggtggcgc tgcgcgcggg gcaggccctg ggcctgcgcg tcccggccga ccctgaccag
601 ccctggccgc ccctgccgcc gcgcggcacg ctcgacgtcc ggcgcgacga cgtgcactgg
661 gcgcgcgagt acctggtgcc gtggatcggg cgccggctgc ggggcgagtc gtcgggcgac
721 cacgtgacgg ccaaggggac gctgtcgccg gacgccatca agacgcggat cgccgcggtg
781 gcctga
```

Figure 18
(SEQ ID No. 22)

```
  1 mqtnpaytsl vavgdsfteg msdllpdgsy rgwadllatr maarspgfry anlavrgkli
 61 gqivdeqvdv aaamgadvit lvgglndtlr pkcdmarvrd lltqaverla phceqlvlmr
121 spgrqgpvle rfrprmealf aviddlagrh gavvvdlyga qsladprmwd vdrlhltaeg
181 hrrvaeavwq slghepedpe whapipatpp pgwvtrrtad vrfarqhllp wigrrltgrs
241 sgdglpakrp dllpyedpar
```

Figure 19 (SEQ ID No. 23)

```
  1 atgcagacga accccgcgta caccagtctc gtcgccgtcg gcgactcctt caccgagggc
 61 atgtcggacc tgctgcccga cggctcctac cgtggctggg ccgacctcct cgccacccgg
121 atggcggccc gctcccccgg cttccggtac gccaacctgg cggtgcgcgg gaagctgatc
181 ggacagatcg tcgacgagca ggtggacgtg gccgccgcca tgggagccga cgtgatcacg
241 ctggtcggcg ggctcaacga cacgctgcgg cccaagtgcg acatggcccg ggtgcgggac
301 ctgctgaccc aggccgtgga acggctcgcc ccgcactgcg agcagctggt gctgatgcgc
361 agtcccggtc gccagggtcc ggtgctggag cgcttccggc cccgcatgga ggccctgttc
421 gccgtgatcg acgacctggc cgggcggcac ggcgccgtgg tcgtcgacct gtacggggcc
481 cagtcgctgg ccgaccctcg gatgtgggac gtggaccggc tgcacctgac cgccgagggc
541 caccgccggg tcgcggaggc ggtgtggcag tcgctcggcc acgagcccga ggacccccgag
601 tggcacgcgc cgatcccggc gacgccgccg ccggggtggg tgacgcgcag gaccgcggac
661 gtccggttcg cccggcagca cctgctgccc tggataggcc gcaggctgac cgggcgctcg
721 tccggggacg gcctgccggc caagcgcccg gacctgctgc cctacgagga ccccgcacgg
781 tga
```

Figure 20 (SEQ ID No. 24)

```
  1 mtrgrdggag apptkhrall aaivtlivai saaiyagasa ddgsrdhalq aggrlprgda
 61 apastgawvg awatapaaae pgtettglag rsvrnvvhts vggtgaritl snlygqsplt
121 vthasialaa gpdtaaaiad tmrrltfggs arviipaggq vmsdtarlai pyganvlvtt
181 yspipsgpvt yhpqarqtsy ladgdrtadv tavayttptp ywryltaldv lsheadgtvv
241 afgdsitdga rsqsdanhrw tdvlaarlhe aagdgrdtpr ysvvnegisg nrlltsrpgr
301 padnpsglsr fqrdvlertn vkavvvvlgv ndvlnspela drdailtglr tlvdraharg
361 lrvvgatitp fggyggytea retmrqevne eirsgrvfdt vvdfdkalrd pydprrmrsd
421 ydsgdhlhpg dkgyarmgav idlaalkgaa pvka
```

Figure 21 (SEQ ID No. 25)

```
   1 atgacccggg gtcgtgacgg gggtgcgggg gcgccccca ccaagcaccg tgccctgctc
  61 gcggcgatcg tcaccctgat agtggcgatc tccgcggcca tatacgccgg agcgtccgcg
 121 gacgacggca gcagggacca cgcgctgcag gccggaggcc gtctcccacg aggagacgcc
 181 gcccccgcgt ccaccggtgc ctgggtgggc gcctgggcca ccgcaccggc cgcggccgag
 241 ccgggcaccg agacgaccgg cctggcgggc cgctccgtgc gcaacgtcgt gcacacctcg
 301 gtcggcggca ccggcgcgcg gatcaccctc tcgaacctgt acgggcagtc gccgctgacc
 361 gtcacacacg cctcgatcgc cctggccgcc gggcccgaca ccgccgccgc gatcgccgac
 421 accatgcgcc ggctcacctt cggcggcagc gcccgggtga tcatcccggc gggcggccag
 481 gtgatgagcg acaccgcccg cctcgccatc ccctacgggg cgaacgtcct ggtcaccacg
 541 tactcccccca tcccgtccgg gccggtgacc taccatccgc aggcccggca gaccagctac
 601 ctggccgacg gcgaccgcac ggcggacgtc accgccgtcg cgtacaccac ccccacgccc
 661 tactggcgct acctgaccgc cctcgacgtg ctgagccacg aggccgacgg cacggtcgtg
 721 gcgttcggcg actccatcac cgacggcgcc cgctcgcaga gcgacgccaa ccaccgctgg
 781 accgacgtcc tcgccgcacg cctgcacgag gcggcgggcg acggccggga cacgccccgc
 841 tacagcgtcg tcaacgaggg catcagcggc aaccggctcc tgaccagcag gccggggcgg
 901 ccggccgaca acccgagcgg actgagccgg ttccagcggg acgtgctgga acgcaccaac
 961 gtcaaggccg tcgtcgtcgt cctcggcgtc aacgacgtcc tgaacagccc ggaactcgcc
1021 gaccgcgacg ccatcctgac cggcctgcgc accctcgtcg accgggcgca cgcccgggga
1081 ctgcgggtcg tcggcgccac gatcacgccg ttcggcggct acggcggcta caccgaggcc
1141 cgcgagacga tgcggcagga ggtcaacgag gagatccgct ccggccgggt cttcgacacg
1201 gtcgtcgact tcgacaaggc cctgcgcgac ccgtacgacc cgcgccggat gcgctccgac
1261 tacgacagcg gcgaccacct gcacccggc gacaaggggt acgcgcgcat gggcgcggtc
1321 atcgacctgg ccgcgctgaa gggcgcggcg ccggtcaagg cgtag
```

## Figure 22 (SEQ ID No. 26)

```
  1 mtsmsrarva rriaagaayg gggiglagaa avglvvaevq larrrvgvgt ptrvpnaqgl
 61 yggtlptagd pplrlmmlgd staagqgvhr agqtpgalla sglaavaerp vrlgsvaqpg
121 acsddldrqv alvlaepdrv pdicvimvga ndvthrmpat rsvrhlssav rrlrtagaev
181 vvgtcpdlgt iervrqplrw larrasrqla aaqtigaveq ggrtvslgdl lgpefaqnpr
241 elfgpdnyhp saegyataam avlpsvcaal glwpadeehp dalrregflp varaaaeaas
301 eagtevaaam ptgprgpwal lkrrrrrrvs eaepsspsgv
```

## Figure 23 (SEQ ID No. 27)

```
   1 atgacgagca tgtcgagggc gagggtggcg cggcggatcg cggccggcgc ggcgtacggc
  61 ggcggcggca tcggcctggc gggagcggcg gcggtcggtc tggtggtggc cgaggtgcag
 121 ctggccagac gcagggtggg ggtgggcacg ccgacccggg tgccgaacgc gcagggactg
 181 tacggcggca ccctgcccac ggccggcgac ccgccgctgc ggctgatgat gctgggcgac
 241 tccacggccg ccgggcaggg cgtgcaccgg gccgggcaga cgccgggcgc gctgctggcg
 301 tccgggctcg cggcggtggc ggagcggccg gtgcggctgg ggtcggtcgc ccagccgggg
 361 gcgtgctcgg acgacctgga ccggcaggtg gcgctggtgc tcgccgagcc ggaccgggtg
 421 cccgacatct gcgtgatcat ggtcggcgcc aacgacgtca cccaccggat gccggcgacc
 481 cgctcggtgc ggcacctgtc ctcggcggta cggcggctgc gcacggccgg tgcggaggtg
 541 gtggtcggca cctgtccgga cctgggcacg atcgagcggg tgcggcagcc gctgcgctgg
 601 ctggcccggc gggcctcacg gcagctcgcg gcggcacaga ccatcggcgc cgtcgagcag
 661 ggcgggcgca cggtgtcgct gggcgacctg ctgggtccgg agttcgcgca gaacccgcgg
 721 gagctcttcg gccccgacaa ctaccacccc tccgccgagg ggtacgccac ggccgcgatg
 781 gcggtactgc cctcggtgtg cgccgcgctc ggcctgtggc cggccgacga ggagcacccg
 841 gacgcgctgc gccgcgaggg cttcctgccg gtggcgcgcg cggcggcgga ggcggcgtcc
 901 gaggcgggta cggaggtcgc cgccgccatg cctacggggc ctcgggggcc ctgggcgctg
 961 ctgaagcgcc ggagacggcg tcgggtgtcg gaggcggaac cgtccagccc gtccggcgtt
1021 tga
```

Figure 24 (SEQ ID No. 28)

```
  1 mgrgtdqrtr ygrrrarval aaltaavlgv gvagcdsvgg dspapsgsps krtrtapawd
 61 tspasvaavg dsitrgfdac avlsdcpevs watgssakvd slavrllgka daaehswnya
121 vtgarmadlt aqvtraaqre pelvavmaga ndacrsttsa mtpvadfraq feeamatlrk
181 klpkaqvyvs sipdlkrlws qgrtnplgkq vwklglcpsm lgdadsldsa atlrrntvrd
241 rvadynevlr evcakdrrcr sddgavhefr fgtdqlshwd wfhpsvdgqa rlaeiayrav
301 taknp
```

Figure 25 (SEQ ID No. 29)

```
  1 atgggtcgag ggacggacca gcggacgcgg tacggccgtc gccgggcgcg tgtcgcgctc
 61 gccgccctga ccgccgccgt cctgggcgtg ggcgtggcgg gctgcgactc cgtgggcggc
121 gactcacccg ctccttccgg cagcccgtcg aagcggacga ggacggcgcc cgcctgggac
181 accagcccgg cgtccgtcgc cgccgtgggc gactccatca cgcgcggctt cgacgcctgt
241 gcggtgctgt cggactgccc ggaggtgtcg tgggcgaccg gcagcagcgc gaaggtcgac
301 tcgctggccg tacggctgct ggggaaggcg gacgcggccg agcacagctg gaactacgcg
361 gtcaccgggg cccggatggc ggacctgacc gctcaggtga cgcgggcggc gcagcgcgag
421 ccggagctgg tggcggtgat ggccggggcg aacgacgcgt gccggtccac gacctcggcg
481 atgacgccgg tggcggactt ccgggcgcag ttcgaggagg cgatggccac cctgcgcaag
541 aagctcccca aggcgcaggt gtacgtgtcg agcatcccgg acctcaagcg gctctggtcc
601 cagggccgca ccaacccgct gggcaagcag gtgtggaagc tcggcctgtg cccgtcgatg
661 ctgggcgacg cggactccct ggactcggcg gcgaccctgc ggcgcaacac ggtgcgcgac
721 cgggtggcgg actacaacga ggtgctgcgg gaggtctgcg cgaaggaccg gcggtgccgc
781 agcgacgacg gcgcggtgca cgagttccgg ttcggcacgg accagttgag ccactgggac
841 tggttccacc cgagtgtgga cggccaggcc cggctggcgg agatcgccta ccgcgcggtc
901 accgcgaaga atccctga
```

## Figure 26 (SEQ ID No. 30)

```
  1 mrlsrraata sallltpala lfgasaavsa priqatdyva lgdsyssgvg agsydsssgs
 61 ckrstksypa lwaashtgtr fnftacsgar tgdvlakqlt pvnsgtdlvs itiggndagf
121 adtmttcnlq gesaclaria karayiqqtl paqldqvyda idsrapaaqv vvlgyprfyk
181 lggscavgls eksraainaa addinavtak raadhgfafg dvnttfaghe lcsgapwlhs
241 vtlpvensyh ptangqskgy lpvlnsat
```

## Figure 27 (SEQ ID No. 31)

```
   1 ttcatcacaa cgatgtcaca acaccggcca tccgggtcat ccctgatcgt gggaatgggt
  61 gacaagcctt cccgtgacga aagggtcctg ctacatcaga aatgacagaa atcctgctca
 121 gggaggttcc atgagactgt cccgacgcgc ggccacggcg tccgcgctcc tcctcacccc
 181 ggcgctcgcg ctcttcggcg cgagcgccgc cgtgtccgcg ccgcgaatcc aggccaccga
 241 ctacgtggcc ctcggcgact cctactcctc gggggtcggc gcgggcagct acgacagcag
 301 cagtggctcc tgtaagcgca gcaccaagtc ctacccggcc ctgtgggccg cctcgcacac
 361 cggtacgcgg ttcaacttca ccgcctgttc gggcgcccgc acaggagacg tgctggccaa
 421 gcagctgacc ccggtcaact ccggcaccga cctggtcagc attaccatcg gcggcaacga
 481 cgcgggcttc gccgacacca tgaccacctg caacctccag ggcgagagcg cgtgcctggc
 541 gcggatcgcc aaggcgcgcg cctacatcca gcagacgctg cccgcccagc tggaccaggt
 601 ctacgacgcc atcgacagcc gggcccccgc agcccaggtc gtcgtcctgg gctacccgcg
 661 cttctacaag ctgggcggca gctgcgccgt cggtctctcg gagaagtccc gcgcggccat
 721 caacgccgcc gccgacgaca tcaacgccgt caccgccaag cgcgccgccg accacggctt
 781 cgccttcggg gacgtcaaca cgaccttcgc cgggcacgag ctgtgctccg gcgcccctg
 841 gctgcacagc gtcacccttc ccgtggagaa ctcctaccac cccacggcca acggacagtc
 901 caagggctac ctgcccgtcc tgaactccgc cacctgatct cgcggctact ccgcccctga
 961 cgaagtcccg cccccgggcg ggcttcgcc gtaggtgcgc gtaccgccgt cgcccgtcgc
1021 gccggtggcc ccgccgtacg tgccgccgcc cccggacgcg gtcggttc
```

Figure 28 (SEQ ID No. 32)

```
  1  MKKWFVCLLG LVALTVQAAD SRPAFSRIVM FGDSLSDTGK MYSKMRGYLP
 51  SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNQ
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIANQY EFLAH*
```

# Figure 29 (SEQ ID No. 33)

```
1    ATGAAAAAAT GGTTTGTGTG TTTATTGGGA TTGGTCGCGC TGACAGTTCA
     TACTTTTTTA CCAAACACAC AAATAACCCT AACCAGCGCG ACTGTCAAGT

51   GGCAGCCGAC AGTCGCCCCG CCTTTTCCCG GATCGTGATG TTCGGCGACA
     CCGTCGGCTG TCAGCGGGGC GGAAAAGGGC CTAGCACTAC AAGCCGCTGT

101  GCCTCTCCGA TACCGGCAAA ATGTACAGCA AGATGCGCGG TTACCTCCCC
     CGGAGAGGCT ATGGCCGTTT TACATGTCGT TCTACGCGCC AATGGAGGGG

151  TCCAGCCCGC CCTACTATGA GGGCCGTTTC TCCAACGGAC CCGTCTGGCT
     AGGTCGGGCG GGATGATACT CCCGGCAAAG AGGTTGCCTG GGCAGACCGA

201  GGAGCAGCTG ACCAAACAGT TCCCGGGTCT GACCATCGCC AACGAAGCGG
     CCTCGTCGAC TGGTTTGTCA AGGGCCCAGA CTGGTAGCGG TTGCTTCGCC

251  AAGGCGGTGC CACTGCCGTG GCTTACAACA AGATCTCCTG GAATCCCAAG
     TTCCGCCACG GTGACGGCAC CGAATGTTGT TCTAGAGGAC CTTAGGGTTC

301  TATCAGGTCA TCAACAACCT GGACTACGAG GTCACCCAGT TCTTGCAGAA
     ATAGTCCAGT AGTTGTTGGA CCTGATGCTC CAGTGGGTCA AGAACGTCTT

351  AGACAGCTTC AAGCCGGACG ATCTGGTGAT CCTCTGGGTC GGTGCCAATG
     TCTGTCGAAG TTCGGCCTGC TAGACCACTA GGAGACCCAG CCACGGTTAC

401  ACTATCTGGC CTATGGCTGG AACACGGAGC AGGATGCCAA GCGGGTTCGC
     TGATAGACCG GATACCGACC TTGTGCCTCG TCCTACGGTT CGCCCAAGCG

451  GATGCCATCA GCGATGCGGC CAACCGCATG GTACTGAACG GTGCCAAGCA
     CTACGGTAGT CGCTACGCCG GTTGGCGTAC CATGACTTGC CACGGTTCGT

501  GATACTGCTG TTCAACCTGC CGGATCTGGG CCAGAACCCG TCAGCTCGCA
     CTATGACGAC AAGTTGGACG GCCTAGACCC GGTCTTGGGC AGTCGAGCGT

551  GTCAGAAGGT GGTCGAGGCG GTCAGCCATG TCTCCGCCTA TCACAACCAG
     CAGTCTTCCA CCAGCTCCGC CAGTCGGTAC AGAGGCGGAT AGTGTTGGTC

601  CTGCTGCTGA ACCTGGCACG CCAGCTGGCC CCCACCGGCA TGGTAAAGCT
     GACGACGACT TGGACCGTGC GGTCGACCGG GGGTGGCCGT ACCATTTCGA

651  GTTCGAGATC GACAAGCAAT TTGCCGAGAT GCTGCGTGAT CCGCAGAACT
     CAAGCTCTAG CTGTTCGTTA AACGGCTCTA CGACGCACTA GGCGTCTTGA

701  TCGGCCTGAG CGACGTCGAG AACCCCTGCT ACGACGGCGG CTATGTGTGG
     AGCCGGACTC GCTGCAGCTC TTGGGGACGA TGCTGCCGCC GATACACACC

751  AAGCCGTTTG CCACCCGCAG CGTCAGCACC GACCGCCAGC TCTCCGCCTT
     TTCGGCAAAC GGTGGGCGTC GCAGTCGTGG CTGGCGGTCG AGAGGCGGAA

801  CAGTCCGCAG GAACGCCTCG CCATCGCCGG CAACCCGCTG CTGGCACAGG
     GTCAGGCGTC CTTGCGGAGC GGTAGCGGCC GTTGGGCGAC GACCGTGTCC

851  CCGTTGCCAG TCCTATGGCC CGCCGCAGCG CCAGCCCCCT CAACTGTGAG
     GGCAACGGTC AGGATACCGG GCGGCGTCGC GGTCGGGGGA GTTGACACTC

901  GGCAAGATGT TCTGGGATCA GGTACACCCG ACCACTGTCG TGCACGCAGC
     CCGTTCTACA AGACCCTAGT CCATGTGGGC TGGTGACAGC ACGTGCGTCG

951  CCTGAGCGAG CGCGCCGCCA CCTTCATCGC GAACCAGTAC GAGTTCCTCG
     GGACTCGCTC GCGCGGCGGT GGAAGTAGCG CTTGGTCATG CTCAAGGAGC

1001 CCCAC TGA
     GGGTG ACT
```

Figure 30 (SEQ ID No. 34)

```
  1  MKKWFVCLLG LIALTVQAAD TRPAFSRIVM FGDSLSDTGK MYSKMRGYLP
 51  SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNK
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIETQY EFLAHG*
```

# Figure 31 (SEQ ID No. 35)

1 ATGAAAAAAT GGTTTGTTTG TTTATTGGGG TTGATCGCGC TGACAGTTCA
TACTTTTTTA CCAAACAAAC AAATAACCCC AACTAGCGCG ACTGTCAAGT

51 GGCAGCCGAC ACTCGCCCCG CCTTCTCCCG GATCGTGATG TTCGGCGACA
CCGTCGGCTG TGAGCGGGGC GGAAGAGGGC CTAGCACTAC AAGCCGCTGT

101 GCCTCTCCGA TACCGGCAAA ATGTACAGCA AGATGCGCGG TTACCTCCCC
CGGAGAGGCT ATGGCCGTTT TACATGTCGT TCTACGCGCC AATGGAGGGG

151 TCCAGCCCGC CCTACTATGA GGGCCGTTTC TCCAACGGAC CCGTCTGGCT
AGGTCGGGCG GGATGATACT CCCGGCAAAG AGGTTGCCTG GGCAGACCGA

201 GGAGCAGCTG ACCAAGCAGT TCCCGGGTCT GACCATCGCC AACGAAGCGG
CCTCGTCGAC TGGTTCGTCA AGGGCCCAGA CTGGTAGCGG TTGCTTCGCC

251 AAGGCGGTGC CACTGCCGTG GCTTACAACA AGATCTCCTG GAATCCCAAG
TTCCGCCACG GTGACGGCAC CGAATGTTGT TCTAGAGGAC CTTAGGGTTC

301 TATCAGGTCA TCAACAACCT GGACTACGAG GTCACCCAGT TCTTGCAGAA
ATAGTCCAGT AGTTGTTGGA CCTGATGCTC CAGTGGGTCA AGAACGTCTT

351 AGACAGCTTC AAGCCGGACG ATCTGGTGAT CCTCTGGGTC GGTGCCAATG
TCTGTCGAAG TTCGGCCTGC TAGACCACTA GGAGACCCAG CCACGGTTAC

401 ACTATCTGGC ATATGGCTGG AATACGGAGC AGGATGCCAA GCGAGTTCGC
TGATAGACCG TATACCGACC TTATGCCTCG TCCTACGGTT CGCTCAAGCG

451 GATGCCATCA GCGATGCGGC CAACCGCATG GTACTGAACG GTGCCAAGCA
CTACGGTAGT CGCTACGCCG GTTGGCGTAC CATGACTTGC CACGGTTCGT

501 GATACTGCTG TTCAACCTGC CGGATCTGGG CCAGAACCCG TCAGCCCGCA
CTATGACGAC AAGTTGGACG GCCTAGACCC GGTCTTGGGC AGTCGGGCGT

551 GTCAGAAGGT GGTCGAGGCG GTCAGCCATG TCTCCGCCTA TCACAACAAG
CAGTCTTCCA CCAGCTCCGC CAGTCGGTAC AGAGGCGGAT AGTGTTGTTC

601 CTGCTGCTGA ACCTGGCACG CCAGCTGGCC CCCACCGGCA TGGTAAAGCT
GACGACGACT TGGACCGTGC GGTCGACCGG GGGTGGCCGT ACCATTTCGA

651 GTTCGAGATC GACAAGCAAT TTGCCGAGAT GCTGCGTGAT CCGCAGAACT
CAAGCTCTAG CTGTTCGTTA AACGGCTCTA CGACGCACTA GGCGTCTTGA

701 TCGGCCTGAG CGACGTCGAG AACCCCTGCT ACGACGGCGG CTATGTGTGG
AGCCGGACTC GCTGCAGCTC TTGGGGACGA TGCTGCCGCC GATACACACC

751 AAGCCGTTTG CCACCCGCAG CGTCAGCACC GACCGCCAGC TCTCCGCCTT
TTCGGCAAAC GGTGGGCGTC GCAGTCGTGG CTGGCGGTCG AGAGGCGGAA

801 CAGTCCGCAG GAACGCCTCG CCATCGCCGG CAACCCGCTG CTGGCACAGG
GTCAGGCGTC CTTGCGGAGC GGTAGCGGCC GTTGGGCGAC GACCGTGTCC

851 CCGTTGCCAG TCCTATGGCC CGCCGCAGCG CCAGCCCCCT CAACTGTGAG
GGCAACGGTC AGGATACCGG GCGGCGTCGC GGTCGGGGGA GTTGACACTC

901 GGCAAGATGT TCTGGGATCA GGTACACCCG ACCACTGTCG TGCACGCAGC
CCGTTCTACA AGACCCTAGT CCATGTGGGC TGGTGACAGC ACGTGCGTCG

951 CCTGAGCGAG CGCGCCGCCA CCTTCATCGA GACCCAGTAC GAGTTCCTCG
GGACTCGCTC GCGCGGCGGT GGAAGTAGCT CTGGGTCATG CTCAAGGAGC

1001 CCCACGGATG A
GGGTGCCTAC T

Figure 32

```
           1       10        20        30        40        50
           |--------+---------+---------+---------+---------|
     satA     ADTRPAFSRIVMFGDSLSDTGKMYSKMRGYLPSSPPYYEGRFSN--G
    R.sol  QSGNPNVAKVQRMVVFGDSLSDIGT-------YTPVAQAVGGGKFTTNPG
Consensus  ...adnraafqRiVmFGDSLSDiGk.......YlPsaqaygeGrFsn..G

           51      60        70        80        90        100
           |--------+---------+---------+---------+---------|
     satA  PVWLEQLTKQFPGLTIANEAEGGATAVAYNKISWNPKYQVINNLDYEVTQ
    R.sol  PIWAETVAAQL-GVTLTPAVMGYATSVQNCPKAGCFDYAQGGSRVTDPNG
Consensus  P!WaEqlaaQl.GlTianaaeGgATaVannkiagnfdYaqgnnrdt#pnq

           101     110       120       130       140       150
           |--------+---------+---------+---------+---------|
     satA  FLQKDSFKPDDLVILWVGANDYLAYG--WNTEQDAKRVRDAISDAANRMV
    R.sol  IGHNGGAGALTYPVQQQLANFYAASNNTFNGNNDVVFVLAGSNDIFFWTT
Consensus  igqndgagaddlp!qqqgANdYaAsn..fNg##DakrVraainDaanrmt

           151     160       170       180       190       200
           |--------+---------+---------+---------+---------|
     satA  LNGAKQILLFNLPDLGQNPSARSQKVVEAVSHVSAYHNKL-LLNLARQLA
    R.sol  AAATSGSGVTPAIATAQVQQAATDLVGYVKDMIAKGATQVYVFNLPDSSL
Consensus  aaaakqiglfnaialaQnqqAas#lVgeakdh!aaganql.llNLarqla

           201     210       220       230       240       250
           |--------+---------+---------+---------+---------|
     satA  PTGMVKLFEIDKQFAEMLRDPQNFGLSDVENPCYDGGYVWKPFATRSVST
    R.sol  TPDGVASGTTGQALLHALVGTFNTTLQSGLAGTSARIIDFNAQLTAAIQN
Consensus  ppdgValgeidqalaeaLrdpqNfgLqdgeagcsargidfnaqaTaa!qn

           251     260       270       280       290       300
           |--------+---------+---------+---------+---------|
     satA  DRQLSAFSPQERLAIAG--NPLLAQAVASPM---ARRSASPLNCEGKMFW
    R.sol  GASFGFANTSARACDATKINALYPSAGGSSLFCSANTLVASGADQSYLFA
Consensus  daqlgaanpqaRaadAg..NaLlaqAgaSp$...Arrlaapgad#gk$Fa

           301     310       320       330
           |--------+---------+---------|
     satA  DQVHPTTVVHAALSERAATFIETQYEFLAH
    R.sol  DGVHPTTAGHRLIASNVLARLLA--DNVAH
Consensus  DqVHPTTagHaaiaeraaariea..#nlAH
```

Figure 33

```
                        ▼
Pfam        *->ivafGDSltdggg..............ayygdsdgggwgagladrltsla..rlrargrgvdv
Sriml  38      YVALGDSYSSGVG............agSYDSSSGSCKRSTKSYPALWAAS..-----HTGTRF 81
Scoe1   5      YVAVGDSFTEG--..............--VGDPGPDGAFVGWADRLAVLL..ADRRPEGDFTY 47
Scoe2  10      LVAVGDSFTEG--..............--MSDLLPDGSYRGWADLLATRM..--AARSPGFRY 50
Scoe3 239      VVAFGDSITDG--..............ARSQSDANHRWTDVLAARLHEAA..GDGRDTPRYSV 283
Scoe4  75      LMMLGDSTAAG--..............------QGVHRAGQTPGALLASG..LAAVAERPVRL 113
Scoe5  66      VAAVGDSITRGFD............acAVLSDCPEVSWATGSSAKVDSLAvrLLGKADAAEHS 116
Ahyd1  28      IVMFGDSLSDTGKmyskmrgylpssppyYEGRFSNGPVWLEQLTNEFPGLTiaNEAEGGPTAVA 91
Asal1  28      IVMFGDSLSDTGKmyskmrgylpssppyYEGRFSNGPVWLEQLTKQF----..------PGLTI 79
Ahyd2  40      IVMFGDSLSDTGKmyskmrgylpssppyYEGRFSNGPVWLEQLTKQFPGLTiaNEAEGGATAVA 103

Pfam        fnrgisGrtsdGrlvvDarlvatllFlaqflGlnlpPYLsgdflrGANFAsagAtIlgtslipflni
Sriml  82 NFTACSGAR---------------------------------------------------------- 90
Scoe1  48 TNLAVRGRL---------------------------------------------------------- 56
Scoe2  51 ANLAVRGKL---------------------------------------------------------- 59
Scoe3 284 VNEGISGNR---------------------------------------------------------- 292
Scoe4 114 GSVAQPGAC---------------------------------------------------------- 122
Scoe5 117 WNYAVTGAR---------------------------------------------------------- 125
Ahyd1  92 YNKISWNPK---------------------------------------------------------- 100
Asal1  80 ANEAEGGAT---------------------------------------------------------- 88
Ahyd2 104 YNKISWNPK---------------------------------------------------------- 112

                                                         ▼
Pfam        QvqFkdfkskvlelrqa.....lgllqellrlvpvldakspdlvtimiGtNDl...itvakfgpks
Sriml  91 -----------------.....---TGDVLAKQLTPVNSGTDLVSITIGGNDAgfaDTMTTCNLQG 131
Scoe1  57 -----------------.....--LDQIVAEQVPRVVGLAPDLVSFAAGGNDI...-----I---- 86
Scoe2  60 -----------------.....--IGQIVDEQVDVAAAMGADVITLVGGLNDT...---------- 88
Scoe3 293 -------LLTSRPGRPA.....DNPSGLSRFQRDVLERTNVKAVVVVLGVNDV...---------- 333
Scoe4 123 -----------------.....SDDLDRQVALVLAEPDRVPDICVIMVGANDV...---------- 153
Scoe5 126 -----------------.....---MADLTAQVTRAAQREPELVAVMAGANDA...--------CR 155
Ahyd1 101 -------------YQVI.....NNLDYEVTQFLQKDSFKPDDLVILWVGANDY...--------LA 137
Asal1  89 -------AVAYNKISWNpkyqvyNNLDYEVTQFLQKDSFKPDDLVILWVGANDY...--------LA 137
Ahyd2 113 -------------YQVI.....NNLDYEVTQFLQKDSFKPDDLVILWVGANDY...--------LA 149

Pfam      .......tksdrnvsvpefrdnlrklikrLrsangariiilitlvllnlpl..........plGCl
Sriml 132 esaclarIAKARAYIQQTLPAQLDQVYDAIDSRAPAA-----QVVVLGYP-..........----- 176
Scoe1  87 .......---RPGTDPDEVAERFELAVAALT-AAAGTVLVTTGFDTRGVP-..........----- 125
Scoe2  89 .......---------LRPKCDMARVRDLLTQAVERLAPHCEQLVLMRSP-..........----- 122
Scoe3 334 .......LNSPELADRDAILTGLRTLVDRAHARGLRVVGATITPFGGYGG-..........----- 376
Scoe4 154 .......---THRMPATRSVRHLSSAVRRLR-TAGAEVVVGTCPDLGTIE-..........----- 192
Scoe5 156 .......STTSAMTPVADFRAQFEEAMATLR-KKLPKAQVYVSSIPDLKRLwsqgrtnplgkQVWKL 214
Ahyd1 138 .......YGWNTEQDAKRVRDAISDAANRMV-LNGAK-----EILLFNLP-..........----- 174
Asal1 138 .......YGWNTEQDAKRVRDAISDAANRMV-LNGAK-----QILLFNLP-..........----- 174
Ahyd2 150 .......YGWNTEQDAKRVRDAISDAANRMV-LNGAK-----QILLFNLP-..........----- 186

Pfam        pq.klalalassknvdatgclerlneavadynealrelaei.ek.l.q.aqlrkdglpdlkeanvpy
Sriml 177 --.RFYKLGGSCAVGLSEKSRAAINAAADDINAVTAKRA--.--.-.-.-----------ADHGFAF 219
Scoe1 126 --.-----------------VLKHLRGKIATYNGHVRAIA--.--.-.-.-----------DRYGCPV 152
Scoe2 123 --.------------GRQGPVLERFRPRMEALFAVIDDLA--.--.-.-.-----------GRHGAVV 154
Scoe3 377 --.YTEARETMRQEVNEEIRSGRVFDTVVDFDKALRDPY--.--.-.-.------------------ 412
Scoe4 193 --.----------------------------RVRQPLRWLaRRaSrQlAAAQTIGAVEQGGRTVSL 227
Scoe5 215 GLcPSMLGDADSLDSAATLRRNTVRDRVADYNEVLREVC--.--.-.AkDRRCRSDDGAVHEFRFGT 273
Ahyd1 175 --.----DLGQNPSARSQKVVEAASHVSAYHNQLLLNLA--.--.-.-.RQLAPTGMVKLFEIDKQF 224
Asal1 175 --.----DLGQNPSARSQKVVEAVSHVSAYHNKLLLNLA--.--.-.-.RQLAPTGMVKLFEIDKQF 224
Ahyd2 187 --.----DLGQNPSARSQKVVEAVSHVSAYHNQLLLNLA--.--.-.-.RQLAPTGMVKLFEIDKQF 236

Pfam        VDlysifqdldgiqnpsayv.y....GFeet.kaCCGyGgr.yNyn.rv.CGnag.l.ck.vtakaC
Sriml 220 GDVNT---------------.-...-----.---------.-TFAgHElCSGAPwL.HS.VT---- 242
Scoe1 153 LDLWSLRSVQDRRA------.-...-----.---------.----.--.-----.-.--.------ 166
Scoe2 155 VDLYGAQSLADPRM------.-...-----.---------.----.--.-----.-.--.------ 168
Scoe3 413 --------------------.-...-----.---------.----.--.-----.-.--.------ 413
Scoe4 228 GDLLGPEFAQNPREL-----.-...-----.---------.----.--.-----.-.--.------ 242
```

```
Scoe5   274 DQL----------------.-...-----.---------.----.--.-----.-.--.------ 276
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303

                         ▼
Pfam      .dassyll.atlfwDgf.HpsekGykavAeal<-*
Srim1 243 .-------.--LPVENSyHPTANGQSKGYLPV    263
Scoe1 167 .-------.--WDADRL.HLSPEGHTRVALRA    186
Scoe2 169 .-------.--WDVDRL.HLTAEGHRRVAEAV    188
Scoe3 413 .-DPRRMRsDYDSGDHL.HPGDKGYARMGAVI    441
Scoe4 243 .-------.--FGPDNY.HPSAEGYATAAMAV    262
Scoe5 277 .-------.--SHWDWF.HPSVDGQARLAEIA    296
Ahyd1 292 rSASTLNCeGKMFWDQV.HPTTVVHAALSEPA    322
Asal1 292 rSASPLNCeGKMFWDQV.HPTTVVHAALSERA    322
Ahyd2 304 rSASPLNCeGKMFWDQV.HPTTVVHAALSERA    334
```

# Figure 34

```
                   ▼
Pfam        *->ivafGDSltdggg...............ayygdsdgggwgagladrltsla..rlrargrgvdv
Sriml  38      YVALGDSYSSGVG.............agSYDSSSGSCKRSTKSYPALWAAS..-----HTGTRF 81
Scoel   5      YVAVGDSFTEG--...............--VGDPGPDGAFVGWADRLAVLL..ADRRPEGDFTY 47
Scoe2  10      LVAVGDSFTEG--...............--MSDLLPDGSYRGWADLLATRM..--AARSPGFRY 50
Ahyd1  28      IVMFGDSLSDTGKmyskmrgylpssppyYEGRFSNGPVWLEQLTNEFPGLTiaNEAEGGPTAVA 91
Asal1  28      IVMFGDSLSDTGKmyskmrgylpssppyYEGRFSNGPVWLEQLTKQF----.------PGLTI 79
Ahyd2  40      IVMFGDSLSDTGKmyskmrgylpssppyYEGRFSNGPVWLEQLTKQFPGLTiaNEAEGGATAVA 103

Pfam        fnrgisGrtsdGrlvvDarlvatllFlaqflGlnlpPYLsgdflrGANFAsagAtIlgtslipflni
Sriml  82 NFTACSGAR----------------------------------------------------------- 90
Scoel  48 TNLAVRGRL----------------------------------------------------------- 56
Scoe2  51 ANLAVRGKL----------------------------------------------------------- 59
Ahyd1   92 YNKISWNPK----------------------------------------------------------- 100
Asal1   80 ANEAEGGAT----------------------------------------------------------- 88
Ahyd2 104 YNKISWNPK----------------------------------------------------------- 112

                                                                    ▼
Pfam        QvqFkdfkskvlelrqa......lgllqellrlvpvldakspdlvtimiGtNDl...itvakfgpks
Sriml  91 ----------------......---TGDVLAKQLTPVNSGTDLVSITIGGNDAgfaDTMTTCNLQG 131
Scoel  57 ----------------......--LDQIVAEQVPRVVGLAPDLVSFAAGGNDI...-----I---- 86
Scoe2  60 ----------------......--IGQIVDEQVDVAAAMGADVITLVGGLNDT...---------- 88
Ahyd1 101 ------------YQVI......NNLDYEVTQFLQKDSFKPDDLVILWVGANDY...--------LA 137
Asal1   89 -------AVAYNKISWNpkyqvyNNLDYEVTQFLQKDSFKPDDLVILWVGANDY...--------LA 137
Ahyd2 113 ------------YQVI......NNLDYEVTQFLQKDSFKPDDLVILWVGANDY...--------LA 149

Pfam        .......tksdrnvsvpefrdnlrklikrLrsangariiilitlvllnlplplGCl
Sriml 132 esaclarIAKARAYIQQTLPAQLDQVYDAIDSRAPAA-----QVVVLGYP------ 176
Scoel  87 .......---RPGTDPDEVAERFELAVAALT-AAAGTVLVTTGFDTRGVP------ 125
Scoe2  89 .......---------LRPKCDMARVRDLLTQAVERLAPHCEQLVLMRSP------ 122
Ahyd1 138 .......YGWNTEQDAKRVRDAISDAANRMV-LNGAK-----EILLFNLP------ 174
Asal1 138 .......YGWNTEQDAKRVRDAISDAANRMV-LNGAK-----QILLFNLP------ 174
Ahyd2 150 .......YGWNTEQDAKRVRDAISDAANRMV-LNGAK-----QILLFNLP------ 186

Pfam        pqklalalassknvdatgclerlneavadynealrelaeieklqaqlrkdglpdlkeanvpy
Sriml 177 --RFYKLGGSCAVGLSEKSRAAINAAADDINAVTAKRA----------------ADHGFAF 219
Scoel 126 ----------------VLKHLRGKIATYNGHVRAIA----------------DRYGCPV 152
Scoe2 123 -------------GRQGPVLERFRPRMEALFAVIDDLA----------------GRHGAVV 154
Ahyd1 175 ------DLGQNPSARSQKVVEAASHVSAYHNQLLLNLA------RQLAPTGMVKLFEIDKQF 224
Asal1 175 ------DLGQNPSARSQKVVEAVSHVSAYHNKLLLNLA------RQLAPTGMVKLFEIDKQF 224
Ahyd2 187 ------DLGQNPSARSQKVVEAVSHVSAYHNQLLLNLA------RQLAPTGMVKLFEIDKQF 236

Pfam        VDlysifqdldgiqnpsayv.y....GFeet.kaCCGyGgr.yNyn.rv.CGnag.l.ck.vtakaC
Sriml 220 GDVNT--------------.-....-----.---------.-TFAgHElCSGAPwL.HS.VT---- 242
Scoel 153 LDLWSLRSVQDRRA------.-....-----.---------.----.--.-----.-.--.------ 166
Scoe2 155 VDLYGAQSLADPRM------.-....-----.---------.----.--.-----.-.--.------ 168
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303

                   ▼
Pfam        .dassyll.atlfwDgf.HpsekGykavAeal<-*
Sriml 243 .-------.--LPVENSyHPTANGQSKGYLPV    263
Scoel 167 .-------.--WDADRL.HLSPEGHTRVALRA    186
Scoe2 169 .-------.--WDVDRL.HLTAEGHRRVAEAV    188
Ahyd1 292 rSASTLNCeGKMFWDQV.HPTTVVHAALSEPA    322
Asal1 292 rSASPLNCeGKMFWDQV.HPTTVVHAALSERA    322
Ahyd2 304 rSASPLNCeGKMFWDQV.HPTTVVHAALSERA    334
```

Figure 35

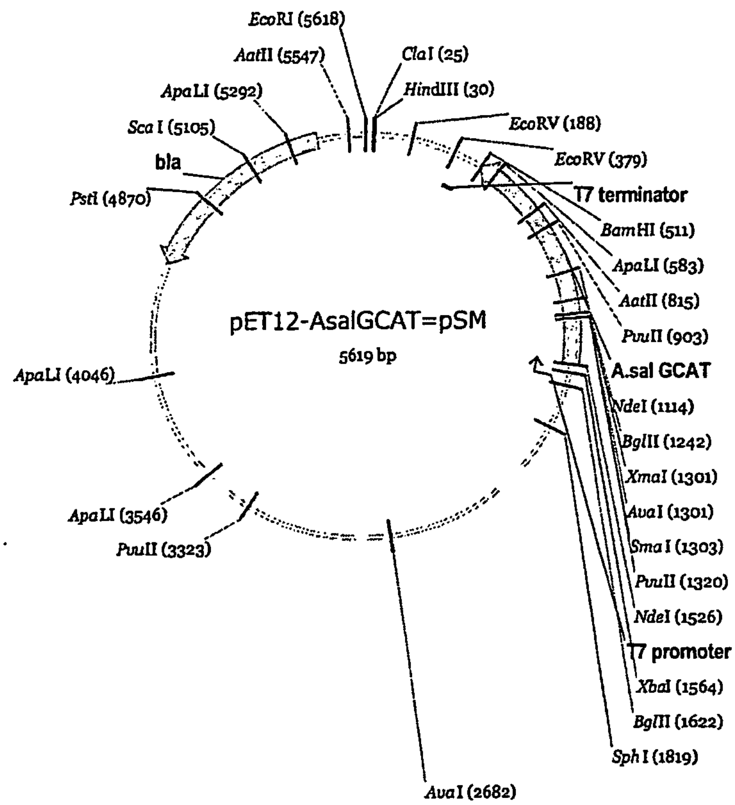

EcoRI (5618)
AatII (5547)
ClaI (25)
HindIII (30)
ApaLI (5292)
EcoRV (188)
ScaI (5105)
EcoRV (379)
bla
T7 terminator
PstI (4870)
BamHI (511)
ApaLI (583)
AatII (815)
pET12-AsalGCAT=pSM
PvuII (903)
5619 bp
A.sal GCAT
ApaLI (4046)
NdeI (1114)
BglII (1242)
XmaI (1301)
AvaI (1301)
ApaLI (3546)
SmaI (1303)
PvuII (3323)
PvuII (1320)
NdeI (1526)
T7 promoter
XbaI (1564)
BglII (1622)
SphI (1819)
AvaI (2682)

Figure 36

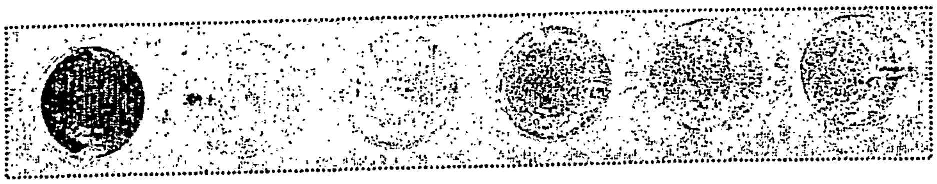

Figure 37

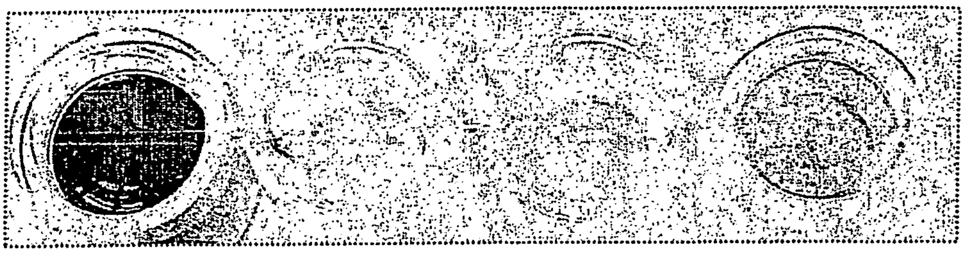

Figure 38

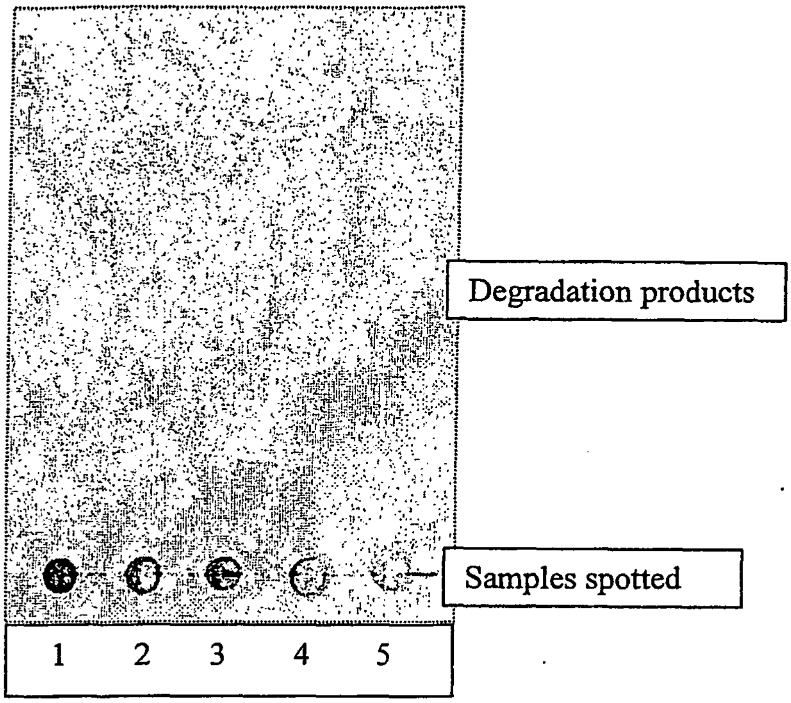

Figure 39

Controls

Pos Neg SE His SE His SE His SE His

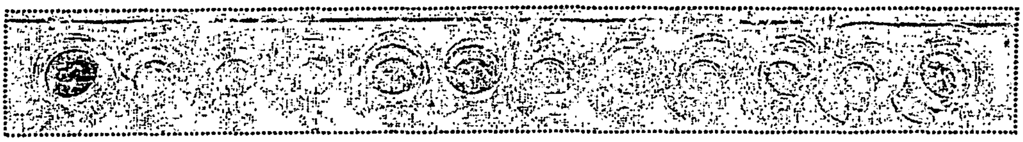

A. hydrophila enzyme

A. salmonicida enzyme

Figure 40.

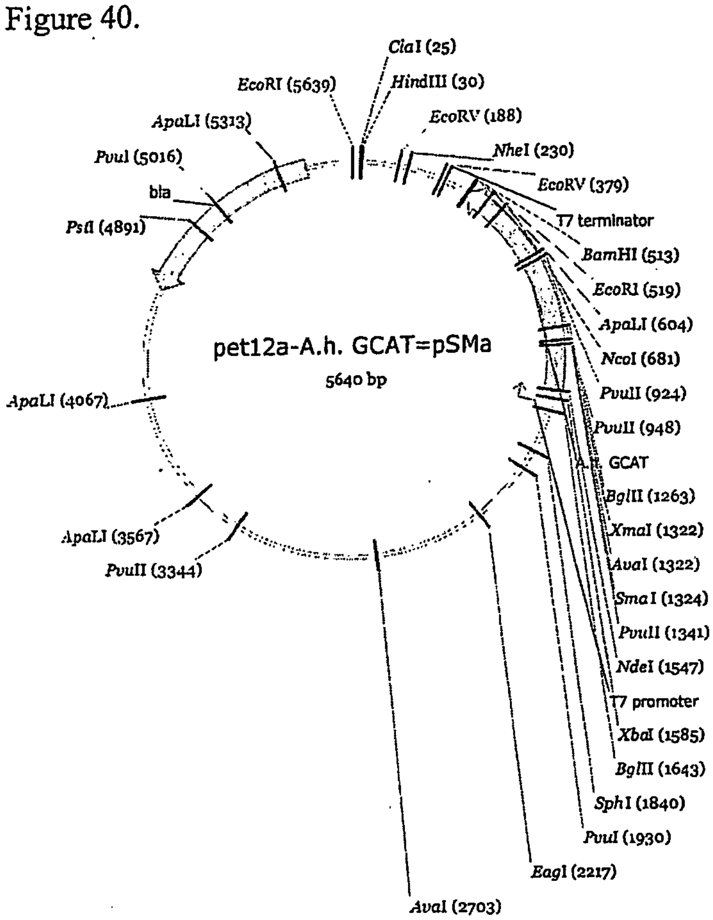
ClaI (25)
HindIII (30)
EcoRI (5639)
EcoRV (188)
ApaLI (5313)
NheI (230)
PvuI (5016)
EcoRV (379)
bla
T7 terminator
PstI (4891)
BamHI (513)
EcoRI (519)
ApaLI (604)
pet12a-A.h. GCAT=pSMa
5640 bp
NcoI (681)
PvuII (924)
ApaLI (4067)
PvuII (948)
A.h. GCAT
BglII (1263)
XmaI (1322)
ApaLI (3567)
AvaI (1322)
PvuII (3344)
SmaI (1324)
PvuII (1341)
NdeI (1547)
T7 promoter
XbaI (1585)
BglII (1643)
SphI (1840)
PvuI (1930)
EagI (2217)
AvaI (2703)

Figure 41:

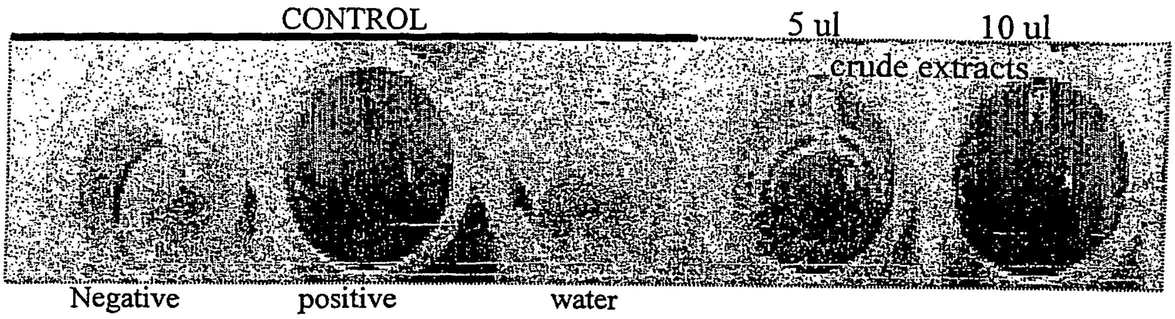

Figure 42

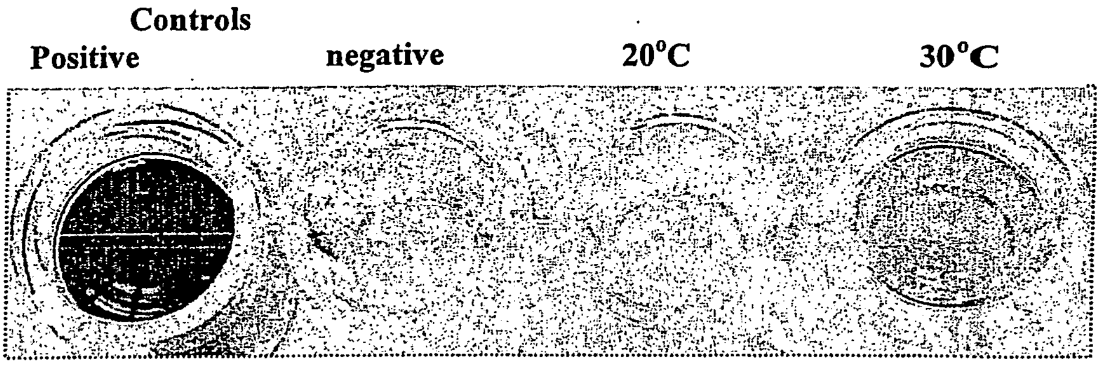

Figure 43

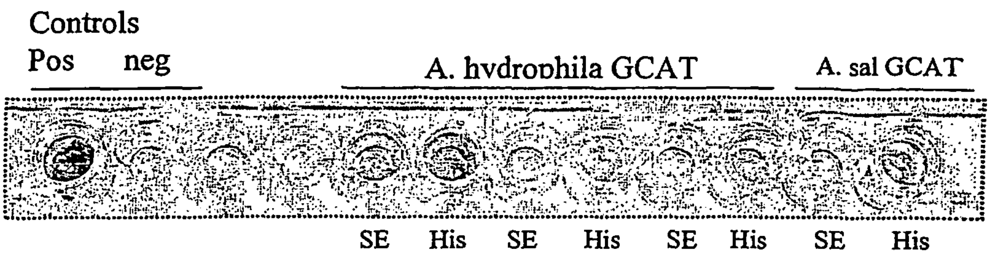
Controls
Pos neg
A. hydrophila GCAT
A. sal GCAT
SE His SE His SE His SE His

Figure 44

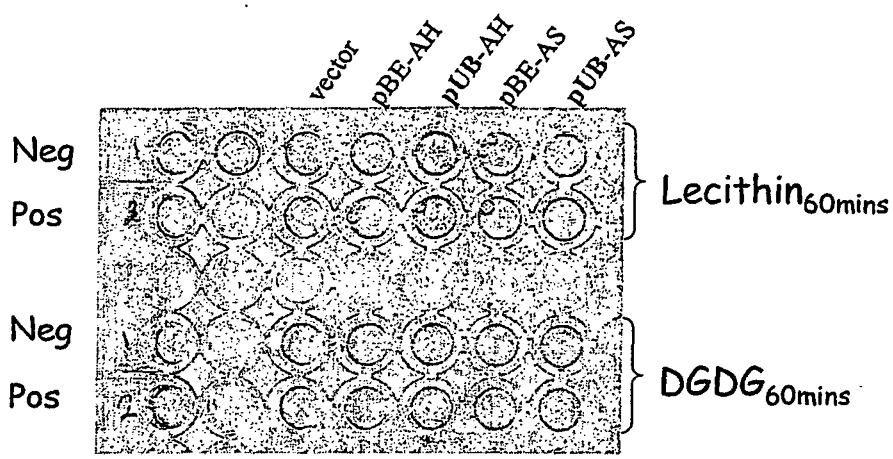
vector
pBE-AH
pUB-AH
pBE-AS
pUB-AS
Neg
Pos
Neg
Pos
Lecithin60mins
DGDG60mins

Figure 45

(SEQ ID No. 36)

```
  1  MFKFKKNFLV GLSAALMSIS LFSATASAAS ADSRPAFSRI VMFGDSLSDT
 51  GKMYSKMRGY LPSSPPYYEG RFSNGPVWLE QLTKQFPGLT IANEAEGGAT
101  AVAYNKISWN PKYQVINNLD YEVTQFLQKD SFKPDDLVIL WVGANDYLAY
151  GWNTEQDAKR VRDAISDAAN RMVLNGAKQI LLFNLPDLGQ NPSARSQKVV
201  EAVSHVSAYH NQLLLNLARQ LAPTGMVKLF EIDKQFAEML RDPQNFGLSD
251  VENPCYDGGY VWKPFATRSV STDRQLSAFS PQERLAIAGN PLLAQAVASP
301  MARRSASPLN CEGKMFWDQV HPTTVVHAAL SERAATFIAN QYEFLAH**
```

Figure 46 (SEQ ID No. 45)

1 ATGTTTAAGT TTAAAAAGAA TTTCTTAGTT GGATTATCGG CAGCTTTAAT
TACAAATTCA AATTTTTCTT AAAGAATCAA CCTAATAGCC GTCGAAATTA

51 GAGTATTAGC TTGTTTTCGG CAACCGCCTC TGCAGCTAGC GCCGACAGCC
CTCATAATCG AACAAAAGCC GTTGGCGGAG ACGTCGATCG CGGCTGTCGG

101 GTCCCGCCTT TTCCCGGATC GTGATGTTCG GCGACAGCCT CTCCGATACC
CAGGGCGGAA AAGGGCCTAG CACTACAAGC CGCTGTCGGA GAGGCTATGG

151 GGCAAAATGT ACAGCAAGAT GCGCGGTTAC CTCCCCTCCA GCCCGCCCTA
CCGTTTTACA TGTCGTTCTA CGCGCCAATG GAGGGGAGGT CGGGCGGGAT

201 CTATGAGGGC CGTTTCTCCA ACGGACCCGT CTGGCTGGAG CAGCTGACCA
GATACTCCCG GCAAAGAGGT TGCCTGGGCA GACCGACCTC GTCGACTGGT

251 AACAGTTCCC GGGTCTGACC ATCGCCAACG AAGCGGAAGG CGGTGCCACT
TTGTCAAGGG CCCAGACTGG TAGCGGTTGC TTCGCCTTCC GCCACGGTGA

301 GCCGTGGCTT ACAACAAGAT CTCCTGGAAT CCCAAGTATC AGGTCATCAA
CGGCACCGAA TGTTGTTCTA GAGGACCTTA GGGTTCATAG TCCAGTAGTT

351 CAACCTGGAC TACGAGGTCA CCCAGTTCTT GCAGAAAGAC AGCTTCAAGC
GTTGGACCTG ATGCTCCAGT GGGTCAAGAA CGTCTTTCTG TCGAAGTTCG

401 CGGACGATCT GGTGATCCTC TGGGTCGGTG CCAATGACTA TCTGGCCTAT
GCCTGCTAGA CCACTAGGAG ACCCAGCCAC GGTTACTGAT AGACCGGATA

451 GGCTGGAACA CGGAGCAGGA TGCCAAGCGG GTTCGCGATG CCATCAGCGA
CCGACCTTGT GCCTCGTCCT ACGGTTCGCC CAAGCGCTAC GGTAGTCGCT

501 TGCGGCCAAC CGCATGGTAC TGAACGGTGC CAAGCAGATA CTGCTGTTCA
ACGCCGGTTG GCGTACCATG ACTTGCCACG GTTCGTCTAT GACGACAAGT

551 ACCTGCCGGA TCTGGGCCAG AACCCGTCAG CTCGCAGTCA GAAGGTGGTC
TGGACGGCCT AGACCCGGTC TTGGGCAGTC GAGCGTCAGT CTTCCACCAG

601 GAGGCGGTCA GCCATGTCTC CGCCTATCAC AACCAGCTGC TGCTGAACCT
CTCCGCCAGT CGGTACAGAG GCGGATAGTG TTGGTCGACG ACGACTTGGA

651 GGCACGCCAG CTGGCCCCCA CCGGCATGGT AAAGCTGTTC GAGATCGACA
CCGTGCGGTC GACCGGGGGT GGCCGTACCA TTTCGACAAG CTCTAGCTGT

701 AGCAATTTGC CGAGATGCTG CGTGATCCGC AGAACTTCGG CCTGAGCGAC
TCGTTAAACG GCTCTACGAC GCACTAGGCG TCTTGAAGCC GGACTCGCTG

751 GTCGAGAACC CCTGCTACGA CGGCGGCTAT GTGTGGAAGC CGTTTGCCAC
CAGCTCTTGG GGACGATGCT GCCGCCGATA CACACCTTCG GCAAACGGTG

801 CCGCAGCGTC AGCACCGACC GCCAGCTCTC CGCCTTCAGT CCGCAGGAAC
GGCGTCGCAG TCGTGGCTGG CGGTCGAGAG GCGGAAGTCA GGCGTCCTTG

851 GCCTCGCCAT CGCCGGCAAC CCGCTGCTGG CACAGGCCGT TGCCAGTCCT
CGGAGCGGTA GCGGCCGTTG GGCGACGACC GTGTCCGGCA ACGGTCAGGA

901 ATGGCCCGCC GCAGCGCCAG CCCCCTCAAC TGTGAGGGCA AGATGTTCTG
TACCGGGCGG CGTCGCGGTC GGGGGAGTTG ACACTCCCGT TCTACAAGAC

951 GGATCAGGTA CACCCGACCA CTGTCGTGCA CGCAGCCCTG AGCGAGCGCG
CCTAGTCCAT GTGGGCTGGT GACAGCACGT GCGTCGGGAC TCGCTCGCGC

1001 CCGCCACCTT CATCGCGAAC CAGTACGAGT TCCTCGCCCA CTGATGA
GGCGGTGGAA GTAGCGCTTG GTCATGCTCA AGGAGCGGGT GACTACT

Figure 47

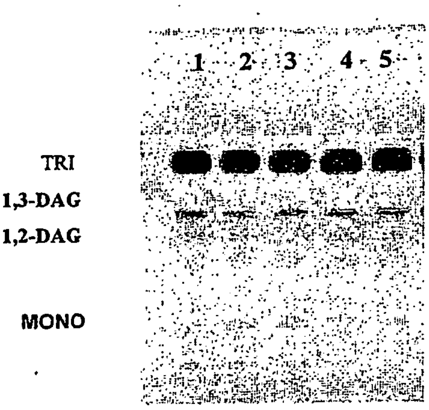


Run order: #196

Palm oil (5% GL)

Lane (5% GL:0% $H_2O$)

Lane (5% GL:1% $H_2O$)

Lane (5% GL:5% $H_2O$)

Lane (10% GL:1% $H_2O$)

NB: 1 : Reference

2-5: With Transferase #196

Figure 48

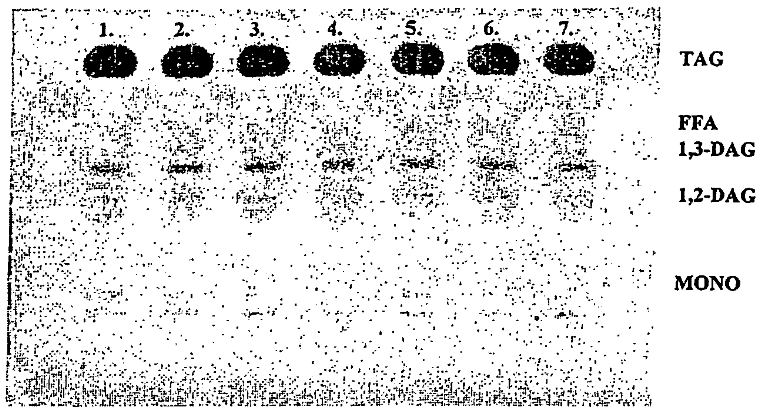


Run order: #196

Lane (5% GL : 1% $H_2O$)

Lane (5% GL : 5% $H_2O$)

Lane (5% GL : 7,5% $H_2O$)

Lane (Reference: 5% GL)

Lane (10% GL : 1% $H_2O$)

Lane (10% GL : 5% $H_2O$)

Lane (10% GL : 7,5% $H_2O$)

Figure 49

**Standard curve - Yield of monoglycerides**

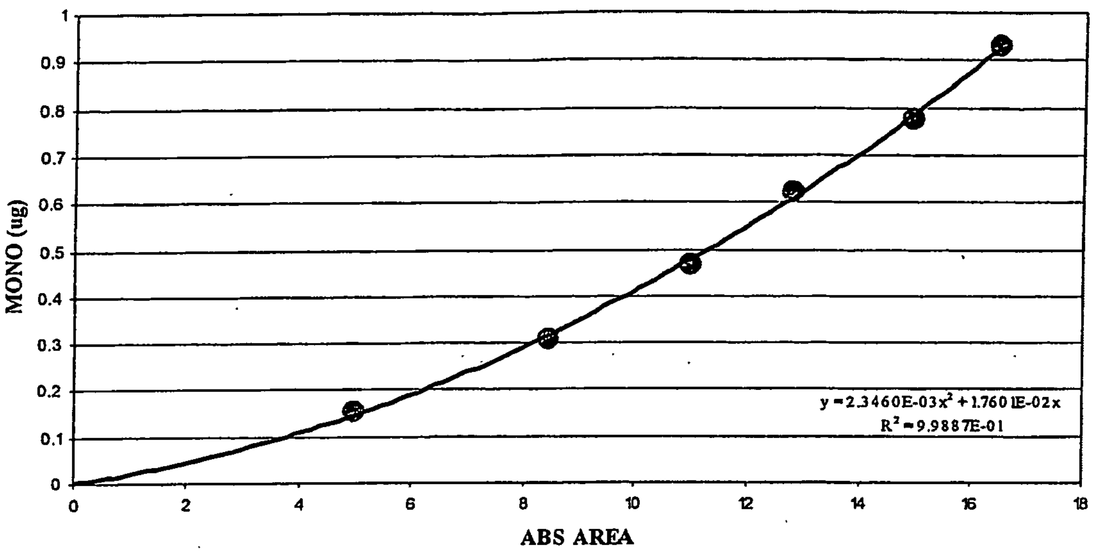

Figure 50

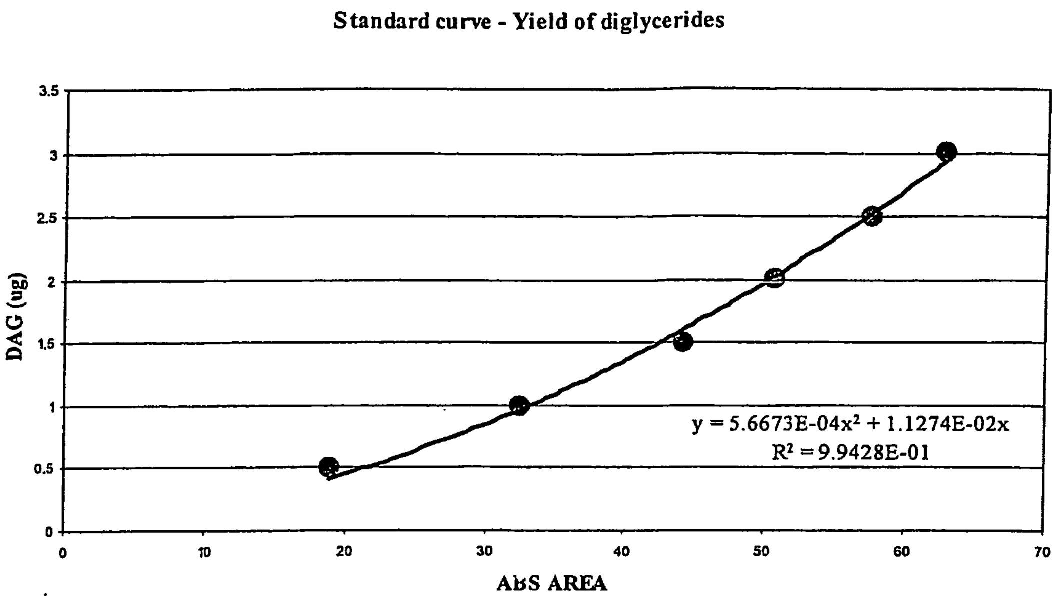

Standard curve - Yield of diglycerides
DAG (ug)
ABS AREA
3.5
3
2.5
2
1.5
1
0.5
0
0
10
20
30
40
50
60
70
y = 5.6673E-04x² + 1.1274E-02x
R² = 9.9428E-01

Figure 51

SEQ ID NO. 54:

ACAGGCCGATGCACGGAACCGTACCTTTCCGCAGTGAAGCGCTCTCCCCCCATCGTTCGC
CGGGACTTCATCCGCGATTTTGGCATGAACACTTCCTTCAACGCGCGTAGCTTGCTACAA
GTGCGGCAGCAGACCCGCTCGTTGGAGGCTCAGTGAGATTGACCCGATCCCTGTCGGCCG
CATCCGTCATCGTCTTCGCCCTGCTGCTCGCGCTGCTGGGCATCAGCCCGGCCCAGGCAG
CCGGCCCGGCCTATGTGGCCCTGGGGGATTCCTATTCCTCGGGCAACGGCGCCGGAAGTT
ACATCGATTCGAGCGGTGACTGTCACCGCAGCAACAACGCGTACCCCGCCCGCTGGGCGG
CGGCCAACGCACCGTCCTCCTTCACCTTCGCGGCCTGCTCGGGAGCGGTGACCACGGATG
TGATCAACAATCAGCTGGGCGCCCTCAACGCGTCCACCGGCCTGGTGAGCATCACCATCG
GCGGCAATGACGCGGGCTTCGCGGACGCGATGACCACCTGCGTCACCAGCTCGGACAGCA
CCTGCCTCAACCGGCTGGCCACCGCCACCAACTACATCAACACCACCCTGCTCGCCCGGC
TCGACGCGGTCTACAGCCAGATCAAGGCCCGTGCCCCCAACGCCCGCGTGGTCGTCCTCG
GCTACCCGCGCATGTACCTGGCCTCGAACCCCTGGTACTGCCTGGGCCTGAGCAACACCA
AGCGCGCGGCCATCAACACCACCGCCGACACCCTCAACTCGGTGATCTCCTCCCGGGCCA
CCGCCCACGGATTCCGATTCGGCGATGTCCGCCCGACCTTCAACAACCACGAACTGTTCT
TCGGCAACGACTGGCTGCACTCACTCACCCTGCCGGTGTGGGAGTCGTACCACCCCACCA
GCACGGGCCATCAGAGCGGCTATCTGCCGGTCCTCAACGCCAACAGCTCGACCTGATCAA
CGCACGGCCGTGCCCGCCCCGCGCGTCACGCTCGGCGCGGGCGCCGCAGCGCGTTGATCA
GCCCACAGTGCCGGTGACGGTCCCACCGTCACGGTCGAGGGTGTACGTCACGGTGGCGCC
GCTCCAGAAGTGGAACGTCAGCAGGACCGTGGAGCCGTCCCTGACCTCGTCGAAGAACTC
CGGGGTCAGCGTGATCACCCCTCCCCCGTAGCCGGGGGCGAAGGCGGCGCCGAACTCCTT
GTAGGACGTCCAGTCGTGCGGCCCGGCGTTGCCACCGTCCGCGTAGACCGCTTCCATGGT
CGCCAGCCGGTCCCCGCGGAACTCGGTGGGATGTCCGTGCCCAAGGTGGTCCCGGTGGT
GTCCGAGAGCACCGGGGGCTCGTACCGGATGATGTGCAGATCCAAAGAATT

FIGURE 52

SEQ ID NO. 55:

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0558112 A
- JP 62061590 B
- EP 0652289 A
- WO 0005396 A
- WO 200036114 A
- US 20030028923 A
- US 20030074695 A
- WO 03100044 A
- EP 0746608 A
- US 5776741 A
- US 4798793 A
- US 5156963 A
- US 6284501 B
- EP 0293194 A
- US 4683202 A
- EP 0583265 A
- EP 0866796 A
- WO 0206457 A
- EP 0752008 A
- EP 1138763 A
- EP 1103606 A
- US 6180406 B
- WO 0134835 A
- WO 0058517 A
- US 6344328 B
- US 6361974 B
- WO 9117243 A
- EP 0238023 A
- EP 0449375 A
- US 5741665 A
- US 5674707 A
- WO 0139544 A
- WO 0116308 A
- US 3817837 A
- US 3850752 A
- US 3939350 A
- US 3996345 A
- US 4277437 A
- US 4275149 A
- US 4366241 A
- US 4816567 A
- GB 0330016 A
- GB 0416023 A
- US 10898775 B


### Non-patent literature cited in the description


- Enzyme Nomenclature Recommendations. *Nomenclature Committee of the International Union of Biochemistry and Molecular Biology,* 1992
- **BRUMLIK ; BUCKLEY.** *Journal of Bacteriology,* April 1996, vol. 178 (7), 2060-2064
- **BATEMAN A et al.** *Nucleic Acids Res.,* 2002, vol. 30, 276-280
- **BATEMAN A ; HAFT DH.** *Brief Bioinform,* 2002, vol. 3, 236-2 45
- **NEEDLEMAN ; WUNSCH.** *J. of Molecular Biology,* 1970, vol. 48, 443-45
- **BALCAO V.M. ; PAIVA A.L. ; MALCATA F.X.** *Enzyme Microb Technol.,* 01 May 1996, vol. 18 (6), 392-416
- **RETZ M.T. ; JAEGER K.E.** *Chem Phys Lipids.,* June 1998, vol. 93 (1-2), 3-14
- **BORNSCHEUER U.T. ; BESSLER C ; SRINIVAS R ; KRISHNA S.H.** *Trends Biotechnol.,* October 2002, vol. 20 (10), 433-7
- **PLOU et al.** *J. Biotechnology,* 2002, vol. 92, 55-66
- **WARMUTH et al.** *Bio Forum,* 1992, vol. 9, 282-283
- **FERRER et al.** *J. Chem. Technol. Biotechnol.,* 2000, vol. 75, 1-8
- **CHRISTENSEN et al.** *Nachwachsende Rohstoff,* 1998, vol. 10, 98-105
- **PETERSEN ; CHRISTENEN.** Applied Biocatalysis. arwood Academic Publishers, 2000
- **BEUCAGE S.L. et al.** *Tetrahedron Letters,* 1981, vol. 22, 1859-1869
- **MATTHES et al.** *EMBO J.,* 1984, vol. 3, 801-805
- **SAIKI R K et al.** *Science,* 1988, vol. 239, 487-491
- **CARUTHERS MH et al.** *Nuc Acids Res Symp Ser,* 1980, 215-23
- **HORN T et al.** *Nuc Acids Res Symp Ser,* 1980, 225-232
- **MORINAGA et al.** *Biotechnology,* 1984, vol. 2, 646-649
- **NELSON ; LONG.** *Analytical Biochemistry,* 1989, vol. 180, 147-151
- **HILTON ; BUCKLEY.** *J Biol. Chem.,* 15 January 1991, vol. 266 (2), 997-1000

- **ROBERTSON et al.** *J. Biol. Chem.,* 21 January 1994, vol. 269 (3), 2146-50
- **BRUMLIK et al.** *J. Bacteriol,* April 1996, vol. 178 (7), 2060-4
- **PEELMAN et al.** *Protein Sci.,* March 1998, vol. 7 (3), 587-99
- **DEVEREUX et al.** *Nuc. Acids Research,* 1984, vol. 12, 387
- **AUSUBEL et al.** Short Protocols in Molecular Biology. 1999
- **ALTSCHUL et al.** *J. Mol. Biol.,* 1990, 403-410
- *FEMS Microbiol Lett,* 1999, vol. 174 (2), 247-50
- *FEMS Microbiol Lett,* 1999, vol. 177 (1), 187-8
- **HIGGINS DG ; SHARP PM.** *Gene,* 1988, vol. 73 (1), 237-244
- **SIMON RJ et al.** *PNAS,* 1992, vol. 89 (20), 9367-9371
- **HORWELL DC.** *Trends Biotechnol.,* 1995, vol. 13 (4), 132-134
- Guide to Molecular Cloning Techniques. **BERGER ; KIMMEL.** Methods in Enzymology. Academic Press, 1987, vol. 152
- **SAMBROOK et al.** Molecular Cloning: A Laboratory Manual. Cold Spring Harbor Laboratory Press, 1989
- **POTRYKUS.** *Annu Rev Plant Physiol Plant Mol Biol,* 1991, vol. 42, 205-225
- **DAVIS ; DE SERRES.** *Methods Enzymol,* 1971, vol. 17A, 79-143
- Aspergillus: 50 years on. Progress in industrial microbiology. Elsevier, 1994, vol. 29, 641-666
- **PUNT et al.** *Trends Biotechnol,* May 2002, vol. 20 (5), 200-6
- **ARCHER ; PEBERDY.** *Crit Rev Biotechnol,* 1997, vol. 17 (4), 273-306
- *Methods Mol Biol,* 1995, vol. 49, 341-54
- *Curr Opin Biotechnol,* October 1997, vol. 8 (5), 554-60
- *FEMS Microbiol Rev,* 2000, vol. 24 (1), 45-66
- Yeast as a vehicle for the expression, of heterologous genes. **E HINCHCLIFFE ; E KENNY.** Yeasts. Academic Press. Ltd, 1993, vol. 5
- **HINNEN et al.** *Proceedings of the National Academy of Sciences of the USA,* 1978, vol. 75, 1929
- **BEGGS, J D.** *Nature,* 1978, vol. 275, 104
- **ITO, H et al.** *J Bacteriology,* 1983, vol. 153, 163-168
- **CHRISTOU.** *Agro-Food-Industry Hi-Tech,* March 1994, 17-27
- *Curr. Opin. Biotechnol.,* 1995, vol. 6 (5), 501-6
- **UPTON C ; BUCKLEY JT.** *Trends Biochem,* 1995, vol. 20, 179-179
- **UPTON C ; BUCKLEY JT.** *Trends Biochem Sci,* 1995, vol. 20, 179-179
- **BERGER, K. G.** Recent developments in palm oil. *Oleagineux,* 1990, vol. 45, 437-443
- **DROZDOWSKI, B.** *General characteristics of eatable fats.,* 1994, 240-243
- **HERNQUIST, L. ; ANJOU, K.** Diglycerides as a stabilizer of the β'-crystal Form in Margarines and Fats. *Fette Seifen Anstrichmittel.,* 1983, vol. 2, 64-66
- **HERNQUIST, L. ; HERSLOF, B. ; LARSSON, K. ; PODLAHA, O.** Polymorphism of rapeseed oil with low content of erucic acid and possibilities to stabilize the β' crystal form in fats. *Journal of Science and Food Agriculture.,* 1981, vol. 32, 1197-1202
- **JACOBSBERG, B. ; OH, C.H.** Studies in Palm Oil Crystallisation. *Journal of the American Oil Chemist Society.,* 1976, vol. 53, 609-616
- **KRISTENSEN, A. C. J.** Preparation of margarine and spreads by enzyme-generated emulsifiers. *Master thesis, The Royal Veterinary and Agricultural University,* 2004
- **MCNEILL, G. P. ; BERGER, R. G.** Enzymatic glycerolysis of palm oil fractions and palm oil based model mixture: Relationship between fatty acid composition and monoglyceride yield. *Food Biotechnology,* 1993, vol. 7, 75-87
- **OKIY, D. A.** Partial glycerides and palm oil Crystallisation. *Journal of Science and Food Agriculture.,* 1977, vol. 28, 955
- **OKIY, D. A.** Interaction of triglycerides and diglycerides of palm oil. *Oleagineux,* 1978, vol. 33, 625-628
- **OKIY, D. A. ; WRIGHT, W. B. ; BERGER, K. G. ; MORTON, I. D.** The physical properties of modified palm oil. *Journal of Science of Food and Agriculture,* 1978, vol. 29, 625-628
- **WALNETT, S. V. ; MEUSEL, D. ; TÜLSNER, M.** Zur kenntnis des diglyceride influsses auf das kristallisationsverhalten von Fetten. *Fat Science Technology,* 1991, vol. 4, 117-121
- **SIEW, N. L.** Understanding the Interactions of Diacylglycerols with oil for better Product performance. *Paper presented at the 2001 PIPOC International Palm Oil Congress - Chemistry and Technology Conference,* 2001
- **SIEW, N. L. ; NG, W. L.** Differential scanning thermograms of palm oil triglycerides in the presence of diglycerides. *Journal of Oil Palm Research,* 2000, vol. 12, 1-7
- **SIEW, N. L. ; NG, W. L.** Influence of diglycerides on crystalisation of palm oil. *Journal of Science of Food and Agriculture,* 1999, vol. 79, 722-726
- **SONNTAG, N. O. V.** Fat splitting, esterification and interesterification. *Bailey's Industrial Oils and Fat products,* 1982, vol. 2, 97-173
- **SONNTAG, N. O. V.** Glycerolysis of Fats and methyl esters - status, review and critique. *Journal of American Oil Chemist Society,* 1982, vol. 59, 795A-802A
- **TIMMS, R.** *Oral presentation (Lecture: Trends & delvlopment) at Danisco A/S,* 2004

SEQUENCE LISTING

<110> Danisco A/S

<120> Method

<130> P021904WO

<140> PCT/IB2004/004374

<141> 2004-12-23

<150> GB0330016.7
<151> 2003-12-24

<150> PCT/IB04/00655
<151> 2004-01-15

<150> GB0416023.0
<151> 2004-07-16

<150> US10/898775
<151> 2004-07-26

<160> 70

<170> PatentIn version 3.1

<210> 1
<211> 361
<212> PRT
<213> Artificial pfam00567 consensus sequence

<400> 1

```
Ile Val Ala Phe Gly Asp Ser Leu Thr Asp Gly Glu Ala Tyr Tyr Gly
1               5                   10                  15
```

Asp Ser Asp Gly Gly Gly Trp Gly Ala Gly Leu Ala Asp Arg Leu Thr
20 25 30

Ala Leu Leu Arg Leu Arg Ala Arg Pro Arg Gly Val Asp Val Phe Asn
35 40 45

Arg Gly Ile Ser Gly Arg Thr Ser Asp Gly Arg Leu Ile Val Asp Ala
50 55 60

---

Leu Val Ala Leu Leu Phe Leu Ala Gln Ser Leu Gly Leu Pro Asn Leu
65 70 75 80

Pro Pro Tyr Leu Ser Gly Asp Phe Leu Arg Gly Ala Asn Phe Ala Ser
85 90 95

Ala Gly Ala Thr Ile Leu Pro Thr Ser Gly Pro Phe Leu Ile Gln Val
100 105 110

Gln Phe Lys Asp Phe Lys Ser Gln Val Leu Glu Leu Arg Gln Ala Leu
115 120 125

Gly Leu Leu Gln Glu Leu Leu Arg Leu Leu Pro Val Leu Asp Ala Lys
130 135 140

Ser Pro Asp Leu Val Thr Ile Met Ile Gly Thr Asn Asp Leu Ile Thr
145 150 155 160

Ser Ala Phe Phe Gly Pro Lys Ser Thr Glu Ser Asp Arg Asn Val Ser
165 170 175

Val Pro Glu Phe Lys Asp Asn Leu Arg Gln Leu Ile Lys Arg Leu Arg
180 185 190

Ser Asn Asn Gly Ala Arg Ile Ile Val Leu Ile Thr Leu Val Ile Leu
195 200 205

Asn Leu Gly Pro Leu Gly Cys Leu Pro Leu Lys Leu Ala Leu Ala Leu
210 215 220

Ala Ser Ser Lys Asn Val Asp Ala Ser Gly Cys Leu Glu Arg Leu Asn
225 230 235 240

Glu Ala Val Ala Asp Phe Asn Glu Ala Leu Arg Glu Leu Ala Ile Ser
245 250 255

Lys Leu Glu Asp Gln Leu Arg Lys Asp Gly Leu Pro Asp Val Lys Gly
260 265 270

Ala Asp Val Pro Tyr Val Asp Leu Tyr Ser Ile Phe Gln Asp Leu Asp
275 280 285

Gly Ile Gln Asn Pro Ser Ala Tyr Val Tyr Gly Phe Glu Thr Thr Lys
    290                 295                 300

Ala Cys Cys Gly Tyr Gly Gly Arg Tyr Asn Tyr Asn Arg Val Cys Gly
305                 310                 315                 320

Asn Ala Gly Leu Cys Asn Val Thr Ala Lys Ala Cys Asn Pro Ser Ser
                325                 330                 335

Tyr Leu Leu Ser Phe Leu Phe Trp Asp Gly Phe His Pro Ser Glu Lys
            340                 345                 350

Gly Tyr Lys Ala Val Ala Glu Ala Leu
        355                 360

<210> 2
<211> 335
<212> PRT
<213> Aeromonas hydrophila

<400> 2

Met Lys Lys Trp Phe Val Cys Leu Leu Gly Leu Val Ala Leu Thr Val
1               5                   10                  15

Gln Ala Ala Asp Ser Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Asn Glu Phe Pro Gly Leu Thr Ile Ala
65                  70                  75                  80

Asn Glu Ala Glu Gly Gly Pro Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145 150 155 160

Val Leu Asn Gly Ala Lys Glu Ile Leu Leu Phe Asn Leu Pro Asp Leu
165 170 175

Gly Gln Asn Pro Ser Ala Arg Ser Gln Lys Val Val Glu Ala Ala Ser
180 185 190

---

His Val Ser Ala Tyr His Asn Gln Leu Leu Leu Asn Leu Ala Arg Gln
195 200 205

Leu Ala Pro Thr Gly Met Val Lys Leu Phe Glu Ile Asp Lys Gln Phe
210 215 220

Ala Glu Met Leu Arg Asp Pro Gln Asn Phe Gly Leu Ser Asp Gln Arg
225 230 235 240

Asn Ala Cys Tyr Gly Gly Ser Tyr Val Trp Lys Pro Phe Ala Ser Arg
245 250 255

Ser Ala Ser Thr Asp Ser Gln Leu Ser Ala Phe Asn Pro Gln Glu Arg
260 265 270

Leu Ala Ile Ala Gly Asn Pro Leu Leu Ala Gln Ala Val Ala Ser Pro
275 280 285

Met Ala Ala Arg Ser Ala Ser Thr Leu Asn Cys Glu Gly Lys Met Phe
290 295 300

Trp Asp Gln Val His Pro Thr Thr Val Val His Ala Ala Leu Ser Glu
305 310 315 320

Pro Ala Ala Thr Phe Ile Glu Ser Gln Tyr Glu Phe Leu Ala His
325 330 335

<210> 3
<211> 336
<212> PRT
<213> Aeromonas salmonicida

<400> 3

Met Lys Lys Trp Phe Val Cys Leu Leu Gly Leu Ile Ala Leu Thr Val
1 5 10 15

Gln Ala Ala Asp Thr Arg Pro Ala Phe Ser Arg Ile Val Met Phe Gly
20 25 30

```
Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Tyr Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175

Gly Gln Asn Pro Ser Ala Arg Ser Gln Lys Val Val Glu Ala Val Ser
            180                 185                 190

His Val Ser Ala Tyr His Asn Lys Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Val Glu
225                 230                 235                 240

Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300
```

Trp Asp Gln Val His Pro Thr Thr Val Val His Ala Ala Leu Ser Glu
305 310 315 320

Arg Ala Ala Thr Phe Ile Glu Thr Gln Tyr Glu Phe Leu Ala His Gly
325 330 335

<210> 4
<211> 295
<212> PRT
<213> Streptomyces coelicolor

<400> 4

Met Pro Lys Pro Ala Leu Arg Arg Val Met Thr Ala Thr Val Ala Ala
1 5 10 15

Val Gly Thr Leu Ala Leu Gly Leu Thr Asp Ala Thr Ala His Ala Ala
20 25 30

Pro Ala Gln Ala Thr Pro Thr Leu Asp Tyr Val Ala Leu Gly Asp Ser
35 40 45

Tyr Ser Ala Gly Ser Gly Val Leu Pro Val Asp Pro Ala Asn Leu Leu
50 55 60

Cys Leu Arg Ser Thr Ala Asn Tyr Pro His Val Ile Ala Asp Thr Thr
65 70 75 80

Gly Ala Arg Leu Thr Asp Val Thr Cys Gly Ala Ala Gln Thr Ala Asp
85 90 95

Phe Thr Arg Ala Gln Tyr Pro Gly Val Ala Pro Gln Leu Asp Ala Leu
100 105 110

Gly Thr Gly Thr Asp Leu Val Thr Leu Thr Ile Gly Gly Asn Asp Asn
115 120 125

Ser Thr Phe Ile Asn Ala Ile Thr Ala Cys Gly Thr Ala Gly Val Leu
130 135 140

Ser Gly Gly Lys Gly Ser Pro Cys Lys Asp Arg His Gly Thr Ser Phe
145 150 155 160

Asp Asp Glu Ile Glu Ala Asn Thr Tyr Pro Ala Leu Lys Glu Ala Leu
165 170 175

Leu Gly Val Arg Ala Arg Ala Pro His Ala Arg Val Ala Ala Leu Gly
180 185 190

Tyr Pro Trp Ile Thr Pro Ala Thr Ala Asp Pro Ser Cys Phe Leu Lys
195 200 205

Leu Pro Leu Ala Ala Gly Asp Val Pro Tyr Leu Arg Ala Ile Gln Ala
210 215 220

His Leu Asn Asp Ala Val Arg Arg Ala Ala Glu Glu Thr Gly Ala Thr
225 230 235 240

Tyr Val Asp Phe Ser Gly Val Ser Asp Gly His Asp Ala Cys Glu Ala
245 250 255

Pro Gly Thr Arg Trp Ile Glu Pro Leu Leu Phe Gly His Ser Leu Val
260 265 270

Pro Val His Pro Asn Ala Leu Gly Glu Arg Arg Met Ala Glu His Thr
275 280 285

Met Asp Val Leu Gly Leu Asp
290 295

<210> 5
<211> 295
<212> PRT
<213> Streptomyces coelicolor

<400> 5

Met Pro Lys Pro Ala Leu Arg Arg Val Met Thr Ala Thr Val Ala Ala
1 5 10 15

Val Gly Thr Leu Ala Leu Gly Leu Thr Asp Ala Thr Ala His Ala Ala
20 25 30

Pro Ala Gln Ala Thr Pro Thr Leu Asp Tyr Val Ala Leu Gly Asp Ser
35 40 45

Tyr Ser Ala Gly Ser Gly Val Leu Pro Val Asp Pro Ala Asn Leu Leu
50 55 60

Cys Leu Arg Ser Thr Ala Asn Tyr Pro His Val Ile Ala Asp Thr Thr
65 70 75 80

Gly Ala Arg Leu Thr Asp Val Thr Cys Gly Ala Ala Gln Thr Ala Asp
85 90 95

Phe Thr Arg Ala Gln Tyr Pro Gly Val Ala Pro Gln Leu Asp Ala Leu
100 105 110

Gly Thr Gly Thr Asp Leu Val Thr Leu Thr Ile Gly Gly Asn Asp Asn
115 120 125

Ser Thr Phe Ile Asn Ala Ile Thr Ala Cys Gly Thr Ala Gly Val Leu
130 135 140

Ser Gly Gly Lys Gly Ser Pro Cys Lys Asp Arg His Gly Thr Ser Phe
145 150 155 160

---

Asp Asp Glu Ile Glu Ala Asn Thr Tyr Pro Ala Leu Lys Glu Ala Leu
165 170 175

Leu Gly Val Arg Ala Arg Ala Pro His Ala Arg Val Ala Ala Leu Gly
180 185 190

Tyr Pro Trp Ile Thr Pro Ala Thr Ala Asp Pro Ser Cys Phe Leu Lys
195 200 205

Leu Pro Leu Ala Ala Gly Asp Val Pro Tyr Leu Arg Ala Ile Gln Ala
210 215 220

His Leu Asn Asp Ala Val Arg Arg Ala Ala Glu Glu Thr Gly Ala Thr
225 230 235 240

Tyr Val Asp Phe Ser Gly Val Ser Asp Gly His Asp Ala Cys Glu Ala
245 250 255

Pro Gly Thr Arg Trp Ile Glu Pro Leu Leu Phe Gly His Ser Leu Val
260 265 270

Pro Val His Pro Asn Ala Leu Gly Glu Arg Arg Met Ala Glu His Thr
275 280 285

Met Asp Val Leu Gly Leu Asp
290 295

<210> 6
<211> 238
<212> PRT
<213> Saccharomyces cerevisiae

<400> 6

Met Asp Tyr Glu Lys Phe Leu Leu Phe Gly Asp Ser Ile Thr Glu Phe
1 5 10 15

Ala Phe Asn Thr Arg Pro Ile Glu Asp Gly Lys Asp Gln Tyr Ala Leu
20 25 30

Gly Ala Ala Leu Val Asn Glu Tyr Thr Arg Lys Met Asp Ile Leu Gln
35 40 45

Arg Gly Phe Lys Gly Tyr Thr Ser Arg Trp Ala Leu Lys Ile Leu Pro
50 55 60

Glu Ile Leu Lys His Glu Ser Asn Ile Val Met Ala Thr Ile Phe Leu
65 70 75 80

Gly Ala Asn Asp Ala Cys Ser Ala Gly Pro Gln Ser Val Pro Leu Pro
85 90 95

Glu Phe Ile Asp Asn Ile Arg Gln Met Val Ser Leu Met Lys Ser Tyr
100 105 110

His Ile Arg Pro Ile Ile Ile Gly Pro Gly Leu Val Asp Arg Glu Lys
115 120 125

Trp Glu Lys Glu Lys Ser Glu Glu Ile Ala Leu Gly Tyr Phe Arg Thr
130 135 140

Asn Glu Asn Phe Ala Ile Tyr Ser Asp Ala Leu Ala Lys Leu Ala Asn
145 150 155 160

Glu Glu Lys Val Pro Phe Val Ala Leu Asn Lys Ala Phe Gln Gln Glu
165 170 175

Gly Gly Asp Ala Trp Gln Gln Leu Leu Thr Asp Gly Leu His Phe Ser
180 185 190

Gly Lys Gly Tyr Lys Ile Phe His Asp Glu Leu Leu Lys Val Ile Glu
195 200 205

Thr Phe Tyr Pro Gln Tyr His Pro Lys Asn Met Gln Tyr Lys Leu Lys
210 215 220

Asp Trp Arg Asp Val Leu Asp Asp Gly Ser Asn Ile Met Ser
225 230 235

<210> 7
<211> 1005
<212> DNA
<213> Aeromonas hydrophila

<400> 7

atgaaaaaat ggtttgtgtg tttattggga ttggtcgcgc tgacagttca ggcagccgac 60

agccgtcccg ccttctcccg gatcgtgatg tttggcgaca gcctctccga taccggcaag 120

```
atgtacagca agatgcgcgg ttacctcccc tccagccccc cctactatga gggccgcttc   180
tccaacgggc ccgtctggct ggagcagctg accaacgagt tcccgggcct gaccatagcc   240
aacgaggcgg aaggcggacc gaccgccgtg gcttacaaca agatctcctg gaatcccaag   300
tatcaggtca tcaacaacct ggactacgag gtcacccagt tcctgcaaaa agacagcttc   360
aagccggacg atctggtgat cctctgggtc ggcgccaacg actatctggc ctatggctgg   420
aacacagagc aggatgccaa gcgggtgcgc gacgccatca gcgatgcggc caaccgcatg   480
gtgctgaacg gcgccaagga gatactgctg ttcaacctgc cggatctggg ccagaacccc   540
tcggcccgca gccagaaggt ggtcgaggcg gccagccatg tctccgccta ccacaaccag   600
ctgctgctga acctggcacg ccagctggct cccaccggca tggtgaagct gttcgagatc   660
gacaagcagt ttgccgagat gctgcgtgat ccgcagaact tcggcctgag cgaccagagg   720
aacgcctgct acggtggcag ctatgtatgg aagccgtttg cctcccgcag cgccagcacc   780
gacagccagc tctccgcctt caacccgcag gagcgcctcg ccatcgccgg caacccgctg   840
ctggcccagg ccgtcgccag ccccatggct gcccgcagcg ccagcaccct caactgtgag   900
ggcaagatgt tctgggatca ggtccacccc accactgtcg tgcacgccgc cctgagcgag   960
cccgccgcca ccttcatcga gagccagtac gagttcctcg cccac                   1005
```

<210> 8
<211> 1011
<212> DNA
<213> Aeromonas salmonicida

<400> 8

```
atgaaaaaat ggtttgtttg tttattgggg ttgatcgcgc tgacagttca ggcagccgac   60
actcgccccg ccttctcccg gatcgtgatg ttcggcgaca gcctctccga taccggcaaa   120
atgtacagca agatgcgcgg ttacctcccc tccagcccgc cctactatga gggccgtttc   180
tccaacggac ccgtctggct ggagcagctg accaagcagt tcccgggtct gaccatcgcc   240
aacgaagcgg aaggcggtgc cactgccgtg gcttacaaca agatctcctg gaatcccaag   300
tatcaggtct acaacaacct ggactacgag gtcacccagt tcttgcagaa agacagcttc   360
aagccggacg atctggtgat cctctgggtc ggtgccaatg actatctggc atatggctgg   420
aatacggagc aggatgccaa gcgagttcgc gatgccatca gcgatgcggc caaccgcatg   480
gtactgaacg gtgccaagca gatactgctg ttcaacctgc cggatctggg ccagaacccg   540
tcagcccgca gtcagaaggt ggtcgaggcg gtcagccatg tctccgccta tcacaacaag   600
ctgctgctga acctggcacg ccagctggcc cccaccggca tggtaaagct gttcgagatc   660
gacaagcaat ttgccgagat gctgcgtgat ccgcagaact tcggcctgag cgacgtcgag   720
aacccctgct acgacggcgg ctatgtgtgg aagccgtttg ccacccgcag cgtcagcacc   780
```

gaccgccagc tctccgcctt cagtccgcag gaacgcctcg ccatcgccgg caacccgctg 840
ctggcacagg ccgttgccag tcctatggcc cgccgcagcg ccagcccccct caactgtgag 900
ggcaagatgt tctgggatca ggtacacccg accactgtcg tgcacgcagc cctgagcgag 960
cgcgccgcca ccttcatcga gacccagtac gagttcctcg cccacggatg a 1011

<210> 9
<211> 888
<212> DNA
<213> Streptomyces coelicolor

<400> 9

atgccgaagc ctgcccttcg ccgtgtcatg accgcgacag tcgccgccgt cggcacgctc 60
gccctcggcc tcaccgacgc caccgcccac gccgcgcccg cccaggccac tccgaccctg 120
gactacgtcg ccctcggcga cagctacagc gccggctccg gcgtcctgcc cgtcgacccc 180
gccaacctgc tctgtctgcg ctcgacggcc aactaccccc acgtcatcgc ggacacgacg 240
ggcgcccgcc tcacggacgt cacctgcggc gccgcgcaga ccgccgactt cacgcgggcc 300
cagtacccgg gcgtcgcacc ccagttggac gcgctcggca ccggcacgga cctggtcacg 360
ctcaccatcg gcggcaacga caacagcacc ttcatcaacg ccatcacggc ctgcggcacg 420
gcgggtgtcc tcagcggcgg caagggcagc cccctgcaagg acaggcacgg cacctccttc 480
gacgacgaga tcgaggccaa cacgtacccc gcgctcaagg aggcgctgct cggcgtccgc 540
gccagggctc cccacgccag ggtggcggct ctcggctacc cgtggatcac cccggccacc 600
gccgacccgt cctgcttcct gaagctcccc ctcgccgccg gtgacgtgcc ctacctgcgg 660
gccatccagg cacacctcaa cgacgcggtc cggcgggccg ccgaggagac cggagccacc 720
tacgtggact tctccggggt gtccgacggc cacgacgcct gcgaggcccc cggcacccgc 780
tggatcgaac cgctgctctt cgggcacagc ctcgttcccg tccaccccaa cgccctgggc 840
gagcggcgca tggccgagca cacgatggac gtcctcggcc tggactga 888

<210> 10
<211> 888
<212> DNA
<213> Streptomyces coelicolor

<400> 10

tcagtccagg ccgaggacgt ccatcgtgtg ctcggccatg cgccgctcgc ccagggcgtt 60
ggggtggacg ggaacgaggc tgtgcccgaa gagcagcggt tcgatccagc gggtgccggg 120
ggcctcgcag gcgtcgtggc cgtcggacac cccggagaag tccacgtagg tggctccggt 180

ctcctcggcg gcccgccgga ccgcgtcgtt gaggtgtgcc tggatggccc gcaggtaggg 240
cacgtcaccg gcggcgaggg ggagcttcag gaagcaggac gggtcggcgg tggccggggt 300
gatccacggg tagccgagag ccgccaccct ggcgtgggga gccctggcgc ggacgccgag 360
cagcgcctcc ttgagcgcgg ggtacgtgtt ggcctcgatc tcgtcgtcga aggaggtgcc 420
gtgcctgtcc ttgcaggggc tgcccttgcc gccgctgagg acacccgccg tgccgcaggc 480
cgtgatggcg ttgatgaagg tgctgttgtc gttgccgccg atggtgagcg tgaccaggtc 540
cgtgccggtg ccgagcgcgt ccaactgggg tgcgacgccc gggtactggg cccgcgtgaa 600
gtcggcggtc tgcgcggcgc cgcaggtgac gtccgtgagg cgggcgcccg tcgtgtccgc 660
gatgacgtgg gggtagttgg ccgtcgagcg cagacagagc aggttggcgg ggtcgacggg 720
caggacgccg gagccggcgc tgtagctgtc gccgagggcg acgtagtcca gggtcggagt 780
ggcctgggcg ggcgcggcgt gggcggtggc gtcggtgagg ccgagggcga gcgtgccgac 840
ggcggcgact gtcgcggtca tgacacggcg aagggcaggc ttcggcat 888

<210> 11
<211> 717
<212> DNA
<213> Saccharomyces cerevisiae

<400> 11

atggattacg agaagtttct gttatttggg gattccatta ctgaatttgc ttttaatact 60
aggcccattg aagatggcaa agatcagtat gctcttggag ccgcattagt caacgaatat 120
acgagaaaaa tggatattct tcaaagaggg ttcaaagggt acacttctag atgggcgttg 180
aaaatacttc ctgagatttt aaagcatgaa tccaatattg tcatggccac aatatttttg 240
ggtgccaacg atgcatgctc agcaggtccc caaagtgtcc ccctccccga atttatcgat 300
aatattcgtc aaatggtatc tttgatgaag tcttaccata tccgtcctat tataatagga 360
ccggggctag tagatagaga gaagtgggaa aaagaaaaat ctgaagaaat agctctcgga 420
tacttccgta ccaacgagaa ctttgccatt tattccgatg ccttagcaaa actagccaat 480
gaggaaaaag ttcccttcgt ggctttgaat aaggcgtttc aacaggaagg tggtgatgct 540
tggcaacaac tgctaacaga tggactgcac ttttccggaa aagggtacaa aatttttcat 600
gacgaattat tgaaggtcat tgagacattc taccccccaat atcatcccaa aaacatgcag 660
tacaaactga aagattggag agatgtgcta gatgatggat ctaacataat gtcttga 717

<210> 12
<211> 347
<212> PRT
<213> Ralstonia

<400> 12

```
Met Asn Leu Arg Gln Trp Met Gly Ala Ala Thr Ala Ala Leu Ala Leu
1               5                   10                  15

Gly Leu Ala Ala Cys Gly Gly Gly Gly Thr Asp Gln Ser Gly Asn Pro
            20                  25                  30

Asn Val Ala Lys Val Gln Arg Met Val Val Phe Gly Asp Ser Leu Ser
        35                  40                  45

Asp Ile Gly Thr Tyr Thr Pro Val Ala Gln Ala Val Gly Gly Gly Lys
    50                  55                  60

Phe Thr Thr Asn Pro Gly Pro Ile Trp Ala Glu Thr Val Ala Ala Gln
65                  70                  75                  80

Leu Gly Val Thr Leu Thr Pro Ala Val Met Gly Tyr Ala Thr Ser Val
                85                  90                  95

Gln Asn Cys Pro Lys Ala Gly Cys Phe Asp Tyr Ala Gln Gly Gly Ser
            100                 105                 110

Arg Val Thr Asp Pro Asn Gly Ile Gly His Asn Gly Gly Ala Gly Ala
        115                 120                 125

Leu Thr Tyr Pro Val Gln Gln Gln Leu Ala Asn Phe Tyr Ala Ala Ser
    130                 135                 140

Asn Asn Thr Phe Asn Gly Asn Asn Asp Val Val Phe Val Leu Ala Gly
145                 150                 155                 160

Ser Asn Asp Ile Phe Phe Trp Thr Thr Ala Ala Ala Thr Ser Gly Ser
                165                 170                 175

Gly Val Thr Pro Ala Ile Ala Thr Ala Gln Val Gln Gln Ala Ala Thr
            180                 185                 190

Asp Leu Val Gly Tyr Val Lys Asp Met Ile Ala Lys Gly Ala Thr Gln
        195                 200                 205

Val Tyr Val Phe Asn Leu Pro Asp Ser Ser Leu Thr Pro Asp Gly Val
    210                 215                 220

Ala Ser Gly Thr Thr Gly Gln Ala Leu Leu His Ala Leu Val Gly Thr
225                 230                 235                 240

Phe Asn Thr Thr Leu Gln Ser Gly Leu Ala Gly Thr Ser Ala Arg Ile
                245                 250                 255
```

Ile Asp Phe Asn Ala Gln Leu Thr Ala Ala Ile Gln Asn Gly Ala Ser
260 265 270

Phe Gly Phe Ala Asn Thr Ser Ala Arg Ala Cys Asp Ala Thr Lys Ile
275 280 285

Asn Ala Leu Val Pro Ser Ala Gly Gly Ser Ser Leu Phe Cys Ser Ala
290 295 300

---

Asn Thr Leu Val Ala Ser Gly Ala Asp Gln Ser Tyr Leu Phe Ala Asp
305 310 315 320

Gly Val His Pro Thr Thr Ala Gly His Arg Leu Ile Ala Ser Asn Val
325 330 335

Leu Ala Arg Leu Leu Ala Asp Asn Val Ala His
340 345

<210> 13
<211> 1044
<212> DNA
<213> Ralstonia

<400> 13

atgaacctgc gtcaatggat gggcgccgcc acggctgccc ttgccttggg cttggccgcg 60

tgcgggggcg gtgggaccga ccagagcggc aatcccaatg tcgccaaggt gcagcgcatg 120

gtggtgttcg gcgacagcct gagcgatatc ggcacctaca cccccgtcgc gcaggcggtg 180

ggcggcggca agttcaccac caacccgggc ccgatctggg ccgagaccgt ggccgcgcaa 240

ctgggcgtga cgctcacgcc ggcggtgatg ggctacgcca cctccgtgca gaattgcccc 300

aaggccggct gcttcgacta tgcgcagggc ggctcgcgcg tgaccgatcc gaacggcatc 360

ggccacaacg gcggcgcggg ggcgctgacc tacccggttc agcagcagct cgccaacttc 420

tacgcggcca gcaacaacac attcaacggc aataacgatg tcgtcttcgt gctggccggc 480

agcaacgaca ttttcttctg gaccactgcg gcggccacca gcggctccgg cgtgacgccc 540

gccattgcca cggcccaggt gcagcaggcc gcgacggacc tggtcggcta tgtcaaggac 600

atgatcgcca agggtgcgac gcaggtctac gtgttcaacc tgcccgacag cagcctgacg 660

ccggacggcg tggcaagcgg cacgaccggc caggcgctgc tgcacgcgct ggtgggcacg 720

ttcaacacga cgctgcaaag cgggctggcc ggcacctcgg cgcgcatcat cgacttcaac 780

gcacaactga ccgcggcgat ccagaatggc gcctcgttcg gcttcgccaa caccagcgcc 840

cgggcctgcg acgccaccaa gatcaatgcc ctggtgccga gcgccggcgg cagctcgctg 900

ttctgctcgg ccaacacgct ggtggcttcc ggtgcggacc agagctacct gttcgccgac 960

ggcgtgcacc cgaccacggc cggccatcgc ctgatcgcca gcaacgtgct ggcgcgcctg 1020

ctggcggata acgtcgcgca ctga 1044

<210> 14
<211> 0
<212> DNA
<213> unknown

<220>
<223> No sequence
<400> 14
000

<210> 15
<211> 0
<212> DNA
<213> unknown

<220>
<223> no sequence
<400> 15
000

<210> 16
<211> 0
<212> DNA
<213> unknown

<220>
<223> No sequence
<400> 16
000

<210> 17
<211> 0
<212> DNA
<213> unknown

<220>
<223> No sequence
<400> 17
000

<210> 18
<211> 0
<212> DNA
<213> unknown

<220>
<223> No sequence
<400> 18
000

<210> 19
<211> 0
<212> DNA
<213> unknown

<220>
<223> No sequence
<400> 19

000

<210> 20
<211> 261
<212> PRT
<213> Artificial conserved hypothetical protein Streptomyces coelicolor

<400> 20

```
Met Ile Gly Ser Tyr Val Ala Val Gly Asp Ser Phe Thr Glu Gly Val
1               5                   10                  15

Gly Asp Pro Gly Pro Asp Gly Ala Phe Val Gly Trp Ala Asp Arg Leu
            20                  25                  30

Ala Val Leu Leu Ala Asp Arg Arg Pro Glu Gly Asp Phe Thr Tyr Thr
        35                  40                  45
```

```
Asn Leu Ala Val Arg Gly Arg Leu Leu Asp Gln Ile Val Ala Glu Gln
    50                  55                  60

Val Pro Arg Val Val Gly Leu Ala Pro Asp Leu Val Ser Phe Ala Ala
65                  70                  75                  80

Gly Gly Asn Asp Ile Ile Arg Pro Gly Thr Asp Pro Asp Glu Val Ala
                85                  90                  95

Glu Arg Phe Glu Leu Ala Val Ala Ala Leu Thr Ala Ala Ala Gly Thr
            100                 105                 110

Val Leu Val Thr Thr Gly Phe Asp Thr Arg Gly Val Pro Val Leu Lys
        115                 120                 125

His Leu Arg Gly Lys Ile Ala Thr Tyr Asn Gly His Val Arg Ala Ile
    130                 135                 140

Ala Asp Arg Tyr Gly Cys Pro Val Leu Asp Leu Trp Ser Leu Arg Ser
145                 150                 155                 160

Val Gln Asp Arg Arg Ala Trp Asp Ala Asp Arg Leu His Leu Ser Pro
                165                 170                 175

Glu Gly His Thr Arg Val Ala Leu Arg Ala Gly Gln Ala Leu Gly Leu
            180                 185                 190

Arg Val Pro Ala Asp Pro Asp Gln Pro Trp Pro Pro Leu Pro Pro Arg
        195                 200                 205

Gly Thr Leu Asp Val Arg Arg Asp Asp Val His Trp Ala Arg Glu Tyr
    210                 215                 220

Leu Val Pro Trp Ile Gly Arg Arg Leu Arg Gly Glu Ser Ser Gly Asp
225                 230                 235                 240

His Val Thr Ala Lys Gly Thr Leu Ser Pro Asp Ala Ile Lys Thr Arg
                245                 250                 255

Ile Ala Ala Val Ala
            260
```

<210> 21
<211> 786
<212> DNA
<213> Artificial conserved hypothetical Streptomyces coelicolor

<400> 21

```
gtgatcgggt cgtacgtggc ggtgggggac agcttcaccg agggcgtcgg cgaccccggc     60
cccgacgggg cgttcgtcgg ctgggccgac cggctcgccg tactgctcgc ggaccggcgc    120
cccgagggcg acttcacgta cacgaacctc gccgtgcgcg gcaggctcct cgaccagatc    180
gtggcggaac aggtcccgcg ggtcgtcgga ctcgcgcccg acctcgtctc gttcgcggcg    240
ggcggcaacg acatcatccg gcccggcacc gatcccgacg aggtcgccga gcggttcgag    300
ctggcggtgg ccgcgctgac cgccgcggcc ggaaccgtcc tggtgaccac cgggttcgac    360
acccgggggg tgcccgtcct caagcacctg cgcggcaaga tcgccacgta caacgggcac    420
gtccgcgcca tcgccgaccg ctacggctgc ccggtgctcg acctgtggtc gctgcggagc    480
gtccaggacc gcagggcgtg ggacgccgac cggctgcacc tgtcgccgga ggggcacacc    540
cgggtggcgc tgcgcgcggg gcaggccctg ggcctgcgcg tcccggccga ccctgaccag    600
ccctggccgc ccctgccgcc gcgcggcacg ctcgacgtcc ggcgcgacga cgtgcactgg    660
gcgcgcgagt acctggtgcc gtggatcggg cgccggctgc ggggcgagtc gtcgggcgac    720
cacgtgacgg ccaaggggac gctgtcgccg gacgccatca agacgcggat cgccgcggtg    780
gcctga                                                               786
```

<210> 22
<211> 260
<212> PRT
<213> artificial conserved hypothetical Streptomyces coelicolor

<400> 22

```
Met Gln Thr Asn Pro Ala Tyr Thr Ser Leu Val Ala Val Gly Asp Ser
1               5                   10                  15

Phe Thr Glu Gly Met Ser Asp Leu Leu Pro Asp Gly Ser Tyr Arg Gly
            20                  25                  30

Trp Ala Asp Leu Leu Ala Thr Arg Met Ala Ala Arg Ser Pro Gly Phe
        35                  40                  45

Arg Tyr Ala Asn Leu Ala Val Arg Gly Lys Leu Ile Gly Gln Ile Val
    50                  55                  60

Asp Glu Gln Val Asp Val Ala Ala Ala Met Gly Ala Asp Val Ile Thr
65                  70                  75                  80

Leu Val Gly Gly Leu Asn Asp Thr Leu Arg Pro Lys Cys Asp Met Ala
                85                  90                  95

Arg Val Arg Asp Leu Leu Thr Gln Ala Val Glu Arg Leu Ala Pro His
            100                 105                 110
```

Cys Glu Gln Leu Val Leu Met Arg Ser Pro Gly Arg Gln Gly Pro Val
115 120 125

Leu Glu Arg Phe Arg Pro Arg Met Glu Ala Leu Phe Ala Val Ile Asp
130 135 140

Asp Leu Ala Gly Arg His Gly Ala Val Val Val Asp Leu Tyr Gly Ala
145 150 155 160

Gln Ser Leu Ala Asp Pro Arg Met Trp Asp Val Asp Arg Leu His Leu
165 170 175

Thr Ala Glu Gly His Arg Arg Val Ala Glu Ala Val Trp Gln Ser Leu
180 185 190

Gly His Glu Pro Glu Asp Pro Glu Trp His Ala Pro Ile Pro Ala Thr
195 200 205

Pro Pro Pro Gly Trp Val Thr Arg Arg Thr Ala Asp Val Arg Phe Ala
210 215 220

Arg Gln His Leu Leu Pro Trp Ile Gly Arg Arg Leu Thr Gly Arg Ser
225 230 235 240

Ser Gly Asp Gly Leu Pro Ala Lys Arg Pro Asp Leu Leu Pro Tyr Glu
245 250 255

Asp Pro Ala Arg
260

<210> 23
<211> 783
<212> DNA
<213> Artificial conserved hypothetical Streptomyces coelicolor

<400> 23

atgcagacga accccgcgta caccagtctc gtcgccgtcg gcgactcctt caccgagggc 60
atgtcggacc tgctgcccga cggctcctac cgtggctggg ccgacctcct cgccacccgg 120
atggcggccc gctcccccgg cttccggtac gccaacctgg cggtgcgcgg gaagctgatc 180
ggacagatcg tcgacgagca ggtggacgtg gccgccgcca tgggagccga cgtgatcacg 240
ctggtcggcg ggctcaacga cacgctgcgg cccaagtgcg acatggcccg ggtgcgggac 300
ctgctgaccc aggccgtgga acggctcgcc ccgcactgcg agcagctggt gctgatgcgc 360
agtcccggtc gccagggtcc ggtgctggag cgcttccggc cccgcatgga ggccctgttc 420
gccgtgatcg acgacctggc cgggcggcac ggcgccgtgg tcgtcgacct gtacggggcc 480

cagtcgctgg ccgaccctcg gatgtgggac gtggaccggc tgcacctgac cgccgagggc 540

caccgccggg tcgcggaggc ggtgtggcag tcgctcggcc acgagcccga ggaccccgag 600

tggcacgcgc cgatcccggc gacgccgccg ccggggtggg tgacgcgcag gaccgcggac 660

gtccggttcg cccggcagca cctgctgccc tggataggcc gcaggctgac cgggcgctcg 720

tccggggacg gcctgccggc caagcgcccg gacctgctgc cctacgagga ccccgcacgg 780

tga 783

<210> 24
<211> 454
<212> PRT
<213> Artificial Streptomyces coelicolor putative secreted protein

<400> 24

Met Thr Arg Gly Arg Asp Gly Gly Ala Gly Ala Pro Pro Thr Lys His
1               5                   10                  15

Arg Ala Leu Leu Ala Ala Ile Val Thr Leu Ile Val Ala Ile Ser Ala
            20                  25                  30

Ala Ile Tyr Ala Gly Ala Ser Ala Asp Asp Gly Ser Arg Asp His Ala
        35                  40                  45

Leu Gln Ala Gly Gly Arg Leu Pro Arg Gly Asp Ala Ala Pro Ala Ser
    50                  55                  60

Thr Gly Ala Trp Val Gly Ala Trp Ala Thr Ala Pro Ala Ala Ala Glu
65                  70                  75                  80

Pro Gly Thr Glu Thr Thr Gly Leu Ala Gly Arg Ser Val Arg Asn Val
                85                  90                  95

Val His Thr Ser Val Gly Gly Thr Gly Ala Arg Ile Thr Leu Ser Asn
            100                 105                 110

Leu Tyr Gly Gln Ser Pro Leu Thr Val Thr His Ala Ser Ile Ala Leu
        115                 120                 125

Ala Ala Gly Pro Asp Thr Ala Ala Ala Ile Ala Asp Thr Met Arg Arg
    130                 135                 140

Leu Thr Phe Gly Gly Ser Ala Arg Val Ile Ile Pro Ala Gly Gly Gln
145                 150                 155                 160

Val Met Ser Asp Thr Ala Arg Leu Ala Ile Pro Tyr Gly Ala Asn Val
                165                 170                 175

Leu Val Thr Thr Tyr Ser Pro Ile Pro Ser Gly Pro Val Thr Tyr His
180 185 190

Pro Gln Ala Arg Gln Thr Ser Tyr Leu Ala Asp Gly Asp Arg Thr Ala
195 200 205

Asp Val Thr Ala Val Ala Tyr Thr Thr Pro Thr Pro Tyr Trp Arg Tyr
210 215 220

---

Leu Thr Ala Leu Asp Val Leu Ser His Glu Ala Asp Gly Thr Val Val
225 230 235 240

Ala Phe Gly Asp Ser Ile Thr Asp Gly Ala Arg Ser Gln Ser Asp Ala
245 250 255

Asn His Arg Trp Thr Asp Val Leu Ala Ala Arg Leu His Glu Ala Ala
260 265 270

Gly Asp Gly Arg Asp Thr Pro Arg Tyr Ser Val Val Asn Glu Gly Ile
275 280 285

Ser Gly Asn Arg Leu Leu Thr Ser Arg Pro Gly Arg Pro Ala Asp Asn
290 295 300

Pro Ser Gly Leu Ser Arg Phe Gln Arg Asp Val Leu Glu Arg Thr Asn
305 310 315 320

Val Lys Ala Val Val Val Val Leu Gly Val Asn Asp Val Leu Asn Ser
325 330 335

Pro Glu Leu Ala Asp Arg Asp Ala Ile Leu Thr Gly Leu Arg Thr Leu
340 345 350

Val Asp Arg Ala His Ala Arg Gly Leu Arg Val Val Gly Ala Thr Ile
355 360 365

Thr Pro Phe Gly Gly Tyr Gly Gly Tyr Thr Glu Ala Arg Glu Thr Met
370 375 380

Arg Gln Glu Val Asn Glu Glu Ile Arg Ser Gly Arg Val Phe Asp Thr
385 390 395 400

Val Val Asp Phe Asp Lys Ala Leu Arg Asp Pro Tyr Asp Pro Arg Arg
405 410 415

Met Arg Ser Asp Tyr Asp Ser Gly Asp His Leu His Pro Gly Asp Lys
420 425 430

Gly Tyr Ala Arg Met Gly Ala Val Ile Asp Leu Ala Ala Leu Lys Gly
435 440 445

Ala Ala Pro Val Lys Ala
450

<210> 25
<211> 1365
<212> DNA
<213> Artificial Streptomyces coelicolor putative secreted protein

<400> 25

```
atgacccggg gtcgtgacgg gggtgcgggg gcgcccccca ccaagcaccg tgccctgctc     60
gcggcgatcg tcaccctgat agtggcgatc tccgcggcca tatacgccgg agcgtccgcg    120
gacgacggca gcagggacca cgcgctgcag gccggaggcc gtctcccacg aggagacgcc    180
gcccccgcgt ccaccggtgc ctgggtgggc gcctgggcca ccgcaccggc cgcggccgag    240
ccgggcaccg agacgaccgg cctggcgggc cgctccgtgc gcaacgtcgt gcacacctcg    300
gtcggcggca ccggcgcgcg gatcaccctc tcgaacctgt acgggcagtc gccgctgacc    360
gtcacacacg cctcgatcgc cctggccgcc gggcccgaca ccgccgccgc gatcgccgac    420
accatgcgcc ggctcacctt cggcggcagc gcccgggtga tcatcccggc gggcggccag    480
gtgatgagcg acaccgcccg cctcgccatc ccctacgggg cgaacgtcct ggtcaccacg    540
tactccccca tcccgtccgg gccggtgacc taccatccgc aggcccggca gaccagctac    600
ctggccgacg gcgaccgcac ggcggacgtc accgccgtcg cgtacaccac ccccacgccc    660
tactggcgct acctgaccgc cctcgacgtg ctgagccacg aggccgacgg cacggtcgtg    720
gcgttcggcg actccatcac cgacggcgcc cgctcgcaga gcgacgccaa ccaccgctgg    780
accgacgtcc tcgccgcacg cctgcacgag gcggcgggcg acggccggga cacgccccgc    840
tacagcgtcg tcaacgaggg catcagcggc aaccggctcc tgaccagcag gccggggcgg    900
ccggccgaca acccgagcgg actgagccgg ttccagcggg acgtgctgga acgcaccaac    960
gtcaaggccg tcgtcgtcgt cctcggcgtc aacgacgtcc tgaacagccc ggaactcgcc   1020
gaccgcgacg ccatcctgac cggcctgcgc accctcgtcg accgggcgca cgcccgggga   1080
ctgcgggtcg tcggcgccac gatcacgccg ttcggcggct acggcggcta caccgaggcc   1140
cgcgagacga tgcggcagga ggtcaacgag gagatccgct ccggccgggt cttcgacacg   1200
gtcgtcgact tcgacaaggc cctgcgcgac ccgtacgacc cgcgccggat gcgctccgac   1260
tacgacagcg gcgaccacct gcaccccggc gacaaggggt acgcgcgcat gggcgcggtc   1320
atcgacctgg ccgcgctgaa gggcgcggcg ccggtcaagg cgtag                   1365
```

<210> 26
<211> 340
<212> PRT
<213> Artificial Steptomyces coelicolor putative secreted protein

<400> 26

```
Met Thr Ser Met Ser Arg Ala Arg Val Ala Arg Arg Ile Ala Ala Gly
1               5                   10                  15

Ala Ala Tyr Gly Gly Gly Gly Ile Gly Leu Ala Gly Ala Ala Ala Val
            20                  25                  30

Gly Leu Val Val Ala Glu Val Gln Leu Ala Arg Arg Arg Val Gly Val
        35                  40                  45

Gly Thr Pro Thr Arg Val Pro Asn Ala Gln Gly Leu Tyr Gly Gly Thr
    50                  55                  60

Leu Pro Thr Ala Gly Asp Pro Pro Leu Arg Leu Met Met Leu Gly Asp
65                  70                  75                  80

Ser Thr Ala Ala Gly Gln Gly Val His Arg Ala Gly Gln Thr Pro Gly
                85                  90                  95

Ala Leu Leu Ala Ser Gly Leu Ala Ala Val Ala Glu Arg Pro Val Arg
            100                 105                 110

Leu Gly Ser Val Ala Gln Pro Gly Ala Cys Ser Asp Asp Leu Asp Arg
        115                 120                 125

Gln Val Ala Leu Val Leu Ala Glu Pro Asp Arg Val Pro Asp Ile Cys
    130                 135                 140

Val Ile Met Val Gly Ala Asn Asp Val Thr His Arg Met Pro Ala Thr
145                 150                 155                 160

Arg Ser Val Arg His Leu Ser Ser Ala Val Arg Arg Leu Arg Thr Ala
                165                 170                 175

Gly Ala Glu Val Val Val Gly Thr Cys Pro Asp Leu Gly Thr Ile Glu
            180                 185                 190

Arg Val Arg Gln Pro Leu Arg Trp Leu Ala Arg Arg Ala Ser Arg Gln
        195                 200                 205

Leu Ala Ala Ala Gln Thr Ile Gly Ala Val Glu Gln Gly Gly Arg Thr
    210                 215                 220

Val Ser Leu Gly Asp Leu Leu Gly Pro Glu Phe Ala Gln Asn Pro Arg
225                 230                 235                 240
```

Glu Leu Phe Gly Pro Asp Asn Tyr His Pro Ser Ala Glu Gly Tyr Ala
245 250 255

Thr Ala Ala Met Ala Val Leu Pro Ser Val Cys Ala Ala Leu Gly Leu
260 265 270

Trp Pro Ala Asp Glu Glu His Pro Asp Ala Leu Arg Arg Glu Gly Phe
275 280 285

Leu Pro Val Ala Arg Ala Ala Ala Glu Ala Ala Ser Glu Ala Gly Thr
290 295 300

Glu Val Ala Ala Ala Met Pro Thr Gly Pro Arg Gly Pro Trp Ala Leu
305 310 315 320

Leu Lys Arg Arg Arg Arg Arg Arg Val Ser Glu Ala Glu Pro Ser Ser
325 330 335

Pro Ser Gly Val
340

<210> 27
<211> 1023
<212> DNA
<213> Artificial Streptomyces coelicolor putative

<400> 27

```
atgacgagca tgtcgagggc gagggtggcg cggcggatcg cggccggcgc ggcgtacggc    60
ggcggcggca tcggcctggc gggagcggcg gcggtcggtc tggtggtggc cgaggtgcag   120
ctggccagac gcagggtggg ggtgggcacg ccgacccggg tgccgaacgc gcagggactg   180
tacggcggca ccctgcccac ggccggcgac ccgccgctgc ggctgatgat gctgggcgac   240
tccacggccg ccgggcaggg cgtgcaccgg gccgggcaga cgccgggcgc gctgctggcg   300
tccgggctcg cggcggtggc ggagcggccg gtgcggctgg ggtcggtcgc ccagccgggg   360
gcgtgctcgg acgacctgga ccggcaggtg gcgctggtgc tcgccgagcc ggaccgggtg   420
cccgacatct gcgtgatcat ggtcggcgcc aacgacgtca cccaccggat gccggcgacc   480
cgctcggtgc ggcacctgtc ctcggcggta cggcggctgc gcacggccgg tgcggaggtg   540
gtggtcggca cctgtccgga cctgggcacg atcgagcggg tgcggcagcc gctgcgctgg   600
ctggcccggc gggcctcacg gcagctcgcg gcggcacaga ccatcggcgc cgtcgagcag   660
ggcgggcgca cggtgtcgct gggcgacctg ctgggtccgg agttcgcgca gaacccgcgg   720
gagctcttcg gccccgacaa ctaccacccc tccgccgagg ggtacgccac ggccgcgatg   780
gcggtactgc cctcggtgtg cgccgcgctc ggcctgtggc cggccgacga ggagcacccg   840
gacgcgctgc gccgcgaggg cttcctgccg gtggcgcgcg cggcggcgga ggcggcgtcc   900
```

gaggcgggta cggaggtcgc cgccgccatg cctacggggc ctcgggggcc ctgggcgctg 960

ctgaagcgcc ggagacggcg tcgggtgtcg gaggcggaac cgtccagccc gtccggcgtt 1020

tga 1023

<210> 28
<211> 305
<212> PRT
<213> Artificial Streptomyces coelicolor putative lipoprotein

<400> 28

```
Met Gly Arg Gly Thr Asp Gln Arg Thr Arg Tyr Gly Arg Arg Arg Ala
1               5                   10                  15

Arg Val Ala Leu Ala Ala Leu Thr Ala Ala Val Leu Gly Val Gly Val
            20                  25                  30

Ala Gly Cys Asp Ser Val Gly Gly Asp Ser Pro Ala Pro Ser Gly Ser
        35                  40                  45

Pro Ser Lys Arg Thr Arg Thr Ala Pro Ala Trp Asp Thr Ser Pro Ala
    50                  55                  60

Ser Val Ala Ala Val Gly Asp Ser Ile Thr Arg Gly Phe Asp Ala Cys
65                  70                  75                  80

Ala Val Leu Ser Asp Cys Pro Glu Val Ser Trp Ala Thr Gly Ser Ser
                85                  90                  95

Ala Lys Val Asp Ser Leu Ala Val Arg Leu Leu Gly Lys Ala Asp Ala
            100                 105                 110

Ala Glu His Ser Trp Asn Tyr Ala Val Thr Gly Ala Arg Met Ala Asp
        115                 120                 125

Leu Thr Ala Gln Val Thr Arg Ala Ala Gln Arg Glu Pro Glu Leu Val
    130                 135                 140

Ala Val Met Ala Gly Ala Asn Asp Ala Cys Arg Ser Thr Thr Ser Ala
145                 150                 155                 160

Met Thr Pro Val Ala Asp Phe Arg Ala Gln Phe Glu Glu Ala Met Ala
                165                 170                 175

Thr Leu Arg Lys Lys Leu Pro Lys Ala Gln Val Tyr Val Ser Ser Ile
            180                 185                 190
```

Pro Asp Leu Lys Arg Leu Trp Ser Gln Gly Arg Thr Asn Pro Leu Gly
195 200 205

Lys Gln Val Trp Lys Leu Gly Leu Cys Pro Ser Met Leu Gly Asp Ala
210 215 220

Asp Ser Leu Asp Ser Ala Ala Thr Leu Arg Arg Asn Thr Val Arg Asp
225 230 235 240

Arg Val Ala Asp Tyr Asn Glu Val Leu Arg Glu Val Cys Ala Lys Asp
245 250 255

Arg Arg Cys Arg Ser Asp Asp Gly Ala Val His Glu Phe Arg Phe Gly
260 265 270

Thr Asp Gln Leu Ser His Trp Asp Trp Phe His Pro Ser Val Asp Gly
275 280 285

Gln Ala Arg Leu Ala Glu Ile Ala Tyr Arg Ala Val Thr Ala Lys Asn
290 295 300

Pro
305

<210> 29
<211> 918
<212> DNA
<213> Artificial Streptomyces coelicolor putative lipoprotein

<400> 29

```
atgggtcgag ggacggacca gcggacgcgg tacggccgtc gccgggcgcg tgtcgcgctc     60
gccgccctga ccgccgccgt cctgggcgtg ggcgtggcgg gctgcgactc cgtgggcggc    120
gactcacccg ctccttccgg cagcccgtcg aagcggacga ggacggcgcc cgcctgggac    180
accagcccgg cgtccgtcgc cgccgtgggc gactccatca cgcgcggctt cgacgcctgt    240
gcggtgctgt cggactgccc ggaggtgtcg tgggcgaccg gcagcagcgc gaaggtcgac    300
tcgctggccg tacggctgct ggggaaggcg gacgcggccg agcacagctg gaactacgcg    360
gtcaccgggg cccggatggc ggacctgacc gctcaggtga cgcgggcggc gcagcgcgag    420
ccggagctgg tggcggtgat ggccggggcg aacgacgcgt gccggtccac gacctcggcg    480
atgacgccgg tggcggactt ccgggcgcag ttcgaggagg cgatggccac cctgcgcaag    540
aagctcccca aggcgcaggt gtacgtgtcg agcatcccgg acctcaagcg gctctggtcc    600
cagggccgca ccaacccgct gggcaagcag gtgtggaagc tcggcctgtg cccgtcgatg    660
ctgggcgacg cggactccct ggactcggcg gcgaccctgc ggcgcaacac ggtgcgcgac    720
cgggtggcgg actacaacga ggtgctgcgg gaggtctgcg cgaaggaccg gcggtgccgc    780
```

agcgacgacg gcgcggtgca cgagttccgg ttcggcacgg accagttgag ccactgggac 840

tggttccacc cgagtgtgga cggccaggcc cggctggcgg agatcgccta ccgcgcggtc 900

accgcgaaga atccctga 918

<210> 30
<211> 268
<212> PRT
<213> Streptomyces rimosus

<400> 30

Met Arg Leu Ser Arg Arg Ala Ala Thr Ala Ser Ala Leu Leu Leu Thr
1 5 10 15

Pro Ala Leu Ala Leu Phe Gly Ala Ser Ala Ala Val Ser Ala Pro Arg
20 25 30

Ile Gln Ala Thr Asp Tyr Val Ala Leu Gly Asp Ser Tyr Ser Ser Gly
35 40 45

Val Gly Ala Gly Ser Tyr Asp Ser Ser Ser Gly Ser Cys Lys Arg Ser
50 55 60

Thr Lys Ser Tyr Pro Ala Leu Trp Ala Ala Ser His Thr Gly Thr Arg
65 70 75 80

Phe Asn Phe Thr Ala Cys Ser Gly Ala Arg Thr Gly Asp Val Leu Ala
85 90 95

Lys Gln Leu Thr Pro Val Asn Ser Gly Thr Asp Leu Val Ser Ile Thr
100 105 110

Ile Gly Gly Asn Asp Ala Gly Phe Ala Asp Thr Met Thr Thr Cys Asn
115 120 125

Leu Gln Gly Glu Ser Ala Cys Leu Ala Arg Ile Ala Lys Ala Arg Ala
130 135 140

Tyr Ile Gln Gln Thr Leu Pro Ala Gln Leu Asp Gln Val Tyr Asp Ala
145 150 155 160

Ile Asp Ser Arg Ala Pro Ala Ala Gln Val Val Val Leu Gly Tyr Pro
165 170 175

Arg Phe Tyr Lys Leu Gly Gly Ser Cys Ala Val Gly Leu Ser Glu Lys
180 185 190

```
Ser Arg Ala Ala Ile Asn Ala Ala Ala Asp Asp Ile Asn Ala Val Thr
        195                 200                 205

Ala Lys Arg Ala Ala Asp His Gly Phe Ala Phe Gly Asp Val Asn Thr
    210                 215                 220

Thr Phe Ala Gly His Glu Leu Cys Ser Gly Ala Pro Trp Leu His Ser
225                 230                 235                 240

Val Thr Leu Pro Val Glu Asn Ser Tyr His Pro Thr Ala Asn Gly Gln
                245                 250                 255

Ser Lys Gly Tyr Leu Pro Val Leu Asn Ser Ala Thr
            260                 265
```

<210> 31
<211> 1068
<212> DNA
<213> Streptomyces rimosus

<400> 31

```
ttcatcacaa cgatgtcaca acaccggcca tccgggtcat ccctgatcgt gggaatgggt      60
gacaagcctt cccgtgacga aagggtcctg ctacatcaga aatgacagaa atcctgctca     120
gggaggttcc atgagactgt cccgacgcgc ggccacggcg tccgcgctcc tcctcacccc     180
ggcgctcgcg ctcttcggcg cgagcgccgc cgtgtccgcg ccgcgaatcc aggccaccga     240
ctacgtggcc ctcggcgact cctactcctc gggggtcggc gcgggcagct acgacagcag     300
cagtggctcc tgtaagcgca gcaccaagtc ctacccggcc ctgtgggccg cctcgcacac     360
cggtacgcgg ttcaacttca ccgcctgttc gggcgcccgc acaggagacg tgctggccaa     420
gcagctgacc ccggtcaact ccggcaccga cctggtcagc attaccatcg gcggcaacga     480
cgcgggcttc gccgacacca tgaccacctg caacctccag ggcgagagcg cgtgcctggc     540
gcggatcgcc aaggcgcgcg cctacatcca gcagacgctg cccgcccagc tggaccaggt     600
ctacgacgcc atcgacagcc gggcccccgc agcccaggtc gtcgtcctgg gctacccgcg     660
cttctacaag ctgggcggca gctgcgccgt cggtctctcg gagaagtccc gcgcggccat     720
caacgccgcc gccgacgaca tcaacgccgt caccgccaag cgcgccgccg accacggctt     780
cgccttcggg gacgtcaaca cgaccttcgc cgggcacgag ctgtgctccg gcgccccctg     840
gctgcacagc gtcacccttc ccgtggagaa ctcctaccac cccacggcca acggacagtc     900
caagggctac ctgcccgtcc tgaactccgc cacctgatct cgcggctact ccgcccctga     960
cgaagtcccg cccccgggcg gggcttcgcc gtaggtgcgc gtaccgccgt cgcccgtcgc    1020
gccggtggcc ccgccgtacg tgccgccgcc cccggacgcg gtcggttc                 1068
```

<210> 32
<211> 335
<212> PRT
<213> Aeromonas hydrophila

<400> 32

```
Met Lys Lys Trp Phe Val Cys Leu Leu Gly Leu Val Ala Leu Thr Val
1               5                   10                  15

Gln Ala Ala Asp Ser Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175

Gly Gln Asn Pro Ser Ala Arg Ser Gln Lys Val Val Glu Ala Val Ser
            180                 185                 190

His Val Ser Ala Tyr His Asn Gln Leu Leu Leu Asn Leu Ala Arg Gln
        195                 200                 205

Leu Ala Pro Thr Gly Met Val Lys Leu Phe Glu Ile Asp Lys Gln Phe
    210                 215                 220
```

Ala Glu Met Leu Arg Asp Pro Gln Asn Phe Gly Leu Ser Asp Val Glu
225 230 235 240

Asn Pro Cys Tyr Asp Gly Gly Tyr Val Trp Lys Pro Phe Ala Thr Arg
245 250 255

Ser Val Ser Thr Asp Arg Gln Leu Ser Ala Phe Ser Pro Gln Glu Arg
260 265 270

Leu Ala Ile Ala Gly Asn Pro Leu Leu Ala Gln Ala Val Ala Ser Pro
275 280 285

Met Ala Arg Arg Ser Ala Ser Pro Leu Asn Cys Glu Gly Lys Met Phe
290 295 300

Trp Asp Gln Val His Pro Thr Thr Val Val His Ala Ala Leu Ser Glu
305 310 315 320

Arg Ala Ala Thr Phe Ile Ala Asn Gln Tyr Glu Phe Leu Ala His
325 330 335

<210> 33
<211> 2016
<212> DNA
<213> Aeromonas hydrophila

<400> 33

atgaaaaaat ggtttgtgtg tttattggga ttggtcgcgc tgacagttca tacttttta 60
ccaaacacac aaataaccct aaccagcgcg actgtcaagt ggcagccgac agtcgccccg 120
ccttttcccg gatcgtgatg ttcggcgaca ccgtcggctg tcagcggggc ggaaaagggc 180
ctagcactac aagccgctgt gcctctccga taccggcaaa atgtacagca agatgcgcgg 240
ttacctcccc cggagaggct atggccgttt tacatgtcgt tctacgcgcc aatggagggg 300
tccagcccgc cctactatga gggccgtttc tccaacggac ccgtctggct aggtcgggcg 360
ggatgatact cccggcaaag aggttgcctg ggcagaccga ggagcagctg accaaacagt 420
tcccgggtct gaccatcgcc aacgaagcgg cctcgtcgac tggtttgtca agggcccaga 480
ctggtagcgg ttgcttcgcc aaggcggtgc cactgccgtg gcttacaaca agatctcctg 540
gaatcccaag ttccgccacg gtgacggcac cgaatgttgt tctagaggac cttagggttc 600
tatcaggtca tcaacaacct ggactacgag gtcacccagt tcttgcagaa atagtccagt 660
agttgttgga cctgatgctc cagtgggtca agaacgtctt agacagcttc aagccggacg 720
atctggtgat cctctgggtc ggtgccaatg tctgtcgaag ttcggcctgc tagaccacta 780
ggagacccag ccacggttac actatctggc ctatggctgg aacacggagc aggatgccaa 840
gcgggttcgc tgatagaccg gataccgacc ttgtgcctcg tcctacggtt cgcccaagcg 900

```
gatgccatca gcgatgcggc caaccgcatg gtactgaacg gtgccaagca ctacggtagt     960
cgctacgccg gttggcgtac catgacttgc cacggttcgt gatactgctg ttcaacctgc    1020
cggatctggg ccagaacccg tcagctcgca ctatgacgac aagttggacg gcctagaccc    1080
ggtcttgggc agtcgagcgt gtcagaaggt ggtcgaggcg gtcagccatg tctccgccta    1140
tcacaaccag cagtcttcca ccagctccgc cagtcggtac agaggcggat agtgttggtc    1200
ctgctgctga acctggcacg ccagctggcc cccaccggca tggtaaagct gacgacgact    1260
tggaccgtgc ggtcgaccgg gggtggccgt accatttcga gttcgagatc gacaagcaat    1320
ttgccgagat gctgcgtgat ccgcagaact caagctctag ctgttcgtta aacggctcta    1380
cgacgcacta ggcgtcttga tcggcctgag cgacgtcgag aacccctgct acgacggcgg    1440
ctatgtgtgg agccggactc gctgcagctc ttggggacga tgctgccgcc gatacacacc    1500
aagccgtttg ccacccgcag cgtcagcacc gaccgccagc tctccgcctt ttcggcaaac    1560
ggtgggcgtc gcagtcgtgg ctggcggtcg agaggcggaa cagtccgcag gaacgcctcg    1620
ccatcgccgg caacccgctg ctggcacagg gtcaggcgtc cttgcggagc ggtagcggcc    1680
gttgggcgac gaccgtgtcc ccgttgccag tcctatggcc cgccgcagcg ccagcccccct   1740
caactgtgag ggcaacggtc aggataccgg gcggcgtcgc ggtcgggggga gttgacactc   1800
ggcaagatgt tctgggatca ggtacacccg accactgtcg tgcacgcagc ccgttctaca    1860
agaccctagt ccatgtgggc tggtgacagc acgtgcgtcg cctgagcgag cgcgccgcca    1920
ccttcatcgc gaaccagtac gagttcctcg ggactcgctc gcgcggcggt ggaagtagcg    1980
cttggtcatg ctcaaggagc cccactgagg gtgact                              2016
```

<210> 34
<211> 336
<212> PRT
<213> Aeromonas salmonicida subsp. Salmonicida

<400> 34

```
Met Lys Lys Trp Phe Val Cys Leu Leu Gly Leu Ile Ala Leu Thr Val
1               5                   10                  15

Gln Ala Ala Asp Thr Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60
```

Val Trp Leu Glu Gln Leu Thr Lys Gln Phe Pro Gly Leu Thr Ile Ala
65 70 75 80

Asn Glu Ala Glu Gly Gly Ala Thr Ala Val Ala Tyr Asn Lys Ile Ser
85 90 95

Trp Asn Pro Lys Tyr Gln Val Ile Asn Asn Leu Asp Tyr Glu Val Thr
100 105 110

Gln Phe Leu Gln Lys Asp Ser Phe Lys Pro Asp Asp Leu Val Ile Leu
115 120 125

Trp Val Gly Ala Asn Asp Tyr Leu Ala Tyr Gly Trp Asn Thr Glu Gln
130 135 140

Asp Ala Lys Arg Val Arg Asp Ala Ile Ser Asp Ala Ala Asn Arg Met
145 150 155 160

Val Leu Asn Gly Ala Lys Gln Ile Leu Leu Phe Asn Leu Pro Asp Leu
165 170 175

Gly Gln Asn Pro Ser Ala Arg Ser Gln Lys Val Val Glu Ala Val Ser
180 185 190

His Val Ser Ala Tyr His Asn Lys Leu Leu Leu Asn Leu Ala Arg Gln
195 200 205

Leu Ala Pro Thr Gly Met Val Lys Leu Phe Glu Ile Asp Lys Gln Phe
210 215 220

Ala Glu Met Leu Arg Asp Pro Gln Asn Phe Gly Leu Ser Asp Val Glu
225 230 235 240

Asn Pro Cys Tyr Asp Gly Gly Tyr Val Trp Lys Pro Phe Ala Thr Arg
245 250 255

Ser Val Ser Thr Asp Arg Gln Leu Ser Ala Phe Ser Pro Gln Glu Arg
260 265 270

Leu Ala Ile Ala Gly Asn Pro Leu Leu Ala Gln Ala Val Ala Ser Pro
275 280 285

Met Ala Arg Arg Ser Ala Ser Pro Leu Asn Cys Glu Gly Lys Met Phe
290 295 300

Trp Asp Gln Val His Pro Thr Thr Val Val His Ala Ala Leu Ser Glu
305 310 315 320

Arg Ala Ala Thr Phe Ile Glu Thr Gln Tyr Glu Phe Leu Ala His Gly
325 330 335

<210> 35
<211> 2022
<212> DNA
<213> Aeromonas Salmonicida sibsp. Salmonicida

<400> 35

```
atgaaaaaat ggtttgtttg tttattgggg ttgatcgcgc tgacagttca tacttttta     60
ccaaacaaac aaataacccc aactagcgcg actgtcaagt ggcagccgac actcgccccg    120
ccttctcccg gatcgtgatg ttcggcgaca ccgtcggctg tgagcggggc ggaagagggc    180
ctagcactac aagccgctgt gcctctccga taccggcaaa atgtacagca agatgcgcgg    240
ttacctcccc cggagaggct atggccgttt tacatgtcgt tctacgcgcc aatggagggg    300
tccagcccgc cctactatga gggccgtttc tccaacggac ccgtctggct aggtcgggcg    360
ggatgatact cccggcaaag aggttgcctg ggcagaccga ggagcagctg accaagcagt    420
tcccgggtct gaccatcgcc aacgaagcgg cctcgtcgac tggttcgtca agggcccaga    480
ctggtagcgg ttgcttcgcc aaggcggtgc cactgccgtg gcttacaaca agatctcctg    540
gaatcccaag ttccgccacg gtgacggcac cgaatgttgt tctagaggac cttagggttc    600
tatcaggtca tcaacaacct ggactacgag gtcacccagt tcttgcagaa atagtccagt    660
agttgttgga cctgatgctc cagtgggtca agaacgtctt agacagcttc aagccggacg    720
atctggtgat cctctgggtc ggtgccaatg tctgtcgaag ttcggcctgc tagaccacta    780
ggagacccag ccacggttac actatctggc atatggctgg aatacggagc aggatgccaa    840
gcgagttcgc tgatagaccg tataccgacc ttatgcctcg tcctacggtt cgctcaagcg    900
gatgccatca gcgatgcggc caaccgcatg gtactgaacg gtgccaagca ctacggtagt    960
cgctacgccg gttggcgtac catgacttgc cacggttcgt gatactgctg ttcaacctgc   1020
cggatctggg ccagaacccg tcagcccgca ctatgacgac aagttggacg gcctagaccc   1080
ggtcttgggc agtcgggcgt gtcagaaggt ggtcgaggcg gtcagccatg tctccgccta   1140
tcacaacaag cagtcttcca ccagctccgc cagtcggtac agaggcggat agtgttgttc   1200
ctgctgctga acctggcacg ccagctggcc cccaccggca tggtaaagct gacgacgact   1260
tggaccgtgc ggtcgaccgg gggtggccgt accatttcga gttcgagatc gacaagcaat   1320
ttgccgagat gctgcgtgat ccgcagaact caagctctag ctgttcgtta aacggctcta   1380
cgacgcacta ggcgtcttga tcggcctgag cgacgtcgag aacccctgct acgacggcgg   1440
ctatgtgtgg agccggactc gctgcagctc ttggggacga tgctgccgcc gatacacacc   1500
aagccgtttg ccacccgcag cgtcagcacc gaccgccagc tctccgcctt ttcggcaaac   1560
ggtgggcgtc gcagtcgtgg ctggcggtcg agaggcggaa cagtccgcag gaacgcctcg   1620
ccatcgccgg caacccgctg ctggcacagg gtcaggcgtc cttgcggagc ggtagcggcc   1680
gttgggcgac gaccgtgtcc ccgttgccag tcctatggcc cgccgcagcg ccagcccccct  1740

caactgtgag ggcaacggtc aggataccgg gcggcgtcgc ggtcggggga gttgacactc   1800
ggcaagatgt tctgggatca ggtacacccg accactgtcg tgcacgcagc ccgttctaca   1860
agaccctagt ccatgtgggc tggtgacagc acgtgcgtcg cctgagcgag cgcgccgcca   1920
ccttcatcga gacccagtac gagttcctcg ggactcgctc gcgcggcggt ggaagtagct   1980
ctgggtcatg ctcaaggagc cccacggatg agggtgccta ct                      2022
```

<210> 36
<211> 347
<212> PRT
<213> Artificial fusion construct

<400> 36

```
Met Phe Lys Phe Lys Lys Asn Phe Leu Val Gly Leu Ser Ala Ala Leu
1               5                   10                  15

Met Ser Ile Ser Leu Phe Ser Ala Thr Ala Ser Ala Ala Ser Ala Asp
            20                  25                  30

Ser Arg Pro Ala Phe Ser Arg Ile Val Met Phe Gly Asp Ser Leu Ser
        35                  40                  45

Asp Thr Gly Lys Met Tyr Ser Lys Met Arg Gly Tyr Leu Pro Ser Ser
    50                  55                  60

Pro Pro Tyr Tyr Glu Gly Arg Phe Ser Asn Gly Pro Val Trp Leu Glu
65                  70                  75                  80

Gln Leu Thr Lys Gln Phe Pro Gly Leu Thr Ile Ala Asn Glu Ala Glu
                85                  90                  95

Gly Gly Ala Thr Ala Val Ala Tyr Asn Lys Ile Ser Trp Asn Pro Lys
            100                 105                 110

Tyr Gln Val Ile Asn Asn Leu Asp Tyr Glu Val Thr Gln Phe Leu Gln
        115                 120                 125

Lys Asp Ser Phe Lys Pro Asp Asp Leu Val Ile Leu Trp Val Gly Ala
    130                 135                 140

Asn Asp Tyr Leu Ala Tyr Gly Trp Asn Thr Glu Gln Asp Ala Lys Arg
145                 150                 155                 160

Val Arg Asp Ala Ile Ser Asp Ala Ala Asn Arg Met Val Leu Asn Gly
                165                 170                 175
```

Ala Lys Gln Ile Leu Leu Phe Asn Leu Pro Asp Leu Gly Gln Asn Pro
180 185 190

Ser Ala Arg Ser Gln Lys Val Val Glu Ala Val Ser His Val Ser Ala
195 200 205

Tyr His Asn Gln Leu Leu Leu Asn Leu Ala Arg Gln Leu Ala Pro Thr
210 215 220

Gly Met Val Lys Leu Phe Glu Ile Asp Lys Gln Phe Ala Glu Met Leu
225 230 235 240

Arg Asp Pro Gln Asn Phe Gly Leu Ser Asp Val Glu Asn Pro Cys Tyr
245 250 255

Asp Gly Gly Tyr Val Trp Lys Pro Phe Ala Thr Arg Ser Val Ser Thr
260 265 270

Asp Arg Gln Leu Ser Ala Phe Ser Pro Gln Glu Arg Leu Ala Ile Ala
275 280 285

Gly Asn Pro Leu Leu Ala Gln Ala Val Ala Ser Pro Met Ala Arg Arg
290 295 300

Ser Ala Ser Pro Leu Asn Cys Glu Gly Lys Met Phe Trp Asp Gln Val
305 310 315 320

His Pro Thr Thr Val Val His Ala Ala Leu Ser Glu Arg Ala Ala Thr
325 330 335

Phe Ile Ala Asn Gln Tyr Glu Phe Leu Ala His
340 345

<210> 37
<211> 27
<212> DNA
<213> Artificial primer asls950new

<400> 37
gtgatggtgg gcgaggaact cgtactg 27

<210> 38
<211> 35
<212> DNA
<213> Artificial primer 1 USNEW

<400> 38
agcatatgaa aaaatggttt gtttgtttat tgggg 35

<210> 39
<211> 39
<212> DNA
<213> Artificial Primer AHLS1001

<400> 39
ttggatccga attcatcaat ggtgatggtg atggtgggc 39

<210> 40
<211> 18
<212> DNA
<213> Atrificial T7 promoter primer

<400> 40
taatacgact cactatag 18

<210> 41
<211> 18
<212> DNA
<213> Artificial T7 terminator primer

<400> 41
ctagttattg ctcagcgg 18

<210> 42
<211> 41
<212> DNA
<213> Artificial pimer AHUS1

<400> 42
gtcatatgaa aaaatggttt gtgtgtttat tgggattggt c 41

<210> 43
<211> 30
<212> DNA
<213> Artificial primer ahls950

<400> 43
atggtgatgg tgggcgagga actcgtactg 30

<210> 44
<211> 41
<212> DNA
<213> Artificial primer AHUS1

<400> 44
gtcatatgaa aaaatggttt gtgtgtttat tgggattggt c 41

<210> 45
<211> 2094
<212> DNA
<213> Aeromonas hydrophila

<400> 45

atgtttaagt ttaaaaagaa tttcttagtt ggattatcgg cagctttaat tacaaattca 60
aatttttctt aaagaatcaa cctaatagcc gtcgaaatta gagtattagc ttgttttcgg 120
caaccgcctc tgcagctagc gccgacagcc ctcataatcg aacaaaagcc gttggcggag 180
acgtcgatcg cggctgtcgg gtcccgcctt ttcccggatc gtgatgttcg gcgacagcct 240
ctccgatacc cagggcggaa aagggcctag cactacaagc cgctgtcgga gaggctatgg 300
ggcaaaatgt acagcaagat gcgcggttac ctcccctcca gcccgcccta ccgttttaca 360
tgtcgttcta cgcgccaatg gaggggaggt cgggcgggat ctatgagggc cgtttctcca 420
acggacccgt ctggctggag cagctgacca gatactcccg gcaaagaggt tgcctgggca 480
gaccgacctc gtcgactggt aacagttccc gggtctgacc atcgccaacg aagcggaagg 540
cggtgccact ttgtcaaggg cccagactgg tagcggttgc ttcgccttcc gccacggtga 600
gccgtggctt acaacaagat ctcctggaat cccaagtatc aggtcatcaa cggcaccgaa 660
tgttgttcta gaggacctta gggttcatag tccagtagtt caacctggac tacgaggtca 720
cccagttctt gcagaaagac agcttcaagc gttggacctg atgctccagt gggtcaagaa 780
cgtctttctg tcgaagttcg cggacgatct ggtgatcctc tgggtcggtg ccaatgacta 840
tctggcctat gcctgctaga ccactaggag acccagccac ggttactgat agaccggata 900
ggctggaaca cggagcagga tgccaagcgg gttcgcgatg ccatcagcga ccgaccttgt 960
gcctcgtcct acggttcgcc caagcgctac ggtagtcgct tgcggccaac cgcatggtac 1020
tgaacggtgc caagcagata ctgctgttca acgccggttg gcgtaccatg acttgccacg 1080
gttcgtctat gacgacaagt acctgccgga tctgggccag aacccgtcag ctcgcagtca 1140

gaaggtggtc tggacggcct agacccggtc ttgggcagtc gagcgtcagt cttccaccag 1200
gaggcggtca gccatgtctc cgcctatcac aaccagctgc tgctgaacct ctccgccagt 1260
cggtacagag gcggatagtg ttggtcgacg acgacttgga ggcacgccag ctggccccca 1320
ccggcatggt aaagctgttc gagatcgaca ccgtgcggtc gaccgggggt ggccgtacca 1380
tttcgacaag ctctagctgt agcaatttgc cgagatgctg cgtgatccgc agaacttcgg 1440
cctgagcgac tcgttaaacg gctctacgac gcactaggcg tcttgaagcc ggactcgctg 1500
gtcgagaacc cctgctacga cggcggctat gtgtggaagc cgtttgccac cagctcttgg 1560
ggacgatgct gccgccgata cacaccttcg gcaaacggtg ccgcagcgtc agcaccgacc 1620
gccagctctc cgccttcagt ccgcaggaac ggcgtcgcag tcgtggctgg cggtcgagag 1680
gcggaagtca ggcgtccttg gcctcgccat cgccggcaac ccgctgctgg cacaggccgt 1740
tgccagtcct cggagcggta gcggccgttg ggcgacgacc gtgtccggca acggtcagga 1800
atggcccgcc gcagcgccag ccccctcaac tgtgagggca agatgttctg taccgggcgg 1860
cgtcgcggtc gggggagttg acactcccgt tctacaagac ggatcaggta cacccgacca 1920
ctgtcgtgca cgcagccctg agcgagcgcg cctagtccat gtgggctggt gacagcacgt 1980
gcgtcgggac tcgctcgcgc ccgccacctt catcgcgaac cagtacgagt tcctcgccca 2040
ctgatgaggc ggtggaagta gcgcttggtc atgctcaagg agcgggtgac tact 2094

<210> 46
<211> 26
<212> DNA
<213> Artificial primer usAHncol

<400> 46
atgccatggc cgacagccgt cccgcc 26

<210> 47
<211> 27
<212> DNA
<213> Artificial primer lsAH

<400> 47
ttggatccga attcatcaat ggtgatg 27

<210> 48
<211> 26
<212> DNA
<213> Artificial primer US-Ahnhel

<400> 48
ttgctagcgc cgacagccgt cccgcc 26

<210> 49
<211> 27
<212> DNA
<213> Artificial primer lsAH

<400> 49
ttggatccga attcatcaat ggtgatg 27

<210> 50
<211> 26
<212> DNA
<213> Artificial primer US-Asncol

<400> 50
ttgccatggc cgacactcgc cccgcc 26

<210> 51
<211> 27
<212> DNA
<213> Artificial lsAH

<400> 51
ttggatccga attcatcaat ggtgatg 27

<210> 52
<211> 26
<212> DNA
<213> Artificial primer US-Asnhel

<400> 52
ttgctagcgc cgacactcgc cccgcc 26

<210> 53
<211> 27

<212> DNA
<213> Artificial primer ISAH

<400> 53
ttggatccga attcatcaat ggtgatg 27

<210> 54
<211> 1371
<212> DNA
<213> Streptomyces

<400> 54

```
acaggccgat gcacggaacc gtacctttcc gcagtgaagc gctctccccc catcgttcgc     60
cgggacttca tccgcgattt tggcatgaac acttccttca acgcgcgtag cttgctacaa    120
gtgcggcagc agacccgctc gttggaggct cagtgagatt gacccgatcc ctgtcggccg    180
catccgtcat cgtcttcgcc ctgctgctcg cgctgctggg catcagcccg gcccaggcag    240
ccggcccggc ctatgtggcc ctgggggatt cctattcctc gggcaacggc gccggaagtt    300
acatcgattc gagcggtgac tgtcaccgca gcaacaacgc gtaccccgcc cgctgggcgg    360
cggccaacgc accgtcctcc ttcaccttcg cggcctgctc gggagcggtg accacggatg    420
tgatcaacaa tcagctgggc gccctcaacg cgtccaccgg cctggtgagc atcaccatcg    480
gcggcaatga cgcgggcttc gcggacgcga tgaccacctg cgtcaccagc tcggacagca    540
cctgcctcaa ccggctggcc accgccacca actacatcaa caccaccctg ctcgcccggc    600
tcgacgcggt ctacagccag atcaaggccc gtgcccccaa cgcccgcgtg gtcgtcctcg    660
gctacccgcg catgtacctg gcctcgaacc cctggtactg cctgggcctg agcaacacca    720
agcgcgcggc catcaacacc accgccgaca ccctcaactc ggtgatctcc tcccgggcca    780
ccgcccacgg attccgattc ggcgatgtcc gcccgacctt caacaaccac gaactgttct    840
tcggcaacga ctggctgcac tcactcaccc tgccggtgtg ggagtcgtac caccccacca    900
gcacgggcca tcagagcggc tatctgccgg tcctcaacgc caacagctcg acctgatcaa    960
cgcacggccg tgcccgcccc gcgcgtcacg ctcggcgcgg gcgccgcagc gcgttgatca   1020
gcccacagtg ccggtgacgg tcccaccgtc acggtcgagg gtgtacgtca cggtggcgcc   1080
gctccagaag tggaacgtca gcaggaccgt ggagccgtcc ctgacctcgt cgaagaactc   1140
cggggtcagc gtgatcaccc ctccccgta gccgggggcg aaggcggcgc cgaactcctt   1200
gtaggacgtc cagtcgtgcg gcccggcgtt gccaccgtcc gcgtagaccg cttccatggt   1260
cgccagccgg tccccgcgga actcggtggg gatgtccgtg cccaaggtgg tcccggtggt   1320
gtccgagagc accgggggct cgtaccggat gatgtgcaga tccaaagaat t            1371
```

<210> 55
<211> 267
<212> PRT
<213> Steptomyces

<400> 55

```
Met Arg Leu Thr Arg Ser Leu Ser Ala Ala Ser Val Ile Val Phe Ala
1               5                   10                  15

Leu Leu Leu Ala Leu Leu Gly Ile Ser Pro Ala Gln Ala Ala Gly Pro
            20                  25                  30

Ala Tyr Val Ala Leu Gly Asp Ser Tyr Ser Ser Gly Asn Gly Ala Gly
        35                  40                  45

Ser Tyr Ile Asp Ser Ser Gly Asp Cys His Arg Ser Asn Asn Ala Tyr
    50                  55                  60

Pro Ala Arg Trp Ala Ala Ala Asn Ala Pro Ser Ser Phe Thr Phe Ala
65                  70                  75                  80

Ala Cys Ser Gly Ala Val Thr Thr Asp Val Ile Asn Asn Gln Leu Gly
                85                  90                  95

Ala Leu Asn Ala Ser Thr Gly Leu Val Ser Ile Thr Ile Gly Gly Asn
            100                 105                 110

Asp Ala Gly Phe Ala Asp Ala Met Thr Thr Cys Val Thr Ser Ser Asp
        115                 120                 125

Ser Thr Cys Leu Asn Arg Leu Ala Thr Ala Thr Asn Tyr Ile Asn Thr
    130                 135                 140

Thr Leu Leu Ala Arg Leu Asp Ala Val Tyr Ser Gln Ile Lys Ala Arg
145                 150                 155                 160

Ala Pro Asn Ala Arg Val Val Val Leu Gly Tyr Pro Arg Met Tyr Leu
                165                 170                 175

Ala Ser Asn Pro Trp Tyr Cys Leu Gly Leu Ser Asn Thr Lys Arg Ala
            180                 185                 190

Ala Ile Asn Thr Thr Ala Asp Thr Leu Asn Ser Val Ile Ser Ser Arg
        195                 200                 205

Ala Thr Ala His Gly Phe Arg Phe Gly Asp Val Arg Pro Thr Phe Asn
    210                 215                 220

Asn His Glu Leu Phe Phe Gly Asn Asp Trp Leu His Ser Leu Thr Leu
225                 230                 235                 240

Pro Val Trp Glu Ser Tyr His Pro Thr Ser Thr Gly His Gln Ser Gly
                245                 250                 255

Tyr Leu Pro Val Leu Asn Ala Asn Ser Ser Thr
            260                 265
```

<210> 56
<211> 35
<212> DNA

<213> Artificial Primer 1USNEW

<400> 56
agcatatgaa aaaatggttt gtttgtttat tgggg 35

<210> 57
<211> 39
<212> DNA
<213> AHLS1001

<400> 57
ttggatccga attcatcaat ggtgatggtg atggtgggc 39

<210> 58
<211> 548
<212> PRT
<213> Thermobifida fusca

<400> 58

```
Met Leu Pro His Pro Ala Gly Glu Arg Gly Glu Val Gly Ala Phe Phe
1               5                   10                  15

Ala Leu Leu Val Gly Thr Pro Gln Asp Arg Arg Leu Arg Leu Glu Cys
            20                  25                  30

His Glu Thr Arg Pro Leu Arg Gly Arg Cys Gly Cys Gly Glu Arg Arg
        35                  40                  45

Val Pro Pro Leu Thr Leu Pro Gly Asp Gly Val Leu Cys Thr Thr Ser
    50                  55                  60
```

```
Ser Thr Arg Asp Ala Glu Thr Val Trp Arg Lys His Leu Gln Pro Arg
65                  70                  75                  80

Pro Asp Gly Gly Phe Arg Pro His Leu Gly Val Gly Cys Leu Leu Ala
                85                  90                  95

Gly Gln Gly Ser Pro Gly Val Leu Trp Cys Gly Arg Glu Gly Cys Arg
            100                 105                 110

Phe Glu Val Cys Arg Arg Asp Thr Pro Gly Leu Ser Arg Thr Arg Asn
        115                 120                 125

Gly Asp Ser Ser Pro Pro Phe Arg Ala Gly Trp Ser Leu Pro Pro Lys
    130                 135                 140

Cys Gly Glu Ile Ser Gln Ser Ala Arg Lys Thr Pro Ala Val Pro Arg
145                 150                 155                 160

Tyr Ser Leu Leu Arg Thr Asp Arg Pro Asp Gly Pro Arg Gly Arg Phe
                165                 170                 175

Val Gly Ser Gly Pro Arg Ala Ala Thr Arg Arg Arg Leu Phe Leu Gly
            180                 185                 190

Ile Pro Ala Leu Val Leu Val Thr Ala Leu Thr Leu Val Leu Ala Val
        195                 200                 205

Pro Thr Gly Arg Glu Thr Leu Trp Arg Met Trp Cys Glu Ala Thr Gln
    210                 215                 220

Asp Trp Cys Leu Gly Val Pro Val Asp Ser Arg Gly Gln Pro Ala Glu
225                 230                 235                 240

Asp Gly Glu Phe Leu Leu Leu Ser Pro Val Gln Ala Ala Thr Trp Gly
                245                 250                 255

Asn Tyr Tyr Ala Leu Gly Asp Ser Tyr Ser Ser Gly Asp Gly Ala Arg
            260                 265                 270

Asp Tyr Tyr Pro Gly Thr Ala Val Lys Gly Gly Cys Trp Arg Ser Ala
        275                 280                 285

Asn Ala Tyr Pro Glu Leu Val Ala Glu Ala Tyr Asp Phe Ala Gly His
    290                 295                 300

Leu Ser Phe Leu Ala Cys Ser Gly Gln Arg Gly Tyr Ala Met Leu Asp
305                 310                 315                 320

Ala Ile Asp Glu Val Gly Ser Gln Leu Asp Trp Asn Ser Pro His Thr
                325                 330                 335
```

```
Ser Leu Val Thr Ile Gly Ile Gly Gly Asn Asp Leu Gly Phe Ser Thr
            340                 345                 350

Val Leu Lys Thr Cys Met Val Arg Val Pro Leu Leu Asp Ser Lys Ala
        355                 360                 365

Cys Thr Asp Gln Glu Asp Ala Ile Arg Lys Arg Met Ala Lys Phe Glu
    370                 375                 380

Thr Thr Phe Glu Glu Leu Ile Ser Glu Val Arg Thr Arg Ala Pro Asp
385                 390                 395                 400

Ala Arg Ile Leu Val Val Gly Tyr Pro Arg Ile Phe Pro Glu Glu Pro
                405                 410                 415

Thr Gly Ala Tyr Tyr Thr Leu Thr Ala Ser Asn Gln Arg Trp Leu Asn
            420                 425                 430

Glu Thr Ile Gln Glu Phe Asn Gln Gln Leu Ala Glu Ala Val Ala Val
        435                 440                 445

His Asp Glu Glu Ile Ala Ala Ser Gly Gly Val Gly Ser Val Glu Phe
    450                 455                 460

Val Asp Val Tyr His Ala Leu Asp Gly His Glu Ile Gly Ser Asp Glu
465                 470                 475                 480

Pro Trp Val Asn Gly Val Gln Leu Arg Asp Leu Ala Thr Gly Val Thr
                485                 490                 495

Val Asp Arg Ser Thr Phe His Pro Asn Ala Ala Gly His Arg Ala Val
            500                 505                 510

Gly Glu Arg Val Ile Glu Gln Ile Glu Thr Gly Pro Gly Arg Pro Leu
        515                 520                 525

Tyr Ala Thr Phe Ala Val Val Ala Gly Ala Thr Val Asp Thr Leu Ala
    530                 535                 540

Gly Glu Val Gly
545
```

<210> 59
<211> 3000
<212> DNA
<213> Thermobifida fusca

<400> 59

```
ggtggtgaac cagaacaccc ggtcgtcggc gtgggcgtcc aggtgcaggt gcaggttctt      60
```

caactgctcc agcaggatgc cgccgtggcc gtgcacgatg gccttgggca ggcctgtggt 120
ccccgacgag tacagcaccc atagcggatg gtcgaacggc agcggggtga actccagttc 180
cgcgccttcg cccgcggctt cgaactccgc ccaggacagg gtgtcggcga cagggccgca 240
gcccaggtac ggcaggacga cggtgtgctg caggctgggc atgccgtcgc gcagggcttt 300
gagcacgtca cggcggtcga agtccttacc gccgtagcgg tagccgtcca cggccagcag 360
cactttcggt tcgatctgcg cgaaccggtc gaggacgctg cgcaccccga agtcggggga 420
~~acaggacgac caggtcgcac cgatcgcggc gcaggcgagg aatgcggccg tcgcctcggc 480~~
gatgttcggc aggtaggcca cgacccggtc gccggggccc accccgaggc tgcggagggc 540
cgcagcgatc gcggcggtgc gggtccgcag ttctccccag gtccactcgg tcaacggccg 600
gagttcggac gcgtgccgga tcgccacggc tgatgggtca cggtcgcgga agatgtgctc 660
ggcgtagttg agggtggcgc cggggaacca gacggcgccg ggcatggcgt cggaggcgag 720
cactgtggtg tacggggtgg cggcgcgcac ccggtagtac tcccagatcg cggaccagaa 780
tccttcgagg tcggttaccg accagcgcca cagtgcctcg tagtccggtg cgtccacacc 840
gcggtgctcc cgcacccagc gggtgaacgc ggtgaggttg gcgcgttctt tgcgctcctc 900
gtcgggactc cacaggatcg gcggctgcgg cttgagtgtc atgaaacgcg acccccttcgt 960
ggacggtgcg gatgcggtga gcgtcgggtg cctcccctaa cgctccccgg tgacggagtg 1020
ttgtgcacca catctagcac gcgggacgcg gaaaccgtat ggagaaaaca cctacaaccc 1080
cggccggacg gtgggtttcg gccacactta ggggtcgggt gcctgcttgc cgggcagggc 1140
agtcccgggg tgctgtggtg cgggcgggag ggctgtcgct tcgaggtgtg ccggcgggac 1200
actccgggcc tcagccgtac ccgcaacggg gacagttctc ctcccttccg ggctggatgg 1260
tcccttcccc cgaaatgcgg cgagatctcc cagtcagccc ggaaaacacc cgctgtgccc 1320
aggtactctt tgcttcgaac agacaggccg gacggtccac gggggaggtt tgtgggcagc 1380
ggaccacgtg cggcgaccag acgacggttg ttcctcggta tccccgctct tgtacttgtg 1440
acagcgctca cgctggtctt ggctgtcccg acggggcgcg agacgctgtg gcgcatgtgg 1500
tgtgaggcca cccaggactg gtgcctgggg gtgccggtcg actcccgcgg acagcctgcg 1560
gaggacggcg agtttctgct gctttctccg gtccaggcag cgacctgggg gaactattac 1620
gcgctcgggg attcgtactc ttcgggggac ggggcccgcg actactatcc cggcaccgcg 1680
gtgaagggcg gttgctggcg gtccgctaac gcctatccgg agctggtcgc cgaagcctac 1740
gacttcgccg gacacttgtc gttcctggcc tgcagcggcc agcgcggcta cgccatgctt 1800
gacgctatcg acgaggtcgg ctcgcagctg gactggaact cccctcacac gtcgctggtg 1860
acgatcggga tcggcggcaa cgatctgggg ttctccacgg ttttgaagac ctgcatggtg 1920
cgggtgccgc tgctggacag caaggcgtgc acggaccagg aggacgctat ccgcaagcgg 1980
atggcgaaat tcgagacgac gtttgaagag ctcatcagcg aagtgcgcac ccgcgcgccg 2040
gacgcccgga tccttgtcgt gggctacccc cggatttttc cggaggaacc gaccggcgcc 2100

tactacacgc tgaccgcgag caaccagcgg tggctcaacg aaaccattca ggagttcaac 2160
cagcagctcg ccgaggctgt cgcggtccac gacgaggaga ttgccgcgtc gggcggggtg 2220
ggcagcgtgg agttcgtgga cgtctaccac gcgttggacg gccacgagat cggctcggac 2280
gagccgtggg tgaacggggt gcagttgcgg gacctcgcca ccggggtgac tgtggaccgc 2340
agtaccttcc accccaacgc cgctgggcac cgggcggtcg gtgagcgggt catcgagcag 2400
atcgaaaccg gcccgggccg tccgctctat gccactttcg cggtggtggc gggggcgacc 2460
~~gtggacactc tcgcgggcga ggtggggtga cccggcttac cgtccggccc gcaggtctgc 2520~~
gagcactgcg gcgatctggt ccactgccca gtgcagttcg tcttcggtga tgaccagcgg 2580
cggggagagc cggatcgttg agccgtgcgt gtctttgacg agcacacccc gctgcaggag 2640
ccgttcgcac agttctcttc cggtggccag agtcgggtcg acgtcgatcc cagcccacag 2700
gccgatgctg cgggccgcga ccacgccgtt gccgaccagt tggtcgaggc gggcgcgcag 2760
cacgggggcg agggcgcgga catggtccag gtaagggccg tcgcggacga ggctcaccac 2820
ggcagtgccg accgcgcagg cgagggcgtt gccgccgaag gtgctgccgt gctggccggg 2880
gcggatcacg tcgaagactt ccgcgtcgcc taccgccgcc gccacgggca ggatgccgcc 2940
gcccagcgct ttgccgaaca ggtagatatc ggcgtcgact ccgctgtggt cgcaggcccg 3000

<210> 60
<211> 372
<212> PRT
<213> Thermobifida fusca

<400> 60

Val Gly Ser Gly Pro Arg Ala Ala Thr Arg Arg Arg Leu Phe Leu Gly
1 5 10 15

Ile Pro Ala Leu Val Leu Val Thr Ala Leu Thr Leu Val Leu Ala Val
20 25 30

Pro Thr Gly Arg Glu Thr Leu Trp Arg Met Trp Cys Glu Ala Thr Gln
35 40 45

Asp Trp Cys Leu Gly Val Pro Val Asp Ser Arg Gly Gln Pro Ala Glu
50 55 60

Asp Gly Glu Phe Leu Leu Leu Ser Pro Val Gln Ala Ala Thr Trp Gly
65 70 75 80

Asn Tyr Tyr Ala Leu Gly Asp Ser Tyr Ser Ser Gly Asp Gly Ala Arg
85 90 95

Asp Tyr Tyr Pro Gly Thr Ala Val Lys Gly Gly Cys Trp Arg Ser Ala
100 105 110

Asn Ala Tyr Pro Glu Leu Val Ala Glu Ala Tyr Asp Phe Ala Gly His
115 120 125

Leu Ser Phe Leu Ala Cys Ser Gly Gln Arg Gly Tyr Ala Met Leu Asp
130 135 140

Ala Ile Asp Glu Val Gly Ser Gln Leu Asp Trp Asn Ser Pro His Thr
145 150 155 160

Ser Leu Val Thr Ile Gly Ile Gly Gly Asn Asp Leu Gly Phe Ser Thr
165 170 175

Val Leu Lys Thr Cys Met Val Arg Val Pro Leu Leu Asp Ser Lys Ala
180 185 190

Cys Thr Asp Gln Glu Asp Ala Ile Arg Lys Arg Met Ala Lys Phe Glu
195 200 205

Thr Thr Phe Glu Glu Leu Ile Ser Glu Val Arg Thr Arg Ala Pro Asp
210 215 220

Ala Arg Ile Leu Val Val Gly Tyr Pro Arg Ile Phe Pro Glu Glu Pro
225 230 235 240

Thr Gly Ala Tyr Tyr Thr Leu Thr Ala Ser Asn Gln Arg Trp Leu Asn
245 250 255

Glu Thr Ile Gln Glu Phe Asn Gln Gln Leu Ala Glu Ala Val Ala Val
260 265 270

His Asp Glu Glu Ile Ala Ala Ser Gly Gly Val Gly Ser Val Glu Phe
275 280 285

Val Asp Val Tyr His Ala Leu Asp Gly His Glu Ile Gly Ser Asp Glu
290 295 300

Pro Trp Val Asn Gly Val Gln Leu Arg Asp Leu Ala Thr Gly Val Thr
305 310 315 320

Val Asp Arg Ser Thr Phe His Pro Asn Ala Ala Gly His Arg Ala Val
325 330 335

Gly Glu Arg Val Ile Glu Gln Ile Glu Thr Gly Pro Gly Arg Pro Leu
340 345 350

Tyr Ala Thr Phe Ala Val Val Ala Gly Ala Thr Val Asp Thr Leu Ala
355 360 365

Gly Glu Val Gly
370

<210> 61
<211> 300
<212> PRT
<213> Corynebacterium efficiens

<400> 61

```
Met Arg Thr Thr Val Ile Ala Ala Ser Ala Leu Leu Leu Leu Ala Gly
1               5                   10                  15

Cys Ala Asp Gly Ala Arg Glu Glu Thr Ala Gly Ala Pro Pro Gly Glu
            20                  25                  30

Ser Ser Gly Gly Ile Arg Glu Glu Gly Ala Glu Ala Ser Thr Ser Ile
        35                  40                  45

Thr Asp Val Tyr Ile Ala Leu Gly Asp Ser Tyr Ala Ala Met Gly Gly
    50                  55                  60

Arg Asp Gln Pro Leu Arg Gly Glu Pro Phe Cys Leu Arg Ser Ser Gly
65                  70                  75                  80

Asn Tyr Pro Glu Leu Leu His Ala Glu Val Thr Asp Leu Thr Cys Gln
                85                  90                  95

Gly Ala Val Thr Gly Asp Leu Leu Glu Pro Arg Thr Leu Gly Glu Arg
            100                 105                 110

Thr Leu Pro Ala Gln Val Asp Ala Leu Thr Glu Asp Thr Thr Leu Val
        115                 120                 125

Thr Leu Ser Ile Gly Gly Asn Asp Leu Gly Phe Gly Glu Val Ala Gly
    130                 135                 140

Cys Ile Arg Glu Arg Ile Ala Gly Glu Asn Ala Asp Asp Cys Val Asp
145                 150                 155                 160

Leu Leu Gly Glu Thr Ile Gly Glu Gln Leu Asp Gln Leu Pro Pro Gln
                165                 170                 175

Leu Asp Arg Val His Glu Ala Ile Arg Asp Arg Ala Gly Asp Ala Gln
            180                 185                 190

Val Val Val Thr Gly Tyr Leu Pro Leu Val Ser Ala Gly Asp Cys Pro
        195                 200                 205
```

```
Glu Leu Gly Asp Val Ser Glu Ala Asp Arg Arg Trp Ala Val Glu Leu
    210                 215                 220

Thr Gly Gln Ile Asn Glu Thr Val Arg Glu Ala Ala Glu Arg His Asp
225                 230                 235                 240

Ala Leu Phe Val Leu Pro Asp Asp Ala Asp Glu His Thr Ser Cys Ala
                245                 250                 255

Pro Pro Gln Gln Arg Trp Ala Asp Ile Gln Gly Gln Gln Thr Asp Ala
            260                 265                 270

Tyr Pro Leu His Pro Thr Ser Ala Gly His Glu Ala Met Ala Ala Ala
        275                 280                 285

Val Arg Asp Ala Leu Gly Leu Glu Pro Val Gln Pro
    290                 295                 300
```

<210> 62
<211> 3000
<212> DNA
<213> Corynebacterium efficiens

<400> 62

```
ttctggggtg ttatggggtt gttatcggct cgtcctgggt ggatcccgcc aggtggggta     60
ttcacggggg acttttgtgt ccaacagccg agaatgagtg ccctgagcgg tgggaatgag    120
gtgggcgggg ctgtgtcgcc atgagggggc ggcgggctct gtggtgcccc gcgacccccg    180
gccccggtga gcggtgaatg aaatccggct gtaatcagca tcccgtgccc accccgtcgg    240
ggaggtcagc gcccggagtg tctacgcagt cggatcctct cggactcggc catgctgtcg    300
gcagcatcgc gctcccgggt cttggcgtcc ctcggctgtt ctgcctgctg tccctggaag    360
gcgaaatgat caccggggag tgatacaccg gtggtctcat cccggatgcc cacttcggcg    420
ccatccggca attcgggcag ctccgggtgg aagtaggtgg catccgatgc gtcggtgacg    480
ccatagtggg cgaagatctc atcctgctcg agggtgctca ggccactctc cggatcgata    540
tcgggggcgt ccttgatggc gtccttgctg aaaccgaggt gcagcttgtg ggcttccaat    600
ttcgcaccac ggagcgggac gaggctggaa tgacggccga agagcccgtg gtggacctca    660
acgaaggtgg gtagtcccgt gtcatcattg aggaacacgc cctccaccgc acccagcttg    720
tggccggagt tgtcgtaggc gctggcatcc agaagggaaa cgatctcata tttgtcggtg    780
tgctcagaca tgatcttcct ttgctgtcgg tgtctggtac taccacggta gggctgaatg    840
caactgttat ttttctgtta ttttaggaat tggtccatat cccacaggct ggctgtggtc    900
aaatcgtcat caagtaatcc ctgtcacaca aaatgggtgg tgggagccct ggtcgcggtt    960
ccgtgggagg cgccgtgccc cgcaggatcg tcggcatcgg cggatctggc cggtaccccg   1020
```

```
cggtgaataa aatcattctg taaccttcat cacggttggt tttaggtatc cgcccctttc    1080
gtcctgaccc cgtccccggc gcgcgggagc ccgcgggttg cggtagacag gggagacgtg    1140
gacaccatga ggacaacggt catcgcagca agcgcattac tccttctcgc cggatgcgcg    1200
gatggggccc gggaggagac cgccggtgca ccgccgggtg agtcctccgg gggcatccgg    1260
gaggaggggg cggaggcgtc gacaagcatc accgacgtct acatcgccct cggggattcc    1320
tatgcggcga tgggcgggcg ggatcagccg ttacggggtg agccgttctg cctgcgctcg    1380
tccggtaatt acccggaact cctccacgca gaggtcaccg atctcacctg ccaggggggcg   1440
gtgaccgggg atctgctcga acccaggacg ctgggggagc gcacgctgcc ggcgcaggtg    1500
gatgcgctga cggaggacac caccctggtc accctctcca tcgggggcaa tgacctcgga    1560
ttcggggagg tggcgggatg catccgggaa cggatcgccg ggagaacgc tgatgattgc     1620
gtggacctgc tgggggaaac catcggggag cagctcgatc agcttccccc gcagctggac    1680
cgcgtgcacg aggctatccg ggaccgcgcc ggggacgcgc aggttgtggt caccggttac    1740
ctgccgctcg tgtctgccgg ggactgcccc gaactggggg atgtctccga ggcggatcgt    1800
cgttgggcgg ttgagctgac cgggcagatc aacgagaccg tgcgcgaggc ggccgaacga    1860
cacgatgccc tctttgtcct gcccgacgat gccgatgagc acaccagttg tgcaccccca    1920
cagcagcgct gggcggatat ccagggccaa cagaccgatg cctatccgct gcacccgacc    1980
tccgccggcc atgaggcgat ggccgccgcc gtccgggacg cgctgggcct ggaaccggtc    2040
cagccgtagc gccgggcgcg cgcttgtcga cgaccaaccc atgccaggct gcagtcacat    2100
ccgcacatag cgcgcgcggg cgatggagta cgcaccatag aggatgagcc cgatgccgac    2160
gatgatgagc agcacactgc cgaagggttg ttccccgagg gtgcgcagag ccgagtccag    2220
acctgcggcc tgctccggat catgggccca accggcgatg acgatcaaca cccccaggat    2280
cccgaaggcg ataccacggg cgacataacc ggctgttccg gtgatgatga tcgcggtccc    2340
gacctgccct gaccccgcac ccgcctccag atcctcccgg aaatcccggg tggccccctt    2400
ccagaggttg tagacacccg cccccagtac caccagcccg gcgaccacaa ccagcaccac    2460
accccagggt tgggatagga cggtggcggt gacatcggtg gcggtctccc catcggaggt    2520
gctgccgccc cgggcgaagg tggaggtggt caccgccagg gagaagtaga ccatggccat    2580
gaccgccccc ttggcccttt ccttgaggtc ctcgcccgcc agcagctggc tcaattgcca    2640
gagtcccagg gccgccaggg cgatgacggc aacccacagg aggaactgcc cacccggagc    2700
ctccgcgatg gtggccaggg cacctgaatt cgaggcctca tcacccgaac cgccggatcc    2760
agtggcgatg cgcaccgcga tccacccgat gaggatgtgc agtatgccca ggacaatgaa    2820
accacctctg gccagggtgg tcagcgcggg gtggtcctcg gcctggtcgg cagcccgttc    2880
gatcgtccgt ttcgcggatc tggtgtcgcc cttatccata gctcccattg aaccgccttg    2940
aggggtgggc ggccactgtc agggcggatt gtgatctgaa ctgtgatgtt ccatcaaccc    3000
```

<210> 63
<211> 284
<212> PRT
<213> Novosphingobium aromaticivorans

<400> 63

```
Met Gly Gln Val Lys Leu Phe Ala Arg Arg Cys Ala Pro Val Leu Leu
1               5                   10                  15

Ala Leu Ala Gly Leu Ala Pro Ala Ala Thr Val Ala Arg Glu Ala Pro
            20                  25                  30

Leu Ala Glu Gly Ala Arg Tyr Val Ala Leu Gly Ser Ser Phe Ala Ala
        35                  40                  45

Gly Pro Gly Val Gly Pro Asn Ala Pro Gly Ser Pro Glu Arg Cys Gly
    50                  55                  60

Arg Gly Thr Leu Asn Tyr Pro His Leu Leu Ala Glu Ala Leu Lys Leu
65                  70                  75                  80

Asp Leu Val Asp Ala Thr Cys Ser Gly Ala Thr Thr His His Val Leu
                85                  90                  95

Gly Pro Trp Asn Glu Val Pro Pro Gln Ile Asp Ser Val Asn Gly Asp
            100                 105                 110

Thr Arg Leu Val Thr Leu Thr Ile Gly Gly Asn Asp Val Ser Phe Val
        115                 120                 125

Gly Asn Ile Phe Ala Ala Ala Cys Glu Lys Met Ala Ser Pro Asp Pro
    130                 135                 140

Arg Cys Gly Lys Trp Arg Glu Ile Thr Glu Glu Glu Trp Gln Ala Asp
145                 150                 155                 160

Glu Glu Arg Met Arg Ser Ile Val Arg Gln Ile His Ala Arg Ala Pro
                165                 170                 175

Leu Ala Arg Val Val Val Val Asp Tyr Ile Thr Val Leu Pro Pro Ser
            180                 185                 190

Gly Thr Cys Ala Ala Met Ala Ile Ser Pro Asp Arg Leu Ala Gln Ser
        195                 200                 205

Arg Ser Ala Ala Lys Arg Leu Ala Arg Ile Thr Ala Arg Val Ala Arg
    210                 215                 220
```

Glu Glu Gly Ala Ser Leu Leu Lys Phe Ser His Ile Ser Arg Arg His
225 230 235 240

His Pro Cys Ser Ala Lys Pro Trp Ser Asn Gly Leu Ser Ala Pro Ala
245 250 255

Asp Asp Gly Ile Pro Val His Pro Asn Arg Leu Gly His Ala Glu Ala
260 265 270

Ala Ala Ala Leu Val Lys Leu Val Lys Leu Met Lys
275 280

<210> 64
<211> 1500
<212> DNA
<213> Novosphingobium aromaticivorans

<400> 64

```
tgccggaact caagcggcgt ctagccgaac tcatgcccga aagcgcgtgg cactatcccg      60
aagaccaggt ctcggacgcc agcgagcgcc tgatggccgc cgaaatcacg cgcgaacagc     120
tctaccgcca gctccacgac gagctgccct atgacagtac cgtacgtccc gagaagtacc     180
tccatcgcaa ggacggttcg atcgagatcc accagcagat cgtgattgcc cgcgagacac     240
agcgtccgat cgtgctgggc aagggtggcg cgaagatcaa ggcgatcgga gaggccgcac     300
gcaaggaact ttcgcaattg ctcgacacca aggtgcacct gttcctgcat gtgaaggtcg     360
acgagcgctg ggccgacgcc aaggaaatct acgaggaaat cggcctcgaa tgggtcaagt     420
gaagctcttc gcgcgccgct gcgccccagt acttctcgcc cttgccgggc tggctccggc     480
ggctacggtc gcgcgggaag caccgctggc cgaaggcgcg cgttacgttg cgctgggaag     540
ctccttcgcc gcaggtccgg gcgtggggcc caacgcgccc ggatcgcccg aacgctgcgg     600
ccggggcacg ctcaactacc cgcacctgct cgccgaggcg ctcaagctcg atctcgtcga     660
tgcgacctgc agcggcgcga cgacccacca cgtgctgggc cctggaacg aggttccccc     720
tcagatcgac agcgtgaatg gcgacacccg cctcgtcacc ctgaccatcg gcggaaacga     780
tgtgtcgttc gtcggcaaca tcttcgccgc cgcttgcgag aagatggcgt cgcccgatcc     840
gcgctgcggc aagtggcggg agatcaccga ggaagagtgg caggccgacg aggagcggat     900
gcgctccatc gtacgccaga tccacgcccg cgcgcctctc gcccgggtgg tggtggtcga     960
ttacatcacg gtcctgccgc catcaggcac ttgcgctgcc atggcgattt cgccggaccg    1020
gctggcccag agccgcagcg ccgcgaaacg gcttgcccgg attaccgcac gggtcgcgcg    1080
agaagagggt gcatcgctgc tcaagttctc gcatatctcg cgccggcacc atccatgctc    1140
tgccaagccc tggagcaacg gcctttccgc cccggccgac gacggcatcc cggtccatcc    1200
gaaccggctc ggacatgctg aagcggcagc ggcgctggtc aagcttgtga aattgatgaa    1260
```

gtagctactg cactgatttc aaatagtatt gcctgtcagc tttccagccc ggattgttgc 1320

agcgcaacag aaacttgtcc gtaatggatt gatggtttat gtcgctcgca aattgccgtc 1380

gaagggaacg ggcgcgtcgc tcgttaacgt cctgggtgca gcagtgacgg agcgcgtgga 1440

tgagtgatac tggcggtgtc atcggtgtac gcgccgccat tcccatgcct gtacgcgccg 1500

<210> 65
<211> 268
<212> PRT
<213> Streptomyces coelicolor

<400> 65

Met Arg Arg Phe Arg Leu Val Gly Phe Leu Ser Ser Leu Val Leu Ala
1 5 10 15

Ala Gly Ala Ala Leu Thr Gly Ala Ala Thr Ala Gln Ala Ala Gln Pro
20 25 30

Ala Ala Ala Asp Gly Tyr Val Ala Leu Gly Asp Ser Tyr Ser Ser Gly
35 40 45

Val Gly Ala Gly Ser Tyr Ile Ser Ser Ser Gly Asp Cys Lys Arg Ser
50 55 60

Thr Lys Ala His Pro Tyr Leu Trp Ala Ala Ala His Ser Pro Ser Thr
65 70 75 80

Phe Asp Phe Thr Ala Cys Ser Gly Ala Arg Thr Gly Asp Val Leu Ser
85 90 95

Gly Gln Leu Gly Pro Leu Ser Ser Gly Thr Gly Leu Val Ser Ile Ser
100 105 110

Ile Gly Gly Asn Asp Ala Gly Phe Ala Asp Thr Met Thr Thr Cys Val
115 120 125

Leu Gln Ser Glu Ser Ser Cys Leu Ser Arg Ile Ala Thr Ala Glu Ala
130 135 140

Tyr Val Asp Ser Thr Leu Pro Gly Lys Leu Asp Gly Val Tyr Ser Ala
145 150 155 160

Ile Ser Asp Lys Ala Pro Asn Ala His Val Val Val Ile Gly Tyr Pro
165 170 175

Arg Phe Tyr Lys Leu Gly Thr Thr Cys Ile Gly Leu Ser Glu Thr Lys
180 185 190

Arg Thr Ala Ile Asn Lys Ala Ser Asp His Leu Asn Thr Val Leu Ala
195 200 205

Gln Arg Ala Ala Ala His Gly Phe Thr Phe Gly Asp Val Arg Thr Thr
210 215 220

Phe Thr Gly His Glu Leu Cys Ser Gly Ser Pro Trp Leu His Ser Val
225 230 235 240

---

Asn Trp Leu Asn Ile Gly Glu Ser Tyr His Pro Thr Ala Ala Gly Gln
245 250 255

Ser Gly Gly Tyr Leu Pro Val Leu Asn Gly Ala Ala
260 265

<210> 66
<211> 2000
<212> DNA
<213> Streptomyces coelicolor

<400> 66

cccggcggcc cgtgcaggag cagcagccgg cccgcgatgt cctcgggcgt cgtcttcatc 60
aggccgtcca tcgcgtcggc gaccggcgcc gtgtagttgg cccggacctc gtcccaggtg 120
cccgcggcga tctggcgggt ggtgcggtgc gggccgcgcc gaggggagac gtaccagaag 180
cccatcgtca cgttctccgg ctgcggttcg ggctcgtccg ccgctccgtc cgtcgcctcg 240
ccgagcacct tctcggcgag gtcggcgctg gtcgccgtca ccgtgacgtc ggcgccccgg 300
ctccagcgcg agatcagcag cgtccagccg tcgccctccg ccagcgtcgc gctgcggtcg 360
tcgtcgcggg cgatccgcag cacgcgcgcg ccgggcggca gcagcgtggc gccggaccgt 420
acgcggtcga tgttcgccgc gtgcgagtac ggctgctcac ccgtggcgaa acggccgagg 480
aacagcgcgt cgacgacgtc ggacggggag tcgctgtcgt ccacgttgag ccggatcggc 540
agggcttcgt gcgggttcac ggacatgtcg ccatgatcgg gcacccggcc gccgcgtgca 600
cccgctttcc cgggcacgca cgacaggggc tttctcgccg tcttccgtcc gaacttgaac 660
gagtgtcagc catttcttgg catggacact tccagtcaac gcgcgtagct gctaccacgg 720
ttgtggcagc aatcctgcta agggaggttc catgagacgt ttccgacttg tcggcttcct 780
gagttcgctc gtcctcgccg ccggcgccgc cctcaccggg gcagcgaccg cccaggcggc 840
ccaacccgcc gccgccgacg gctatgtggc cctcggcgac tcctactcct ccggggtcgg 900
agcgggcagc tacatcagct cgagcggcga ctgcaagcgc agcacgaagg cccatcccta 960
cctgtgggcg gccgcccact cgccctccac gttcgacttc accgcctgtt ccggcgcccg 1020
tacgggtgat gttctctccg gacagctcgg cccgctcagc tccggcaccg gcctcgtctc 1080

```
gatcagcatc ggcggcaacg acgccggttt cgccgacacc atgacgacct gtgtgctcca   1140
gtccgagagc tcctgcctgt cgcggatcgc caccgccgag gcgtacgtcg actcgacgct   1200
gcccggcaag ctcgacggcg tctactcggc aatcagcgac aaggcgccga acgcccacgt   1260
cgtcgtcatc ggctacccgc gcttctacaa gctcggcacc acctgcatcg gcctgtccga   1320
gaccaagcgg acggcgatca acaaggcctc cgaccacctc aacaccgtcc tcgcccagcg   1380
cgccgccgcc cacggcttca ccttcggcga cgtacgcacc accttcaccg gccacgagct   1440
gtgctccggc agcccctggc tgcacagcgt caactggctg aacatcggcg agtcgtacca   1500
ccccaccgcg gccggccagt ccggtggcta cctgccggtc ctcaacggcg ccgcctgacc   1560
tcaggcggaa ggagaagaag aaggagcgga gggagacgag gagtgggagg ccccgcccga   1620
cggggtcccc gtccccgtct ccgtctccgt cccggtcccg caagtcaccg agaacgccac   1680
cgcgtcggac gtggcccgca ccggactccg cacctccacg cgcacggcac tctcgaacgc   1740
gccggtgtcg tcgtgcgtcg tcaccaccac gccgtcctgg cgcgagcgct cgccgcccga   1800
cgggaaggac agcgtccgcc accccggatc ggagaccgac ccgtccgcgg tcacccaccg   1860
gtagccgacc tccgcgggca gccgcccgac cgtgaacgtc gccgtgaacg cgggtgcccg   1920
gtcgtgcggc ggcggacagg cccccgagta gtgggtgcgc gagcccacca cggtcacctc   1980
caccgactgc gctgcggggc                                                2000
```

<210> 67
<211> 269
<212> PRT
<213> Streptomyces avermitilis

<400> 67

```
Met Arg Arg Ser Arg Ile Thr Ala Tyr Val Thr Ser Leu Leu Leu Ala
1               5                   10                  15

Val Gly Cys Ala Leu Thr Gly Ala Ala Thr Ala Gln Ala Ser Pro Ala
            20                  25                  30

Ala Ala Ala Thr Gly Tyr Val Ala Leu Gly Asp Ser Tyr Ser Ser Gly
        35                  40                  45

Val Gly Ala Gly Ser Tyr Leu Ser Ser Ser Gly Asp Cys Lys Arg Ser
    50                  55                  60

Ser Lys Ala Tyr Pro Tyr Leu Trp Gln Ala Ala His Ser Pro Ser Ser
65                  70                  75                  80

Phe Ser Phe Met Ala Cys Ser Gly Ala Arg Thr Gly Asp Val Leu Ala
                85                  90                  95
```

Asn Gln Leu Gly Thr Leu Asn Ser Ser Thr Gly Leu Val Ser Leu Thr
100 105 110

Ile Gly Gly Asn Asp Ala Gly Phe Ser Asp Val Met Thr Thr Cys Val
115 120 125

Leu Gln Ser Asp Ser Ala Cys Leu Ser Arg Ile Asn Thr Ala Lys Ala
130 135 140

---

Tyr Val Asp Ser Thr Leu Pro Gly Gln Leu Asp Ser Val Tyr Thr Ala
145 150 155 160

Ile Ser Thr Lys Ala Pro Ser Ala His Val Ala Val Leu Gly Tyr Pro
165 170 175

Arg Phe Tyr Lys Leu Gly Gly Ser Cys Leu Ala Gly Leu Ser Glu Thr
180 185 190

Lys Arg Ser Ala Ile Asn Asp Ala Ala Asp Tyr Leu Asn Ser Ala Ile
195 200 205

Ala Lys Arg Ala Ala Asp His Gly Phe Thr Phe Gly Asp Val Lys Ser
210 215 220

Thr Phe Thr Gly His Glu Ile Cys Ser Ser Ser Thr Trp Leu His Ser
225 230 235 240

Leu Asp Leu Leu Asn Ile Gly Gln Ser Tyr His Pro Thr Ala Ala Gly
245 250 255

Gln Ser Gly Gly Tyr Leu Pro Val Met Asn Ser Val Ala
260 265

<210> 68
<211> 1980
<212> DNA
<213> Streptomyces avermitilis

<400> 68

ccaccgccgg gtcggcggcg agtctcctgg cctcggtcgc ggagaggttg gccgtgtagc 60
cgttcagcgc ggcgccgaac gtcttcttca ccgtgccgcc gtactcgttg atcaggccct 120
tgcccttgct cgacgcggcc ttgaagccgg tgcccttctt gagcgtgacg atgtagctgc 180
ccttgatcgc ggtgggggag ccggcggcga gcaccgtgcc ctcggccggg gtggcctggg 240
cgggcagtgc ggtgaatccg cccacgaggg cgccggtcgc cacggcggtt atcgcggcga 300
tccggatctt cttgctacgc agctgtgcca tacgagggag tcctcctctg ggcagcggcg 360

cgcctgggtg gggcgcacgg ctgtgggggg tgcgcgcgtc atcacgcaca cggccctgga 420
gcgtcgtgtt ccgccctggg ttgagtaaag cctcggccat ctacgggggt ggctcaaggg 480
agttgagacc ctgtcatgag tctgacatga gcacgcaatc aacggggccg tgagcacccc 540
ggggcgaccc cggaaagtgc cgagaagtct tggcatggac acttcctgtc aacacgcgta 600
gctggtacga cggttacggc agagatcctg ctaaagggag gttccatgag acgttcccga 660
attacggcat acgtgacctc actcctcctc gccgtcggct gcgccctcac cggggcagcg 720
acggcgcagg cgtccccagc cgccgcggcc acgggctatg tggccctcgg cgactcgtac 780
tcgtccggtg tcggcgccgg cagctacctc agctccagcg gcgactgcaa gcgcagttcg 840
aaggcctatc cgtacctctg gcaggccgcg cattcaccct cgtcgttcag tttcatggct 900
tgctcgggcg ctcgtacggg tgatgtcctg gccaatcagc tcggcaccct gaactcgtcc 960
accggcctgg tctccctcac catcggaggc aacgacgcgg gcttctccga cgtcatgacg 1020
acctgtgtgc tccagtccga cagcgcctgc ctctcccgca tcaacacggc gaaggcgtac 1080
gtcgactcca ccctgcccgg ccaactcgac agcgtgtaca cggcgatcag cacgaaggcc 1140
ccgtcggccc atgtggccgt gctgggctac ccccgcttct acaaactggg cggctcctgc 1200
ctcgcgggcc tctcggagac caagcggtcc gccatcaacg acgcggccga ctatctgaac 1260
agcgccatcg ccaagcgcgc cgccgaccac ggcttcacct tcggcgacgt caagagcacc 1320
ttcaccggcc atgagatctg ctccagcagc acctggctgc acagtctcga cctgctgaac 1380
atcggccagt cctaccaccc gaccgcggcc ggccagtccg gcggctatct gccggtcatg 1440
aacagcgtgg cctgagctcc cacggcctga atttttaagg cctgaatttt taaggcgaag 1500
gtgaaccgga agcggaggcc ccgtccgtcg ggtctccgt cgcacaggtc accgagaacg 1560
gcacggagtt ggacgtcgtg cgcaccgggt cgcgcacctc gacggcgatc tcgttcgaga 1620
tcgttccgct cgtgtcgtac gtggtgacga acacctgctt ctgctgggtc tttccgccgc 1680
tcgccgggaa ggacagcgtc ttccagcccg gatccgggac ctcgcccttc ttggtcaccc 1740
agcggtactc cacctcgacc ggcacccggc ccaccgtgaa ggtcgccgtg aacgtgggcg 1800
cctgggcggt gggcggcggg caggcaccgg agtagtcggt gtgcacgccg gtgaccgtca 1860
ccttcacgga ctgggccggc ggggtcgtcg taccgccgcc gccaccgccg cctcccggag 1920
tggagcccga gctgtggtcg cccccgccgt cggcgttgtc gtcctcgggg gttttcgaac 1980

<210> 69
<211> 1371
<212> DNA
<213> Streptomyces

<400> 69

acaggccgat gcacggaacc gtacctttcc gcagtgaagc gctctccccc catcgttcgc 60

```
cgggacttca tccgcgattt tggcatgaac acttccttca acgcgcgtag cttgctacaa    120
gtgcggcagc agacccgctc gttggaggct cagtgagatt gacccgatcc ctgtcggccg    180
catccgtcat cgtcttcgcc ctgctgctcg cgctgctggg catcagcccg gcccaggcag    240
ccggcccggc ctatgtggcc ctgggggatt cctattcctc gggcaacggc gccggaagtt    300
acatcgattc gagcggtgac tgtcaccgca gcaacaacgc gtaccccgcc cgctgggcgg    360
cggccaacgc accgtcctcc ttcaccttcg cggcctgctc gggagcggtg accacggatg    420
tgatcaacaa tcagctgggc gccctcaacg cgtccaccgg cctggtgagc atcaccatcg    480
gcggcaatga cgcgggcttc gcggacgcga tgaccacctg cgtcaccagc tcggacagca    540
cctgcctcaa ccggctggcc accgccacca actacatcaa caccaccctg ctcgcccggc    600
tcgacgcggt ctacagccag atcaaggccc gtgcccccaa cgcccgcgtg gtcgtcctcg    660
gctacccgcg catgtacctg gcctcgaacc cctggtactg cctgggcctg agcaacacca    720
agcgcgcggc catcaacacc accgccgaca ccctcaactc ggtgatctcc tcccgggcca    780
ccgcccacgg attccgattc ggcgatgtcc gcccgacctt caacaaccac gaactgttct    840
tcggcaacga ctggctgcac tcactcaccc tgccggtgtg ggagtcgtac caccccacca    900
gcacgggcca tcagagcggc tatctgccgg tcctcaacgc caacagctcg acctgatcaa    960
cgcacggccg tgcccgcccc gcgcgtcacg ctcggcgcgg gcgccgcagc gcgttgatca   1020
gcccacagtg ccggtgacgg tcccaccgtc acggtcgagg gtgtacgtca cggtggcgcc   1080
gctccagaag tggaacgtca gcaggaccgt ggagccgtcc ctgacctcgt cgaagaactc   1140
cggggtcagc gtgatcaccc ctcccccgta gccgggggcg aaggcggcgc cgaactcctt   1200
gtaggacgtc cagtcgtgcg gcccggcgtt gccaccgtcc gcgtagaccg cttccatggt   1260
cgccagccgg tccccgcgga actcggtggg gatgtccgtg cccaaggtgg tcccggtggt   1320
gtccgagagc accgggggct cgtaccggat gatgtgcaga tccaaagaat t            1371
```

<210> 70
<211> 267
<212> PRT
<213> Streptomyces

<400> 70

```
Met Arg Leu Thr Arg Ser Leu Ser Ala Ala Ser Val Ile Val Phe Ala
1               5                   10                  15

Leu Leu Leu Ala Leu Leu Gly Ile Ser Pro Ala Gln Ala Ala Gly Pro
            20                  25                  30

Ala Tyr Val Ala Leu Gly Asp Ser Tyr Ser Ser Gly Asn Gly Ala Gly
        35                  40                  45
```

```
Ser Tyr Ile Asp Ser Ser Gly Asp Cys His Arg Ser Asn Asn Ala Tyr
    50                  55                  60

Pro Ala Arg Trp Ala Ala Ala Asn Ala Pro Ser Ser Phe Thr Phe Ala
65                  70                  75                  80

Ala Cys Ser Gly Ala Val Thr Thr Asp Val Ile Asn Asn Gln Leu Gly
                85                  90                  95

Ala Leu Asn Ala Ser Thr Gly Leu Val Ser Ile Thr Ile Gly Gly Asn
            100                 105                 110

Asp Ala Gly Phe Ala Asp Ala Met Thr Thr Cys Val Thr Ser Ser Asp
        115                 120                 125

Ser Thr Cys Leu Asn Arg Leu Ala Thr Ala Thr Asn Tyr Ile Asn Thr
    130                 135                 140

Thr Leu Leu Ala Arg Leu Asp Ala Val Tyr Ser Gln Ile Lys Ala Arg
145                 150                 155                 160

Ala Pro Asn Ala Arg Val Val Val Leu Gly Tyr Pro Arg Met Tyr Leu
                165                 170                 175

Ala Ser Asn Pro Trp Tyr Cys Leu Gly Leu Ser Asn Thr Lys Arg Ala
            180                 185                 190

Ala Ile Asn Thr Thr Ala Asp Thr Leu Asn Ser Val Ile Ser Ser Arg
        195                 200                 205

Ala Thr Ala His Gly Phe Arg Phe Gly Asp Val Arg Pro Thr Phe Asn
    210                 215                 220

Asn His Glu Leu Phe Phe Gly Asn Asp Trp Leu His Ser Leu Thr Leu
225                 230                 235                 240

Pro Val Trp Glu Ser Tyr His Pro Thr Ser Thr Gly His Gln Ser Gly
                245                 250                 255

Tyr Leu Pro Val Leu Asn Ala Asn Ser Ser Thr
            260                 265
```